# EUROPEAN PATENT APPLICATION

(11) **EP 4 147 700 A1**
(43) Date of publication of application: **15.03.2023**
(21) Application number: 21306230.0
(22) Date of filing: 08.09.2021
(51) Int. Cl.: A61K 31/4985, A61P 7/06

(54) **N-(4-(AZAINDAZOL-6-YL)-PHENYL)-SULFONAMIDES FOR USE IN THE TREATMENT OF SICKLE CELL DISEASE**

(71) Applicant: LQT Therapeutics Inc., Laval, Québec H7V 5B7 (CA); Sanofi, 75008 Paris (FR)
(72) Inventor: HARA, Yannis, Cambridge, 02139 (US); HALLAND, Nis, DE-65926 Frankfurt am Main (DE)
(74) Representative: Cabinet Becker et Associés

(57) **Abstract**

N-(4-(azaindazol-6-yl)-phenyl)-sulfonamides of the formula I, 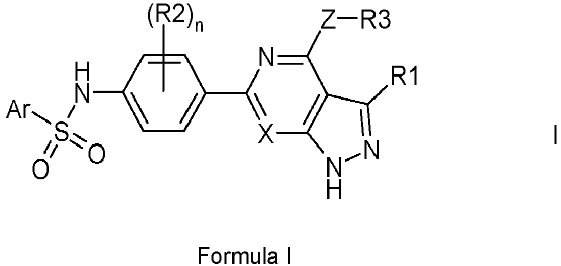 and pharmaceutically acceptable salts thereof, as well as pharmaceutical compositions comprising a compound of the formula I or a pharmaceutically acceptable salt thereof, are described for use in the treatment of a β-hemoglobinopathy, particularly for use in the treatment of sickle cell disease or β-thalassemia, more particularly for use in the treatment of sickle cell disease.

## Description

### TECHNICAL FIELD

The technical field relates to compounds, pharmaceutical compositions, uses and methods for the treatment of β-hemoglobinopathies, and more particularly relates to N-(4-(azaindazol-6-yl)-phenyl)-sulfonamides of the formula I, corresponding pharmaceutical compositions, uses and methods, for the treatment of sickle cell disease.

### BACKGROUND

Sickle cell disease (SCD), also known as sickle cell anemia, is a common monogenic disease with more than 300 000 affected individuals born annually worldwide (Piel, F.B., et al., Lancet, 2013. 381(9861): p. 142-51.). This condition is the result of substitution of glutamic acid by valine at position 6 of the β-globin subunit of hemoglobin. The presence of this mutant β-globin subunit leads to the production of abnormal hemoglobin S (HbS) that polymerizes in red blood cells under conditions of low oxygen, which affects blood flow rheology and ultimately leads to vaso-occlusive crises and organ damage (Barabino et al. Annu. Rev. Biomed. Eng., 2010. 12: p. 345-67.).

Long-term irreversible complications of SCD, such as vaso-occlusive crisis (VOC) and hemolysis, are the most common causes of morbidity and death. The red blood cells (RBCs) affected by SCD have a shorter life span, which results in chronic hemolytic anemia. Administering hydroxyurea is common to treat the symptoms associated with SCD. However, patients taking hydroxyurea can experience adverse effects such as nausea, anorexia and myelosuppression. Other recently approved drugs for the treatment of sickle cell disease include L-glutamine, crizanlizumab and voxelotor. However, challenges still exist in the treatment of sickle cell disease.

### SUMMARY

In one aspect of the present description, there is provided a compound of the formula I, in any of its stereoisomeric forms or a mixture of stereoisomeric forms in any ratio, or a pharmaceutically acceptable salt thereof, 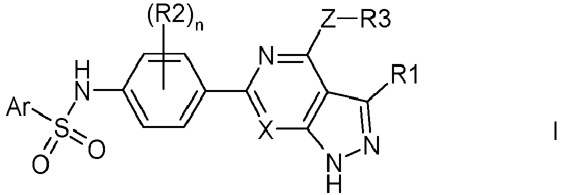 for use in the treatment of sickle cell disease,
wherein
Ar is selected from the series consisting of phenyl and a 5-membered or 6-membered, monocyclic, aromatic, heterocyclic group which comprises 1, 2 or 3 identical or different ring heteroatoms selected from the series consisting of nitrogen, oxygen and sulfur, and is bonded via a ring carbon atom, which all are unsubstituted or substituted by one or more identical or different substituents R5;
n is selected from the series consisting of 0, 1 and 2;
X is selected from the series consisting of N and CH;
Z is selected from the series consisting of a direct bond, O, S and N(R10);
R1 is selected from the series consisting of H, -N(R11)-R12, -N(R13)-C(O)-R14, -N(R13)-S(O)₂-R15, -N(R13)-C(O)-NH-R16, (C₁-C₄)-alkyl and -(C₁-C₄)-alkyl-O-R17;
R2 is selected from the series consisting of halogen, (C₁-C₄)-alkyl, -O-(C₁-C₄)-alkyl and -CN;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, (C₃-C₇)-cycloalkyl, -(C₁-C₄)-alkyl-(C₃-C₇)-cycloalkyl, -O-(C₁-C₄)-alkyl, -O-(C₃-C₇)-cycloalkyl, -O-(C₁-C₄)-alkyl-(C₃-C₇)-cycloalkyl, -C(O)-N(R6)-R7 and -CN,
and two groups R5 bonded to adjacent ring carbon atoms in Ar, together with the carbon atoms carrying them, can form a 5-membered to 8-membered, monocyclic, unsaturated ring which comprises 0, 1 or 2 identical or different ring heteroatoms selected from the series consisting of nitrogen, oxygen and sulfur, and which is unsubstituted or substituted by one or more identical or different substituents R8;
R6 and R7 are independently of one another selected from the series consisting of H and (C₁-C₄)-alkyl;
R8 is selected from the series consisting of halogen, (C₁-C₄)-alkyl, -O-(C₁-C₄)-alkyl and -CN;
R10 is selected from the series consisting of H and (C₁-C₄)-alkyl;
R11 and R12 are independently of one another selected from the series consisting of H, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl, wherein phenyl is unsubstituted or substituted by one or more identical or different substituents R50,
or R11 and R12, together with the nitrogen atom carrying them, form a 4-membered to 7-membered, monocyclic, saturated, heterocyclic group which, in addition to the nitrogen atom carrying R11 and R12, comprises 0 or 1 further ring heteroatom selected from the series consisting of nitrogen, oxygen and sulfur, and which is unsubstituted or substituted by one or more identical or different substituents selected from the series consisting of fluorine and (C₁-C₄)-alkyl;
R13 is selected from the series consisting of H, (C₁-C₄)-alkyl and (C₃-C₇)-cycloalkyl;
R14 and R16 are independently of one another selected from the series consisting of (C₁-C₈)-alkyl, (C₃-C₇)-cycloalkyl, -(C₁-C₄)-alkyl-(C₃-C₇)-cycloalkyl, phenyl, -(C₁-C₄)-alkyl-phenyl, Het2 and -(C₁-C₄)-alkyl-Het2, wherein (C₁-C₈)-alkyl and (C₃-C₇)-cycloalkyl all are unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R15 is selected from the series consisting of (C₁-C₈)-alkyl, phenyl and Het3, wherein phenyl and Het3 all are unsubstituted or substituted by one or more identical or different substituents R50;
R17 is selected from the series consisting of H and (C₁-C₄)-alkyl;
R30 is a 3-membered to 12-membered, monocyclic or bicyclic, saturated, partially unsaturated or aromatic, cyclic group which comprises 0, 1, 2 or 3 identical or different ring heteroatoms selected from the series consisting of nitrogen, oxygen and sulfur, which is unsubstituted or substituted by one or more identical or different substituents R32;
R31 is selected from the series consisting of halogen, -OH, -O-(C₁-C₄)-alkyl, -O-(C₃-C₇)-cycloalkyl, -O-(C₁-C₄)-alkyl-(C₃-C₇)-cycloalkyl, -N(R33)-R34, -CN and -C(O)-N(R35)-R36;
R32 is selected from the series consisting of halogen, (C₁-C₄)-alkyl, (C₃-C₇)-cycloalkyl, -(C₁-C₄)-alkyl-(C₃-C₇)-cycloalkyl, -(C₁-C₄)-alkyl-O-R37, -(C₁-C₄)-alkyl-N(R38)-R39, -(C₁-C₄)-alkyl-CN, - C(O)-(C₁-C₄)-alkyl, -CN, -OH, =O, -O-(C₁-C₄)-alkyl, -N(R40)-R41, -C(O)-O-(C₁-C₄)-alkyl and - C(O)-N(R42)-R43;
R33, R34, R35, R36, R37, R38, R39, R40, R41, R42 and R43 are independently of one another selected from the series consisting of H and (C₁-C₄)-alkyl;
R50 is selected from the series consisting of halogen, (C₁-C₄)-alkyl, -O-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen, oxygen and sulfur, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated, partially unsaturated or aromatic, heterocyclic group which comprises 1 or 2 identical or different ring heteroatoms selected from the series consisting of nitrogen, oxygen and sulfur, and is bonded via a ring carbon atom;
Het3 is a 5-membered or 6-membered, monocyclic, aromatic, heterocyclic group which comprises 1, 2 or 3 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

In another aspect of the present description, there is provided the use of a SGK1 inhibitor, for increasing fetal hemoglobin (HbF) in erythrocytes.

In another aspect of the present description, there is provided the use of a SGK1 inhibitor, for the treatment of a β-hemoglobinopathy.

In another aspect of the present description, there is provided the use of a SGK1 inhibitor, for the treatment of sickle cell disease or β-thalassemia.

In another aspect of the present description, there is provided the use of a SGK1 inhibitor, for the treatment of sickle cell disease.

### BRIEF DESCRIPTION OF THE DRAWINGS

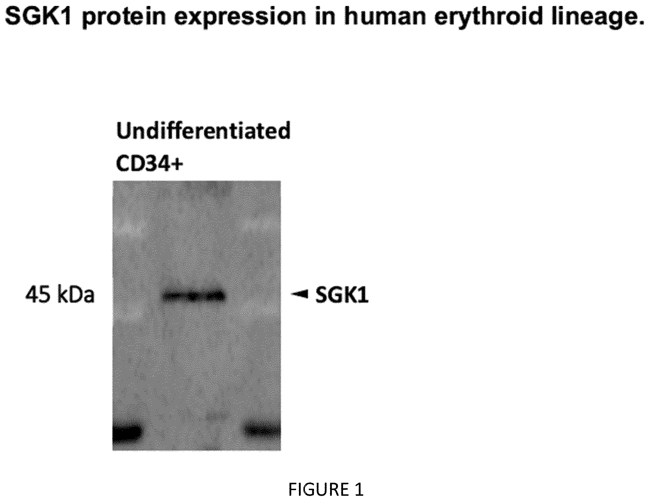

FIG. 1 shows the expression of SGK1 in human CD34+ cells from a healthy donor.

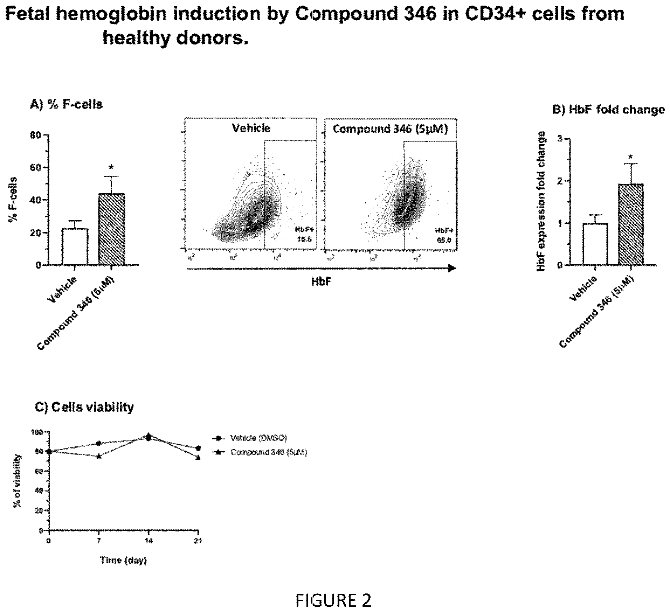

FIG. 2: FIG. 2A shows the result of fetal hemoglobin induction in human CD34+ cells when mobilized CD34+ HSPC from healthy individuals are cultured for 21 days under conditions to promote erythroid differentiation and are exposed to SGK1 inhibitor Compound 346 at the indicated doses (µM). Exposure to Compound 346 increases F-cell (HbF+) frequency in enucleated GlyA+ cells, compared to control (DMSO indicated as Vehicle). FIG. 2B shows the HbF+ fold change to control (DMSO) cells. FIG. 2C: Representative plots from a single donor are shown. All data are expressed as mean ± SEM (* p < 0.05, N= 3 independent donors).

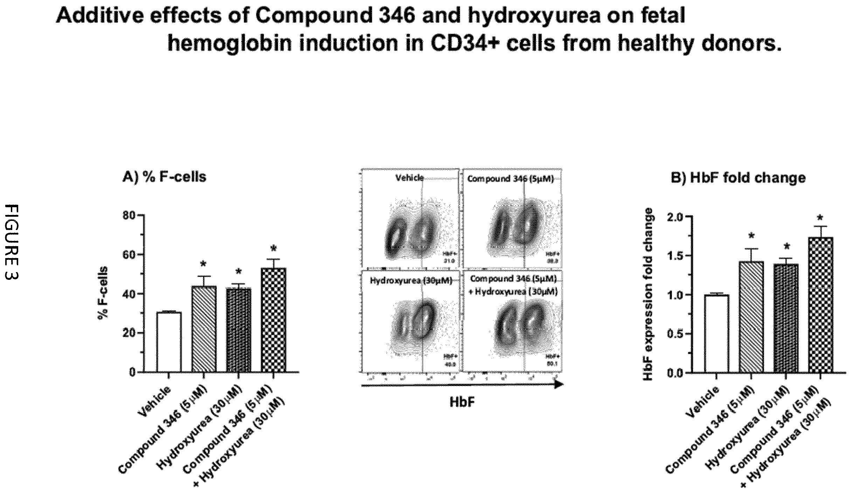

FIG. 3: FIG. 3A shows the result of fetal hemoglobin induction in human CD34+ cells when mobilized CD34+ HSPC from healthy individuals are cultured for 14 days under conditions promoting erythroid differentiation and are exposed to Compound 346 or Hydroxyurea or a combination of both. Exposure to Compound 346 or Hydroxyurea increases F-cell (HbF+) frequency in enucleated GlyA+ cells, compared to control cells (DMSO indicated as Vehicle). The combination of both leads to a higher frequency of F-cell compared to control and Compound 346 or HU alone. FIG. 3B shows the HbF+ fold change to control. Representative plots from one healthy donor are shown in FIG. 3C. All data are expressed as mean ± SEM (* p < 0.05, N= 3 independent donors).

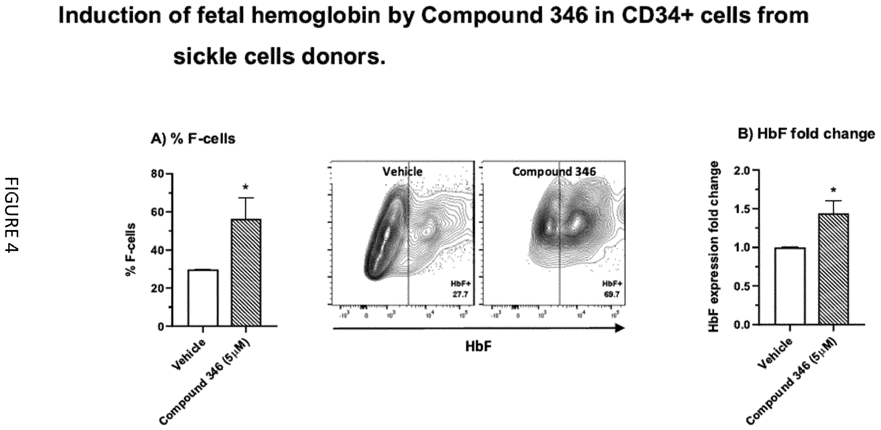

FIG. 4: FIG. 4A shows the result of fetal hemoglobin induction in human CD34+ cells from patients with sickle cell disease after exposure to Compound 346, Hydroxyurea, and a combination of Compound 346 and Hydroxyurea. Exposure to Compound 346 or Hydroxyurea alone increase F-cell (HbF+) frequency in enucleated GlyA+ cells, compared to control cells (DMSO indicated as Vehicle). The combination of both leads to a higher frequency of F-cell compared to Compound 346 or Hydroxyurea alone. FIG. 4B shows the HbF+ fold change to control. Representative plots from one donor with sickle cell disease are shown in FIG. 4C. All data are expressed as mean ± SEM (* p < 0.05, N= 3 independent donors).

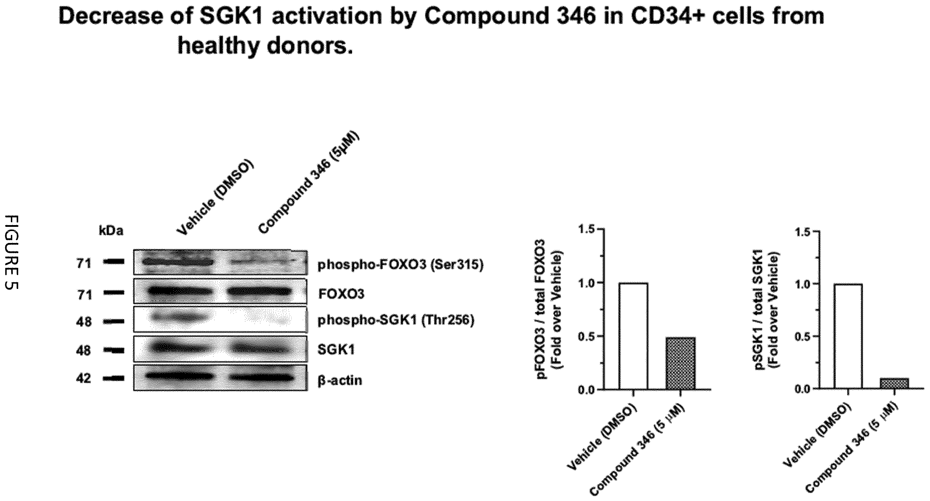

FIG. 5 shows the measurement of phosphorylated residue Threonine 256 on SGK1 in mobilized CD34+ HSPC from healthy donors at day 11 of erythroid differentiation and the phosphorylation of FOX03 at Serine 315, assessed by Western blotting and using cell lysates from the 1-hour time point. The blot confirms the inhibition of SGK1 by Compound 346, revealed by a decrease in Thr256 phosphorylation. This shows that the phosphorylation of FOX03 on Ser315 decreases in the cells exposed to Compound 346 normalized to β-actin and assessed by densitometry.

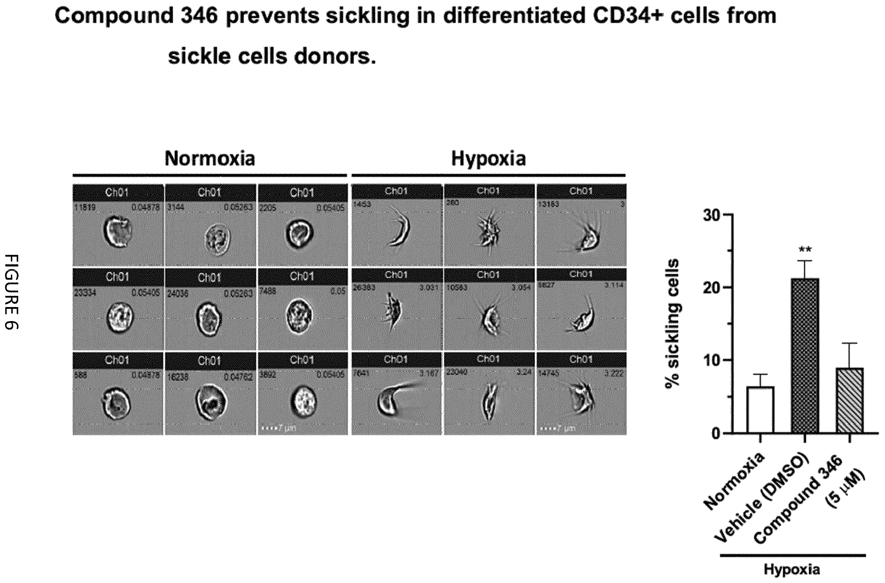

FIG. 6 shows the effect of Compound 346 on the sickling of erythroid cells induced by hypoxia, after 21 days of differentiation, and derived from CD34+ cells from sickle patients. Cells treated with Compound 346 were significantly protected from sickling under hypoxia compared to controls (vehicle). All data are expressed as mean ± SEM (** p < 0.01, N= 3 independent donors).

### DETAILED DESCRIPTION

It was previously shown that increased fetal hemoglobin (HbF), whether endogenous or drug induced, ameliorates the symptoms and complications of SCD by preventing red blood cell sickling through dilution of the concentration of HbS in erythrocytes and inhibiting the polymerization of HbS (Piel et al. Lancet, 2013. 381(9861): p. 142-51.). Although hydroxyurea (HU) is the only US Food and Drug Administration approved HbF-inducing agent for use in adults with SCD (Steinberg, M.H., et al., JAMA, 2003. 289(13): p. 1645-51.), up to 50% of patients do not experience clinical improvement on HU. This variability of HbF-inducing response to HU is still under investigation (Platt, et al., N Engl J Med, 2008. 358(13): p. 1362-9.). New HbF inducing drugs are therefore urgently needed. Finally, Increasing HbF in erythrocytes is also beneficial to treat clinical manifestations of other β-hemoglobinopathies, including β-thalassemia (Ye, L., et al., Proc Natl Acad Sci U S A, 2016. 113(38): p. 10661-5.).

FOX03 (forkhead box 0-3) is a transcription factor hypothesized to be involved in the increase of HbF when overexpressed or activated in CD34+ erythroid cells (Zhang, Y., et al., Blood, 2018. 132(3): p. 321-333.). FOX03 is in the forkhead/winged helix box gene, group O (FoxO) family of proteins that are evolutionarily conserved transcription factors. FOXO transcription factors integrate many important cellular functions and were originally identified as regulators of insulin-related genes. FOXO transcription factors regulate genes controlling several biological processes, including substrate and energy metabolism, protein turnover, cell survival, oxidative stress, proteostasis, apoptosis and cell death, cell cycle regulation, metabolic processes, immunity, inflammation and stem cell maintenance (Morris, B.J., et al., Gerontology, 2015. 61(6): p. 515-25; and Stefanetti, R.J., et al., F1000Res, 2018. 7.). More specifically, FOX03 is required for the maintenance of murine hematopoietic stem cells and functions (Yalcin, S., et al., J Biol Chem, 2008. 283(37): p. 25692-25705; and Rimmele, P., et al., EMBO Rep, 2015. 16(9): p. 1164-76.). Moreover, FOX03 is a key mediator of erythroid terminal maturation, regulating cell cycle in early stages of erythropoiesis and critical for reactive oxygen species (ROS) regulation, enucleation and mitochondrial clearance in late stages (Marinkovic, D., et al., J Clin Invest, 2007. 117(8): p. 2133-44; and Liang, R., et al., PLoS Genet, 2015. 11(10): p. e1005526.). In this context, modulation of FOX03 might influence erythroid disorders as has been reported specifically for hemoglobinopathies (Zhang, Y., et al., Blood, 2018. 132(3): p. 321-333; Pourfarzad, F., et al., Cell Rep, 2013. 4(3): p. 589-600; and Franco, S.S., et al., 2014. 99(2): p. 267-75.). A previous *in vitro* study showed that decreasing FOX03 by shRNA delivery in differentiating human CD34+ erythroid cells decreased expression of HbF, and conversely, over-expression and activation of FOX03 increased HbF expression in these cells. In this study, it was theorized that AMPK phosphorylates and activates FOXO3, and metformin was used to increase cellular AMP and indirectly activate AMPK to increase HbF expression (Zhang, Y., et al., Blood, 2018. 132(3): p. 321-333.). Finally, metformin was shown to prevent RBC sickling in SCD (Zhang, Paikari et al. 2018).

Serum- and glucocorticoid-regulated kinase 1 (SGK1) is a serine/threonine kinase in the AGK kinase family that controls physiological processes such as cell growth, proliferation, migration, and apoptosis (Hayashi, Tapping et al. 2001, Sang, Kong et al. 2020). SGK1 is regulated by multiple ligands (insulin, cAMP, IGF-1, steroids, IL-2 and TGF-β) and phosphorylation by SGK1 modulates the activity of downstream effectors including ion channels (ENaC), Na-Cl cotransporters (NCC), membrane transporters, cellular enzymes (GSK3B) and transcription factors (FOX03a, β-catenin, NF-κB and SP1) (Brunet, Park et al. 2001, Snyder, Olson et al. 2002, Loffing, Flores et al. 2006, Bruhn, Pearson et al. 2010, Boccitto and Kalb 2011, Wang, Hu et al. 2017). It was previously shown that SGK1 mediates survival signals in HEK cells by inhibiting FOXO3a through phosphorylation at Ser-315 (Brunet, Park et al. 2001).

Thus, as it was shown that SGK1 mediates survival signals in HEK cells by inhibiting FOXO3a through phosphorylation at Ser-315 (Brunet, Park et al. 2001), and recently, metformin was shown to induce HbF in erythroid cells through FOXO3a activation and metformin prevents RBC sickling in SCD (Zhang, Paikari et al. 2018), it was hypothesized that inhibition of SGK1 and subsequent alleviation of SGK1-induced FOXO3a inhibition, may induce expression of erythroid cell HbF.

In the present description, the ability of SGK1 to inhibit HbF induction in erythroid cells by culturing CD34+ hematopoietic progenitor stem cells from both healthy and SCD blood donors using a 21-day differentiation protocol is shown. After confirming expression of SGK1 in CD34+ cells by Western blot, SGK1 activity was inhibited using the selective SGK1 inhibitor Compound 346 (Halland, Schmidt et al. 2015, and PCT application Pub. No. WO 2014/140065A1). SGK1 is activated by phosphorylation at Thr256 and target engagement was confirmed through measurement of Thr256 phosphorylation on Western blots. To decipher the effect of SGK1 inhibition on the SGK1 downstream pathway, the inhibition of FOXO3a triggered by SGK1 through evaluation of FOXO3a phosphorylation Ser315 was assessed. In parallel, the level of HbF protein was assessed and F-cells were quantified by flow cytometry. Finally, to evaluate the effect of SGK1 inhibition on RBC sickling, a cell sickling assay was performed upon completion of erythroid differentiation in culture. Fully differentiated CD34+ cells from SCD blood donors were incubated under in hypoxia (2% O₂) for 4 hours and then abnormal shaped cells were analyzed using the Amnis^{®} ImageStream^{®} flow cytometer.

By day 21 of differentiation, HbF protein expression in CD34+ cells increased significantly in cells treated with Compound 346, versus untreated controls. In addition, a combination of SGK1 inhibition and hydroxyurea treatment reveals a potential synergistic induction of HbF. Western blot analysis shows a decrease in phospho-SGK1 phosphorylated at Thr-256 with SGK1 inhibition, confirming target engagement and loss of SGK1 activity. Downstream of SGK1, phospho-FOXO3a phosphorylated at Ser-315 was also decreased significantly following SGK1 inhibition, demonstrating alleviation of FOXO3a inhibition. Finally, in the RBC sickling assay, cells treated with Compound 346 were significantly protected from sickling under hypoxia compared to controls.

In one aspect of the present invention, the use of an SGK1 inhibitor for the treatment of β-hemoglobinopathies such as sickle cell disease or β-thalassemia, is provided. The present invention also relates to the use of an SGK1 inhibitor for increasing fetal hemoglobin (HbF) in erythrocytes.

In one aspect of the present invention, the SGK1 inhibitor is a N-(4-(azaindazol-6-yl)-phenyl)-sulfonamide of the formula I 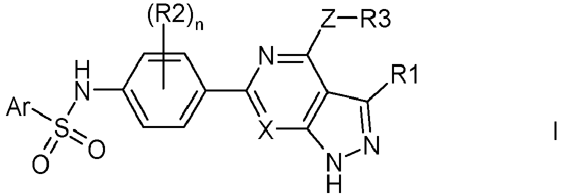 wherein Ar, n, X, Z, R1, R2 and R3 have the meanings indicated below.

It should be understood that all the compounds of the formula I described herein are provided for use for increasing fetal hemoglobin (HbF) in erythrocytes; and/or
for use in the treatment of a β-hemoglobinopathy, particularly for use in the treatment of sickle cell disease or β-thalassemia, more particularly for use in the treatment of sickle cell disease.

Similarly, the pharmaceutical compositions comprising the compounds of the formula I described herein are provided for use for increasing fetal hemoglobin (HbF) in erythrocytes; and/or for use in the treatment of a β-hemoglobinopathy, particularly for use in the treatment of sickle cell disease or β-thalassemia, more particularly for use in the treatment of sickle cell disease.

Similarly, one aspect of the present invention is to provide a method for increasing fetal hemoglobin (HbF) in erythrocytes; and/or a method for the treatment of a β-hemoglobinopathy, particularly a method for the treatment of sickle cell disease or β-thalassemia, more particularly a method for the treatment of sickle cell disease, the method comprising administering a N-(4-(azaindazol-6-yl)-phenyl)-sulfonamide of the formula I 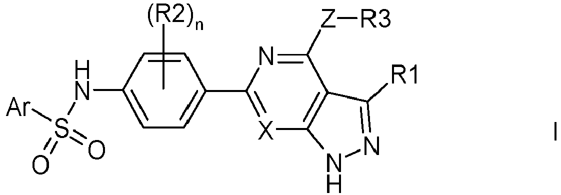 wherein Ar, n, X, Z, R1, R2 and R3 have the meanings indicated below. Thus, a subject of the present invention are the compounds of the formula I, in any of their stereoisomeric forms or a mixture of stereoisomeric forms in any ratio, and the pharmaceutically acceptable salts thereof, 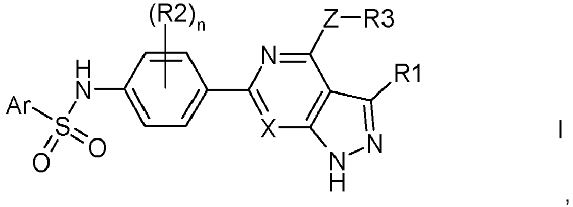 for use for increasing fetal hemoglobin (HbF) in erythrocytes; and/or for use in the treatment of a β-hemoglobinopathy, particularly for use in the treatment of sickle cell disease or β-thalassemia, more particularly for use in the treatment of sickle cell disease,
wherein
Ar is selected from the series consisting of phenyl and a 5-membered or 6-membered, monocyclic, aromatic, heterocyclic group which comprises 1, 2 or 3 identical or different ring heteroatoms selected from the series consisting of nitrogen, oxygen and sulfur, and is bonded via a ring carbon atom, which all are unsubstituted or substituted by one or more identical or different substituents R5;
n is selected from the series consisting of 0, 1 and 2;
X is selected from the series consisting of N and CH;
Z is selected from the series consisting of a direct bond, O, S and N(R10);
R1 is selected from the series consisting of H, -N(R11)-R12, -N(R13)-C(O)-R14, -N(R13)-S(O)₂-R15, -N(R13)-C(O)-NH-R16, (C₁-C₄)-alkyl and -(C₁-C₄)-alkyl-O-R17;
R2 is selected from the series consisting of halogen, (C₁-C₄)-alkyl, -O-(C₁-C₄)-alkyl and -CN;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, (C₃-C₇)-cycloalkyl, -(C₁-C₄)-alkyl-(C₃-C₇)-cycloalkyl, -O-(C₁-C₄)-alkyl, -O-(C₃-C₇)-cycloalkyl, -O-(C₁-C₄)-alkyl-(C₃-C₇)-cycloalkyl, -C(O)-N(R6)-R7 and -CN,
and two groups R5 bonded to adjacent ring carbon atoms in Ar, together with the carbon atoms carrying them, can form a 5-membered to 8-membered, monocyclic, unsaturated ring which comprises 0, 1 or 2 identical or different ring heteroatoms selected from the series consisting of nitrogen, oxygen and sulfur, and which is unsubstituted or substituted by one or more identical or different substituents R8;
R6 and R7 are independently of one another selected from the series consisting of H and (C₁-C₄)-alkyl;
R8 is selected from the series consisting of halogen, (C₁-C₄)-alkyl, -O-(C₁-C₄)-alkyl and -CN;
R10 is selected from the series consisting of H and (C₁-C₄)-alkyl;
R11 and R12 are independently of one another selected from the series consisting of H, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl, wherein phenyl is unsubstituted or substituted by one or more identical or different substituents R50,
or R11 and R12, together with the nitrogen atom carrying them, form a 4-membered to 7-membered, monocyclic, saturated, heterocyclic group which, in addition to the nitrogen atom carrying R11 and R12, comprises 0 or 1 further ring heteroatom selected from the series consisting of nitrogen, oxygen and sulfur, and which is unsubstituted or substituted by one or more identical or different substituents selected from the series consisting of fluorine and (C₁-C₄)-alkyl;
R13 is selected from the series consisting of H, (C₁-C₄)-alkyl and (C₃-C₇)-cycloalkyl;
R14 and R16 are independently of one another selected from the series consisting of (C₁-C₈)-alkyl, (C₃-C₇)-cycloalkyl, -(C₁-C₄)-alkyl-(C₃-C₇)-cycloalkyl, phenyl, -(C₁-C₄)-alkyl-phenyl, Het2 and -(C₁-C₄)-alkyl-Het2, wherein (C₁-C₈)-alkyl and (C₃-C₇)-cycloalkyl all are unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R15 is selected from the series consisting of (C₁-C₈)-alkyl, phenyl and Het3, wherein phenyl and Het3 all are unsubstituted or substituted by one or more identical or different substituents R50;
R17 is selected from the series consisting of H and (C₁-C₄)-alkyl;
R30 is a 3-membered to 12-membered, monocyclic or bicyclic, saturated, partially unsaturated or aromatic, cyclic group which comprises 0, 1, 2 or 3 identical or different ring heteroatoms selected from the series consisting of nitrogen, oxygen and sulfur, which is unsubstituted or substituted by one or more identical or different substituents R32;
R31 is selected from the series consisting of halogen, -OH, -O-(C₁-C₄)-alkyl, -O-(C₃-C₇)-cycloalkyl, -O-(C₁-C₄)-alkyl-(C₃-C₇)-cycloalkyl, -N(R33)-R34, -CN and -C(O)-N(R35)-R36;
R32 is selected from the series consisting of halogen, (C₁-C₄)-alkyl, (C₃-C₇)-cycloalkyl, -(C₁-C₄)-alkyl-(C₃-C₇)-cycloalkyl, -(C₁-C₄)-alkyl-O-R37, -(C₁-C₄)-alkyl-N(R38)-R39, -(C₁-C₄)-alkyl-CN,-C(O)-(C₁-C₄)-alkyl, -CN, -OH, =O, -O-(C₁-C₄)-alkyl, -N(R40)-R41, -C(O)-O-(C₁-C₄)-alkyl and-C(O)-N(R42)-R43;
R33, R34, R35, R36, R37, R38, R39, R40, R41, R42 and R43 are independently of one another selected from the series consisting of H and (C₁-C₄)-alkyl;
R50 is selected from the series consisting of halogen, (C₁-C₄)-alkyl, -O-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen, oxygen and sulfur, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated, partially unsaturated or aromatic, heterocyclic group which comprises 1 or 2 identical or different ring heteroatoms selected from the series consisting of nitrogen, oxygen and sulfur, and is bonded via a ring carbon atom;
Het3 is a 5-membered or 6-membered, monocyclic, aromatic, heterocyclic group which comprises 1, 2 or 3 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

If structural elements such as groups, substituents or numbers, for example, can occur several times in the compounds of the formula I, they are all independent of each other and can in each case have any of the indicated meanings, and they can in each case be identical to or different from any other such element. In a dialkylamino group, for example, the alkyl groups can be identical or different.

Alkyl groups, i.e. saturated hydrocarbon residues, can be linear (straight-chain) or branched. This also applies if these groups are substituted or are part of another group, for example an -O-alkyl group (alkyloxy group, alkoxy group) or an HO-substituted alkyl group (-alkyl-OH, hydroxyalkyl group). Depending on the respective definition, the number of carbon atoms in an alkyl group can be 1, 2, 3, 4, 5, 6, 7 or 8, or 1, 2, 3, 4, 5 or 6, or 1, 2, 3 or 4, or 1, 2 or 3, or 1 or 2, or 1. Examples of alkyl are methyl, ethyl, propyl including n-propyl and isopropyl, butyl including n-butyl, sec-butyl, isobutyl and tert-butyl, pentyl including n-pentyl, 1-methylbutyl, isopentyl, neopentyl and tert-pentyl, hexyl including n-hexyl, 2,2-dimethylbutyl, 3,3-dimethylbutyl, 2-methylpentyl, 3-methylpentyl and isohexyl, heptyl including n-heptyl, and octyl including n-octyl and 2,2-dimethylhexyl. Examples of -O-alkyl groups are methoxy, ethoxy, n-propoxy, isopropoxy, n-butoxy, isobutoxy, tert-butoxy, n-pentoxy.

A substituted alkyl group can be substituted in any positions, provided that the respective compound is sufficiently stable and is suitable as a pharmaceutical active compound. The prerequisite that a specific group and a compound of the formula I are sufficiently stable and suitable as a pharmaceutical active compound, applies in general with respect to the definitions of all groups in the compounds of the formula I. Independently of any other substituents which can be present on an alkyl group, and unless specified otherwise, alkyl groups can be substituted by one or more fluorine substituents, for example by 1, 2, 3, 4, 5, 6, 7, 8, 9, 10 or 11 fluorine substituents, or by 1, 2, 3, 4 or 5 fluorine substituents, or by 1, 2 or 3 fluorine substituents, which can be located in any positions. I.e., independently of any other substituents which can be present on an alkyl group, an alkyl group can be unsubstituted by fluorine substituents, i.e. not carry fluorine substituents, or substituted by fluorine substituents, wherein all alkyl groups in the compounds of the formula I are independent of one another with regard to the optional substitution by fluorine substituents. For example, in a fluoro-substituted alkyl group one or more methyl groups can carry three fluorine substituents each and be present as trifluoromethyl groups, and/or one or more methylene groups (CH₂) can carry two fluorine substituents each and be present as difluoromethylene groups. The explanations with respect to the substitution of a group by fluorine also apply if the group additionally carries other substituents and/or is part of another group, for example of an -O-alkyl group. Examples of fluoro-substituted alkyl groups are -CF₃ (trifluoromethyl), -CHF₂, -CH₂F, -CHF-CF₃, -CHF-CHF₂, -CHF-CH₂F, -CH₂-CF₃, -CH₂-CHF₂, -CH₂-CH₂F, -CF₂-CF₃, -CF₂-CHF₂, -CF₂-CH₂F, -CH₂-CHF-CF₃, -CH₂-CHF-CHF₂, -CH₂-CHF-CH₂F,-CH₂-CH₂-CF₃, -CH₂-CH₂-CHF₂, -CH₂-CH₂-CH₂F, -CH₂-CF₂-CF₃, -CH₂-CF₂-CHF₂, -CH₂-CF₂-CH₂F, -CHF-CHF-CF₃, -CHF-CHF-CHF₂, -CHF-CHF-CH₂F, -CHF-CH₂-CF₃, -CHF-CH₂-CHF₂, -CHF-CH₂-CH₂F, -CHF-CF₂-CF₃, -CHF-CF₂-CHF₂, -CHF-CF₂-CH₂F, -CF₂-CHF-CF₃, -CF₂-CHF-CHF₂,-CF₂-CHF-CH₂F, -CF₂-CH₂-CF₃, -CF₂-CH₂-CHF₂, -CF₂-CH₂-CH₂F, -CF₂-CF₂-CF₃, -CF₂-CF₂-CHF₂, or -CF₂-CF₂-CH₂F. Examples of fluoro-substituted -O-alkyl groups are trifluoromethoxy (-O-CF₃), 2,2,2-trifluoroethoxy, pentafluoroethoxy and 3,3,3-trifluoropropoxy. With respect to all groups or substituents in the compounds of the formula I which can be an alkyl group which can generally contain one or more fluorine substituents, as an example of groups or substituents containing fluorine-substituted alkyl, which may be included in the definition of the group or substituent, the group CF₃ (trifluoromethyl), or a respective group such as -O-CF₃, may be mentioned.

The above explanations with respect to alkyl groups apply correspondingly to alkyl groups which in the definition of a group in the compounds of the formula I are bonded to two adjacent groups, or linked to two groups, and may be regarded as divalent alkyl groups (alkanediyl groups), like in the case of the alkyl part of a substituted alkyl group. Thus, such groups can also be linear or branched, the bonds to the adjacent groups can be located in any positions and can start from the same carbon atom or from different carbon atoms, and they can be unsubstituted or substituted by fluorine substituents independently of any other substituents. Examples of such divalent alkyl groups are -CH₂-, -CH₂-CH₂-, -CH₂-CH₂-CH₂-, -CH₂-CH₂-CH₂-CH₂-, -CH₂-CH₂-CH₂-CH₂-CH₂-, -CH₂-CH₂-CH₂-CH₂-CH₂-CH₂-, -CH(CH₃)-, -C(CH₃)₂-, -CH(CH₃)-CH₂-, -CH₂-CH(CH₃)-, -C(CH₃)₂-CH₂-, -CH₂-C(CH₃)₂-. Examples of fluoro-substituted alkanediyl groups, which can contain 1, 2, 3, 4, 5 or 6 fluorine substituents, or 1, 2, 3 or 4 fluorine substituents, or 1 or 2 fluorine substituents, for example, are -CF₂-, -CHF-, -CHF-CHF₂-, -CHF-CHF-, -CH₂-CF₂-, -CH₂-CHF-, -CF₂-CF₂-, -CF₂-CHF-, -CH₂-CHF-CF₂-, -CH₂-CHF-CHF-, -CH₂-CH₂-CF₂-, -CH₂-CH₂-CHF, -CH₂-CF₂-CF₂-, -CH₂-CF₂-CHF-, -CHF-CHF-CF₂-, -CHF-CHF-CHF-, -CHF-CH₂-CF₂-, -CHF-CH₂-CHF-, -CHF-CF₂-CF₂-, -CHF-CF₂-CHF-, -CF₂-CHF-CF₂-, -CF₂-CHF-CHF-, -CF₂-CH₂-CF₂-, -CF₂-CH₂-CHF-, -CF₂-CF₂-CF₂-, or -CF₂-CF₂-CHF-.

The number of ring carbon atoms in a (C₃-C₇)-cycloalkyl group can be 3, 4, 5, 6 or 7. Examples of cycloalkyl are cyclopropyl, cyclobutyl, cyclopentyl, cyclohexyl and cycloheptyl. Independently of any other substituents which can be present on a cycloalkyl group, and unless specified otherwise, cycloalkyl groups can be substituted by one or more (C₁-C₄)-alkyl substituents, for example by 1, 2, 3 or 4 identical or different (C₁-C₄)-alkyl substituents, for example by methyl groups, which can be located in any positions. I.e., independently of any other substituents which can be present on a cycloalkyl group, a cycloalkyl group can be unsubstituted by (C₁-C₄)-alkyl substituents, i.e. not carry (C₁-C₄)-alkyl substituents, or substituted by (C₁-C₄)-alkyl substituents, wherein all cycloalkyl groups in the compounds of the formula I are independent of one another with regard to the optional substitution by (C₁-C₄)-alkyl substituents. Examples of such alkyl-substituted cycloalkyl groups are 1-methylcyclopropyl, 2,2-dimethylcyclopropyl, 1-methylcyclopentyl, 2,3-dimethylcyclopentyl, 1-methylcyclohexyl, 4-methylcyclohexyl, 4-isopropylcyclohexyl, 4-tert-butylcyclohexyl, 3,3,5,5-tetramethylcyclohexyl. Independently of any other substituents including (C₁-C₄)-alkyl substituents which can be present on a cycloalkyl group, and unless specified otherwise, cycloalkyl groups can further be substituted by one or more fluorine substituents, for example by 1, 2, 3, 4, 5, 6, 7, 8, 9, 10 or 11 fluorine substituents, or by 1, 2, 3, 4 or 5 fluorine substituents, or by 1, 2 or 3 fluorine substituents, which can be located in any positions and can also be present in a (C₁-C₄)-alkyl substituent. I.e., independently of any other substituents which can be present on a cycloalkyl group, a cycloalkyl group can be unsubstituted by fluorine substituents, i.e. not carry fluorine substituents, or substituted by fluorine substituents, wherein all cycloalkyl groups in the compounds of the formula I are independent of one another with regard to the optional substitution by fluorine substituents. Examples of fluoro-substituted cycloalkyl groups are 1-fluorocyclopropyl, 2,2-difluorocyclopropyl, 3,3-difluorocyclobutyl, 1-fluorocyclohexyl, 4,4-difluorocyclohexyl, 3,3,4,4,5,5-hexafluorocyclohexyl. Cycloalkyl groups can also be substituted simultaneously by fluorine and alkyl. Examples of the group -(C₁-C₄)-alkyl-(C₃-C₇)-cycloalkyl are cyclopropylmethyl-, cyclobutylmethyl-, cyclopentylmethyl-, cyclohexylmethyl-, cycloheptylmethyl-, 1-cyclopropylethyl-, 2-cyclopropylethyl-, 1-cyclobutylethyl-, 2-cyclobutylethyl-, 1-cyclopentylethyl-, 2-cyclopentylethyl-, 1-cyclohexylethyl-, 2-cyclohexylethyl-, 1-cycloheptylethyl-, 2-cycloheptylethyl-. In one embodiment of the invention, a -(C₁-C₄)-alkyl-(C₃-C₇)-cycloalkyl group in any one or more occurrences of such a group, independently of any other occurrences, is a -(C₁-C₂)-alkyl-(C₃-C₇)-cycloalkyl group, in another embodiment a -CH₂-(C₃-C₇)-cycloalkyl group. In the group -(C₁-C₄)-alkyl-(C₃-C₇)-cycloalkyl, and likewise in all other groups, the terminal hyphen denotes the free bond via which the group is bonded, and thus indicates via which subgroup a group composed of subgroups is bonded.

In substituted phenyl groups, including phenyl groups representing Ar, for example, the substituents can be located in any positions. In monosubstituted phenyl groups, the substituent can be located in position 2, in position 3 or in position 4. In disubstituted phenyl groups, the substituents can be located in positions 2 and 3, in positions 2 and 4, in positions 2 and 5, in positions 2 and 6, in positions 3 and 4, or in positions 3 and 5. In trisubstituted phenyl groups, the substituents can be located in positions 2, 3 and 4, in positions 2, 3 and 5, in positions 2, 3 and 6, in positions 2, 4 and 5, in positions 2, 4 and 6, or in positions 3, 4 and 5. If a phenyl group carries four substituents, some of which can be fluorine atoms, for example, the substituents can be located in positions 2, 3, 4 and 5, in positions 2, 3, 4 and 6, or in positions 2, 3, 5 and 6. If a polysubstituted phenyl group or any other polysubstituted group carries different substituents, each substituent can be located in any suitable position, and the present invention comprises all positional isomers. The number of substituents in a substituted phenyl group can be 1, 2, 3, 4 or 5. In one embodiment of the invention, the number of substituents in a substituted phenyl group, is 1, 2, 3 or 4, in another embodiment 1, 2 or 3, in another embodiment 1 or 2, in another embodiment 1, wherein the number of substituents in any occurrence of such a substituted group is independent of the number of substituents in other occurrences.

In heterocyclic groups, including the groups Het1, Het2, Het3, heterocyclic groups representing Ar, heterocyclic groups R30 and other heterocyclic rings which can be present in the compounds of the formula I, such as rings formed by two group together with the atom or atoms carrying them, the hetero ring members can be present in any combination and located in any suitable ring positions, provided that the resulting group and the compound of the formula I are suitable and sufficiently stable as a pharmaceutical active compound. In one embodiment of the invention, two oxygen atoms in any heterocyclic ring in the compounds of the formula I cannot be present in adjacent ring positions. In another embodiment of the invention, two hetero ring members selected from the series consisting of oxygen atoms and sulfur atoms cannot be present in adjacent ring positions in any heterocyclic ring in the compounds of the formula I. In another embodiment of the invention, two hetero ring members selected from the series consisting of nitrogen atoms carrying an exocyclic group like a hydrogen atom or a substituent, sulfur atoms and oxygen atoms cannot be present in adjacent ring positions in any heterocyclic ring in the compounds of the formula I. The choice of hetero ring members in an aromatic heterocyclic ring is limited by the prerequisite that the ring is aromatic, i.e. it comprises a cyclic system of six delocalized pi electrons in case of a monocycle or 10 delocalized pi electrons in case of a bicycle. Monocyclic aromatic heterocycles are 5-membered or 6-membered rings and, in the case of a 5-membered ring, comprise one ring heteroatom selected from the series consisting of oxygen, sulfur and nitrogen, wherein this ring nitrogen carries an exocyclic group like a hydrogen atom or a substituent, and optionally one or more further ring nitrogen atoms, and, in the case of a 6-membered ring, comprise one or more nitrogen atoms as ring heteroatoms, but no oxygen atoms and sulfur atoms as ring heteroatoms. Heterocyclic groups in the compounds of the formula I can be bonded via a ring carbon atom or a ring nitrogen atom, unless specified otherwise in the definition of the respective group, wherein a heterocyclic group can be bonded via any suitable carbon atom or nitrogen atom, respectively, in the ring. In substituted heterocyclic groups, the substituents can be located in any positions.

The number of ring heteroatoms which can be present in a heterocyclic group in the compounds of the formula I, the number of ring members which can be present, and the degree of saturation, or hydrogenation, i.e. whether the heterocyclic group is saturated and does not contain a double bond within the ring, or whether it is partially unsaturated and contains one or more, for example one or two, double bonds within the ring but is not aromatic, or whether it is aromatic and thus contains two double bonds within the ring in the case of a 5-membered monocyclic aromatic heterocycle and three double bonds within the ring in the case of a 6-membered monocyclic aromatic heterocycle, for example, is specified in the definitions of the individual groups in the compounds of the formula I. Examples of heterocyclic ring systems, from which heterocyclic groups in the compounds of the formula I including, for example, Het1, Het2, Het3, heterocyclic groups representing Ar, heterocyclic groups R30 and rings formed by two groups together with the atom or atoms carrying them, can be derived, and from any one or more of which any of the heterocyclic groups in the compounds of the formula I is selected in one embodiment of the invention, provided that the ring system is comprised by the definition of the group, are oxetane, thietane, azetidine, furan, tetrahydrofuran, thiophene, tetrahydrothiophene, pyrrole, pyrroline, pyrrolidine, [1,3]dioxole, [1,3]dioxolane, isoxazole ([1,2]oxazole), isoxazoline, isoxazolidine, oxazole ([1,3]oxazole), oxazoline, oxazolidine, isothiazole ([1,2]thiazole), isothiazoline, isothiazolidine, thiazole ([1,3]thiazole), thiazoline, thiazolidine, pyrazole, pyrazoline, pyrazolidine, imidazole, imidazoline, imidazolidine, [1,2,3]triazole, [1,2,4]triazole, [1,2,4]oxadiazole, [1,3,4]oxadiazole, [1,2,5]oxadiazole, [1,2,4]thiadiazole, pyran, tetrahydropyran, thiopyran, tetrahydrothiopyran, 2,3-dihydro[1,4]dioxine, 1,4-dioxane, pyridine, 1,2,5,6-tetrahydropyridine, piperidine, morpholine, thiomorpholine, piperazine, pyridazine, pyrimidine, pyrazine, [1,2,4]triazine, oxepane, thiepane, azepane, [1,3]diazepane, [1,4]diazepane, [1,4]oxazepane, [1,4]thiazepane, benzofuran, isobenzofuran, benzothiophene (benzo[b]thiophene), 1H-indole, 2,3-dihydro-1H-indole, 2H-isoindole, 2-aza-spiro[4.4]nonane, 2-aza-spiro[4.5]decane, 2-azaspiro[4.6]undecane, 2-aza-spiro[5.5]undecane, 3-aza-spiro[5.5]undecane, 6-azaspiro[2.5]octane, 7-aza-spiro[3.5]nonane, 8-aza-spiro[4.5]decane, benzo[1,3]dioxole, benzoxazole, benzthiazole, 1H-benzimidazole, chroman, isochroman, thiochroman, benzo[1,4]dioxane, 3,4-dihydro-2H-benzo[b][1,4]dioxepine (3,4-dihydro-2H-1,5-benzodioxepine), 3,4-dihydro-2H-benzo[1,4]oxazine, 1-oxa-8-aza-spiro[4.5]decane, 2-oxa-6-azaspiro[3,3]heptane, 2-oxa-6-aza-spiro[3.4]octane, 2-oxa-6-aza-spiro[3.5]nonane, 2-oxa-7-azaspiro[3,5]nonane, 8-oxa-2-aza-spiro[4.5]decane, 3,4-dihydro-2H-benzo[1,4]thiazine, quinoline, 5,6,7,8-tetrahydroquinoline, isoquinoline, 5,6,7,8-tetrahydroisoquinoline, cinnoline, quinazoline, quinoxaline, phthalazine and [1,8]naphthyridine, which can all be unsubstituted or substituted in any suitable positions as specified in the definition of the respective group in the compounds of the formula I, wherein the given degree of unsaturation is by way of example only, and in the individual groups also ring systems with a higher or lower degree of saturation or unsaturation can be present as specified in the definition of the group. Ring sulfur atoms, in particular in saturated and partially unsaturated heterocycles, can generally carry one or two oxo groups, i.e. doubly bonded oxygen atoms (=O), and in such heterocycles, besides a ring sulfur atom, also an S(O) group (S(=O)) and an S(O)₂ group (S(=O)₂) can be present as hetero ring member.

As mentioned, unless specified otherwise in the definition of the respective group in the compounds of the formula I, heterocyclic groups can be bonded via any suitable ring carbon atom and ring nitrogen atom, for example in the case of heterocyclic groups representing R30. Thus, for example, among others can an oxetane and a thietane ring be bonded via positions 2 and 3, an azetidine ring via positions 1, 2 and 3, a furan ring, a tetrahydrofuran ring, a thiophene ring and a tetrahydrothiophene ring via positions 2 and 3, a pyrrole ring and a pyrrolidine ring via positions 1, 2 and 3, an isoxazole ring and an isothiazole ring via positions 3, 4 and 5, a pyrazole ring via positions 1, 3, 4 and 5, an oxazole ring and a thiazole ring via positions 2, 4 and 5, an imidazole ring and an imidazolidine ring via positions 1, 2, 4 and 5, a tetrahydropyran ring and a tetrahydrothiopyran ring via positions 2, 3 and 4, a 1,4-dioxane ring via position 2, a pyridine ring via positions 2, 3 and 4, a piperidine ring via positions 1, 2, 3 and 4, a morpholine ring and a thiomorpholine ring via positions 2, 3 and 4, a piperazine ring via positions 1 and 2, a pyrimidine ring via positions 2, 4 and 5, a pyrazine ring via position 2, an azepane ring via positions 1, 2, 3 and 4, a benzofuran ring and a benzothiophene ring via positions 2, 3, 4, 5, 6 and 7, a 1H-indole ring and a 2,3-dihydro-1H-indole ring via positions 1, 2, 3, 4, 5, 6 and 7, a benzo[1,3]dioxole ring via positions 4, 5, 6 and 7, a benzoxazole ring and a benzthiazole ring via positions 2, 4, 5, 6 and 7, a 1H-benzimidazole ring via positions 1, 2, 4, 5, 6 and 7, a benzo[1,4]dioxane ring via positions 5, 6, 7 and 8, a quinoline ring via positions 2, 3, 4, 5, 6, 7 and 8, a 5,6,7,8-tetrahydroquinoline ring via positions 2, 3 and 4, an isoquinoline ring via positions 1, 3, 4, 5, 6, 7 and 8, a 5,6,7,8-tetrahydroisoquinoline ring via positions 1, 3 and 4, wherein the resulting residues of the heterocyclic groups can all be unsubstituted or substituted in any suitable positions as specified in the definition of the respective group in the compounds of the formula I.

Halogen is fluorine, chlorine, bromine or iodine. In one embodiment of the invention, halogen is in any of its occurrences fluorine, chlorine or bromine, in another embodiment fluorine or chlorine, in another embodiment fluorine, in another embodiment chlorine, wherein all occurrences of halogen are independent of each other.

The present invention comprises all stereoisomeric forms of the compounds of the formula I, for example all enantiomers and diastereomers including cis/trans isomers. The invention likewise comprises mixtures of two or more stereoisomeric forms, for example mixtures of enantiomers and/or diastereomers including cis/trans isomers, in all ratios. Asymmetric centers contained in the compounds of the formula I can all independently of each other have S configuration or R configuration. The invention relates to enantiomers, both the levorotatory and the dextrorotatory antipode, in enantiomerically pure form and essentially enantiomerically pure form, and in the form of their racemate, i.e. a mixture of the two enantiomers in molar ratio of 1:1, and in the form of mixtures of the two enantiomers in all ratios. The invention likewise relates to diastereomers in the form of pure and essentially pure diastereomers and in the form of mixtures of two or more diastereomers in all ratios. The invention also comprises all cis/trans isomers of the compounds of the formula I in pure form and essentially pure form, and in the form of mixtures of the cis isomer and the trans isomer in all ratios. Cis/trans isomerism can occur in substituted rings. The preparation of individual stereoisomers, if desired, can be carried out by resolution of a mixture according to customary methods, for example, by chromatography or crystallization, or by use of stereochemically uniform starting compounds in the synthesis, or by stereoselective reactions. Optionally, before a separation of stereoisomers a derivatization can be carried out. The separation of a mixture of stereoisomers can be carried out at the stage of the compound of the formula I or at the stage of an intermediate in the course of the synthesis. For example, in the case of a compound of the formula I containing an asymmetric center the individual enantiomers can be prepared by preparing the racemate of the compound of the formula I and resolving it into the enantiomers by high pressure liquid chromatography on a chiral phase according to standard procedures, or resolving the racemate of any intermediate in the course of its synthesis by such chromatography or by crystallization of a salt thereof with an optically active amine or acid and converting the enantiomers of the intermediate into the enantiomeric forms of the final compound of the formula I, or by performing an enantioselective reaction in the course of the synthesis. The invention also comprises all tautomeric forms of the compounds of the formula I.

Besides the free compounds of the formula I, i.e. compounds in which acidic and basic groups are not present in the form of a salt, the present invention comprises also salts of the compounds of the formula I, in particular their physiologically acceptable salts, or toxicologically acceptable salts, or pharmaceutically acceptable salts, which can be formed on one or more acidic or basic groups in the compounds of the formula I, for example on basic heterocyclic moieties. The compounds of the formula I may thus be deprotonated on an acidic group by an inorganic or organic base and be used, for example, in the form of the alkali metal salts. Compounds of the formula I comprising at least one basic group may also be prepared and used in the form of their acid addition salts, for example in the form of pharmaceutically acceptable salts with inorganic acids and organic acids, such as salts with hydrochloric acid and thus be present in the form of the hydrochlorides, for example. Salts can in general be prepared from acidic and basic compounds of the formula I by reaction with an acid or base in a solvent or diluent according to customary procedures. If the compounds of the formula I simultaneously contain an acidic and a basic group in the molecule, the invention also includes internal salts (betaines, zwitterions) in addition to the salt forms mentioned. The present invention also comprises all salts of the compounds of the formula I which, because of low physiological tolerability, are not directly suitable for use as a pharmaceutical, but are suitable as intermediates for chemical reactions or for the preparation of physiologically acceptable salts, for example by means of anion exchange or cation exchange.

In one embodiment of the invention, an aromatic heterocycle representing the group Ar comprises 1 or 2 identical or different ring heteroatoms, in another embodiment 1 or 2 identical or different ring heteroatoms which are selected from the series consisting of nitrogen and sulfur. In another embodiment, an aromatic heterocycle representing Ar is a 5-membered heterocycle which comprises 1 or 2 identical or different ring heteroatoms which are selected from the series consisting of nitrogen and sulfur, or it is a 6-membered heterocycle which comprises 1 or 2 ring heteroatoms which are nitrogen atoms, in another embodiment it is a 5-membered heterocycle which comprises 1 or 2 identical or different ring heteroatoms which are selected from the series consisting of nitrogen and sulfur, which heterocycles are all unsubstituted or substituted by one or more substituents identical or different R5. In another embodiment, an aromatic heterocycle representing Ar is selected from the series consisting of thiophene, thiazole, pyrazole, imidazole, pyridine, pyridazine, pyrimidine and pyrazine, in another embodiment from the series consisting of thiophene, thiazole, pyrazole, imidazole and pyridine, in another embodiment from the series consisting of thiophene, thiazole, pyrazole and imidazole, in another embodiment from the series consisting of thiophene and pyrazole, in another embodiment it is thiophene, and in another embodiment it is pyrazole, which heterocycles are all unsubstituted or substituted by one or more identical or different substituents R5. In one embodiment of the invention, Ar is phenyl which is unsubstituted or substituted by one or more identical or different substituents R5, in another embodiment Ar is phenyl which is substituted by one or more identical or different substituents R5, in another embodiment Ar is a 5-membered or 6-membered aromatic heterocycle which is unsubstituted or substituted by one or more identical or different substituents R5, and in another embodiment Ar is a 5-membered or 6-membered aromatic heterocycle which is substituted by one or more identical or different substituents R5. In one embodiment, Ar is substituted by one or more identical or different substituents R5. In one embodiment of the invention, the number of identical or different substituents R5 which can be present in the group Ar is 1, 2, 3 or 4, in another embodiment it is 1, 2 or 3, in another embodiment it is 1 or 2, in another embodiment it is 1, in another embodiment it is 2, 3 or 4, in another embodiment it is 2 or 3, in another embodiment it is 3, in another embodiment it is 2.

In one embodiment of the invention, the number n is selected from the series consisting of 0 and 1, in another embodiment from the series consisting of 1 and 2, in another embodiment it is 1, in another embodiment it is 0.

In one embodiment of the invention, X is N, and the compounds of the formula I thus are N-[4-(1H-pyrazolo[3,4-d]pyrimidin-6-yl)-phenyl]-sulfonamides. In another embodiment of the invention, X is CH, and the compounds of the formula I thus are N-[4-(1H-pyrazolo[4,3-c]pyridin-6-yl)-phenyl]-sulfonamides.

In case the divalent group Z is a direct bond, the group R3 is directly bonded via a single bond to the ring carbon in position 4 of the bicyclic ring system depicted in formula I which carries Z, and the compound of the formula I thus is a compound of the formula la, wherein Ar, n, X, R1, R2 and R3 are defined as in the compounds of the formula I. In one embodiment of the invention, Z is selected from the series 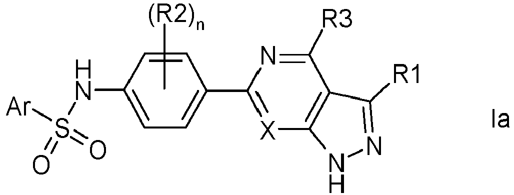 consisting of a direct bond, O and N(R10), in another embodiment from the series consisting of a direct bond and O, in another embodiment from the series consisting of a direct bond and N(R10), in another embodiment from the series consisting of O, S and N(R10), in another embodiment from the series consisting of O and N(R10), in another embodiment Z is a direct bond, in another embodiment Z is O, i.e. an oxygen atom, in another embodiment Z is S, i.e. a sulfur atom, and in another embodiment Z is N(R10), i.e. a nitrogen atom carrying the atom or group R10.

In one embodiment of the invention, R1 is selected from the series consisting of H, -N(R11)-R12, -N(R13)-C(O)-R14, -N(R13)-S(O)₂-R15, -N(R13)-C(O)-NH-R16 and (C₁-C₄)-alkyl, in another embodiment from the series consisting of H, -N(R11)-R12, -N(R13)-C(O)-R14, -N(R13)-S(O)₂-R15 and -N(R13)-C(O)-NH-R16, in another embodiment from the series consisting of -N(R11)-R12, -N(R13)-C(O)-R14, -N(R13)-S(O)₂-R15, -N(R13)-C(O)-NH-R16 and (C₁-C₄)-alkyl, in another embodiment from the series consisting of-N(R11)-R12, -N(R13)-C(O)-R14, -N(R13)-S(O)₂-R15 and -N(R13)-C(O)-NH-R16, in another embodiment from the series consisting of -N(R11)-R12 and N(R13)-C(O)-R14, and in another embodiment R1 is -N(R11)-R12. In another embodiment, R1 is selected from the series consisting of H, -N(R11)-R12, -N(R13)-C(O)-R14 and (C₁-C₄)-alkyl, in another embodiment from the series consisting of H, -N(R11)-R12 and (C₁-C₄)-alkyl, and in another embodiment from the series consisting of - N(R11)-R12 and (C₁-C₄)-alkyl. In another embodiment, R1 is selected from the series consisting of H, (C₁-C₄)-alkyl and -(C₁-C₄)-alkyl-O-R17, in another embodiment from the series consisting of H and (C₁-C₄)-alkyl, in another embodiment R1 is H, in another embodiment R1 is (C₁-C₄)-alkyl, and in another embodiment R1 is -(C₁-C₄)-alkyl-O-R17. In one embodiment, a (C₁-C₄)-alkyl group representing R1 or present in -(C₁-C₄)-alkyl-O-R17 is (C₁-C₃)-alkyl, in another embodiment it is (C₁-C₂)-alkyl, in another embodiment it is methyl. As applies to alkyl groups in general, in all these embodiments an alkyl group representing R1 or present in R1, for example the group (C₁-C₄)-alkyl representing R1, can be substituted by one or more fluorine substituents, i.e., independently of any other substituents on the alkyl group it is unsubstituted by fluorine substituents or it is substituted by fluorine substituents. In one embodiment, an alkyl group representing R1 or present in R1, for example the group (C₁-C₄)-alkyl representing R1, independently of any other substituents on the alkyl group, is unsubstituted by fluorine substituents. In another embodiment, an alkyl group representing R1 or present in R1, for example the group (C₁-C₄)-alkyl representing R1, independently of any other substituents on the alkyl group, is substituted by one or more fluorine substituents, for example by 1, 2, 3, 4 or 5 fluorine substituents or by 1, 2 or 3 fluorine substituents.

In one embodiment of the invention, R2 is selected from the series consisting of halogen, (C₁-C₄)-alkyl and -O-(C₁-C₄)-alkyl, in another embodiment from the series consisting of halogen and (C₁-C₄)-alkyl, in another embodiment from the series consisting of halogen and -O-(C₁-C₄)-alkyl, in another embodiment from the series consisting of halogen, -O-(C₁-C₄)-alkyl and -CN, in another embodiment from the series consisting of halogen and -CN, in another embodiment from the series consisting of halogen, wherein in all these embodiments alkyl can be substituted by one or more, for example by 1, 2, 3, 4 or 5, or by 1, 2 or 3, fluorine substituents, as applies to alkyl groups in general. In one embodiment, a (C₁-C₄)-alkyl group representing R2 or present in R2 is (C₁-C₃)-alkyl, in another embodiment it is (C₁-C₂)-alkyl, in another embodiment it is methyl. In one embodiment, halogen representing R2 is selected from the series consisting of fluorine and chlorine, in another embodiment it is fluorine. Ring carbon atoms in the divalent phenyl group depicted in formula I which are not bonded to adjacent groups depicted in formula I, and which do not carry a group R2, carry hydrogen atoms. Thus, in case the number n is 0 and hence no group R2 is present, all four carbon atoms in the ring positions of the divalent phenyl group depicted in formula I, which in formula I' are designated as positions 2', 3', 5' and 6', carry hydrogen atoms. In case the number n is 1 and hence one group R2 is present, one of the four carbon atoms in the ring positions of the divalent phenyl group depicted in formula I, which in formula I' are designated as 2', 3', 5' and 6', carries the group R2 and the other three said carbon atoms carry hydrogen atoms. In case the number n is 2 and hence two groups R2 are present, two of the four carbon atoms in the ring positions of the divalent phenyl group depicted in formula I, which in formula I' are designated as positions 2', 3', 5' and 6', carry the groups R2 and the other two said carbon atoms carry hydrogen atoms.

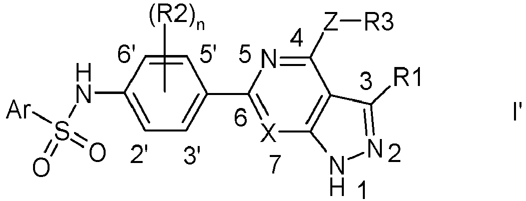

Groups R2 can be present in any positions of the divalent phenyl group depicted in formula I which in formula I' are designated as 2', 3', 5' and 6'. If one group R2 is present, in one embodiment of the invention the group R2 is present in the position which in formula I' is designated as position 2', which is equivalent to position 6', and in another embodiment it is present in the position which in formula I' is designated as position 3', which is equivalent to position 5'. If two groups R2 are present, in one embodiment of the invention the groups R2 are present in the positions which in formula I' are designated as positions 2' and 3', in another embodiment in the positions which in formula I' are designated as positions 2' and 5', in another embodiment in the positions which in formula I' are designated as positions 2' and 6', in another embodiment in the positions which in formula I' are designated as positions 3' and 5'.

In one embodiment of the invention, R3 is selected from the series consisting of H, (C₁-C₈)-alkyl and R30, in another embodiment from the series consisting of H, (C₁-C₈)-alkyl and -(C₁-C₄)-alkyl-R30, in another embodiment from the series consisting of H and (C₁-C₈)-alkyl, in another embodiment from the series consisting of H and R30, in another embodiment from the series consisting of (C₁-C₈)-alkyl, R30 and -(C₁-C₄)-alkyl-R30, in another embodiment from the series consisting of (C₁-C₈)-alkyl and R30, in another embodiment from the series consisting of R30 and -(C₁-C₄)-alkyl-R30, in another embodiment R3 is H, in another embodiment R3 is (C₁-C₈)-alkyl, in another embodiment R3 is R30, and in another embodiment R3 is -(C₁-C₄)-alkyl-R30, wherein in all these embodiments (C₁-C₈)-alkyl is unsubstituted or substituted by one or more identical or different substituents R31, and wherein in one embodiment of the invention all these embodiments independently apply to compounds of the formula I in which Z is a direct bond on the one hand, and to compounds of the formula I in which Z is selected from the series consisting of O, S and N(R10) on the other hand, and R3 can thus be defined differently for such compounds. For example, in one embodiment R3 is selected from the series consisting of H, (C₁-C₈)-alkyl and R30 in case Z is a direct bond, and R3 is selected from the series consisting of H, (C₁-C₈)-alkyl, R30 and -(C₁-C₄)-alkyl-R30 in case Z is selected from the series consisting of O, S and N(R10), in another embodiment R3 is selected from the series consisting of H, (C₁-C₈)-alkyl and R30 in case Z is a direct bond, and R3 is selected from the series consisting of H, (C₁-C₈)-alkyl and R30 in case Z is selected from the series consisting of O, S and N(R10), in another embodiment R3 is selected from the series consisting of H, (C₁-C₈)-alkyl and R30 in case Z is a direct bond, and R3 is selected from the series consisting of (C₁-C₈)-alkyl, R30 and -(C₁-C₄)-alkyl-R30 in case Z is selected from the series consisting of O, S and N(R10), in another embodiment R3 is selected from the series consisting of H, (C₁-C₈)-alkyl and R30 in case Z is a direct bond, and R3 is selected from the series consisting of (C₁-C₈)-alkyl and R30 in case Z is selected from the series consisting of O, S and N(R10), in another embodiment R3 is selected from the series consisting of H and R30 in case Z is a direct bond, and R3 is selected from the series consisting of (C₁-C₈)-alkyl, R30 and -(C₁-C₄)-alkyl-R30 in case Z is selected from the series consisting of O, S and N(R10), and in another embodiment R3 is selected from the series consisting of H and R30 in case Z is a direct bond, and R3 is selected from the series consisting of (C₁-C₈)-alkyl and R30 in case Z is selected from the series consisting of O, S and N(R10), wherein in all these embodiments (C₁-C₈)-alkyl is unsubstituted or substituted by one or more identical or different substituents R31. In one embodiment, the number of substituents R31 which is optionally present in alkyl groups representing R3, is 1, 2, 3, 4 or 5, in another embodiment it is 1, 2, 3 or 4, in another embodiment it is 1, 2 or 3, in another embodiment it is 1 or 2, in another embodiment it is 1, wherein independently of substituents R31 an alkyl group representing R3 can be substituted by one or more fluorine substituents, as applies to alkyl groups in general. In one embodiment, a (C₁-C₈)-alkyl group representing R3 is (C₁-C₆)-alkyl, in another embodiment it is (C₁-C₄)-alkyl, in another embodiment it is (C₁-C₃)-alkyl, in another embodiment it is (C₁-C₂)-alkyl, which groups all are unsubstituted or substituted by one or more identical or different substituents R31 and/or fluorine substituents. In one embodiment, the (C₁-C₄)-alkyl moiety in the group -(C₁-C₄)-alkyl-R30 representing R3 is (C₁-C₃)-alkyl, in another embodiment it is (C₁-C₂)-alkyl, in another embodiment it is methyl.

If two groups R5 bonded to adjacent ring carbon atoms in Ar together with the ring carbon atoms carrying them form a 5-membered to 8-membered ring, this ring is at least mono-unsaturated, i.e., the resulting ring contains at least one double bond within the ring, which double bond is present between the said two adjacent ring carbon in the aromatic ring Ar which are common to the ring Ar and the ring formed by the two groups R5, and because of the rules of nomenclature for fused rings is regarded as a double bond present in both rings. The ring formed by two groups R5 together with the carbon atoms carrying them can contain 1, 2 or 3 double bonds within the ring. In one embodiment, the formed ring contains 1 or 2 double bonds, in another embodiment 1 double bond within the ring. In the case of a 6-membered carbocyclic or heterocyclic ring or a 5-membered heterocyclic ring the formed ring can be aromatic and, together with the aromatic ring Ar, form a bicyclic aromatic ring system, for example a naphthalene ring system, a quinoline ring system, an isoquinoline ring system or a benzothiophene ring system. In one embodiment of the invention, not more than two substituents R5 on Ar, together with the ring carbon atoms in Ar carrying them, form a ring, i.e., in this embodiment not more than one ring formed by two groups R5 together with the ring carbon atoms in Ar carrying them is fused to Ar. If paired groups R5 forming a ring are present, further individual groups R5 can additionally be present on Ar, for example groups like halogen, (C₁-C₄)-alkyl or -O-(C₁-C₄)-alkyl.

The case that two groups R5 bonded to adjacent ring carbon atoms in Ar together with the carbon atoms carrying them form a 5-membered to 8-membered unsaturated ring, can in other terms be regarded as two groups R5 together forming a divalent residue comprising a chain of 3 to 6 atoms of which 0, 1 or 2 are identical or different heteroatoms selected from the series consisting of nitrogen, oxygen and sulfur, the terminal atoms of which are bonded to the two adjacent ring carbon atoms in Ar. Examples of such divalent residues, from any one or more of which two groups R5 bonded to adjacent ring carbon atoms in Ar are selected in one embodiment of the invention, are the residues -CH₂-CH₂-CH₂-, -CH₂-CH₂-CH₂-CH₂-, -CH₂-CH₂-CH₂-CH₂-CH₂-, -CH=CH-CH=CH-, -N=CH-CH=CH-, -CH=CH-CH=N-, -CH=N-CH=CH-, -CH=CH-N=CH-,-O-CH₂-CH₂-,-CH₂-CH₂-O-, -O-CH₂-O-, -O-CH₂-CH₂-O-, -O-CH₂-CH₂-CH₂-O-, -O-CH₂-CH₂-CH₂-CH₂-O-,-S-CH=CH-, -CH=CH-S-, =CH-S-CH=, -N=CH-S-, -S-CH=N-, -N=CH-O-, -O-CH=N-, -NH-CH₂-CH₂-O-, -O-CH₂-CH₂-NH-, -S-CH₂-CH₂-NH- and -NH-CH₂-CH₂-S-, which can all be substituted by one or more identical or different substituents R8, and can thus also be present, for example, as the divalent residues -O-CF₂-O-,-O-C(CH₃)₂-O-, -S-C(Cl)=CH-, -CH=C(Cl)-S-, -N(CH₃)-CH₂-CH₂-O-, -O-CH₂-CH₂-N(CH₃)-, -S-CH₂-CH₂-N(CH₃)- and -N(CH₃)-CH₂-CH₂-S-. In one embodiment of the invention, the ring heteroatoms which are optionally present in a ring formed by two groups R5 bonded to adjacent ring carbon atoms in Ar together with the carbon atoms carrying them, are selected from the series consisting of nitrogen and oxygen, in another embodiment from the series consisting of oxygen and sulfur, and in another embodiment they are oxygen atoms. In one embodiment of the invention, the ring which can be formed by two groups R5 bonded to adjacent ring carbon atoms in Ar together with the ring carbon atoms carrying them, is a 5-membered to 7-membered, in another embodiment a 5-membered to 6-membered, in another embodiment a 6-membered to 7-membered, in another embodiment a 5-membered, in another embodiment a 6-membered ring, in another embodiment a 7-membered ring. In one embodiment of the invention, the ring which can be formed by two groups R5 bonded to adjacent carbon atoms in Ar together with the carbon atoms carrying them, comprises 0 ring heteroatoms, i.e. it is a carbocyclic ring, and in another embodiment it comprises 1 or 2 identical or different ring heteroatoms. In one embodiment of the invention, the number of substituents R8 which can be present in a ring formed by two groups R5 bonded to adjacent ring carbon atoms in Ar together with the carbon atoms carrying them, is 1, 2, 3 or 4, in another embodiment 1, 2 or 3, in another embodiment 1 or 2, in another embodiment 1, in another embodiment it is 0.

In one embodiment of the invention, R5 is selected from the series consisting of halogen, (C₁-C₄)-alkyl, (C₃-C₇)-cycloalkyl, -O-(C₁-C₄)-alkyl, -O-(C₃-C₇)-cycloalkyl, -C(O)-N(R6)-R7 and -CN, in another embodiment from the series consisting of halogen, (C₁-C₄)-alkyl, (C₃-C₇)-cycloalkyl, -O-(C₁-C₄)-alkyl, -C(O)-N(R6)-R7 and -CN, in another embodiment from the series consisting of halogen, (C₁-C₄)-alkyl, -O-(C₁-C₄)-alkyl, -O-(C₃-C₇)-cycloalkyl, -C(O)-N(R6)-R7 and -CN, in another embodiment from the series consisting of halogen, (C₁-C₄)-alkyl, - O-(C₁-C₄)-alkyl, -C(O)-N(R6)-R7 and -CN, in another embodiment from the series consisting of halogen, (C₁-C₄)-alkyl, -O-(C₁-C₄)-alkyl and -CN, in another embodiment from the series consisting of halogen, -(C₁-C₄)-alkyl and -CN, in another embodiment from the series consisting of halogen, -O-(C₁-C₄)-alkyl and -CN, in another embodiment from the series consisting of -O-(C₁-C₄)-alkyl and -CN, in another embodiment from the series consisting of halogen and -CN, in another embodiment from the series consisting of halogen, and in all these embodiments two groups R5 bonded to adjacent ring carbon atoms in Ar, together with the carbon atoms carrying them, can form a 5-membered to 8-membered unsaturated ring which comprises 0, 1 or 2 identical or different ring heteroatoms selected from the series consisting of nitrogen, oxygen and sulfur, and which is unsubstituted or substituted by one or more identical or different substituents R8.

In one embodiment of the invention, R5 is selected from the series consisting of halogen, (C₁-C₄)-alkyl, (C₃-C₇)-cycloalkyl, -O-(C₁-C₄)-alkyl, -O-(C₃-C₇)-cycloalkyl, -C(O)-N(R6)-R7 and -CN, in another embodiment from the series consisting of halogen, (C₁-C₄)-alkyl, (C₃-C₇)-cycloalkyl, -O-(C₁-C₄)-alkyl, -C(O)-N(R6)-R7 and -CN, in another embodiment from the series consisting of halogen, (C₁-C₄)-alkyl, -O-(C₁-C₄)-alkyl, -O-(C₃-C₇)-cycloalkyl, -C(O)-N(R6)-R7 and -CN, in another embodiment from the series consisting of halogen, (C₁-C₄)-alkyl,-O-(C₁-C₄)-alkyl, -C(O)-N(R6)-R7 and -CN, in another embodiment from the series consisting of halogen, (C₁-C₄)-alkyl, -O-(C₁-C₄)-alkyl and -CN, in another embodiment from the series consisting of halogen, -(C₁-C₄)-alkyl and -CN, in another embodiment from the series consisting of halogen, -O-(C₁-C₄)-alkyl and -CN, in another embodiment from the series consisting of -O-(C₁-C₄)-alkyl and -CN, in another embodiment from the series consisting of halogen and -CN, in another embodiment from the series consisting of halogen.

In one embodiment, substituents R5 which are bonded to a ring nitrogen atom in Ar, such as in the case of a pyrrole, pyrazole or imidazole ring representing Ar, are selected from the series consisting of (C₁-C₄)-alkyl, (C₃-C₇)-cycloalkyl, -(C₁-C₄)-alkyl-(C₃-C₇)-cycloalkyl and -C(O)-N(R6)-R7, in another embodiment from the series consisting of (C₁-C₄)-alkyl, (C₃-C₇)-cycloalkyl and -(C₁-C₄)-alkyl-(C₃-C₇)-cycloalkyl, in another embodiment from the series consisting of (C₁-C₄)-alkyl.

In one embodiment of the invention, a (C₁-C₄)-alkyl group which represents R5 or is present in the group -O-(C₁-C₄)-alkyl representing R5, is a (C₁-C₃)-alkyl group, in another embodiment a (C₁-C₂)-alkyl group, in another embodiment a methyl group, wherein all these alkyl groups can optionally be substituted by fluorine substituents as applies to alkyl groups in general, and also occur as a trifluoromethyl group, for example. In one embodiment, an alkyl group representing R5 or present in a group representing R5 is, independently of any other alkyl group occurring in R5, not substituted by fluorine substituents. In one embodiment, a (C₃-C₇)-cycloalkyl group representing R5 or present in a group representing R5, is a (C₃-C₆)-cycloalkyl group, in another embodiment a (C₃-C₄)-cycloalkyl group, in another embodiment a cyclopropyl group. In one embodiment, halogen representing R5 is selected from the series consisting of fluorine and chlorine.

Examples of groups Ar, including the optional substituents R5 on Ar, from any one or more of which Ar is selected in one embodiment of the invention, are 2-chloro-phenyl, 2-fluoro-phenyl, 3-fluoro-phenyl, 2,3-dichloro-phenyl, 2,5-dichloro-phenyl, 2,5-difluoro-phenyl, 2-chloro-3-fluoro-phenyl, 2-chloro-4-fluoro-phenyl, 3-chloro-2-fluoro-phenyl, 5-chloro-2-fluoro-phenyl, 2,3,5-trifluoro-phenyl, 2,4,5-trifluoro-phenyl, 2-chloro-3,5-difluoro-phenyl, 2-chloro-4,5-difluoro-phenyl, 3-chloro-2,5-difluoro-phenyl, 3-chloro-2,6-difluoro-phenyl, 5-chloro-2,4-difluoro-phenyl, 2-fluoro-5-methyl-phenyl, 2-fluoro-5-methoxy-phenyl, 2-chloro-5-methoxy-phenyl, 2-bromo-4,5-dimethoxy-phenyl, 2-fluoro-4,5-dimethoxy-phenyl, 4,5-dimethoxy-2-methyl-phenyl, 2-cyano-phenyl, 3-cyano-phenyl, 2-cyano-3-fluoro-phenyl, 2-cyano-5-fluoro-phenyl, 3-cyano-4-fluoro-phenyl, 5-cyano-2-fluoro-phenyl, 3-chloro-2-cyano-phenyl, 5-chloro-2-cyano-phenyl, 2-cyano-5-methyl-phenyl, 5-cyano-2-methyl-phenyl, 2-cyano-5-methoxy-phenyl, 2-carbamoyl-phenyl, 4-bromo-thiophen-2-yl, 4-chloro-thiophen-3-yl, 5-bromo-thiophen-2-yl, 5-chloro-thiophen-2-yl, 2,5-dichloro-thiophen-3-yl, 4,5-dichloro-thiophen-2-yl, 5-chloro-1,3-dimethyl-pyrazol-4-yl, 7-chloro-2,3-dihydro-benzo[1,4]dioxin-6-yl, 8-bromo-3,4-dihydro-2H-benzo[b][1,4]dioxepin-7-yl, 8-chloro-3,4-dihydro-2H-benzo[b][1,4]dioxepin-7-yl.

In one embodiment of the invention, R6 and R7 are independently of one another selected from the series consisting of hydrogen and (C₁-C₃)-alkyl, in another embodiment from the series consisting of hydrogen and (C₁-C₂)-alkyl, in another embodiment from the series consisting of hydrogen and methyl, and in another embodiment R6 and R7 are hydrogen.

In one embodiment of the invention, substituents R8 which can be present in a ring formed by two groups R5 bonded to adjacent ring carbon atoms in Ar together with the carbon atoms carrying them, are selected from the series consisting of halogen, (C₁-C₄)-alkyl and -CN, in another embodiment from the series consisting of halogen, -O-(C₁-C₄)-alkyl and -CN, in another embodiment from the series consisting of halogen and (C₁-C₄)-alkyl, in another embodiment from the series consisting of (C₁-C₄)-alkyl. In one embodiment, substituents R8 which are bonded to a ring nitrogen atom in a ring from by two groups R5 bonded to adjacent ring carbon atoms in Ar together with the carbon atoms carrying them, are selected from the series consisting of (C₁-C₄)-alkyl.

In one embodiment of the invention, R10 is selected from the series consisting of hydrogen and (C₁-C₃)-alkyl, in another embodiment from the series consisting of hydrogen and (C₁-C₂)-alkyl, in another embodiment from the series consisting of hydrogen and methyl, and in another embodiment R10 is hydrogen.

The monocyclic heterocycle which can be formed by the groups R11 and R12 together with the nitrogen atom carrying them, which heterocycle is thus bonded via a ring nitrogen atom, can be 4-membered, 5-membered, 6-membered or 7-membered. In one embodiment of the invention, the heterocycle formed by the groups R11 and R12 together with the nitrogen atom carrying them, is 4-membered to 6-membered, in another embodiment it is 5-membered or 6-membered, in another embodiment it is 6-membered. In one embodiment, the further ring heteroatom which is optionally present in a heterocycle formed by the groups R11 and R12 together with the nitrogen atom carrying them, is selected from the series consisting of nitrogen and oxygen, in another embodiment it is a nitrogen atom, and in another embodiment it is an oxygen atom, and in another embodiment no further ring heteroatom is present. In one embodiment of the invention, the number of substituents selected from the series consisting of fluorine and (C₁-C₄)-alkyl, which can be present in a ring formed by the groups R11 and R12 together with the nitrogen atom carrying them, is 1, 2 or 3, in another embodiment it is 1 or 2, in another embodiment it is 1. In one embodiment of the invention, substituents which can be present in a ring formed by the groups R11 and R12 together with the nitrogen atom carrying them, are fluorine substituents, and in another embodiment they are (C₁-C₄)-alkyl substituents, for example methyl substituents. In one embodiment are substituents in a ring formed by the groups R11 and R12 together with the nitrogen atom carrying them, which are bonded to a ring nitrogen atom, selected from the series consisting of (C₁-C₄)-alkyl. Examples of heterocyclic groups, from any one or more of which the heterocyclic group formed by the groups R11 and R12 together with the nitrogen atom carrying them is selected in one embodiment of the invention, are azetidin-1-yl, pyrrolidin-1-yl, piperidin-1-yl, morpholin-4-yl, thiomorpholin-4-yl, and 4-methylpiperazin-1-yl.

In one embodiment of the invention, one of the groups R11 and R12 is selected from the series consisting of hydrogen and (C₁-C₄)-alkyl, and the other of the groups R11 and R12 is selected from the series consisting of hydrogen, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl, in another embodiment the other of the groups R11 and R12 is selected from the series consisting of hydrogen, (C₁-C₄)-alkyl, -(C₁-C₄)-alkyl-(C₃-C₇)-cycloalkyl and -(C₁-C₄)-alkyl-Het1, in another embodiment the other of the groups R11 and R12 is selected from the series consisting of hydrogen, (C₁-C₄)-alkyl and -(C₁-C₄)-alkyl-Het1, in another embodiment the groups R11 and R12 are independently of one another selected from the series consisting of hydrogen, (C₁-C₄)-alkyl, -(C₁-C₄)-alkyl-(C₃-C₇)-cycloalkyl and -(C₁-C₄)-alkyl-Het1, in another embodiment the groups R11 and R12 are independently of one another selected from the series consisting of hydrogen, (C₁-C₄)-alkyl and -(C₁-C₄)-alkyl-Het1, in another embodiment the groups R11 and R12 are independently of one another selected from the series consisting of hydrogen and (C₁-C₄)-alkyl, and in another embodiment the groups R11 and R12 are both hydrogen, i.e., in this latter embodiment the group -N(R11)-R12 representing R1 is the group -NH₂ (amino), or in all these embodiments R11 and R12, together with the nitrogen atom carrying them, form a monocyclic, 4-membered to 7-membered, saturated heterocycle which, in addition to the nitrogen atom carrying R11 and R12, comprises 0 or 1 further ring heteroatom selected from the series consisting of nitrogen, oxygen and sulfur, and which is unsubstituted or substituted by one or more identical or different substituents selected from the series consisting of fluorine and (C₁-C₄)-alkyl.

In one embodiment of the invention, R11 and R12 are independently of one another selected from the series consisting of hydrogen (H), (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl, wherein phenyl is unsubstituted or substituted by one or more identical or different substituents R50. In another embodiment, one of the groups R11 and R12 is selected from the series consisting of hydrogen and (C₁-C₄)-alkyl, and the other of the groups R11 and R12 is selected from the series consisting of hydrogen, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl, in another embodiment the other of the groups R11 and R12 is selected from the series consisting of hydrogen, (C₁-C₄)-alkyl, -(C₁-C₄)-alkyl-(C₃-C₇)-cycloalkyl and -(C₁-C₄)-alkyl-Het1, and in another embodiment the other of the groups R11 and R12 is selected from the series consisting of hydrogen, (C₁-C₄)-alkyl and -(C₁-C₄)-alkyl-Het1. In one embodiment, the groups R11 and R12 are independently of one another selected from the series consisting of hydrogen, (C₁-C₄)-alkyl, -(C₁-C₄)-alkyl-(C₃-C₇)-cycloalkyl and -(C₁-C₄)-alkyl-Het1, in another embodiment the groups R11 and R12 are independently of one another selected from the series consisting of hydrogen, (C₁-C₄)-alkyl and -(C₁-C₄)-alkyl-Het1, in another embodiment the groups R11 and R12 are independently of one another selected from the series consisting of hydrogen and (C₁-C₄)-alkyl, and in another embodiment the groups R11 and R12 are both hydrogen, i.e., in this latter embodiment the group -N(R11)-R12 representing R1 is the group -NH₂.

In one embodiment, one of the groups R11 and R12 is hydrogen, and the other of the groups R11 and R12 is selected from the series consisting of hydrogen, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl, in another embodiment the other of the groups R11 and R12 is selected from the series consisting of hydrogen, (C₁-C₄)-alkyl, -(C₁-C₄)-alkyl-(C₃-C₇)-cycloalkyl and -(C₁-C₄)-alkyl-Het1, in another embodiment the other of the groups R11 and R12 is selected from the series consisting of hydrogen, (C₁-C₄)-alkyl and -(C₁-C₄)-alkyl-Het1, and in another embodiment the other of the groups R11 and R12 is selected from the series consisting of hydrogen and (C₁-C₄)-alkyl.

In one embodiment of the invention, R13 is selected from the series consisting of hydrogen and (C₁-C₄)-alkyl, in another embodiment from the series consisting of hydrogen and (C₁-C₃)-alkyl, in another embodiment from the series consisting of hydrogen and (C₁-C₂)-alkyl, in another embodiment from the series consisting of hydrogen and methyl, and in another embodiment R13 is hydrogen.

In one embodiment of the invention, R14 is selected from the series consisting of (C₃-C₇)-cycloalkyl, -(C₁-C₄)-alkyl-(C₃-C₇)-cycloalkyl, phenyl, -(C₁-C₄)-alkyl-phenyl, Het2 and -(C₁-C₄)-alkyl-Het2, in another embodiment from the series consisting of (C₃-C₇)-cycloalkyl, -(C₁-C₄)-alkyl-(C₃-C₇)-cycloalkyl, phenyl, -(C₁-C₄)-alkyl-phenyl and Het2, in another embodiment from the series consisting of (C₃-C₇)-cycloalkyl, phenyl, -(C₁-C₄)-alkyl-phenyl, Het2 and -(C₁-C₄)-alkyl-Het2, in another embodiment from the series consisting of (C₃-C₇)-cycloalkyl, phenyl, -(C₁-C₄)-alkyl-phenyl and Het2, in another embodiment from the series consisting of (C₃-C₇)-cycloalkyl, phenyl and Het2, in another embodiment from the series consisting of (C₃-C₇)-cycloalkyl and Het2, in another embodiment R14 is (C₃-C₇)-cycloalkyl, in another embodiment R14 is Het2, and in another embodiment R14 is phenyl, wherein in all these embodiments (C₃-C₇)-cycloalkyl groups all are unsubstituted or substituted by one or more identical or different substituents selected from the series consisting of -OH and -O-(C₁-C₄)-alkyl and, independently thereof, one or more identical or different substituents selected from the series consisting of fluorine and (C₁-C₄)-alkyl as applies to cycloalkyl groups in general, and phenyl and Het2 groups all are unsubstituted or substituted by one or more identical or different substituents R50. In one embodiment, the number of substituents selected from the series consisting of -OH and -O-(C₁-C₄)-alkyl, which can be present in a (C₁-C₈)-alkyl group representing R14 or a (C₃-C₇)-cycloalkyl group occurring in R14, is 1, 2 or 3, in another embodiment it is 1 or 2, in another embodiment it is 1. In one embodiment, the number of substituents R50 which can be present in a phenyl group or Het2 group representing R14 or occurring in R14, is 1, 2 or 3, in another embodiment it is 1 or 2, in another embodiment it is 1.

In one embodiment of the invention, R15 is selected from the series consisting of phenyl and Het3, in another embodiment from the series consisting of (C₁-C₈)-alkyl and phenyl, and in another embodiment R15 is phenyl, wherein in all these embodiments phenyl and Het3 all are unsubstituted or substituted by one or more identical or different substituents R50. In one embodiment, the number of substituents R50 which can be present in a phenyl group or Het3 group representing R15 is 1, 2, 3 or 4, in another embodiment it is 1, 2 or 3, in another embodiment it is 1 or 2, in another embodiment it is 1.

In one embodiment of the invention, R16 is selected from the series consisting of (C₁-C₈)-alkyl, (C₃-C₇)-cycloalkyl, -(C₁-C₄)-alkyl-(C₃-C₇)-cycloalkyl, -(C₁-C₄)-alkyl-phenyl, Het2 and -(C₁-C₄)-alkyl-Het2, in another embodiment from the series consisting of (C₃-C₇)-cycloalkyl, -(C₁-C₄)-alkyl-(C₃-C₇)-cycloalkyl, -(C₁-C₄)-alkyl-phenyl, Het2 and -(C₁-C₄)-alkyl-Het2, in another embodiment from the series consisting of -(C₁-C₄)-alkyl-phenyl, Het2 and -(C₁-C₄)-alkyl-Het2, in another embodiment from the series consisting of -(C₁-C₄)-alkylphenyl and -(C₁-C₄)-alkyl-Het2, wherein (C₁-C₈)-alkyl and (C₃-C₇)-cycloalkyl all are unsubstituted or substituted by one or more identical or different substituents selected from the series consisting of -OH and -O-(C₁-C₄)-alkyl and, independently thereof, fluorine substituents and, in case of cycloalkyl groups, (C₁-C₄)-alkyl substituents, and wherein phenyl and Het2 all are unsubstituted or substituted by one or more identical or different substituents R50. In one embodiment, the number of substituents selected from the series consisting of -OH and -O-(C₁-C₄)-alkyl, which can be present in a (C₁-C₈)-alkyl group representing R16 or a (C₃-C₇)-cycloalkyl group occurring in R16, is 1 or2, in another embodiment it is 1, and in another embodiment it is 0. In one embodiment, the number of substituents R50 which can be present in a phenyl group or Het2 group representing R16 or occurring in R16, is 1, 2 or 3, in another embodiment it is 1 or 2, in another embodiment it is 1, and in another embodiment it is 0.

In one embodiment of the invention, R17 is (C₁-C₄)-alkyl, in another embodiment R17 is hydrogen. In one embodiment, a (C₁-C₄)-alkyl group representing R17 is (C₁-C₃)-alkyl, in another embodiment it is (C₁-C₂)-alkyl, in another embodiment it is methyl.

The cyclic group R30, which can be monocyclic and bicyclic, can contain 3, 4, 5, 6, 7, 8, 9, 10, 11 or 12 ring members. In one embodiment of the invention, R30 contains 3, 4, 5, 6, 7, 8, 9, 10 or 11 ring members, in another embodiment 3, 4, 5, 6, 7, 8, 9 or 10 ring members, in another embodiment 3, 4, 5, 6, 7, 8 or 9 ring members. In one embodiment, the number of ring members in a monocyclic group R30 is 3, 4, 5, 6 or 7, in another embodiment 3, 4, 5 or 6, in another embodiment 3 or 4, in another embodiment 5, 6 or 7, in another embodiment 5 or 6, in another embodiment 3, in another embodiment 4, in another embodiment 5, in another embodiment 6, and the number of ring members in a bicyclic group R30 is 6, 7, 8, 9, 10, 11 or 12, in another embodiment 6, 7, 8, 9, 10 or 11, in another embodiment 6, 7, 8, 9 or 10, in another embodiment 7, 8, 9, 10 or 11, in another embodiment 7, 8, 9 or 10, in another embodiment 7, 8 or 9, in another embodiment 8, 9 or 10. In one embodiment, the number of ring members of the cyclic group R30 is from 3 to 12 in the case of a carbocyclic ring, and from 4 to 12 in the case of a heterocyclic ring. In one embodiment, the cyclic group R30 is monocyclic, in another embodiment it is bicyclic. A bicyclic group R30 can be a fused ring system or a bridged ring system or a spirocyclic ring system. In one embodiment, a bicyclic group R30 is a fused or bridged ring system, in another embodiment it is a fused or spirocyclic ring system, in another embodiment it is a bridged or spirocyclic ring system, in another embodiment it is a fused ring system, in another embodiment it is a bridged ring system, and in another embodiment it is a spirocyclic ring system. In one embodiment, the cyclic group R30 is a saturated group, i.e. it does not contain a double bond within the ring, or it is an aromatic group, i.e. it contains two double bonds within the ring in the case of a 5-membered monocyclic aromatic heterocycle which double bonds, together with an electron pair on a ring heteroatom, form a delocalized cyclic system of six pi electrons, and three double bonds within the ring in the case of a phenyl group or a 6-membered monocyclic aromatic heterocycle, or two, three, four or five double bonds within two fused rings in the case of a bicyclic group comprising one or two aromatic rings. In another embodiment, R30 is a partially unsaturated group, i.e. it contains one or more, for example one or two, double bonds within the ring via which it is bonded, but is not aromatic within this ring. In another embodiment, R30 is a saturated group or it is a partially unsaturated group, in another embodiment R30 is an aromatic group or it is a partially unsaturated group, in another embodiment R30 is a saturated group, and in another embodiment R30 is an aromatic group.

The cyclic group R30 can be a carbocyclic group, i.e. comprise 0 (zero) ring heteroatoms, or a heterocyclic group, i.e. comprise 1, 2 or 3 identical or different ring heteroatoms. In one embodiment, R30 comprises 0, 1 or 2 identical or different ring heteroatoms, in another embodiment 0 or 1 ring heteroatom. In another embodiment, R30 comprises 0 ring heteroatoms, i.e. R30 is a carbocyclic group. In another embodiment R30 is a heterocyclic group which comprises 1, 2 or 3 identical or different ring heteroatoms, in another embodiment 1 or 2 identical or different ring heteroatoms, in another embodiment 1 ring heteroatom. In one embodiment, the ring heteroatoms in R30 are selected from the series consisting of nitrogen and oxygen, in another embodiment from the series consisting of oxygen and sulfur, in another embodiment they are nitrogen atoms, and in another embodiment they are oxygen atoms. Heterocyclic groups representing R30 can be bonded to the group Z via a ring carbon atom or a ring nitrogen atom. In one embodiment, a heterocyclic group representing R30 is bonded via a ring carbon atom, in another embodiment it is bonded via a ring nitrogen atom.

Examples of carbocyclic groups, which may represent R30 and any one or more of which may be included in the definition of R30 in one embodiment of the invention, and from any one or more of which R30 is selected in another embodiment, are cycloalkyl groups such as (C₃-C₇)-cycloalkyl, including cyclopropyl, cyclobutyl, cyclopentyl, cyclohexyl and cycloheptyl, cycloalkenyl groups such as (C₅-C₇)-cycloalkenyl, including cyclopentenyl, cyclohexenyl and cycloheptenyl, bicycloalkyl groups such as (C₆-C₁₂)-bicycloalkyl, phenyl groups, indanyl groups, including indan-1-yl and indan-2-yl, and naphthyl groups, including naphthalen-1-yl and naphthalen-2-yl, for example, which can all be unsubstituted or substituted by one or more identical or different substituents R32. The explanations given above, for example with respect to cycloalkyl groups and phenyl groups, apply also to such groups representing R30.

Examples of heterocyclic groups, which may represent R30 and any one or more of which may be included in the definition of R30 in one embodiment of the invention, and from any one or more of which R30 is selected in another embodiment, are 4-membered to 7-membered, monocyclic, saturated, partially unsaturated or aromatic, heterocyclic groups which comprise 1, 2 or 3 identical or different ring heteroatoms selected from the series consisting of nitrogen, oxygen and sulfur and are bonded via a nitrogen atom, 6-membered to 12-membered, bicyclic, saturated or partially unsaturated, heterocyclic groups which comprise 1, 2 or 3 identical or different ring heteroatoms selected from the series consisting of nitrogen, oxygen and sulfur and are bonded via a nitrogen atom, and the groups Het1, Het2 and Het3, and more specifically oxetanyl including oxetan-2-yl and oxetan-3-yl, tetrahydrofuranyl including tetrahydrofuran-2-yl and tetrahydrofuran-3-yl, tetrahydropyranyl including tetrahydropyran-2-yl, tetrahydropyran-3-yl and tetrahydropyran-4-yl, oxepanyl including oxepan-2-yl, oxepan-3-yl and oxepan-4-yl, azetidinyl including azetidin-1-yl, azetidin-2-yl and azetidin-3-yl, pyrrolidinyl including pyrrolidin-1-yl, pyrrolidin-2-yl and pyrrolidin-3-yl, piperidinyl including piperidin-1-yl, piperidin-2-yl, piperidin-3-yl and piperidin-4-yl, azepanyl including azepan-1-yl, azepan-2-yl, azepan-3-yl and azepan-4-yl, morpholinyl including morpholin-2-yl, morpholin-3-yl and morpholin-4-yl, thiomorpholinyl including thiomorpholin-2-yl, thiomorpholin-3-yl and thiomorpholin-4-yl, piperazinyl including piperazin-1-yl and piperazin-2-yl, furanyl including furan-2-yl and furan-3-yl, thiophenyl (thienyl) including thiophen-2-yl and thiophen-3-yl, pyrrolyl including pyrrol-1-yl, pyrrol-2-yl and pyrrol-3-yl, isoxazolyl including isoxazol-3-yl, isoxazol-4-yl and isoxazol-5-yl, oxazolyl including oxazol-2-yl, oxazol-4-yl and oxazol-5-yl, thiazolyl including thiazol-2-yl, thiazol-4-yl and thiazol-5-yl, pyrazolyl including pyrazol-1-yl, pyrazol-3-yl, pyrazol-4-yl and pyrazol-5-yl, imidazolyl including imidazolyl-1-yl, imidazol-2-yl, imidazol-4-yl and imidazol-5-yl, [1,2,4]triazolyl including [1,2,4]triazol-1-yl, [1,2,4]triazol-3-yl and [1,2,4]triazol-5-yl, pyridinyl (pyridyl) including pyridin-2-yl, pyridin-3-yl and pyridin-4-yl, pyrazinyl including pyrazin-2-yl, for example, which can all be unsubstituted or substituted by one or more identical or different substituents R32. The explanations given above and below, for example with respect to heterocyclic groups in general and the groups Het1, Het2 and Het3, apply also to such groups representing R30.

In one embodiment of the invention, the number of substituents R32 which can be present in R30, is 1, 2, 3, 4, 5 or 6, in another embodiment it is 1, 2, 3, 4 or 5, in another embodiment it is 1, 2, 3 or 4, in another embodiment it is 1, 2 or 3, in another embodiment it is 1 or 2, in another embodiment it is 1. In another embodiment, R30 is unsubstituted.

In one embodiment of the invention, R31 is selected from the series consisting of halogen, -OH,-O-(C₁-C₄)-alkyl, -O-(C₃-C₇)-cycloalkyl, -O-(C₁-C₄)-alkyl-(C₃-C₇)-cycloalkyl, -N(R33)-R34 and -CN, in another embodiment from the series consisting of halogen, -OH, -O-(C₁-C₄)-alkyl, -O-(C₃-C₇)-cycloalkyl, -N(R33)-R34 and -CN, in another embodiment from the series consisting of halogen,-OH, -O-(C₁-C₄)-alkyl, -O-(C₃-C₇)-cycloalkyl, -N(R33)-R34, -CN and -C(O)-N(R35)-R36, in another embodiment from the series consisting of halogen, -OH, -O-(C₁-C₄)-alkyl, -O-(C₃-C₇)-cycloalkyl, -O-(C₁-C₄)-alkyl-(C₃-C₇)-cycloalkyl and -N(R33)-R34, in another embodiment from the series consisting of halogen, -OH, -O-(C₁-C₄)-alkyl, -O-(C₃-C₇)-cycloalkyl, -O-(C₁-C₄)-alkyl-(C₃-C₇)-cycloalkyl, -N(R33)-R34 and -C(O)-N(R35)-R36, in another embodiment from the series consisting of halogen, -OH, -O-(C₁-C₄)-alkyl, -O-(C₃-C₇)-cycloalkyl and -N(R33)-R34, in another embodiment from the series consisting of halogen, -OH, -O-(C₁-C₄)-alkyl, -O-(C₃-C₇)-cycloalkyl, -N(R33)-R34 and -C(O)-N(R35)-R36, in another embodiment from the series consisting of halogen, -OH, -O-(C₁-C₄)-alkyl and -N(R33)-R34, in another embodiment from the series consisting of halogen, - OH, -O-(C₁-C₄)-alkyl, -N(R33)-R34 and -C(O)-N(R35)-R36, in another embodiment from the series consisting of halogen, -OH, -O-(C₁-C₄)-alkyl, -N(R33)-R34 and -CN, in another embodiment from the series consisting of halogen, -OH, -O-(C₁-C₄)-alkyl, -N(R33)-R34, -CN and -C(O)-N(R35)-R36, in another embodiment from the series consisting of halogen, -OH, -O-(C₁-C₄)-alkyl, -O-(C₃-C₇)-cycloalkyl and -O-(C₁-C₄)-alkyl-(C₃-C₇)-cycloalkyl, in another embodiment from the series consisting of halogen, -OH, -O-(C₁-C₄)-alkyl and-O-(C₃-C₇)-cycloalkyl, in another embodiment from the series consisting of halogen, -OH and -O-(C₁-C₄)-alkyl, in another embodiment from the series consisting of halogen and -N(R33)-R34, in another embodiment from the series consisting of -OH, -O-(C₁-C₄)-alkyl and -N(R33)-R34, in another embodiment from the series consisting of - OH, -O-(C₁-C₄)-alkyl, -N(R33)-R34 and -C(O)-N(R35)-R36, in another embodiment from the series consisting of -OH, -O-(C₁-C₄)-alkyl, -O-(C₃-C₇)-cycloalkyl and -O-(C₁-C₄)-alkyl-(C₃-C₇)-cycloalkyl, in another embodiment from the series consisting of -OH, -O-(C₁-C₄)-alkyl and-O-(C₃-C₇)-cycloalkyl. In one embodiment, halogen representing R31 is selected from the series consisting of fluorine and chlorine, in another embodiment halogen representing R31 is fluorine.

In one embodiment of the invention, R32 is selected from the series consisting of halogen, (C₁-C₄)-alkyl, (C₃-C₇)-cycloalkyl, -(C₁-C₄)-alkyl-(C₃-C₇)-cycloalkyl, -(C₁-C₄)-alkyl-O-R37, -(C₁-C₄)-alkyl-N(R38)-R39, -C(O)-(C₁-C₄)-alkyl, -OH, =O, -O-(C₁-C₄)-alkyl, -N(R40)-R41, -C(O)-O-(C₁-C₄)-alkyl and -C(O)-N(R42)-R43, in another embodiment from the series consisting of halogen, (C₁-C₄)-alkyl, (C₃-C₇)-cycloalkyl, -(C₁-C₄)-alkyl-(C₃-C₇)-cycloalkyl, -(C₁-C₄)-alkyl-O-R37, -(C₁-C₄)-alkyl-N(R38)-R39, -OH, =O, -O-(C₁-C₄)-alkyl, -N(R40)-R41, -C(O)-O-(C₁-C₄)-alkyl and -C(O)-N(R42)-R43, in another embodiment from the series consisting of halogen, (C₁-C₄)-alkyl, (C₃-C₇)-cycloalkyl, -(C₁-C₄)-alkyl-(C₃-C₇)-cycloalkyl, -(C₁-C₄)-alkyl-O-R37, -(C₁-C₄)-alkyl-N(R38)-R39, -OH, =O, -O-(C₁-C₄)-alkyl and -N(R40)-R41, in another embodiment from the series consisting of halogen, (C₁-C₄)-alkyl, (C₃-C₇)-cycloalkyl, -(C₁-C₄)-alkyl-O-R37, -(C₁-C₄)-alkyl-N(R38)-R39, -OH, =O, -O-(C₁-C₄)-alkyl and -N(R40)-R41, in another embodiment from the series consisting of halogen, (C₁-C₄)-alkyl, (C₃-C₇)-cycloalkyl, -(C₁-C₄)-alkyl-O-R37, -(C₁-C₄)-alkyl-N(R38)-R39, -OH, =O and -O-(C₁-C₄)-alkyl, in another embodiment from the series consisting of halogen, (C₁-C₄)-alkyl, (C₃-C₇)-cycloalkyl, -(C₁-C₄)-alkyl-O-R37, -OH, =O and -O-(C₁-C₄)-alkyl, in another embodiment from the series consisting of halogen, (C₁-C₄)-alkyl, (C₃-C₇)-cycloalkyl, -(C₁-C₄)-alkyl-O-R37, -(C₁-C₄)-alkyl-N(R38)-R39, -OH and -O-(C₁-C₄)-alkyl, in another embodiment from the series consisting of halogen, (C₁-C₄)-alkyl, (C₃-C₇)-cycloalkyl, -(C₁-C₄)-alkyl-O-R37, -(C₁-C₄)-alkyl-N(R38)-R39, -OH and =O, in another embodiment from the series consisting of halogen, (C₁-C₄)-alkyl, (C₃-C₇)-cycloalkyl, -(C₁-C₄)-alkyl-O-R37 and -OH, in another embodiment from the series consisting of halogen, (C₁-C₄)-alkyl, (C₃-C₇)-cycloalkyl and -OH, in another embodiment from the series consisting of halogen, (C₁-C₄)-alkyl and (C₃-C₇)-cycloalkyl, in another embodiment from the series consisting of halogen, -OH and -O-(C₁-C₄)-alkyl, in another embodiment from the series consisting of halogen and -OH, in another embodiment from the series consisting of halogen, (C₁-C₄)-alkyl, (C₃-C₇)-cycloalkyl, -(C₁-C₄)-alkyl-O-R37, -OH and -O-(C₁-C₄)-alkyl, in another embodiment from the series consisting of halogen, (C₁-C₄)-alkyl, (C₃-C₇)-cycloalkyl, -OH and -O-(C₁-C₄)-alkyl, in another embodiment from the series consisting of halogen, (C₁-C₄)-alkyl, -OH and -O-(C₁-C₄)-alkyl, in another embodiment from the series consisting of halogen, -OH and -O-(C₁-C₄)-alkyl, in another embodiment from the series consisting of -OH and -O-(C₁-C₄)-alkyl, and in another embodiment R32 is -OH. In another embodiment R32 is selected from the series of halogen, (C₁-C₄)-alkyl, (C₃-C₇)-cycloalkyl, -(C₁-C₄)-alkyl-(C₃-C₇)-cycloalkyl, -(C₁-C₄)-alkyl-O-R37, -(C₁-C₄)-alkyl-N(R38)-R39, -C(O)-(C₁-C₄)-alkyl, -OH, =O, -O-(C₁-C₄)-alkyl, -N(R40)-R41, -C(O)-O-(C₁-C₄)-alkyl and -C(O)-N(R42)-R43, in another embodiment from the series consisting of halogen, (C₁-C₄)-alkyl, (C₃-C₇)-cycloalkyl, -(C₁-C₄)-alkyl-(C₃-C₇)-cycloalkyl, -(C₁-C₄)-alkyl-O-R37, -(C₁-C₄)-alkyl-N(R38)-R39,-C(O)-(C₁-C₄)-alkyl, -OH, =O, -O-(C₁-C₄)-alkyl and -N(R40)-R41, in another embodiment from the series consisting of halogen, (C₁-C₄)-alkyl, (C₃-C₇)-cycloalkyl, -(C₁-C₄)-alkyl-O-R37, -(C₁-C₄)-alkyl-N(R38)-R39, -C(O)-(C₁-C₄)-alkyl, -OH, =O, -O-(C₁-C₄)-alkyl and -N(R40)-R41, in another embodiment from the series consisting of halogen, (C₁-C₄)-alkyl, (C₃-C₇)-cycloalkyl, -(C₁-C₄)-alkyl-O-R37, -(C₁-C₄)-alkyl-N(R38)-R39, -C(O)-(C₁-C₄)-alkyl, -OH, =O and -O-(C₁-C₄)-alkyl, in another embodiment from the series consisting of halogen, (C₁-C₄)-alkyl, (C₃-C₇)-cycloalkyl, -(C₁-C₄)-alkyl-O-R37,-OH, =O and -O-(C₁-C₄)-alkyl, in another embodiment from the series consisting of halogen, (C₁-C₄)-alkyl, (C₃-C₇)-cycloalkyl, -(C₁-C₄)-alkyl-O-R37, -(C₁-C₄)-alkyl-N(R38)-R39, -C(O)-(C₁-C₄)-alkyl, -OH and -O-(C₁-C₄)-alkyl, in another embodiment from the series consisting of halogen, (C₁-C₄)-alkyl, (C₃-C₇)-cycloalkyl, -(C₁-C₄)-alkyl-O-R37, -(C₁-C₄)-alkyl-N(R38)-R39, -C(O)-(C₁-C₄)-alkyl, -OH and =O, in another embodiment from the series consisting of halogen, (C₁-C₄)-alkyl, (C₃-C₇)-cycloalkyl, -(C₁-C₄)-alkyl-O-R37, -C(O)-(C₁-C₄)-alkyl and -OH, in another embodiment from the series consisting of halogen, (C₁-C₄)-alkyl, (C₃-C₇)-cycloalkyl, -C(O)-(C₁-C₄)-alkyl and -OH, in another embodiment from the series consisting of halogen, (C₁-C₄)-alkyl and (C₃-C₇)-cycloalkyl and -C(O)-(C₁-C₄)-alkyl, in another embodiment from the series consisting of halogen, (C₁-C₄)-alkyl, (C₃-C₇)-cycloalkyl, -(C₁-C₄)-alkyl-O-R37, -C(O)-(C₁-C₄)-alkyl, -OH and -O-(C₁-C₄)-alkyl, and in another embodiment from the series consisting of halogen, (C₁-C₄)-alkyl, (C₃-C₇)-cycloalkyl, -C(O)-(C₁-C₄)-alkyl, -OH and -O-(C₁-C₄)-alkyl.

In one embodiment, substituents R32 which are bonded to ring nitrogen atoms in R30, are selected from the series consisting of (C₁-C₄)-alkyl, (C₃-C₇)-cycloalkyl, -(C₁-C₄)-alkyl-(C₃-C₇)-cycloalkyl, -(C₁-C₄)-alkyl-O-R37, -(C₁-C₄)-alkyl-N(R38)-R39, -(C₁-C₄)-alkyl-CN, -C(O)-(C₁-C₄)-alkyl, -C(O)-O-(C₁-C₄)-alkyl and -C(O)-N(R42)-R43, in another embodiment from the series consisting of (C₁-C₄)-alkyl, (C₃-C₇)-cycloalkyl, -(C₁-C₄)-alkyl-(C₃-C₇)-cycloalkyl, -(C₁-C₄)-alkyl-O-R37, -(C₁-C₄)-alkyl-N(R38)-R39 and -(C₁-C₄)-alkyl-CN, in another embodiment from the series consisting of (C₁-C₄)-alkyl, (C₃-C₇)-cycloalkyl,-(C₁-C₄)-alkyl-(C₃-C₇)-cycloalkyl and -(C₁-C₄)-alkyl-O-R37, in another embodiment from the series consisting of (C₁-C₄)-alkyl, (C₃-C₇)-cycloalkyl and -(C₁-C₄)-alkyl-O-R37. In one embodiment, the number of oxo substituents (=O) occurring in the cyclic group R30 is not greater than two, in another embodiment it is not greater than one. In one embodiment, halogen representing R32 is selected from the series consisting of fluorine and chlorine, in another embodiment halogen representing R32 is fluorine. In another embodiment, substituents R32 which are bonded to ring nitrogen atoms in R30, are selected from the series consisting of (C₁-C₄)-alkyl, (C₃-C₇)-cycloalkyl, -(C₁-C₄)-alkyl-(C₃-C₇)-cycloalkyl, -(C₁-C₄)-alkyl-O-R37, -(C₁-C₄)-alkyl-N(R38)-R39 and -C(O)-(C₁-C₄)-alkyl, in another embodiment from the series consisting of (C₁-C₄)-alkyl, (C₃-C₇)-cycloalkyl, -(C₁-C₄)-alkyl-(C₃-C₇)-cycloalkyl, -(C₁-C₄)-alkyl-O-R37 and -C(O)-(C₁-C₄)-alkyl, in another embodiment from the series consisting of (C₁-C₄)-alkyl, (C₃-C₇)-cycloalkyl, -(C₁-C₄)-alkyl-O-R37 and -C(O)-(C₁-C₄)-alkyl, in another embodiment from the series consisting of (C₁-C₄)-alkyl, (C₃-C₇)-cycloalkyl and -C(O)-(C₁-C₄)-alkyl. In one embodiment, the number of oxo (=O) substituents R32 occurring in the cyclic group R30 is not greater than two, in another embodiment it is not greater than one. In one embodiment, halogen representing R32 is selected from the series consisting of fluorine and chlorine, in another embodiment halogen representing R32 is fluorine.

In one embodiment of the invention, R33, R34, R35, R36, R37, R38, R39, R40, R41, R42 and R43 are independently of one another selected from the series consisting of hydrogen and (C₁-C₃)-alkyl, in another embodiment from the series consisting of hydrogen and (C₁-C₂)-alkyl, in another embodiment from the series consisting of hydrogen and methyl. In another embodiment, any of the groups R33, R34, R35, R36, R37, R38, R39, R40, R41, R42 and R43 is independently of any other group hydrogen, in another embodiment it is (C₁-C₄)-alkyl, in another embodiment (C₁-C₃)-alkyl, in another embodiment (C₁-C₂)-alkyl, and in another embodiment methyl.

In one embodiment of the invention, R50 is in any of its occurrences, independently of its other occurrences, selected from the series consisting of halogen, (C₁-C₄)-alkyl and -CN; in another embodiment from the series consisting of halogen, (C₁-C₄)-alkyl and -O-(C₁-C₄)-alkyl, in another embodiment from the series consisting of halogen and (C₁-C₄)-alkyl, in another embodiment from the series consisting of halogen and -CN, in another embodiment from the series consisting of halogen. In one embodiment, a group R50 which is bonded to ring nitrogen atom in a group Het2 or Het3, is selected from the series consisting of (C₁-C₄)-alkyl. In one embodiment, a (C₁-C₄)-alkyl group representing R50 or occurring in R50 is in any occurrence of R50, independently of other occurrences, selected from (C₁-C₃)-alkyl, in another embodiment from (C₁-C₂)-alkyl, and in another embodiment it is methyl.

The group Het1 can contain 4, 5, 6 or 7 ring members. In one embodiment of the invention, Het1 is 4-membered to 6-membered, in another embodiment 5-membered or 6-membered, in another embodiment 6-membered. In one embodiment, Het1 comprises 1 ring heteroatom. In one embodiment, the ring heteroatoms in Het1 are selected from the series consisting of nitrogen and oxygen, in another embodiment from the series consisting of oxygen and sulfur, in another embodiment they are nitrogen atoms, and in another embodiment they are oxygen atoms. Examples of heterocycles, from any one or more of which Het1 is chosen in one embodiment, are oxetanyl, tetrahydrofuranyl, tetrahydropyranyl, oxepanyl, tetrahydrothiophenyl, tetrahydrothiopyranyl, azetidinyl, pyrrolidinyl, piperidinyl, azepanyl, morpholinyl, thiomorpholinyl and piperazinyl. In one embodiment, the number of optional substituents in a group Het1 is 1, 2, 3 or 4, in another embodiment it is 1, 2 or 3, in another embodiment it is 1 or 2, in another embodiment it is 1, and in another embodiment Het1 is unsubstituted. In one embodiment, substituents which are bonded to a ring nitrogen atom in Het1, are selected from the series consisting of (C₁-C₄)-alkyl.

The group Het2 can contain 4, 5, 6 or 7 ring members. In one embodiment of the invention, Het2 is 4-membered to 6-membered, in another embodiment 5-membered or 6-membered, in another embodiment 5-membered, in another embodiment 6-membered. In one embodiment, Het2 is a saturated or aromatic group, in another embodiment a saturated group, in another embodiment an aromatic group. In one embodiment, Het2 comprises 1 ring heteroatom. In one embodiment, the ring heteroatoms in Het2 are selected from the series consisting of nitrogen and oxygen, in another embodiment from the series consisting of nitrogen and sulfur, in another embodiment from the series consisting of oxygen and sulfur, in another embodiment they are nitrogen atoms, in another embodiment they are oxygen atoms, and in another embodiment they are sulfur atoms. Examples of heterocycles, from any one or more of which Het2 is chosen in one embodiment, are oxetanyl, tetrahydrofuranyl, furanyl, tetrahydropyranyl, oxepanyl, tetrahydrothiophenyl, thiophenyl, tetrahydrothiopyranyl, azetidinyl, pyrrolidinyl, pyrrolyl, piperidinyl, pyridinyl, azepanyl, pyrazolyl, imidazolyl, oxazolyl, isoxazolyl, thiazolyl, morpholinyl, thiomorpholinyl and piperazinyl.

In one embodiment of the invention, the group Het3 is 5-membered, in another embodiment it is 6-membered. In one embodiment, Het3 comprises 1 or 2 identical or different ring heteroatoms, in another embodiment 1 ring heteroatom. In one embodiment, the ring heteroatoms in Het3 are selected from the series consisting of nitrogen and oxygen, in another embodiment from the series consisting of nitrogen and sulfur, in another embodiment they are nitrogen atoms, and in another embodiment they are sulfur atoms. Examples of heterocycles, from any one or more of which Het3 is chosen in one embodiment, are furanyl, thiophenyl, pyrrolyl, pyridinyl, pyrazolyl, imidazolyl, [1,2,4]triazolyl, oxazolyl, isoxazolyl and thiazolyl.

A subject of the invention are all compounds of the formula I wherein any one or more structural elements such as groups, residues, substituents and numbers are defined as in any of the specified embodiments or definitions of the elements, or have one or more of the specific meanings which are mentioned herein as examples of elements, wherein all combinations of one or more definitions of compounds or elements and/or specified embodiments and/or specific meanings of elements are a subject of the present invention. Also with respect to all such compounds of the formula I, all their stereoisomeric forms and mixtures of stereoisomeric forms in any ratio, and their pharmaceutically acceptable salts are a subject of the present invention.

As an example of compounds of the invention which with respect to any structural elements are defined as in specified embodiments of the invention or definitions of such elements, compounds of the formula I may be mentioned, in any of their stereoisomeric forms or a mixture of stereoisomeric forms in any ratio, and the pharmaceutically acceptable salts thereof, wherein
Ar is selected from the series consisting of phenyl and a 5-membered or 6-membered, monocyclic, aromatic, heterocyclic group which comprises 1 or 2 identical or different ring heteroatoms selected from the series consisting of nitrogen, oxygen and sulfur, and is bonded via a ring carbon atom, which all are unsubstituted or substituted by one or more identical or different substituents R5;
n is selected from the series consisting of 0, 1 and 2;
X is selected from the series consisting of N and CH;
Z is selected from the series consisting of a direct bond, O, S and N(R10);
R1 is selected from the series consisting of H, -N(R11)-R12, -N(R13)-C(O)-R14, -N(R13)-S(O)₂-R15, -N(R13)-C(O)-NH-R16 and (C₁-C₄)-alkyl;
R2 is selected from the series consisting of halogen, (C₁-C₄)-alkyl, -O-(C₁-C₄)-alkyl and -CN;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, (C₃-C₇)-cycloalkyl, -O-(C₁-C₄)-alkyl, -O-(C₃-C₇)-cycloalkyl, -C(O)-N(R6)-R7 and -CN, and two groups R5 bonded to adjacent ring carbon atoms in Ar, together with the carbon atoms carrying them, can form a 5-membered to 8-membered, monocyclic, unsaturated ring which comprises 0, 1 or 2 identical or different ring heteroatoms selected from the series consisting of nitrogen, oxygen and sulfur, and which is unsubstituted or substituted by one or more identical or different substituents R8;
R6 and R7 are independently of one another selected from the series consisting of H and (C₁-C₄)-alkyl;
R8 is selected from the series consisting of halogen and (C₁-C₄)-alkyl;
R10 is selected from the series consisting of H and (C₁-C₄)-alkyl;
R11 and R12 are independently of one another selected from the series consisting of H, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl, wherein phenyl is unsubstituted or substituted by one or more identical or different substituents R50;
R13 is selected from the series consisting of H and (C₁-C₄)-alkyl;
R14 and R16 are independently of one another selected from the series consisting of (C₁-C₈)-alkyl, (C₃-C₇)-cycloalkyl, -(C₁-C₄)-alkyl-(C₃-C₇)-cycloalkyl, phenyl, -(C₁-C₄)-alkyl-phenyl, Het2 and -(C₁-C₄)-alkyl-Het2, wherein (C₁-C₈)-alkyl and (C₃-C₇)-cycloalkyl all are unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R15 is selected from the series consisting of phenyl and Het3, wherein phenyl and Het3 all are unsubstituted or substituted by one or more identical or different substituents R50;
R30 is a 3-membered to 12-membered, monocyclic or bicyclic, saturated, partially unsaturated or aromatic, cyclic group which comprises 0, 1, 2 or 3 identical or different ring heteroatoms selected from the series consisting of nitrogen, oxygen and sulfur, which is unsubstituted or substituted by one or more identical or different substituents R32;
R31 is selected from the series consisting of halogen, -OH, -O-(C₁-C₄)-alkyl, -O-(C₃-C₇)-cycloalkyl, -O-(C₁-C₄)-alkyl-(C₃-C₇)-cycloalkyl, -N(R33)-R34 and -CN;
R32 is selected from the series consisting of halogen, (C₁-C₄)-alkyl, (C₃-C₇)-cycloalkyl, -(C₁-C₄)-alkyl-(C₃-C₇)-cycloalkyl, -(C₁-C₄)-alkyl-O-R37, -(C₁-C₄)-alkyl-N(R38)-R39, -(C₁-C₄)-alkyl-CN,-C(O)-(C₁-C₄)-alkyl, -CN, -OH, =O, -O-(C₁-C₄)-alkyl, -N(R40)-R41, -C(O)-O-(C₁-C₄)-alkyl and-C(O)-N(R42)-R43;
R33, R34, R37, R38, R39, R40, R41, R42 and R43 are independently of one another selected from the series consisting of H and (C₁-C₄)-alkyl;
R50 is selected from the series consisting of halogen, (C₁-C₄)-alkyl, -O-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen, oxygen and sulfur, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated, partially unsaturated or aromatic, heterocyclic group which comprises 1 or 2 identical or different ring heteroatoms selected from the series consisting of nitrogen, oxygen and sulfur, and is bonded via a ring carbon atom;
Het3 is a 5-membered or 6-membered, monocyclic, aromatic, heterocyclic group which comprises 1 or 2 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

As another such example, compounds of the formula I may be mentioned, in any of their stereoisomeric forms or a mixture of stereoisomeric forms in any ratio, and the pharmaceutically acceptable salts thereof, wherein

Ar is selected from the series consisting of phenyl and a 5-membered monocyclic, aromatic, heterocyclic group which comprises 1 or 2 identical or different ring heteroatoms selected from the series consisting of nitrogen and sulfur, and is bonded via a ring carbon atom, which all are unsubstituted or substituted by one or more identical or different substituents R5;
n is selected from the series consisting of 0, 1 and 2;
X is selected from the series consisting of N and CH;
Z is selected from the series consisting of a direct bond, O, S and N(R10);
R1 is selected from the series consisting of H, -N(R11)-R12, -N(R13)-C(O)-R14, -N(R13)-S(O)₂-R15, -N(R13)-C(O)-NH-R16 and (C₁-C₄)-alkyl;
R2 is selected from the series consisting of halogen, (C₁-C₄)-alkyl and -O-(C₁-C₄)-alkyl;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, (C₃-C₇)-cycloalkyl, -O-(C₁-C₄)-alkyl, -O-(C₃-C₇)-cycloalkyl and -CN,
and two groups R5 bonded to adjacent ring carbon atoms in Ar, together with the carbon atoms carrying them, can form a 5-membered to 7-membered, monocyclic, unsaturated ring which comprises 0, 1 or 2 oxygen atoms as ring heteroatoms, and which is unsubstituted or substituted by one or more identical or different substituents R8;
R8 is selected from the series consisting of halogen and (C₁-C₄)-alkyl;
R10 is selected from the series consisting of H and (C₁-C₄)-alkyl;
one of the groups R11 and R12 is selected from the series consisting of hydrogen and (C₁-C₄)-alkyl, and the other of the groups R11 and R12 is selected from the series consisting of hydrogen, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and - (C₁-C₄)-alkyl-phenyl;
R13 is selected from the series consisting of H and (C₁-C₄)-alkyl;
R14 and R16 are independently of one another selected from the series consisting of (C₁-C₈)-alkyl, (C₃-C₇)-cycloalkyl, -(C₁-C₄)-alkyl-(C₃-C₇)-cycloalkyl, phenyl, -(C₁-C₄)-alkyl-phenyl, Het2 and -(C₁-C₄)-alkyl-Het2, wherein (C₁-C₈)-alkyl and (C₃-C₇)-cycloalkyl all are unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R15 is phenyl which is unsubstituted or substituted by one or more identical or different substituents R50;
R30 is a 3-membered to 12-membered, monocyclic or bicyclic, saturated, partially unsaturated or aromatic, cyclic group which comprises 0, 1, 2 or 3 identical or different ring heteroatoms selected from the series consisting of nitrogen and oxygen, which is unsubstituted or substituted by one or more identical or different substituents R32;
R31 is selected from the series consisting of halogen, -OH, -O-(C₁-C₄)-alkyl, -O-(C₃-C₇)-cycloalkyl, -O-(C₁-C₄)-alkyl-(C₃-C₇)-cycloalkyl and -N(R33)-R34;
R32 is selected from the series consisting of halogen, (C₁-C₄)-alkyl, (C₃-C₇)-cycloalkyl, -(C₁-C₄)-alkyl-(C₃-C₇)-cycloalkyl, -(C₁-C₄)-alkyl-O-R37, -(C₁-C₄)-alkyl-N(R38)-R39, -OH, =O, -O-(C₁-C₄)-alkyl and -N(R40)-R41;
R33, R34, R37, R38, R39, R40 and R41 are independently of one another selected from the series consisting of H and (C₁-C₄)-alkyl;
R50 is selected from the series consisting of halogen, (C₁-C₄)-alkyl, -O-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen and oxygen, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated, partially unsaturated or aromatic, heterocyclic group which comprises 1 or 2 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

As another such example, compounds of the formula I may be mentioned, in any of their stereoisomeric forms or a mixture of stereoisomeric forms in any ratio, and the pharmaceutically acceptable salts thereof, wherein
Ar is phenyl, which is unsubstituted or substituted by one or more identical or different substituents R5;
n is selected from the series consisting of 0 and 1;
X is selected from the series consisting of N and CH;
Z is selected from the series consisting of a direct bond, O and N(R10);
R1 is selected from the series consisting of H, -N(R11)-R12, -N(R13)-C(O)-R14 and (C₁-C₄)-alkyl;
R2 is selected from the series consisting of halogen and -O-(C₁-C₄)-alkyl;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, -O-(C₁-C₄)-alkyl and -CN,
and two groups R5 bonded to adjacent ring carbon atoms in Ar, together with the carbon atoms carrying them, can form a 5-membered to 7-membered, monocyclic, unsaturated ring which comprises 0, 1 or 2 oxygen atoms as ring heteroatoms, and which is unsubstituted or substituted by one or more identical or different substituents R8;
R8 is selected from the series consisting of halogen and (C₁-C₄)-alkyl;
R10 is selected from the series consisting of H and (C₁-C₄)-alkyl;
one of the groups R11 and R12 is selected from the series consisting of hydrogen and (C₁-C₄)-alkyl, and the other of the groups R11 and R12 is selected from the series consisting of hydrogen, (C₁-C₄)-alkyl, -(C₁-C₄)-alkyl-(C₃-C₇)-cycloalkyl and -(C₁-C₄)-alkyl-Het1;
R13 is selected from the series consisting of H and (C₁-C₄)-alkyl;
R14 is selected from the series consisting of (C₃-C₇)-cycloalkyl, phenyl and Het2, wherein (C₃-C₇)-cycloalkyl is unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R30 is a 3-membered to 10-membered, monocyclic or bicyclic, saturated, partially unsaturated or aromatic, cyclic group which comprises 0, 1, 2 or 3 identical or different ring heteroatoms selected from the series consisting of nitrogen and oxygen, which is unsubstituted or substituted by one or more identical or different substituents R32;
R31 is selected from the series consisting of halogen, -OH, -O-(C₁-C₄)-alkyl, -O-(C₃-C₇)-cycloalkyl and -N(R33)-R34;
R32 is selected from the series consisting of halogen, (C₁-C₄)-alkyl, (C₃-C₇)-cycloalkyl, -(C₁-C₄)-alkyl-O-R37, -(C₁-C₄)-alkyl-N(R38)-R39, -OH, =O, -O-(C₁-C₄)-alkyl and -N(R40)-R41;
R33, R34, R37, R38, R39, R40 and R41 are independently of one another selected from the series consisting of H and (C₁-C₄)-alkyl;
R50 is selected from the series consisting of halogen, (C₁-C₄)-alkyl, -O-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen and oxygen, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated or aromatic, heterocyclic group which comprises 1 or 2 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

As another such example, compounds of the formula I may be mentioned, in any of their stereoisomeric forms or a mixture of stereoisomeric forms in any ratio, and the pharmaceutically acceptable salts thereof, wherein
Ar is phenyl, which is unsubstituted or substituted by one or more identical or different substituents R5;
n is selected from the series consisting of 0 and 1;
X is selected from the series consisting of N and CH;
Z is selected from the series consisting of a direct bond and O;
R1 is selected from the series consisting of H, -N(R11)-R12 and (C₁-C₄)-alkyl;
R2 is selected from the series consisting of halogen;
R3 is selected from the series consisting of H, R30 and -(C₁-C₄)-alkyl-R30;
R5 is selected from the series consisting of halogen, (C₁-C₄)-alkyl, -O-(C₁-C₄)-alkyl and -CN;
R11 and R12 are independently of one another selected from the series consisting of hydrogen and (C₁-C₄)-alkyl;
R30 is a 3-membered to 7-membered, monocyclic saturated or aromatic, cyclic group which comprises 0, 1 or 2 identical or different ring heteroatoms selected from the series consisting of nitrogen and oxygen, which is unsubstituted or substituted by one or more identical or different substituents R32;
R32 is selected from the series consisting of halogen, (C₁-C₄)-alkyl, (C₃-C₇)-cycloalkyl, -(C₁-C₄)-alkyl-O-R37, -(C₁-C₄)-alkyl-N(R38)-R39, -OH and =O;
R37, R38 and R39 are independently of one another selected from the series consisting of H and (C₁-C₄)-alkyl;
wherein all cycloalkyl groups can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

A subject of the invention also is a compound of the formula I,
for use for increasing fetal hemoglobin (HbF) in erythrocytes; and/or
for use in the treatment of a β-hemoglobinopathy, particularly for use in the treatment of sickle cell disease or β-thalassemia, more particularly for use in the treatment of sickle cell disease,
the compound of the formula I being selected from any of the specific compounds of the formula I which are disclosed herein, or is any one of the specific compounds of the formula I which are disclosed herein, irrespective thereof whether they are disclosed as a free compound and/or as a specific salt, or a pharmaceutically acceptable salt thereof, wherein the compound of the formula I is a subject of the invention in any of its stereoisomeric forms or a mixture of stereoisomeric forms in any ratio, if applicable. For example, a subject of the invention is a compound of the formula I which is selected from the series consisting of:
   N-[4-(3-Amino-4-cyclopropyl-1H-pyrazolo[3,4-d]pyrimidin-6-yl)-phenyl]-2-cyano-5-methoxy-benzenesulfonamide,
   N-[4-(3-Amino-1H-pyrazolo[3,4-d]pyrimidin-6-yl)-phenyl]-2,5-difluoro-benzenesulfonamide,
   N-[4-(3-Amino-1H-pyrazolo[3,4-d]pyrimidin-6-yl)-phenyl]-5-chloro-2-cyano-benzenesulfonamide,
   2-Chloro-N-{4-[4-(1-ethyl-piperidin-3-yloxy)-3-methyl-1H-pyrazolo[3,4-d]pyrimidin-6-yl]-phenyl}-5-methoxy-benzenesulfonamide,
   5-Chloro-N-{4-[4-(1-ethyl-piperidin-3-yloxy)-3-methyl-1H-pyrazolo[3,4-d]pyrimidin-6-yl]-phenyl}-2-fluoro-benzenesulfonamide,
   4-{6-[4-(2,5-Difluoro-benzenesulfonylamino)-phenyl]-3-methyl-1H-pyrazolo[3,4-d]pyrimidin-4-yloxy}-piperidine-1-carboxylic acid ethyl ester,
   N-[4-(3-Amino-4-propoxy-1H-pyrazolo[3,4-d]pyrimidin-6-yl)-phenyl]-2,5-difluoro-benzenesulfonamide,
   N-[4-(3-Amino-4-ethoxy-1H-pyrazolo[3,4-d]pyrimidin-6-yl)-phenyl]-5-chloro-2-fluoro-benzenesulfonamide,
   N-[4-(3-Amino-4-propoxy-1H-pyrazolo[3,4-d]pyrimidin-6-yl)-phenyl]-5-chloro-2-fluoro-benzenesulfonamide,
   N-[4-(3-Amino-4-ethoxy-1H-pyrazolo[3,4-d]pyrimidin-6-yl)-phenyl]-2,5-difluoro-benzenesulfonamide,
   2-Fluoro-N-(4-{4-[1-(2-methoxy-ethyl)-piperidin-4-yloxy]-3-methyl-1H-pyrazolo[3,4-d]pyrimidin-6-yl}-phenyl)-5-methyl-benzenesulfonamide,
   2,5-Difluoro-N-(4-{4-[1-(2-methoxy-ethyl)-piperidin-4-yloxy]-3-methyl-1 H-pyrazolo[3,4-d]pyrimidin-6-yl}-phenyl)-benzenesulfonamide,
   5-Chloro-2-fluoro-N-(4-{4-[1-(2-methoxy-ethyl)-piperidin-4-yloxy]-3-methyl-1H-pyrazolo[3,4-d]pyrimidin-6-yl}-phenyl)-benzenesulfonamide,
   N-{4-[4-(1-Ethyl-piperidin-4-yloxy)-3-methyl-1H-pyrazolo[3,4-d]pyrimidin-6-yl]-phenyl}-2-fluoro-5-methoxy-benzenesulfonamide,
   2,5-Dichloro-N-{4-[4-(1-ethyl-piperidin-4-yloxy)-3-methyl-1H-pyrazolo[3,4-d]pyrimidin-6-yl]-phenyl}-benzenesulfonamide,
   N-{4-[4-(1-Ethyl-piperidin-4-yloxy)-3-methyl-1H-pyrazolo[3,4-d]pyrimidin-6-yl]-phenyl}-2-fluoro-5-methyl-benzenesulfonamide,
   N-{4-[4-(1-Ethyl-piperidin-4-yloxy)-3-methyl-1H-pyrazolo[3,4-d]pyrimidin-6-yl]-phenyl}-2-fluoro-benzenesulfonamide,
   5-Chloro-N-{4-[4-(1-ethyl-piperidin-4-yloxy)-3-methyl-1H-pyrazolo[3,4-d]pyrimidin-6-yl]-phenyl}-2-fluoro-benzenesulfonamide,
   N-{4-[4-(1-Cyclobutyl-piperidin-4-yloxy)-3-methyl-1H-pyrazolo[3,4-d]pyrimidin-6-yl]-phenyl}-2,5-difluoro-benzenesulfonamide,
   2,5-Difluoro-N-(4-{4-[1-(3-methoxy-propyl)-piperidin-4-yloxy]-3-methyl-1H-pyrazolo[3,4-d]pyrimidin-6-yl}-phenyl)-benzenesulfonamide,
   5-Chloro-2-fluoro-N-{4-[4-(3-hydroxy-propoxy)-3-methyl-1H-pyrazolo[3,4-d]pyrimidin-6-yl]-phenyl}-benzenesulfonamide,
   2,5-Difluoro-N-{4-[4-(1-isopropyl-piperidin-4-yloxy)-3-methyl-1H-pyrazolo[3,4-d]pyrimidin-6-yl]-phenyl}-benzenesulfonamide,
   2-Fluoro-N-(4-{4-[1-(2-fluoro-ethyl)-piperidin-4-yloxy]-3-methyl-1H-pyrazolo[3,4-d]pyrimidin-6-yl}-phenyl)-benzenesulfonamide,
   5-Chloro-2-fluoro-N-{4-[4-(1-isopropyl-piperidin-4-yloxy)-3-methyl-1H-pyrazolo[3,4-d]pyrimidin-6-yl]-phenyl}-benzenesulfonamide,
   2,5-Difluoro-N-(4-{4-[1-(2-fluoro-ethyl)-piperidin-4-yloxy]-3-methyl-1H-pyrazolo[3,4-d]pyrimidin-6-yl}-phenyl)-benzenesulfonamide,
   N-[4-(3-Amino-4-isopropoxy-1H-pyrazolo[3,4-d]pyrimidin-6-yl)-phenyl]-2,5-dichloro-benzenesulfonamide,
   N-[4-(3-Amino-4-isobutoxy-1H-pyrazolo[3,4-d]pyrimidin-6-yl)-phenyl]-2,5-difluoro-benzenesulfonamide,
   N-[4-(3-Amino-4-isobutoxy-1H-pyrazolo[3,4-d]pyrimidin-6-yl)-phenyl]-2-fluoro-5-methoxy-benzenesulfonamide,
   2,5-Dichloro-N-{4-[3-methyl-4-(piperidin-3-yloxy)-1H-pyrazolo[3,4-d]pyrimidin-6-yl]-phenyl}-benzenesulfonamide,
   2,5-Difluoro-N-{4-[3-methyl-4-(piperidin-3-yloxy)-1H-pyrazolo[3,4-d]pyrimidin-6-yl]-phenyl}-benzenesulfonamide,
   2-Fluoro-5-methyl-N-{4-[3-methyl-4-(morpholin-2-ylmethoxy)-1H-pyrazolo[3,4-d]pyrimidin-6-yl]-phenyl}-benzenesulfonamide,
   N-{4-[4-(3-Aminomethyl-oxetan-3-ylmethoxy)-1H-pyrazolo[3,4-d]pyrimidin-6-yl]-phenyl}-5-chloro-2-fluoro-benzenesulfonamide,
   N-[4-(3-Amino-4-ethoxymethyl-1H-pyrazolo[3,4-d]pyrimidin-6-yl)-phenyl]-2-fluoro-5-methyl-benzenesulfonamide,
   N-[4-(3-Amino-4-trifluoromethyl-1H-pyrazolo[3,4-d]pyrimidin-6-yl)-phenyl]-2,5-difluoro-benzenesulfonamide,
   2-Fluoro-N-{4-[4-(piperidin-4-yloxy)-1H-pyrazolo[3,4-d]pyrimidin-6-yl]-phenyl}-benzenesulfonamide,
   N-[4-(3-Amino-1 H-pyrazolo[3,4-d]pyrimidin-6-yl)-phenyl]-2-fluoro-5-methoxy-benzenesulfonamide,
   N-[4-(3-Amino-4-methoxymethyl-1H-pyrazolo[3,4-d]pyrimidin-6-yl)-phenyl]-5-chloro-2-fluoro-benzenesulfonamide,
   N-{4-[4-(3-Amino-propoxy)-3-methyl-1H-pyrazolo[3,4-d]pyrimidin-6-yl]-phenyl}-5-chloro-2-fluoro-benzenesulfonamide,
   N-[4-(3-Amino-1H-pyrazolo[3,4-d]pyrimidin-6-yl)-phenyl]-2,5-difluoro-benzenesulfonamide,
   N-[4-(3-Amino-1H-pyrazolo[3,4-d]pyrimidin-6-yl)-phenyl]-2,4,5-trifluoro-benzenesulfonamide,
   N-[4-(3-Amino-1H-pyrazolo[3,4-d]pyrimidin-6-yl)-phenyl]-2-chloro-4,5-difluoro-benzenesulfonamide,
   N-{4-[3-Amino-4-(2,2,2-trifluoro-ethoxy)-1H-pyrazolo[3,4-d]pyrimidin-6-yl]-phenyl}-2-cyano-5-methyl-benzenesulfonamide,
   N-[4-(3-Amino-4-trifluoromethyl-1H-pyrazolo[3,4-d]pyrimidin-6-yl)-phenyl]-5-chloro-2-fluoro-benzenesulfonamide,
   N-{4-[3-Amino-4-(2-methoxy-ethyl)-1H-pyrazolo[3,4-d]pyrimidin-6-yl]-phenyl}-2-cyano-5-methyl-benzenesulfonamide,
   2-Cyano-5-methyl-N-{4-[4-(2,2,2-trifluoro-ethoxy)-1H-pyrazolo[3,4-d]pyrimidin-6-yl]-phenyl}-benzenesulfonamide,
   N-[4-(3-Amino-4-cyclopropyl-1H-pyrazolo[3,4-d]pyrimidin-6-yl)-phenyl]-2,4,5-trifluoro-benzenesulfonamide,
   N-[4-(3-Amino-4-cyclopropyl-1H-pyrazolo[3,4-d]pyrimidin-6-yl)-phenyl]-2-fluoro-benzenesulfonamide,
   N-[4-(3-Amino-4-cyclopropyl-1H-pyrazolo[3,4-d]pyrimidin-6-yl)-phenyl]-2,5-difluoro-benzenesulfonamide,
   N-[4-(3-Amino-4-methoxy-1H-pyrazolo[3,4-d]pyrimidin-6-yl)-phenyl]-5-chloro-2-fluoro-benzenesulfonamide,
   N-[4-(3-Amino-4-methoxy-1H-pyrazolo[3,4-d]pyrimidin-6-yl)-phenyl]-5-chloro-2-cyano-benzenesulfonamide,
   N-[4-(3-Amino-4-methyl-1H-pyrazolo[3,4-d]pyrimidin-6-yl)-phenyl]-2-chloro-3,5-difluoro-benzenesulfonamide,
   2-Cyano-N-{4-[4-(4-hydroxy-cyclohexyloxy)-1H-pyrazolo[3,4-d]pyrimidin-6-yl]-phenyl}-5-methoxy-benzenesulfonamide,
   N-[4-(3-Amino-1H-pyrazolo[4,3-c]pyridin-6-yl)-phenyl]-5-chloro-2,4-difluoro-benzenesulfonamide, and
   5-Chloro-2-cyano-N-{4-[4-(4-hydroxy-cyclohexyloxy)-3-methyl-1H-pyrazolo[3,4-d]pyrimidin-6-yl]-phenyl}-benzenesulfonamide, and/or the series consisting of:
      N-{4-[4-(1-Cyclopropyl-piperidin-4-yloxy)-3-methyl-1H-pyrazolo[3,4-d]pyrimidin-6-yl]-phenyl}-2,5-difluoro-benzenesulfonamide,
      5-Chloro-N-{4-[4-(1-cyclopropyl-piperidin-4-yloxy)-3-methyl-1H-pyrazolo[3,4-d]pyrimidin-6-yl]-phenyl}-2-fluoro-benzenesulfonamide,
      N-{4-[4-(1-Acetyl-piperidin-4-yloxy)-3-methyl-1H-pyrazolo[3,4-d]pyrimidin-6-yl]-phenyl}-2-fluoro-5-methoxy-benzenesulfonamide,
      N-{4-[4-(1-Acetyl-piperidin-4-yloxy)-3-methyl-1H-pyrazolo[3,4-d]pyrimidin-6-yl]-phenyl}-2,5-difluoro-benzenesulfonamide,
      N-{4-[4-(1-Acetyl-piperidin-4-yloxy)-3-methyl-1H-pyrazolo[3,4-d]pyrimidin-6-yl]-phenyl}-5-chloro-2-fluoro-benzenesulfonamide,
      5-Chloro-2-fluoro-N-{4-[4-(6-hydroxy-pyridin-3-yloxy)-3-methyl-1H-pyrazolo[3,4-d]pyrimidin-6-yl]-phenyl}-benzenesulfonamide, and
      2-Fluoro-N-{4-[4-(6-hydroxy-pyridin-3-yloxy)-3-methyl-1H-pyrazolo[3,4-d]pyrimidin-6-yl]-phenyl}-5-methyl-benzenesulfonamide,
   or which is any one of these compounds, and its pharmaceutically acceptable salts.

The compounds of the formula I and their pharmaceutically acceptable salts can therefore be used in animals, in particular in mammals and specifically in humans, as a pharmaceutical or medicament on their own, in mixtures with one another, or in the form of pharmaceutical compositions. A subject of the present invention also are the compounds of the formula I and their pharmaceutically acceptable salts for use as a pharmaceutical. A subject of the present invention also are pharmaceutical compositions and medicaments which comprise at least one compound of the formula I and/or a pharmaceutically acceptable salt thereof as an active ingredient, in an effective dose for the desired use, and a pharmaceutically acceptable carrier, i.e. one or more pharmaceutically innocuous, or nonhazardous, vehicles and/or excipients, and optionally one or more other pharmaceutical active compounds.

A subject of the present invention also are the compounds of the formula I and their pharmaceutically acceptable salts for use in the treatment of the diseases mentioned above or below, including the treatment of any one of the mentioned diseases, for example the treatment of a β-hemoglobinopathy, particularly for use in the treatment of sickle cell disease or β-thalassemia, more particularly for use in the treatment of sickle cell disease. A subject of the present invention also are the use of the compounds of the formula I and their pharmaceutically acceptable salts for the manufacture of a medicament for the treatment of the diseases mentioned above or below, including the treatment of any one of the mentioned diseases, for example the treatment of a β-hemoglobinopathy, particularly for use in the treatment of sickle cell disease or β-thalassemia, more particularly for use in the treatment of sickle cell disease. A subject of the present invention also are methods for the treatment of the diseases mentioned above or below, including the treatment of any one of the mentioned diseases, for example the treatment of a β-hemoglobinopathy, particularly for use in the treatment of sickle cell disease or β-thalassemia, more particularly for use in the treatment of sickle cell disease, which comprise administering an efficacious amount of at least one compound of the formula I and/or a pharmaceutically acceptable salt thereof to a human or an animal which is in need thereof.

The compounds of the formula I and their pharmaceutically acceptable salts, and pharmaceutical compositions and medicaments comprising them, can be administered enterally, for example by oral or rectal administration in the form of pills, tablets, lacquered tablets, coated tablets, granules, hard and soft gelatin capsules, solutions, syrups, emulsions, suspensions, aerosol mixtures or suppositories, or parenterally. Parenteral administration can be carried out, for example, intravenously, intraarticularly, intraperitoneally, intramuscularly or subcutaneously, in the form of injection solutions or infusion solutions, microcapsules, implants or rods, or percutaneously, transdermally or topically, for example in the form of ointments, solutions or tinctures, or in other ways, for example in the form of aerosols or nasal sprays. The preferred form of administration depends on the particulars of the specific case.

Pharmaceutical formulations adapted for transdermal administration can be administered as plasters for extended, close contact with the epidermis of the recipient. For topical administration, formulations such as ointments, creams, suspensions, lotions, powders, solutions, pastes, gels, sprays, aerosols or oils can be used. For the treatment of the eye or other external tissue, for example mouth and skin, suitable formulations are topical ointments or creams, for example. In the case of ointments, the active ingredient can be employed either with a paraffinic or a water-miscible cream base. Alternatively, the active ingredient can be formulated to give a cream with an oil-in-water cream base or a water-in-oil base. Pharmaceutical formulations adapted for topical application to the eye include eye drops, in which the active ingredient is dissolved or suspended in a suitable carrier, in particular an aqueous solvent.

The pharmaceutical compositions according to the invention are prepared in a manner known per se and familiar to the person skilled in the art by admixing one or more pharmaceutically acceptable inert inorganic and/or organic vehicles and excipients with one or more compounds of the formula I and/or pharmaceutically acceptable salts thereof, and bringing them into a suitable form for dosage and administration, which can then be used in human medicine or veterinary medicine. For the production of pills, tablets, coated tablets and hard gelatin capsules it is possible to use, for example, lactose, cornstarch or derivatives thereof, talc, stearic acid or its salts. For the production of gelatin capsules and suppositories fats, waxes, semisolid and liquid polyols, natural or hardened oils, for example, can be used. For the production of solutions, for example injection solutions, or of emulsions or syrups water, saline, alcohols, glycerol, polyols, sucrose, invert sugar, glucose, vegetable oils, for example, can be used, and for the production of microcapsules, implants or rods copolymers of glycolic acid and lactic acid, for example, can be used. The pharmaceutical compositions normally contain from about 0.5% to 90% by weight of the compounds of the formula I and/or their pharmaceutically acceptable salts. The amount of the active ingredient of the formula I and/or its pharmaceutically acceptable salts in the pharmaceutical compositions normally is from about 0.5 mg to about 1000 mg, preferably from about 1 mg to about 500 mg per unit dose. Depending on the kind of the pharmaceutical composition and other particulars of the specific case, the amount may deviate from the indicated ones.

In addition to the active ingredients of the formula I and/or their pharmaceutically acceptable salts and to vehicles, or carrier substances, the pharmaceutical compositions can contain excipients, or auxiliaries or additives, such as, for example, fillers, disintegrants, binders, lubricants, wetting agents, stabilizers, emulsifiers, preservatives, sweeteners, colorants, flavorings, aromatizers, thickeners, diluents, buffer substances, solvents, solubilizers, agents for achieving a depot effect, salts for altering the osmotic pressure, coating agents or antioxidants. They can also contain two or more compounds of the formula I, and/or their pharmaceutically acceptable salts. In case a pharmaceutical composition contains two or more compounds of the formula I, the selection of the individual compounds can aim at a specific overall pharmacological profile of the pharmaceutical composition. For example, a highly potent compound with a shorter duration of action may be combined with a long-acting compound of lower potency. The flexibility permitted with respect to the choice of substituents in the compounds of the formula I allows a great deal of control over the biological and physico-chemical properties of the compounds and thus allows the selection of such desired compounds.

When using the compounds of the formula I, the dose can vary within wide limits and, as is customary and is known to the physician, is to be suited to the individual conditions in each individual case. It depends, for example, on the specific compound employed, on the nature and severity of the disease to be treated, on the mode and the schedule of administration, or on whether an acute or chronic condition is treated or whether prophylaxis is carried out. An appropriate dosage can be established using clinical approaches known to the person skilled in the art. In general, the daily dose for achieving the desired results in an adult weighing about 75 kg is from about 0.01 mg/kg to about 100 mg/kg, preferably from about 0.1 mg/kg to about 50 mg/kg, in particular from about 0.1 mg/kg to about 10 mg/kg, in each case in mg per kg of body weight. The daily dose can be divided, in particular in the case of the administration of relatively large amounts, into several, for example 2, 3 or 4, part administrations. As usual, depending on individual behavior it may be necessary to deviate upwards or downwards from the daily dose indicated.

The compounds of the formula I described herein can be used in combination with one or more additional active therapeutic agents. For the treatment of sickle cell disease, preferably, the one or more additional active therapeutic agents are known to have a beneficial effect against sickle cell disease or against one or more adverse effects of sickle cell disease. Non-limiting examples of these one or more additional active therapeutic agents include hydroxyurea, L-glutamine, voxelotor and crizanlizumab.

### EXAMPLES

### Synthesis of SGK1 inhibitors

The N-(4-(Azaindazol-6-yl)-phenyl)-sulfonamides can be obtained according to the procedures described in PCT application Pub. No. WO 2014/140065A1, which is hereby incorporated by reference in its entirety.

When in the final step of the synthesis of an example compound an acid such as trifluoroacetic acid or acetic acid was used, for example when trifluoroacetic acid was employed to remove an acid-labile protecting group containing a tert-butyl group, or when a compound was purified by chromatography using an eluent which contained such an acid, as is usual in HPLC (High Performance Liquid Chromatography) purifications on reversed phase columns, in some cases, depending on the work-up procedure, for example the details of a freeze-drying process, the compound was obtained partially or completely in the form of a salt of the acid used, for example in the form of the acetic acid salt or trifluoroacetic acid salt. In the names of the example compounds and the structural formulae such contained trifluoroacetic acid or acetic acid is not specified. Likewise, the acid component of other acid addition salts, such as hydrochlorides, for example, in the form of which example compounds have in part been isolated, is not specified in the names and formulae.

The prepared compounds were in general characterized by spectroscopic data and chromatographic data, in particular mass spectra (MS) and/or nuclear magnetic resonance (NMR) spectra. ¹H-NMR spectra were generally recorded at 400 MHz. In the NMR characterization, the chemical shift δ (in ppm), the number of hydrogen atoms (H), the coupling constant J (in Hz) and the multiplicity (s: singlet, d: doublet, dd: double doublet, t: triplet, dt: double triplet, m: multiplet; br: broad) of the peaks are given. In the MS characterization, the mass number (m/e) of the peak of the molecular ion (M) or of a related ion such as the ion (M+1), i.e. the protonated molecular ion (M+H), or the ion (M-1), which was formed depending on the ionization method used, is given. Generally, the ionization method was electrospray ionization (ES+ or ES-).

### Abbreviations

BDFP 1,1'-Bis(diphenylphosphino)ferrocene-palladium(II) dichloride (Pd(dppf)₂Cl₂)
DCM Dichloromethane
Diox [1,4]Dioxane
DMF N,N-Dimethylformamide
DMSO Dimethyl sulfoxide
EtOAc Ethyl acetate
Hep n-Heptane
iPrOH Isopropanol
MeCN Acetonitrile
RT Room temperature (20°C to 25°C)
TFA Trifluoroacetic acid
THF Tetrahydrofuran

### Example 1: 2,5-Dichloro-N-[4-(3-methyl-1H-pyrazolo[3,4-d]pyrimidin-6-yl)-phenyl]-benzenesulfonamide

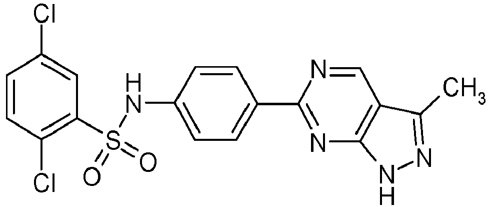

(i) 2,5-Dichloro-N-[4-(4,4,5,5-tetramethyl-[1,3,2]dioxaborolan-2-yl)-phenyl]-benzenesulfonamide 2,5-Dichloro-benzenesulfonyl chloride (11.7 g) and 4-(4,4,5,5-tetramethyl-[1,3,2]dioxaborolan-2-yl)-phenylamine (10.0 g) were added to a reaction vessel containing a magnetic stirring bar, followed by 200 ml dry DCM and 4.1 ml pyridine. The reaction mixture was stirred at RT for 20 h before being cooled on an ice-bath and quenched with 1M aqueous sodium hydroxide solution. The organic phase was separated and the aqueous phase acidified with 2M aqueous hydrochloric acid and extracted three times with EtOAc. The combined organic phases were washed with brine and dried over sodium sulfate and evaporated to afford the crude product. Purification by flash chromatography on silica gel using a mixture of EtOAc and Hep as the eluent afforded 2,5-dichloro-N-[4-(4,4,5,5-tetramethyl-[1,3,2]dioxaborolan-2-yl)-phenyl]-benzenesulfonamide as a colorless solid after evaporation of the solvents under reduced pressure. Yield: 13.67 g (70%). MS (ES-): m/e = 426.2 (M-H), chloro pattern.
(ii) 2,5-Dichloro-N-[4-(3-methyl-1H-pyrazolo[3,4-d]pyrimidin-6-yl)-phenyl]-benzenesulfonamide 2,5-Dichloro-N-[4-(4,4,5,5-tetramethyl-[1,3,2]dioxaborolan-2-yl)-phenyl]-benzenesulfonamide (514 mg) was added to a reaction vessel containing a magnetic stirring bar together with 6-chloro-3-methyl-1-(tetrahydro-pyran-2-yl)-1H-pyrazolo[3,4-d]pyrimidine (303 mg) (prepared from 6-chloro-3-methyl-1H-pyrazolo[3,4-d]pyrimidine (WO 2005/121107) analogously to the procedure described in example 3 (i)), BDFP (70 mg) and cesium carbonate (1.17 g), followed by 12 ml Diox and 2 ml water, and the mixture heated to 100°C under stirring. After 3 h the reaction mixture was cooled to RT and quenched with a saturated aqueous sodium hydrogencarbonate solution (100 ml) and extracted with EtOAc (3 x 200 ml). The combined aqueous phases were dried over sodium sulfate, filtered and evaporated to afford the crude product as a brown oil. The crude product was dissolved in a mixture of 4M HCI in Diox (6 ml) and iPrOH (6 ml) and stirred for 2 h at RT before evaporation of the solvent. The crude product was purified by preparative HPLC (C18 reversed phase column, elution with a water/MeCN gradient with 0.1% TFA). The fractions containing the product were lyophilized to yield pure 2,5-dichloro-N-[4-(3-methyl-1H-pyrazolo[3,4-d]pyrimidin-6-yl)-phenyl]-benzenesulfonamide. Yield: 83 mg (16%).

¹H-NMR (DMSO-d₆): δ (ppm) = 2.55 (s, 3H), 7.27 (d, J = 8.8 Hz, 2H), 7.69 (d, J = 8.5 Hz, 1H), 7.74 (dd, J = 2.5, 8.5 Hz, 1H), 8.07 (d, J = 2.5 Hz, 1H), 8.34 (d, J = 8.8 Hz, 2H), 9.31 (s, 1H), 11.12 (s, 1H), 13.56 (s, 1H).

MS (ES+): m/e = 434.1 (M+H), chloro pattern.

### Example 2: 2,5-Dichloro-N-[4-(4-morpholin-4-yl-1H-pyrazolo[3,4-d]pyrimidin-6-yl)-phenyl]-benzenesulfonamide

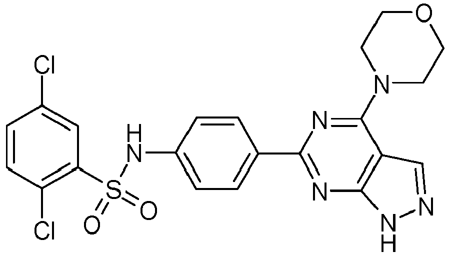

(i) 6-Chloro-4-morpholin-4-yl-1H-pyrazolo[3,4-d]pyrimidine Morpholine (1.19 ml) was added to a reaction vessel at RT equipped with a magnetic stirring bar and containing a solution of commercially available 4,6-dichloro-1H-pyrazolo[3,4-d]pyrimidine (2.46 g) in DCM (100 ml) and triethylamine (3.61 ml). The reaction mixture was stirred at RT for 2 h during which a white precipitate was formed, and then evaporated to dryness. The residue was stirred in water (100 ml) for 1 h, filtered and the solid washed with water and dried under vacuum to afford 2.84 g 6-chloro-4-morpholin-4-yl-1H-pyrazolo[3,4-d]pyrimidine as a colorless solid (91%). MS (ES+): m/e = 240.1 (M+H), chloro pattern.
(ii) 6-Chloro-4-morpholin-4-yl-1-(tetrahydro-pyran-2-yl)-1H-pyrazolo[3,4-d]pyrimidine 6-Chloro-4-morpholin-4-yl-1H-pyrazolo[3,4-d]pyrimidine (1.2 g) was dissolved in THF (30 ml) followed by addition of 3,4-dihydro-2H-pyran (2.29 ml) and pyridinium 4-toluenesulfonate (63 mg) at RT. The reaction mixture was heated to 60°C for 3 h and allowed to cool down before evaporation of the volatiles. The residue was dissolved in DCM (80 ml) and washed with a saturated aqueous sodium hydrogencarbonate solution (3 x 50 ml), dried over sodium sulfate, filtered and evaporated to afford 6-chloro-4-morpholin-4-yl-1-(tetrahydro-pyran-2-yl)-1H-pyrazolo[3,4-d]pyrimidine in quantitative yield. MS (ES+): m/e = 324.1 (M+H), chloro pattern.
(iii) 2,5-Dichloro-N-[4-(4-morpholin-4-yl-1H-pyrazolo[3,4-d]pyrimidin-6-yl)-phenyl]-benzenesulfonamide 2,5-Dichloro-N-[4-(4,4,5,5-tetramethyl-[1,3,2]dioxaborolan-2-yl)-phenyl]-benzenesulfonamide (343 mg) was added to a reaction vessel containing a magnetic stirring bar together with 6-chloro-4-morpholin-4-yl-1-(tetrahydro-pyran-2-yl)-1H-pyrazolo[3,4-d]pyrimidine (259 mg), BDFP (47 mg) and cesium carbonate (782 mg), followed by 8 ml Diox and 2 ml water, and the mixture heated to 100°C under stirring. After 3 h the reaction mixture was cooled to RT and quenched with a saturated aqueous sodium hydrogencarbonate solution (100 ml) and extracted with EtOAc (3 x 200 ml). The combined aqueous phases were dried over sodium sulfate, filtered and evaporated to afford the crude product as a brown oil. The crude product was dissolved in a mixture of 4M HCI in Diox (6 ml) and iPrOH (6 ml) and stirred for 2 h at RT before evaporation of the solvent. The crude product was purified by preparative HPLC (C18 reversed phase column, elution with a water/MeCN gradient with 0.1% TFA). The fractions containing the product were lyophilized to yield pure 2,5-dichloro-N-[4-(4-morpholin-4-yl-1H-pyrazolo[3,4-d]pyrimidin-6-yl)-phenyl]-benzenesulfonamide. Yield: 163 mg (40%).

¹H-NMR (DMSO-d₆): δ (ppm) = 3.76-3.79 (m, 2H), 4.00 (br, 2H), 7.24 (d, J = 8.8 Hz, 2H), 7.69 (d, J = 8.6 Hz, 1H), 7.74 (dd, J = 2.5, 8.6 Hz, 1H), 8.06 (d, J = 2.5 Hz, 1H), 8.26 (d, J = 8.8 Hz, 2H), 8.39 (br, 1H), 11.12 (br s, 1H).

MS (ES+): m/e = 505.3 (M+H), chloro pattern.

### Example 3: 5-Chloro-2-fluoro-N-{4-[4-(4-hydroxy-cyclohexyloxy)-1H-pyrazolo[3,4-d]pyrimidin-6-yl]-phenyl}-benzenesulfonamide

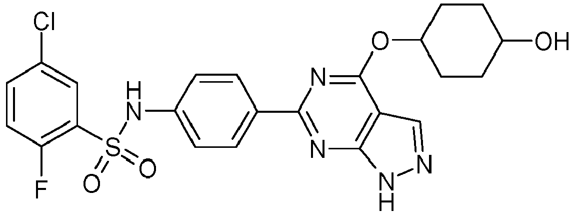

(i) 4,6-Dichloro-1-(tetrahydro-pyran-2-yl)-1H-pyrazolo[3,4-d]pyrimidine Commercially available 4,6-dichloro-1H-pyrazolo[3,4-d]pyrimidine (30 g) was dissolved in THF (400 ml) in a reaction vessel containing a magnetic stirring bar, followed by addition of 3,4-dihydro-2H-pyran (72.5 ml) and pyridinium 4-toluenesulfonate (1.99 g) at RT. The reaction mixture was heated to 60°C for 2 h and allowed to cool down before evaporation of the volatiles. The residue was dissolved in EtOAc (200 ml) and washed with a saturated aqueous sodium hydrogencarbonate solution (3 x 100 ml), dried over sodium sulfate, filtered and evaporated to afford 4,6-dichloro-1-(tetrahydro-pyran-2-yl)-1H-pyrazolo[3,4-d]pyrimidine in quantitative yield.
(ii) 4-[6-Chloro-1-(tetrahydro-pyran-2-yl)-1H-pyrazolo[3,4-d]pyrimidin-4-yloxy]-cyclohexanol 1,4-Cyclohexanediol (1.91 g, cis-trans mixture) was dissolved in 25 ml dry THF in a reaction vessel containing a magnetic stirring bar under a argon atmosphere, and the mixture cooled on an ice bath. Then sodium hydride (132 mg, 60% suspension in mineral oil) was added and the mixture stirred on an ice bath for approximately 30 min before addition of 4,6-dichloro-1-(tetrahydro-pyran-2-yl)-1H-pyrazolo[3,4-d]pyrimidine (948 mg) dissolved in 10 ml THF. The ice bath was removed and the mixture stirred at RT until complete conversion of the starting material as monitored by HPLC/MS. Then the reaction mixture was quenched with water (50 ml) and extracted with EtOAc (3 x 100 ml) and the combined organic phases dried over sodium sulfate, filtered and evaporated. The crude product was purified by flash chromatography on silica gel using a mixture of EtOAc and Hep as the eluent to afford 4-[6-chloro-1-(tetrahydro-pyran-2-yl)-1H-pyrazolo[3,4-d]pyrimidin-4-yloxy]-cyclohexanol as a colorless oil after evaporation. Yield: 755 mg (65%). MS (ES+): m/e = 353.0 (M+H), chloro pattern.
(iii) 5-Chloro-2-fluoro-N-{4-[4-(4-hydroxy-cyclohexyloxy)-1H-pyrazolo[3,4-d]pyrimidin-6-yl]-phenyl}-benzenesulfonamide 5-Chloro-2-fluoro-N-[4-(4,4,5,5-tetramethyl-[1,3,2]dioxaborolan -2-yl)-phenyl]-benzenesulfonamide (205 mg) was added to a reaction vessel containing a magnetic stirring bar together with 4-[6-chloro-1-(tetrahydro-pyran-2-yl)-1H-pyrazolo[3,4-d]pyrimidin-4-yloxy]-cyclohexanol (177 mg), BDFP (29 mg) and cesium carbonate (489 mg), followed by 5 ml Diox and 1 ml water, and the mixture heated to 100°C under stirring. After 3 h the reaction mixture was cooled to RT and quenched with a saturated aqueous sodium hydrogencarbonate solution (50 ml) and extracted with EtOAc (3 x 75 ml). The combined aqueous phases were dried over sodium sulfate, filtered and evaporated to afford the crude product as a brown oil. The crude product was dissolved in a mixture of 4M HCI in Diox (6 ml) and iPrOH (6 ml) and stirred for 2 h at RT before evaporation of the solvent. The crude product was purified by preparative HPLC (C18 reversed phase column, elution with a water/MeCN gradient with 0.1% TFA). The fractions containing the product were lyophilized to yield pure 5-chloro-2-fluoro-N-{4-[4-(4-hydroxy-cyclohexyloxy)-1H-pyrazolo[3,4-d]pyrimidin-6-yl]-phenyl}-benzenesulfonamide. Yield: 49 mg (19%).

¹H-NMR (DMSO-d₆): δ (ppm) = 1.39-2.20 (m, 8H), 3.55-3.63 (m, 0.67H), 3.66-3.72 (m, 0.33H), 4.55 (br, 0.33H), 4.63 (br, 0.67H), 5.40-5.48 (m, 0.67H), 5.50-5.55 (m, 0.33H), 7.26-7.31 (m, 2H), 7.49-7.54 (m, 1H), 7.77-7.81 (m, 1H), 7.86-7.89 (m, 1H), 8.11 (br s, 0.67H), 8.14 (br s, 0.33H), 8.29-8.34 (m, 2H), 11.10 (br s, 0.33H), 11.11 (br s, 0.67H), 13.86 (br s, 1H).

MS (ES+): m/e = 518.1 (M+H), chloro pattern.

### Example 4: 2-Cyano-N-{4-[4-(4-hydroxy-cyclohexyloxy)-1H-pyrazolo[3,4-d]pyrimidin-6-yl]-phenyl}-5-methyl-benzenesulfonamide

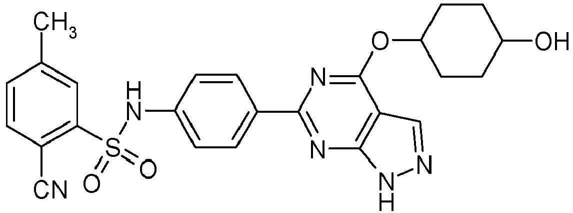

(i) 4-[6-(4-Amino-phenyl)-1H-pyrazolo[3,4-d]pyrimidin-4-yloxy]-cyclohexanol 4-[6-Chloro-1-(tetrahydro-pyran-2-yl)-1H-pyrazolo[3,4-d]pyrimidin-4-yloxy]-cyclohexanol (2.0 g) (example 3, step (ii)) was added to a reaction vessel containing a magnetic stirring bar together with 4-(4,4,5,5-tetramethyl-[1,3,2]dioxaborolan-2-yl)-phenylamine (1.24 g), BDFP (331 mg) and cesium carbonate (5.5 g), followed by 50 ml Diox and 5 ml water, and the mixture heated to 100°C under stirring. After 1 h the reaction mixture was cooled to RT and quenched with a saturated aqueous sodium hydrogencarbonate solution (50 ml) and extracted with EtOAc (3 x 75 ml). The combined aqueous phases were dried over sodium sulfate, filtered and evaporated to afford the crude product as a brown oil. The crude product was purified by flash chromatography on silica gel using a mixture of EtOAc and Hep as the eluent to afford 4-[6-(4-amino-phenyl)-1H-pyrazolo[3,4-d]pyrimidin-4-yloxy]-cyclohexanol as a brown foam. Yield: 1.1 g (47%). MS (ES+): m/e = 410.2 (M+H).
(ii) 2-Cyano-N-{4-[4-(4-hydroxy-cyclohexyloxy)-1H-pyrazolo[3,4-d]pyrimidin-6-yl]-phenyl}-5-methyl-benzenesulfonamide 4-[6-(4-Amino-phenyl)-1H-pyrazolo[3,4-d]pyrimidin-4-yloxy]-cyclohexanol (280 mg) was dissolved in pyridine (3 ml) and 2-cyano-5-methyl-benzenesulfonyl chloride (147 mg) was added and the mixture heated to 100°C for 1 h and allowed to cool down before evaporation of the volatiles. The crude product was purified by preparative HPLC (C18 reversed phase column, elution with a water/MeCN gradient with 0.1% TFA). The fractions containing the product were lyophilized to yield pure 2-cyano-N-{4-[4-(4-hydroxy-cyclohexyloxy)-1H-pyrazolo[3,4-d]pyrimidin-6-yl]-phenyl}-5-methyl-benzenesulfonamide. Yield: 166.7 mg (48%).

¹H-NMR (DMSO-d₆): δ (ppm) = 1.39-1.50 (m, 2H), 1.55-1.66 (m, 2H), 1.87-1.95 (m, 2H), 2.11-2.20 (m, 2H), 3.56-3.62 (m, 1H), 5.41-5.47 (m, 1H), 7.27 (d, J = 8.7 Hz, 2H), 7.64 (d, J = 7.8 Hz, 1H), 7.97 (d, J = 7.8 Hz, 1H), 7.97 (s, 1H), 8.12 (s, 1H), 8.30 (d, J = 8.7 Hz, 2H), 11.13 (s, 1H), 13.87 (br s, 1H).

MS (ES+): m/e = 505.2 (M+H).

### Example 5: 2,5-Dichloro-N-[4-(4-pyridin-3-yl-1H-pyrazolo[3,4-d]pyrimidin-6-yl)-phenyl]-benzenesulfonamide

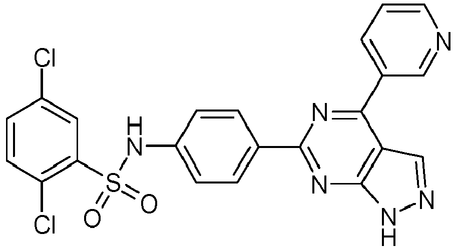

(i) 6-Chloro-4-pyridin-3-yl-1-(tetrahydro-pyran-2-yl)-1H-pyrazolo[3,4-d]pyrimidine In a reaction vessel containing a magnetic stirrer bar, 4,6-dichloro-1-(tetrahydro-pyran-2-yl)-1H-pyrazolo[3,4-d]pyrimidine (1.0 g) (example 3, step (i)) was dissolved in dry toluene (50 ml) before adding lithium chloride (434 mg), tetrakis(triphenylphosphine)palladium (338 mg) and 3-(tributylstannyl)pyridine (1.17 ml). The reaction mixture was heated to 100°C for 20 h before being cooled to RT and quenched with a saturated aqueous sodium hydrogencarbonate solution (50 ml) and extracted with EtOAc (3 x 100 ml). The combined aqueous phases were dried over sodium sulfate, filtered and evaporated to afford the crude product that was purified by flash chromatography on silica gel using a mixture of EtOAc and Hep as the eluent to afford pure 6-chloro-4-pyridin-3-yl-1-(tetrahydro-pyran-2-yl)-1H-pyrazolo[3,4-d]pyrimidine. Yield: 45 mg (4%). MS (ES+): m/e = 316.1 (M+H), chloro pattern.
(ii) 2,5-Dichloro-N-[4-(4-pyridin-3-yl-1H-pyrazolo[3,4-d]pyrimidin-6-yl)-phenyl]-benzenesulfonamide 2,5-Dichloro-N-[4-(4,4,5,5-tetramethyl-[1,3,2]dioxaborolan-2-yl)-phenyl]-benzenesulfonamide (67.1 mg) was added to a reaction vessel containing a magnetic stirring bar together with 6-chloro-4-pyridin-3-yl-1-(tetrahydro-pyran-2-yl)-1H-pyrazolo[3,4-d]pyrimidine (45 mg), BDFP (8 mg) and cesium carbonate (140 mg), followed by 3 ml Diox and 0.5 ml water, and the mixture heated to 100°C under stirring. After 20 h the reaction mixture was cooled to RT and quenched with a saturated aqueous sodium hydrogencarbonate solution (25 ml) and extracted with EtOAc (3 x 25 ml). The combined aqueous phases were dried over sodium sulfate, filtered and evaporated to afford the crude product as a brown oil. The crude product was dissolved in a mixture of 4M HCI in Diox (6 ml) and iPrOH (6 ml) and stirred for 2 h at RT before evaporation of the solvent. The crude product was purified by preparative HPLC (C18 reversed phase column, elution with a water/MeCN gradient with 0.1% TFA). The fractions containing the product were lyophilized to yield pure 2,5-dichloro-N-[4-(4-pyridin-3-yl-1H-pyrazolo[3,4-d]pyrimidin-6-yl)-phenyl]-benzenesulfonamide. Yield: 15.7 mg (21%).

¹H-NMR (DMSO-d₆): δ (ppm) = 7.33 (d, J = 8.9 Hz, 2H), 7.70 (d, J = 8.5 Hz, 1H), 7.75 (dd, J = 2.5, 8.5 Hz, 1H), 7.83-7.88 (m, 1H), 8.09 (d, J = 2.5 Hz, 1H), 8.50 (d, J = 8.9 Hz, 2H), 8.78 (s, 1H), 8.92 (d, J = 5.0 Hz, 1H), 8.98 (d, J = 7.8 Hz, 1H), 9.63 (d, J = 2.0 Hz, 1H), 11.19 (s, 1H).

MS (ES-): m/e = 495.3 (M-H), chloro pattern.

### Example 6: 5-Chloro-2-fluoro-N-[4-(4-methyl-1H-pyrazolo[3,4-d]pyrimidin-6-yl)-phenyl]-benzenesulfonamide

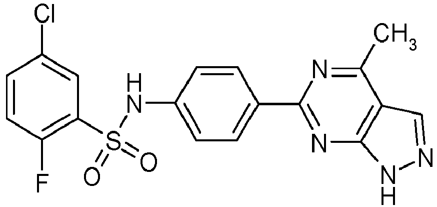

(i) 6-Chloro-4-methyl-1-(tetrahydro-pyran-2-yl)-1H-pyrazolo[3,4-d]pyrimidine 4,6-Dichloro-1-(tetrahydro-pyran-2-yl)-1H-pyrazolo[3,4-d]pyrimidine (1.0 g) (example 3, step (i)) was dissolved in dry THF (20 ml) under an argon atmosphere in a reaction vessel containing a magnetic stirrer bar. The solution was cooled on a dry ice acetone bath and methylmagnesium bromide (1.22 ml, 3M in diethyl ether) was added slowly by syringe and the cooling bath was removed. Incomplete conversion was observed at RT and another equivalent methylmagnesium bromide (1.22 ml, 3M in diethyl ether) was added. After 2 h the reaction mixture was quenched with a saturated aqueous sodium hydrogencarbonate solution (50 ml) and extracted with EtOAc (3 x 100 ml). The combined aqueous phases were dried over sodium sulfate, filtered and evaporated to afford the 6-chloro-4-methyl-1-(tetrahydro-pyran-2-yl)-1H-pyrazolo[3,4-d]pyrimidine. Yield: 920 mg (99%). MS (ES+): m/e = 253.1 (M+H), chloro pattern.
(ii) 5-Chloro-2-fluoro-N-[4-(4-methyl-1H-pyrazolo[3,4-d]pyrimidin-6-yl)-phenyl]-benzenesulfonamide 5-Chloro-2-fluoro-N-[4-(4,4,5,5-tetramethyl-[1,3,2]dioxaborolan-2-yl)-phenyl]-benzenesulfonamide (325 mg) was added to a reaction vessel containing a magnetic stirring bar together with 6-chloro-4-methyl-1-(tetrahydro-pyran-2-yl)-1H-pyrazolo[3,4-d]pyrimidine (200 mg), BDFP (46 mg) and cesium carbonate (773 mg), followed by 6 ml Diox and 1.0 ml water, and the mixture heated to 100°C under stirring. After 4 h the reaction mixture was cooled to RT and quenched with a saturated aqueous sodium hydrogencarbonate solution (35 ml) and extracted with EtOAc (3 x 75 ml). The combined aqueous phases were dried over sodium sulfate, filtered and evaporated to afford the crude product as a brown oil. The crude product was dissolved in a mixture of 4M HCI in Diox (6 ml) and iPrOH (6 ml) and stirred for 2 h at RT before evaporation of the solvent. The crude product was purified by preparative HPLC (C18 reversed phase column, elution with a water/MeCN gradient with 0.1% TFA). The fractions containing the product were lyophilized to yield pure 5-chloro-2-fluoro-N-[4-(4-methyl-1H-pyrazolo[3,4-d]pyrimidin-6-yl)-phenyl]-benzenesulfonamide. Yield: 48.3 mg (15%).

¹H-NMR (DMSO-d₆): δ (ppm) = 2.98 (s, 3H), 7.29 (d, J = 8.8 Hz, 2H), 7.51 (d, J = 8.9 Hz, 1H), 7.76-7.81 (m, 1H), 7.88 (dd, J = 2.5, 6.0 Hz, 1H), 8.35 (d, J = 8.8 Hz, 2H), 8.40 (s, 1H), 11.11 (s, 1H), 13.56 (s, 1H).

MS (ES+): m/e = 417.9 (M+H), chloro pattern.

### Example 7: N-[4-(3-Amino-4-methoxy-1H-pyrazolo[3,4-d]pyrimidin-6-yl)-phenyl]-5-chloro-2-fluoro-benzenesulfonamide

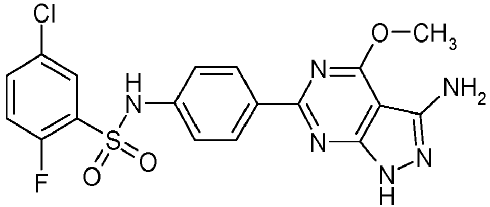

(i) 6-Chloro-4-methoxy-1H-pyrazolo[3,4-d]pyrimidine
   To commercially available 4,6-dichloro-1H-pyrazolo[3,4-d]pyrimidine (5.0 g) dissolved in dry THF (100 ml) was added cesium carbonate (17.2 g) and methanol (60 ml) and the mixture heated to 60°C. After 30 min the reaction mixture was quenched with water and extracted three times with EtOAc. The combined organic phases were dried over sodium sulfate, filtered and evaporated to afford 6-chloro-4-methoxy-1H-pyrazolo[3,4-d]pyrimidine as a brown solid. Yield: 3.34 g (68%). MS (ES+): m/e = 185.0 (M+H), chloro pattern.
(ii) 6-Chloro-3-iodo-4-methoxy-1H-pyrazolo[3,4-d]pyrimidine
   To 6-chloro-4-methoxy-1H-pyrazolo[3,4-d]pyrimidine (2.45 g) dissolved in dry DMF (60 ml) was added N-iodosuccinimide (3.45 g) and the reaction mixture heated to 80°C under stirring. After 3 h the reaction mixture was cooled to RT and the DMF removed by rotary evaporation. Water was added to the residue which was then extracted three times with tert-butyl methyl ether. The combined organic phases were washed with water and brine and dried over sodium sulfate, filtered and evaporated to afford 6-chloro-3-iodo-4-methoxy-1H-pyrazolo[3,4-d]pyrimidine as a brown solid. Yield: 4.13 g (100%). MS (ES+): m/e = 310.9 (M+H), chloro pattern.
(iii) 6-Chloro-3-iodo-4-methoxy-1-(tetrahydro-pyran-2-yl)-1H-pyrazolo[3,4-d]pyrimidine 6-Chloro-3-iodo-4-methoxy-1H-pyrazolo[3,4-d]pyrimidine (4.1 g) was dissolved in THF (60 ml), in a reaction vessel containing a magnetic stirring bar, followed by addition of 3,4-dihydro-2H-pyran (11.4 ml) and pyridinium 4-toluenesulfonate (170 mg) at RT. The reaction mixture was heated to 60°C for 3 h and allowed to cool down before evaporation of the volatiles. The residue was dissolved in EtOAc (200 ml) and washed with a saturated aqueous sodium hydrogencarbonate solution (3 x 100 ml), dried over sodium sulfate, filtered and evaporated to afford 6-chloro-3-iodo-4-methoxy-1-(tetrahydro-pyran-2-yl)-1H-pyrazolo[3,4-d]pyrimidine in quantitative yield. MS (ES+): m/e = 395.0 (M+H), chloro pattern.
(iv) Benzhydrylidene-[6-chloro-4-methoxy-1-(tetrahydro-pyran-2-yl)-1H-pyrazolo[3,4-d]pyrimidin-3-yl]-amine
   To a mixture of palladium acetate (305 mg) and 9,9-dimethyl-4,5-bis(diphenylphosphino)xanthene (923 mg) was added Diox (40 ml) and benzophenone imine (2.98 g) under argon, and the mixture heated to 100°C for 5 min and then cooled to RT. Then cesium carbonate (13.1 g) and 6-chloro-3-iodo-4-methoxy-1-(tetrahydro-pyran-2-yl)-1H-pyrazolo[3,4-d]pyrimidine (5.25 g) in 90 ml Diox was added and the reaction mixture heated to 100°C for 3 h. The reaction mixture was cooled, evaporated and diluted with water (400 ml) and extracted three times with tert-butyl methyl ether. The combined organic phases were washed with water and brine and dried over sodium sulfate, filtered and evaporated to afford the crude product that was purified by flash chromatography on silica gel using a mixture of EtOAc and Hep as the eluent to afford pure benzhydrylidene-[6-chloro-4-methoxy-1-(tetrahydro-pyran-2-yl)-1H-pyrazolo[3,4-d]pyrimidin-3-yl]-amine. Yield: 3.23 g (54%). MS (ES+): m/e = 448.3 (M+H), chloro pattern.
(v) N-{4-[3-(Benzhydrylidene-amino)-4-methoxy-1-(tetrahydro-pyran-2-yl)-1H-pyrazolo[3,4-d]pyrimidin-6-yl]-phenyl}-5-chloro-2-fluoro-benzenesulfonamide
   To a reaction vessel containing 5-chloro-2-fluoro-N-[4-(4,4,5,5-tetramethyl-[1,3,2]dioxaborolan-2-yl)-phenyl]-benzenesulfonamide (288 mg) a magnetic stirring bar and benzhydrylidene-[6-chloro-4-methoxy-1-(tetrahydro-pyran-2-yl)-1H-pyrazolo[3,4-d]pyrimidin-3-yl]-amine (313 mg) was added BDFP (41 mg) and cesium carbonate (688 mg), followed by 6 ml Diox and 1.0 ml water, and the mixture heated to 80°C under stirring. After 3 h the reaction mixture was cooled to RT and quenched with a saturated aqueous sodium hydrogencarbonate solution (35 ml) and extracted with EtOAc (3 x 75 ml). The combined aqueous phases were dried over sodium sulfate, filtered and evaporated to afford the crude product that was purified by flash chromatography on silica gel using a mixture of EtOAc and Hep as the eluent to afford pure N-{4-[3-(benzhydrylidene-amino)-4-methoxy-1-(tetrahydro-pyran-2-yl)-1H-pyrazolo[3,4-d]pyrimidin-6-yl]-phenyl}-5-chloro-2-fluoro-benzenesulfonamide. Yield: 430 mg (88%). MS (ES+): m/e = 697.2 (M+H), chloro pattern.
(vi) N-[4-(3-Amino-4-methoxy-1H-pyrazolo[3,4-d]pyrimidin-6-yl)-phenyl]-5-chloro-2-fluoro-benzenesulfonamide
   N-{4-[3-(Benzhydrylidene-amino)-4-methoxy-1-(tetrahydro-pyran-2-yl)-1H-pyrazolo[3,4-d]pyrimidin-6-yl]-phenyl}-5-chloro-2-fluoro-benzenesulfonamide (430 mg) was dissolved in a mixture of 4M HCI in Diox (6 ml) and iPrOH (6 ml) and stirred for 2 h at RT before evaporation of the solvent. The crude product was purified by preparative HPLC (C18 reversed phase column, elution with a water/MeCN gradient with 0.1% TFA). The fractions containing the product were lyophilized to yield pure N-[4-(3-amino-4-methoxy-1H-pyrazolo[3,4-d]pyrimidin-6-yl)-phenyl]-5-chloro-2-fluoro-benzenesulfonamide. Yield: 45 mg (13%).

¹H-NMR (DMSO-d₆): δ (ppm) = 4.12 (s, 3H), 5.39 (br s, 2H), 7.26 (d, J = 8.7 Hz, 2H), 7.49-7.54 (m, 1H), 7.76-7.81 (m, 1H), 7.87 (dd, J = 2.6, 6.0 Hz, 1H), 8.30 (d, J = 8.8 Hz, 2H), 11.10 (s, 1H), 12.35 (s, 1H).

MS (ES+): m/e = 449.1 (M+H), chloro pattern.

### Analogously to the procedures described in the examples above, the example compounds of the formula Ib

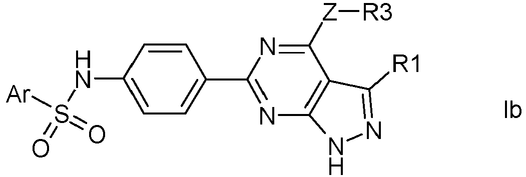

listed in Table 1 were synthesized. In the formulae of the groups -Z-R3 in Table 1 the line crossed with the symbol 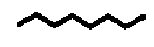 represents the free bond via which the group -Z-R3 is bonded to the carbon atom in the 4-position of the pyrazolo[3,4-d]pyrimidine ring system. I.e., in the formula of the complete molecule the terminal endpoint of the line crossed with the said symbol ends at the carbon atom in the 4-position of the pyrazolo[3,4-d]pyrimidine ring system. If the respective starting compound of the formula H-Z-R3 contained two primary or secondary amino groups, in the employed starting material one of them was protected by a tertbutoxycarbonyl group. If the respective starting compound of the formula H-Z-R3 contained a hydroxy group and a primary or secondary amino group, and a reaction at the hydroxy group was intended, in the employed starting material the amino group was protected by a tertbutoxycarbonyl group. Deprotections were generally performed using either hydrogen chloride in Diox and/or iPrOH or TFA in DCM, for example a 1:1 mixture or TFA and DCM. In the column "Synthesis" the number of the example is specified in analogy to which the synthesis was performed, and in parentheses a reference to footnotes. The ionization method in the MS characterization was ES+ if the specified ion is M+H, and ES- if the specified ion is M-H. CP means chloro pattern, BP means bromo pattern in the mass spectrum.

**Table 1. Example compounds of the formula Ib**

| Example no. | Ar | R1 | 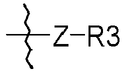 | Synthesis | Yield (%) | MS (m/e) |
|---|---|---|---|---|---|---|
| 8 | 5-chloro-2-fluoro-phenyl | H | 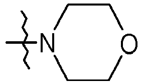 | 2 | 2 | 489.2 (M+H), CP |
| 9 | 2,3-dichlorophenyl | H | 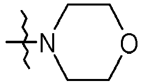 | 2 | 49 | 505.2 (M+H), CP |
| 10 | 5-chloro-2-fluoro-phenyl | CH₃ | 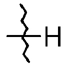 | 1 | 40 | 418.2 (M+H), CP |
| 11 | 2,3-dichlorophenyl | CH₃ | 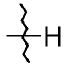 | 1 | 35 | 434.1 (M+H), CP |
| 12 | 2,3-dichlorophenyl | H | 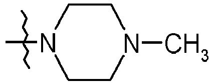 | 2 | 41 | 518.3 (M+H), CP |
| 13 | 5-chloro-2-fluoro-phenyl | H | 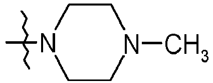 | 2 | 19 | 502.2 (M+H), CP |
| 14 | 2,5-dichlorophenyl | H | 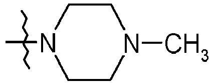 | 2 | 40 | 518.3 (M+H), CP |
| 15 | 2,3-dichlorophenyl | H | 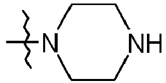 | 2 | 66 | 502.2 (M-H), CP |
| 16 | 5-chloro-2-fluoro-phenyl | H | 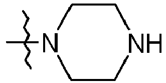 | 2 | 56 | 486.3 (M-H), CP |
| 17 | 2,5-dichlorophenyl | H | 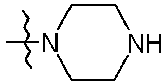 | 2 | 65 | 502.1 (M-H), CP |
| 18 | 2,3-dichlorophenyl | H | 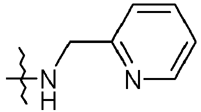 | 2 | 88 | 526.3 (M+H), CP |
| 19 | 5-chloro-2-fluoro-phenyl | H | 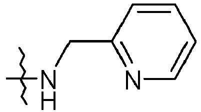 | 2 | 14 | 510.3 (M+H), CP |
| 20 | 5-chloro-2-fluoro-phenyl | H | 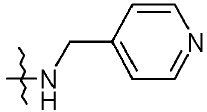 | 2 | 31 | 510.1 (M+H), CP |
| 21 | 2,5-dichlorophenyl | H | 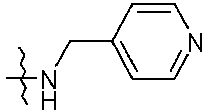 | 2 | 22 | 526.1 (M+H), CP |
| 22 | 5-chloro-2-fluoro-phenyl | H | 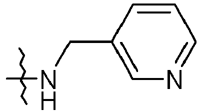 | 2 | 22 | 510.1 (M+H), CP |
| 23 | 5-chloro-2-fluoro-phenyl | H | 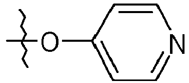 | 3 | 18 | 497.1 (M+H), CP |
| 24 | 2,5-dichlorophenyl | H | 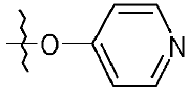 | 3 | 11 | 513.0 (M+H), CP |
| 25 | 2,5-dichlorophenyl | H | 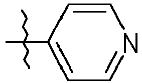 | 5 | 7 | 497.1 (M+H), CP |
| 26 | 5-chloro-2-fluoro-phenyl | H | 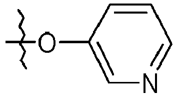 | 3 | 11 | 495.3 (M-H), CP |
| 27 | 2,5-dichlorophenyl | H | 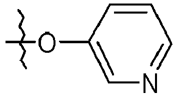 | 3 | 20 | 511.1 (M-H), CP |
| 28 | 2,3-dichlorophenyl | H | 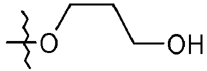 | 3 | 51 | 492.3 (M-H), CP |
| 29 | 5-chloro-2-fluoro-phenyl | H | 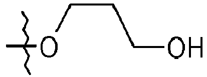 | 3 | 49 | 476.2 (M-H), CP |
| 30 | 2,5-dichlorophenyl | H | 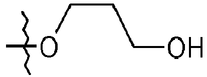 | 3 | 62 | 494.1 (M+H), CP |
| 31 | 2,3-dichlorophenyl | H | 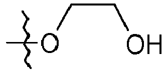 | 3 | 64 | 480.1 (M+H), CP |
| 32 | 5-chloro-2-fluoro-phenyl | H | 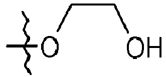 | 3 | 84 | 464.1 (M+H), CP |
| 33 | 2,5-dichlorophenyl | H | 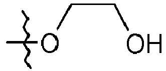 | 3 | 53 | 480.1 (M+H), CP |
| 34 | 2,3-dichlorophenyl | H | 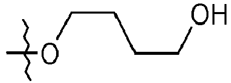 | 3 | 60 | 508.1 (M+H), CP |
| 35 | 5-chloro-2-fluoro-phenyl | H | 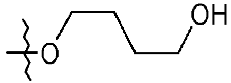 | 3 | 72 | 492.1 (M+H), CP |
| 36 | 2,5-dichlorophenyl | H | 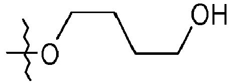 | 3 | 69 | 508.1 (M+H), CP |
| 37 | 5-chloro-2-fluoro-phenyl | H | 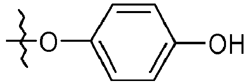 | 3 | 5 | 512.1 (M+H), CP |
| 38 | 5-chloro-2-fluoro-phenyl | H | 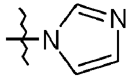 | 3 | 22 | 470.1 (M+H), CP |
| 39 | 2,5-dichlorophenyl | H | 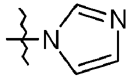 | 3 | 20 | 486.0 (M+H), CP |
| 40 | 5-chloro-2-fluoro-phenyl | H | 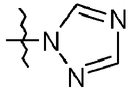 | 3 | 13 | 471.1 (M+H), CP |
| 41 | 2,5-dichlorophenyl | H | 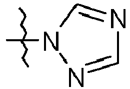 | 3 | 7 | 487.1 (M+H), CP |
| 42 | 5-chloro-2-fluoro-phenyl | H | 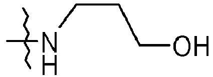 | 2 | 18 | 477.1 (M+H), CP |
| 43 | 2,5-dichlorophenyl | H | 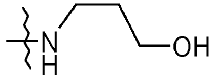 | 2 | 6 | 493.1 (M+H), CP |
| 44 | 2,3-dichlorophenyl | H | 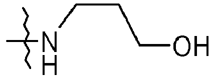 | 2 | 15 | 493.1 (M+H), CP |
| 45 | 2,3-dichlorophenyl | H | 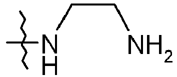 | 2 | 29 | 478.0 (M+H), CP |
| 46 | 5-chloro-2-fluoro-phenyl | H | 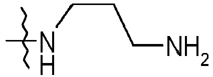 | 2 | 29 | 476.1 (M+H), CP |
| 47 | 5-chloro-2-fluoro-phenyl | H | 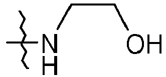 | 2 | 14 | 463.1 (M+H), CP |
| 48 | 2,5-dichlorophenyl | H | 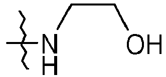 | 2 | 24 | 479.1 (M+H), CP |
| 49 | 2,3-dichlorophenyl | H | 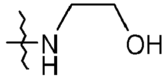 | 2 | 13 | 479.0 (M+H), CP |
| 50 | 5-chloro-2-fluoro-phenyl | H | 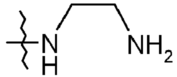 | 2 | 32 | 462.0 (M+H), CP |
| 51 | 2,5-dichlorophenyl | H | 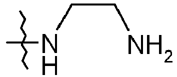 | 2 | 22 | 478.0 (M+H), CP |
| 52 | 2,5-dichlorophenyl | H | 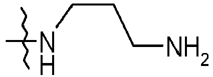 | 2 | 21 | 492.0 (M+H), CP |
| 53 | 2,3-dichlorophenyl | H | 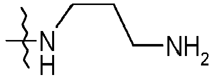 | 2 | 23 | 492.0 (M+H), CP |
| 54 | 5-chloro-2-fluoro-phenyl | H | 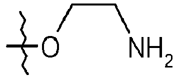 | 3 | 10 | 461.2 (M-H), CP |
| 55 | 2,5-dichlorophenyl | H | 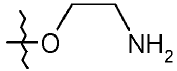 | 3 | 9 | 477.0 (M-H), CP |
| 56 | 2,3-dichlorophenyl | H | 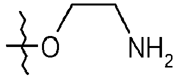 | 3 | 10 | 477.1 (M-H), CP |
| 57 | 5-chloro-2-fluoro-phenyl | H | 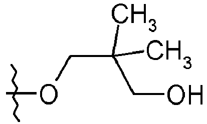 | 3 | 14 | 506.1 (M+H), CP |
| 58 | 2,5-dichlorophenyl | H | 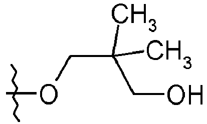 | 3 | 22 | 522.2 (M+H), CP |
| 59 | 2,3-dichlorophenyl | H | 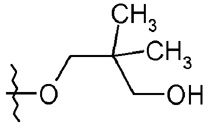 | 3 | 23 | 522.0 (M+H), CP |
| 60 | 2,5-dichlorophenyl | H | 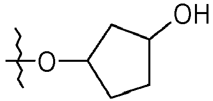 | 3 | 25 | 520.0 (M+H), CP |
| 61 | 2,5-dichlorophenyl | H | 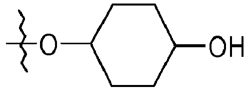 | 3 | 10 | 534.0 (M+H), CP |
| 62 | 5-chloro-2-fluoro-phenyl | H | 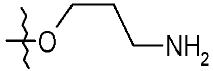 | 3 | 25 | 477.0 (M+H), CP |
| 63 | 2,5-dichlorophenyl | H | 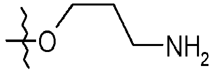 | 3 | 23 | 493.0 (M+H), CP |
| 64 | 2,3-dichlorophenyl | H | 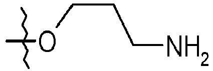 | 3 | 22 | 493.0 (M+H), CP |
| 65 | 5-chloro-2-fluoro-phenyl | H | 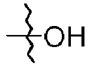 | 3 | 8 | 420.0 (M+H), CP |
| 66 | 5-chloro-2-fluoro-phenyl | H | 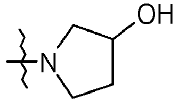 | 2 | 24 | 489.0 (M+H), CP |
| 67 | 2,5-dichlorophenyl | H | 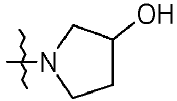 | 2 | 27 | 505.0 (M+H), CP |
| 68 | 5-chloro-2-fluoro-phenyl | H | 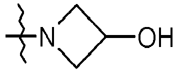 | 2 | 57 | 475.0 (M+H), CP |
| 69 | 2,5-dichlorophenyl | H | 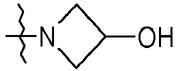 | 2 | 40 | 491.0 (M+H), CP |
| 70 | 5-chloro-2-fluoro-phenyl | H | 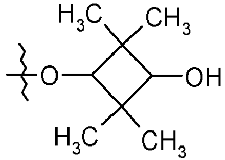 | 3 | 15 | 546.1 (M+H), CP |
| 71 | 5-chloro-2-fluoro-phenyl | H | 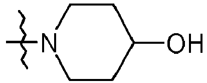 | 2 | 53 | 503.0 (M+H), CP |
| 72 | 2,5-dichlorophenyl | H | 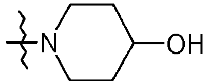 | 2 | 16 | 517.0 (M-H), CP |
| 73 | 5-chloro-2-fluoro-phenyl | H | 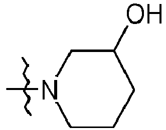 | 2 | 47 | 512.0 (M+H), CP |
| 74 | 2,5-dichlorophenyl | H | 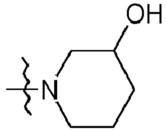 | 2 | 14 | 519.0 (M+H), CP |
| 75 | 5-chloro-2-fluoro-phenyl | H | 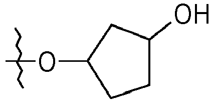 | 3 | 45 | 504.0 (M+H), CP |
| 76 | 2,5-dichlorophenyl | H | 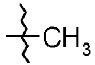 | 6 | 20 | 433.9 (M+H), CP |
| 77 | 5-chloro-2-fluoro-phenyl | H | 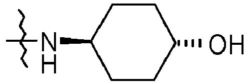 trans configuration | 2 | 32 | 517.0 (M+H), CP |
| 78 | 2,5-dichlorophenyl | H | 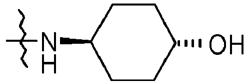 trans configuration | 2 | 18 | 533.0 (M+H), CP |
| 79 | 5-chloro-2-fluoro-phenyl | H | 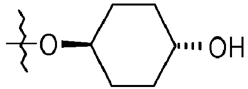 trans configuration | 3 | 54 | 518.0 (M+H), CP |
| 80 | 2,5-dichlorophenyl | H | 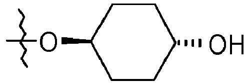 trans configuration | 3 | 43 | 534.0 (M+H), CP |
| 81 | 5-chloro-2-fluoro-phenyl | H | 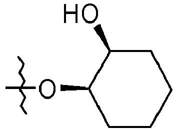 cis configuration, racemic | 3 | 48 | 518.0 (M+H), CP |
| 82 | 2,5-dichlorophenyl | H | 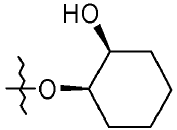 cis configuration, racemic | 3 | 39 | 534.0 (M+H), CP |
| 83 | 5-chloro-2-cyano-phenyl | H | 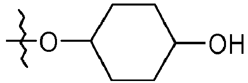 | 3 | 44 | 525.0 (M+H), CP |
| 84 | 5-chloro-2-fluoro-phenyl | H | 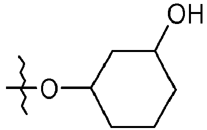 | 3 | 48 | 518.0 (M+H), CP |
| 85 | 2,5-dichlorophenyl | H | 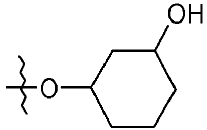 | 3 | 50 | 534.0 (M+H), CP |
| 86 | 5-chloro-2-fluoro-phenyl | H | 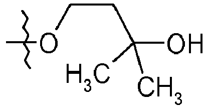 | 3 | 46 | 506.0 (M+H), CP |
| 87 | 5-chloro-2-fluoro-phenyl | H | 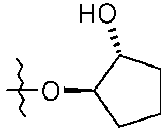 trans, R,R-configuration | 3 | 47 | 504.0 (M+H), CP |
| 88 | 2,5-dichlorophenyl | H | 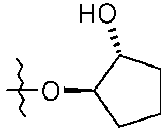 trans, R,R-configuration | 3 | 45 | 520.0 (M+H), CP |
| 89 | 2,5-dichlorophenyl | H | 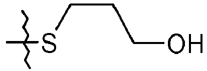 | 3 (a) | 19 | 510.0 (M+H), CP |
| 90 | 2-chloro-5-methoxyphenyl | H | 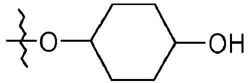 | 3 | 9 | 530.0 (M+H), CP |
| 91 | 2-chloro-5-methoxyphenyl | H | 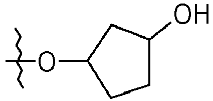 | 3 | 49 | 516.0 (M+H), CP |
| 92 | 2-fluoro-5-methyl-phenyl | H | 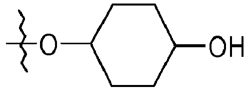 | 4 | 36 | 496.3 (M-H) |
| 93 | 2-cyano-5-methyl-phenyl | CH₃ | 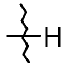 | 1 | 66 | 405.2 (M+H) |
| 94 | 5-chloro-1,3-dimethyl-1H-pyrazol-4-yl | CH₃ | 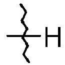 | 1 | | 418.2 (M+H), CP |
| 95 | 2-fluoro-5-methyl-phenyl | CH₃ | 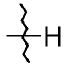 | 1 | 90 | 398.2 (M+H) |
| 96 | 2-cyano-5-methyl-phenyl | CH₃ | 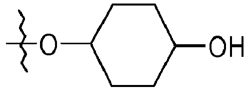 | 3 | 35 | 519.3 (M+H) |
| 97 | 2-cyano-5-methyl-phenyl | CH₃ | 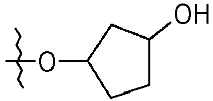 | 3 | 42 | 505.3 (M+H) |
| 98 | 2-cyano-5-methyl-phenyl | CH₃ | 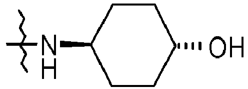 trans configuration | 2 | 38 | 518.3 (M+H) |
| 99 | 2-cyano-5-methyl-phenyl | CH₃ | 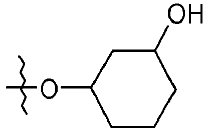 | 3 | 15 | 519.3 (M+H) |
| 100 | 5-chloro-2-cyano-phenyl | CH₃ | 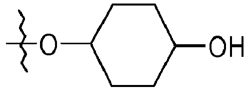 | 3 | 15 | 539.3 (M+H), CP |
| 101 | 2-fluoro-5-methyl-phenyl | CH₃ | 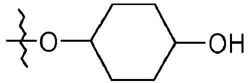 | 3 | 34 | 512.3 (M+H) |
| 102 | 2-chloro-5-methoxyphenyl | CH₃ | 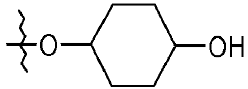 | 3 | 40 | 544.3 (M+H), CP |
| 103 | 7-chloro-2,3-dihydro-benzo[1,4]dioxin-6-yl | CH₃ | 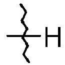 | 4 | 67 | 458.1 (M+H), CP |
| 104 | 2-fluoro-4,5-dimethoxyphenyl | CH₃ | 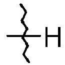 | 4 | 80 | 444.2 (M+H) |
| 105 | 2-bromo-4,5-dimethoxyphenyl | CH₃ | 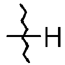 | 4 | 53 | 504.1 (M+H), BP |
| 106 | 4,5-dimethoxy-2-methylphenyl | CH₃ | 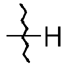 | 4 | 54 | 440.2 (M+H) |
| 107 | 2-fluoro-5-methyl-phenyl | H | 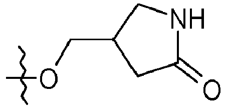 | 3 | 24 | 497.2 (M+H) |
| 108 | 2-fluoro-5-methyl-phenyl | H | 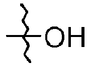 | 3 (b) | 19 | 399.2 (M+H) |
| 109 | 2-fluoro-5-methyl-phenyl | H | 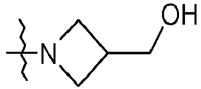 | 2 | 21 | 469.2 (M+H) |
| 110 | 2-chloro-5-methoxyphenyl | H | 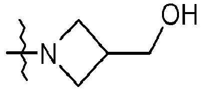 | 2 | 10 | 501.2 (M+H), CP |
| 111 | 5-chloro-2-cyano-phenyl | H | 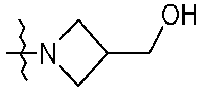 | 2 | 9 | 496.2 (M+H), CP |
| 112 | 2-fluoro-5-methyl-phenyl | H | 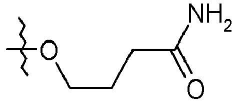 | 3 | 38 | 485.2 (M+H) |
| 113 | 2-chloro-5-methoxyphenyl | H | 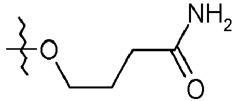 | 3 | 25 | 517.2 (M+H), CP |
| 114 | 5-chloro-2-cyano-phenyl | H | 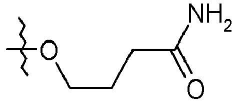 | 3 | 20 | 512.2 (M+H), CP |
| 115 | 5-chloro-2-cyano-phenyl | H | 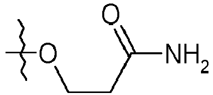 | 3 | 29 | 498.1 (M+H), CP |
| 116 | 2-fluoro-5-methyl-phenyl | H | 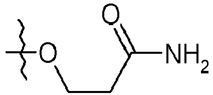 | 3 | 21 | 471.2 (M+H) |
| 117 | 2-chloro-5-methoxyphenyl | H | 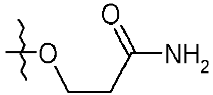 | 3 | 23 | 503.2 (M+H), CP |
| 118 | 2-cyano-5-methyl-phenyl | H | 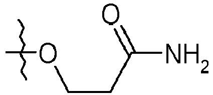 | 3 | 12 | 478.2 (M+H) |
| 119 | 2-fluoro-5-methyl-phenyl | H | 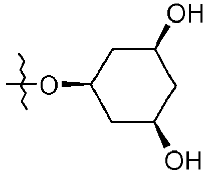 all-cis configuration | 3 | 67 | 514.2 (M+H) |
| 120 | 2-cyano-5-methyl-phenyl | H | 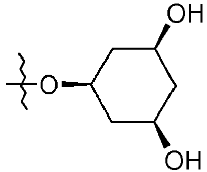 all-cis configuration | 3 | 23 | 521.3 (M+H) |
| 121 | 2-fluoro-5-methyl-phenyl | H | 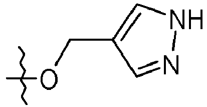 | 3 | 30 | 480.2 (M+H) |
| 122 | 2-cyano-5-methyl-phenyl | H | 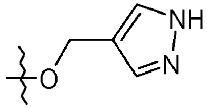 | 3 | 28 | 487.3 (M+H) |
| 123 | 2-fluoro-5-methyl-phenyl | H | 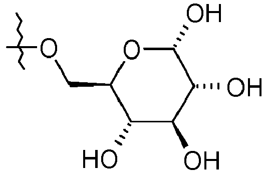 | 3 | 52 | 562.2 (M+H) |
| 124 | 2-cyano-5-methyl-phenyl | H | 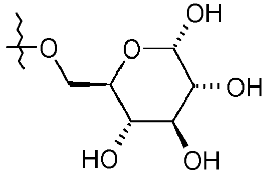 | 3 | 18 | 569.2 (M+H) |
| 125 | 2-cyano-5-methyl-phenyl | H | 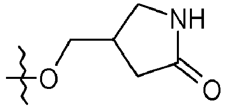 | 3 | 4 | 504.2 (M+H) |
| 126 | 2-chloro-5-methoxyphenyl | H | 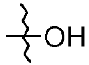 | 3 (b) | | 432.1 (M+H), CP |
| 127 | 5-chloro-2-cyano-phenyl | H | 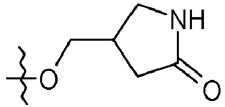 | 3 | 1 | 524.2 (M+H), CP |
| 128 | 5-cyano-2-fluoro-phenyl | CH₃ | 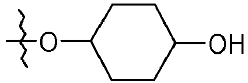 | 3 | 42 | 523.3 (M+H) |
| 129 | 2-fluoro-5-methoxyphenyl | CH₃ | 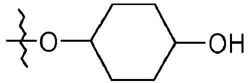 | 3 | 40 | 528.3 (M+H) |
| 130 | 2-cyano-5-methoxyphenyl | CH₃ | 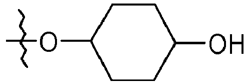 | 3 | 40 | 528.3 (M+H) |
| 131 | 2-cyano-5-fluoro-phenyl | CH₃ | 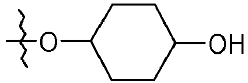 | 3 | 5 | 523.3 (M+H) |
| 132 | 5-cyano-2-fluoro-phenyl | H | 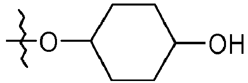 | 3 | 21 | 509.3 (M+H) |
| 133 | 2-fluoro-5-methoxyphenyl | H | 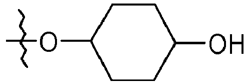 | 3 | 23 | 514.3 (M+H) |
| 134 | 2-cyano-5-methoxyphenyl | H | 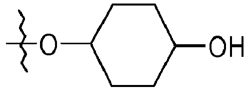 | 3 | 16 | 521.3 (M+H) |
| 135 | 2-cyano-5-methyl-phenyl | H | 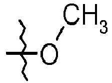 | 3 | 3 | 421.2 (M+H) |
| 136 | 5-chloro-2-fluoro-phenyl | H | 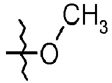 | 3 | 13 | 434.1 (M+H), CP |
| 137 | 2-fluoro-5-methyl-phenyl | H | 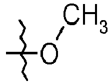 | 3 | 35 | 414.1 (M+H) |
| 138 | 5-chloro-2-cyano-phenyl | H | 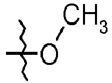 | 3 | 49 | 441.1 (M+H), CP |
| 139 | 2,5-dichlorophenyl | H | 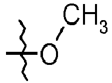 | 3 | 48 | 450.0 (M+H), CP |
| 140 | 2-fluoro-5-methyl-phenyl | H | 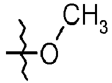 | 3 | 27 | 429.1 (M+H) |
| 141 | 2-fluoro-5-methyl-phenyl | H | 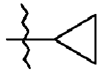 | 6 | 59 | 424.1 (M+H) |
| 142 | 5-chloro-2-fluoro-phenyl | H | 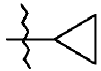 | 6 | 42 | 444.1 (M+H) |
| 143 | 2-chloro-5-methoxyphenyl | H | 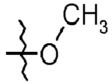 | 3 | 82 | 444.1 (M-H), CP |
| 144 | 2-fluoro-5-methoxyphenyl | H | 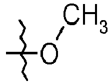 | 3 | 84 | 430.0 (M+H) |
| 145 | 2-cyano-5-methyl-phenyl | H | 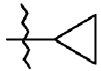 | 6 | 5 | 431.3 (M+H) |
| 146 | 2-cyano-5-methyl-phenyl | H | 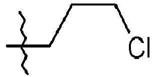 | 6 | 10 | 467.2 (M+H), CP |
| 147 | 2-fluoro-5-methoxyphenyl | H | 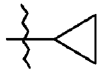 | 6 | 5 | 440.2 (M+H) |
| 148 | 2-fluoro-5-methoxyphenyl | H | 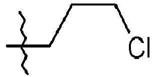 | 6 | 12 | 476.2 (M+H), CP |
| 149 | 2-cyano-5-methyl-phenyl | NH₂ | 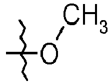 | 7 | 7 | 436.2 (M+H) |
| 150 | 2-chloro-5-methoxyphenyl | NH₂ | 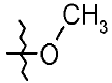 | 7 | 12 | 461.2 (M+H), CP |
| 151 | 5-chloro-2-cyano-phenyl | NH₂ | 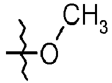 | 7 | 12 | 456.1 (M+H), CP |
| 152 | 2-fluoro-5-methoxyphenyl | NH₂ | 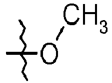 | 7 | 15 | 445.2 (M+H) |
| 153 | 2,5-dichlorophenyl | NH₂ | 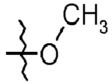 | 7 | 29 | 465.1 (M+H), CP |
| 154 | 2-fluoro-5-methyl-phenyl | H | 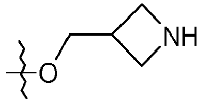 | 6 | 2 | 469.2 (M+H) |
| 155 | 2-chloro-5-methoxyphenyl | H | 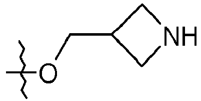 | 6 | 4 | 501.2 (M+H), CP |
| 156 | 2,5-dichlorophenyl | H | 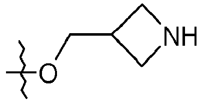 | 6 | 1 | 505.1 (M+H), CP |
| 157 | 2-cyano-5-methyl-phenyl | H | 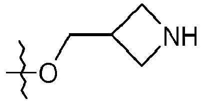 | 6 | 1 | 476.2 (M+H) |
| 158 | 2-fluoro-5-methyl-phenyl | H | 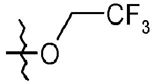 | 6 | 36 | 482.1 (M+H) |
| 159 | 2-chloro-5-methoxyphenyl | H | 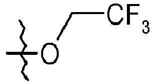 | 6 | 42 | 514.1 (M+H), CP |
| 160 | 5-chloro-2-fluoro-phenyl | H | 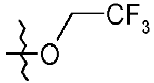 | 6 | 32 | 502.1 (M+H), CP |
| 161 | 2,5-dichlorophenyl | H | 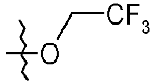 | 6 | 13 | 518.1 (M+H), CP |
| 162 | 5-chloro-2-cyano-phenyl | H | 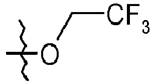 | 6 | 27 | 509.1 (M+H), CP |
| 163 | 2-cyano-5-methyl-phenyl | H | 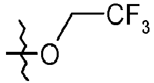 | 6 | 40 | 489.1 (M+H) |
| 164 | 2-chloro-4-fluoro-phenyl | NH₂ | 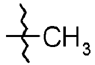 | 6 | 26 | 433.1 (M+H), CP |
| 165 | 2,4,5-trifluorophenyl | NH₂ | 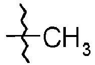 | 6 | 19 | 435.1 (M+H) |
| 166 | 5-bromo-thiophen-2-yl | NH₂ | 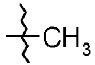 | 6 | 21 | 465.0 (M+H), BP |
| 167 | 5-chloro-thiophen-2-yl | NH₂ | 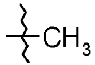 | 6 | 26 | 421.0 (M+H), CP |
| 168 | 4,5-dichloro-thiophen-2-yl | NH₂ | 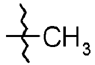 | 6 | 23 | 455.0 (M+H), CP |
| 169 | 2-fluoro-phenyl | NH₂ | 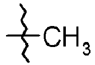 | 6 | 25 | 399.1 (M+H) |
| 170 | 2-chloro-4,5-difluoro-phenyl | NH₂ | 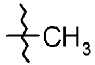 | 6 | 25 | 451.1 (M+H), CP |
| 171 | 3-chloro-2-fluoro-phenyl | NH₂ | 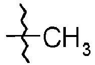 | 6 | 20 | 433.1 (M+H), CP |
| 172 | 4-bromo-thiophen-2-yl | NH₂ | 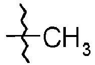 | 6 | 31 | 465.0 (M+H), BP |
| 173 | 4-bromo-thiophen-3-yl | NH₂ | 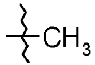 | 6 | 13 | 465.0 (M+H), BP |
| 174 | 4-chloro-thiophen-3-yl | NH₂ | 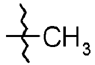 | 6 | 30 | 419.1 (M-H), CP |
| 175 | 2,5-dichloro-thiophen-3-yl | NH₂ | 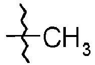 | 6 | 31 | 454.9 (M+H), CP |
| 176 | 3-chloro-2-cyano-phenyl | H | 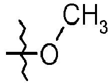 | 3 | 21 | 441.1 (M+H), CP |
| 177 | 2-chloro-3,5-difluoro-phenyl | H | 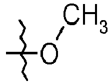 | 3 | 31 | 452.0 (M+H), CP |
| 178 | 5-chloro-2,4-difluoro-phenyl | H | 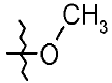 | 3 | 37 | 452.0 (M+H), CP |
| 179 | 2,4,5-trifluorophenyl | H | 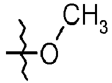 | 3 | 38 | 436.1 (M+H) |
| 180 | 2-chloro-4-fluoro-phenyl | H | 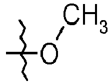 | 3 | 33 | 434.0 (M+H), CP |
| 181 | 3-chloro-2-fluoro-phenyl | H | 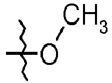 | 3 | 42 | 434.0 (M+H), CP |
| 182 | 2-chloro-4,5-difluoro-phenyl | H | 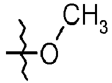 | 3 | 38 | 452.0 (M+H), CP |
| 183 | 4,5-dichloro-thiophen-2-yl | H | 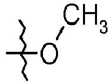 | 3 | 28 | 455.9 (M+H), CP |
| 184 | 2,5-dichloro-thiophen-3-yl | H | 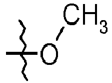 | 3 | 18 | 455.9 (M+H), CP |
| 185 | 2,5-dichlorophenyl | H | 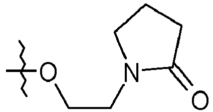 | 3 | 35 | 547.1 (M+H), CP |
| 186 | 5-chloro-2-fluoro-phenyl | H | 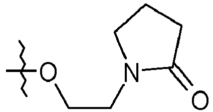 | 3 | 46 | 531.1 (M+H), CP |
| 187 | 5-chloro-2-cyano-phenyl | H | 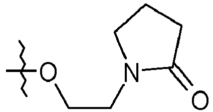 | 3 | 24 | 538.1 (M+H), CP |
| 188 | 2-cyano-5-methyl-phenyl | H | 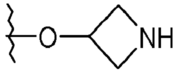 | 3 | 12 | 462.1 (M+H) |
| 189 | 2-chloro-5-methoxyphenyl | H | 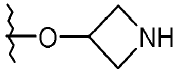 | 3 | 13 | 487.1 (M+H), CP |
| 190 | 2,5-dichlorophenyl | H | 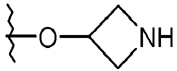 | 3 | 12 | 490.9 (M+H), CP |
| 191 | 5-chloro-2-cyano-phenyl | H | 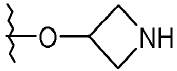 | 3 | 10 | 482.1 (M+H), CP |
| 192 | 2-fluoro-5-methoxyphenyl | H | 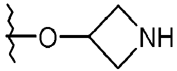 | 3 | 12 | 471.1 (M+H) |
| 193 | 2-cyano-5-methyl-phenyl | NH₂ | 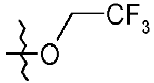 | 7 (c) | 9 | 504.1 (M+H) |
| 194 | 2-chloro-5-methoxyphenyl | NH₂ | 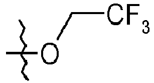 | 7 (c) | 12 | 529.0 (M+H), CP |
| 195 | 2,5-dichlorophenyl | NH₂ | 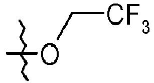 | 7 (c) | 13 | 533.0 (M+H), CP |
| 196 | 5-chloro-2-cyano-phenyl | NH₂ | 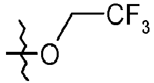 | 7 (c) | 11 | 524.0 (M+H), CP |
| 197 | 2-fluoro-5-methoxyphenyl | NH₂ | 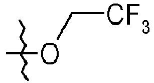 | 7 (c) | 13 | 513.0 (M+H) |
| 198 | 5-chloro-2-fluoro-phenyl | H | 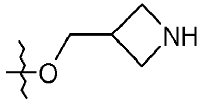 | 3 | 5 | 489.1 (M+H), CP |
| 199 | 2,5-difluorophenyl | H | 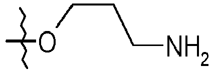 | 3 | 1 | 461.1 (M+H) |
| 200 | 2,5-difluorophenyl | H | 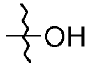 | 3 (b) | 10 | 404.1 (M+H) |
| 201 | 2-fluoro-phenyl | H | 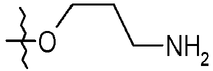 | 3 | 7 | 443.2 (M+H) |
| 202 | 2-fluoro-phenyl | H | 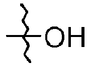 | 3 | 18 | 386.1 (M+H) |
| 203 | 2-chloro-5-methoxyphenyl | H | 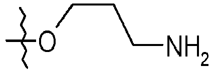 | 3 | 5 | 489.1 (M+H), CP |
| 204 | 2-fluoro-5-methyl-phenyl | H | 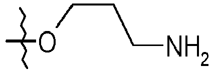 | 3 | 42 | 457.2 (M+H) |
| 205 | 2,5-difluoro-phenyl | H | 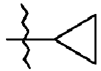 | 6 | 30 | 428.1 (M+H) |
| 206 | 2-fluoro-phenyl | H | 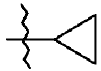 | 6 | 35 | 410.1 (M+H) |
| 207 | 2-fluoro-5-methyl-phenyl | CH₃ | 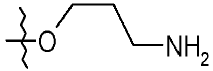 | 3 | 59 | 471.1 (M+H) |
| 208 | 2,5-difluoro-phenyl | CH₃ | 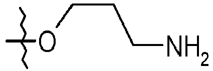 | 3 | 58 | 475.1 (M+H) |
| 209 | 5-chloro-2-fluoro-phenyl | CH₃ | 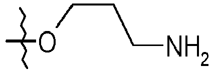 | 3 | 67 | 491.1 (M+H), CP |
| 210 | 2,5-dichloro-phenyl | CH₃ | 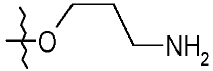 | 3 | 78 | 505.0 (M-H), CP |
| 211 | 2-fluoro-phenyl | CH₃ | 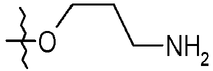 | 3 | 52 | 457.1 (M+H) |
| 212 | 2-chloro-5-methoxyphenyl | CH₃ | 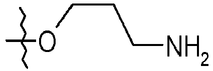 | 3 | 78 | 503.1 (M+H), CP |
| 213 | 2-cyano-5-methyl-phenyl | CH₃ | 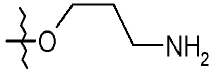 | 3 | 57 | 478.1 (M+H) |
| 214 | 2,5-dichloro-phenyl | CH₃ | 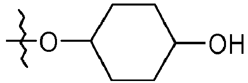 | 3 | 53 | 548.0 (M+H), CP |
| 215 | 5-chloro-2-fluoro-phenyl | CH₃ | 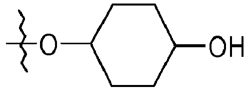 | 3 | 70 | 532.1 (M+H), CP |
| 216 | 2-fluoro-phenyl | CH₃ | 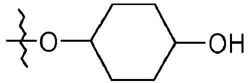 | 3 | 51 | 498.2 (M+H) |
| 217 | 2,5-difluoro-phenyl | CH₃ | 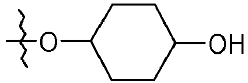 | 3 | 38 | 516.2 (M+H) |
| 218 | 2-fluoro-5-methoxyphenyl | CH₃ | 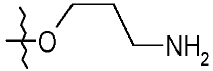 | 3 | 74 | 487.1 (M+H) |
| 219 | 2-fluoro-5-methoxyphenyl | H | 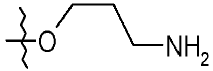 | 3 | 25 | 473.1 (M+H) |
| 220 | 5-chloro-2-cyano-phenyl | CH₃ | 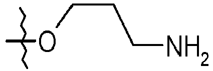 | 3 | 3 | 498.1 (M+H), CP |
| 221 | 2-fluoro-5-methyl-phenyl | H | 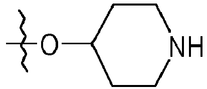 | 3 | 40 | 483.2 (M+H) |
| 222 | 2,5-difluoro-phenyl | H | 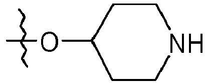 | 3 | 62 | 487.1 (M+H) |
| 223 | 2-chloro-5-methoxyphenyl | H | 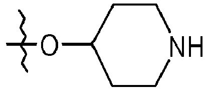 | 3 | 68 | 515.1 (M+H), CP |
| 224 | 5-chloro-2-fluoro-phenyl | H | 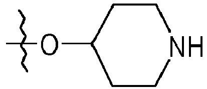 | 3 | 70 | 503.1 (M+H), CP |
| 225 | 2,5-dichloro-phenyl | H | 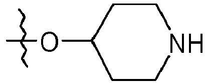 | 3 | 10 | 517.0 (M-H), CP |
| 226 | 2-cyano-5-methyl-phenyl | H | 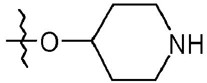 | 3 | 42 | 490.2 (M+H) |
| 227 | 2-fluoro-5-methoxy-.phenyl | H | 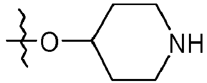 | 3 | 78 | 499.2 (M+H) |
| 228 | 2-fluoro-phenyl | H | 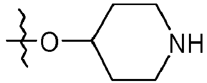 | 3 | 42 | 469.1 (M+H) |
| 229 | 2,5-difluoro-phenyl | H | 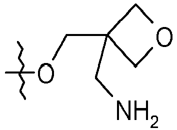 | 3 | 7 | 503.2 (M+H) |
| 230 | 2-fluoro-5-methyl-phenyl | H | 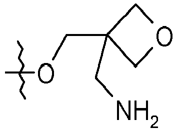 | 3 | 23 | 499.2 (M+H) |
| 231 | 2-fluoro-phenyl | H | 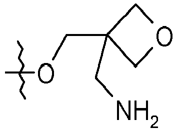 | 3 | 22 | 485.2 (M+H) |
| 232 | 5-chloro-2-fluoro-phenyl | H | 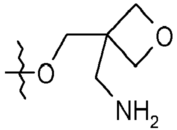 | 3 | 24 | 519.2 (M+H), CP |
| 233 | 2-chloro-5-methoxyphenyl | H | 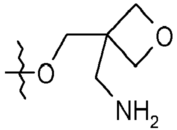 | 3 | 10 | 531.2 (M+H), CP |
| 234 | 2-fluoro-5-methoxyphenyl | H | 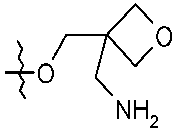 | 3 | 18 | 515.2 (M+H) |
| 235 | 5-chloro-2-cyano-phenyl | H | 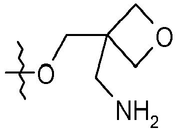 | 3 | 7 | 526.2 (M+H), CP |
| 236 | 2,5-dichloro-phenyl | H | 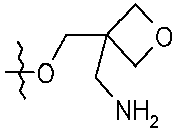 | 3 | 8 | 535.1 (M+H), CP |
| 237 | 2-cyano-5-methyl-phenyl | H | 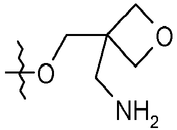 | 3 | 11 | 506.2 (M+H) |
| 238 | 2-fluoro-phenyl | H | 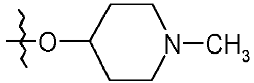 | 3 | 35 | 483.2 (M+H) |
| 239 | 2,5-dichloro-phenyl | H | 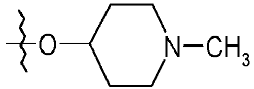 | 3 | 58 | 533.1 (M+H) |
| 240 | 2,5-difluoro-phenyl | H | 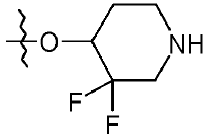 | 3 | 68 | 523.1 (M+H) |
| 241 | 2-fluoro-phenyl | H | 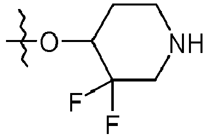 | 3 | 66 | 505.1 (M+H) |
| 242 | 2-fluoro-5-methyl-phenyl | H | 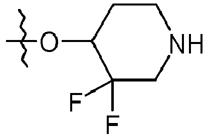 | 3 | 70 | 519.2 (M+H) |
| 243 | 5-chloro-2-fluoro-phenyl | H | 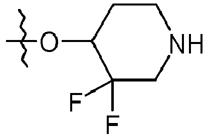 | 3 | 60 | 539.1 (M+H), CP |
| 244 | 2-fluoro-5-methoxyphenyl | H | 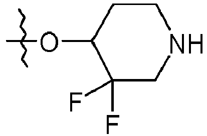 | 3 | 65 | 535.2 (M+H) |
| 245 | 2-chloro-5-methoxyphenyl | H | 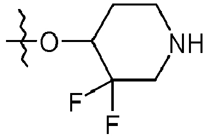 | 3 | 71 | 551.1 (M+H), CP |
| 246 | 5-chloro-2-cyano-phenyl | H | 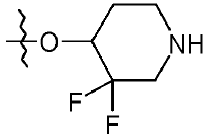 | 3 | 18 | 546.1 (M+H), CP |
| 247 | 2,5-dichloro-phenyl | H | 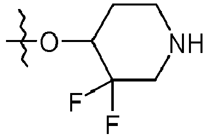 | 3 | 11 | 555.1 (M+H), CP |
| 248 | 2-cyano-5-methyl-phenyl | H | 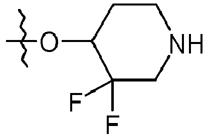 | 3 | 64 | 526.2 (M+H) |
| 249 | 2-fluoro-5-methoxyphenyl | H | 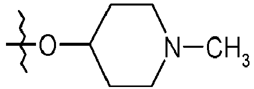 | 3 | 50 | 513.2 (M+H) |
| 250 | 2,5-difluoro-phenyl | H | 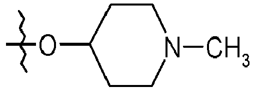 | 3 | 89 | 501.2 (M+H) |
| 251 | 2-fluoro-5-methoxyphenyl | H | 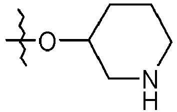 | 3 | 19 | 499.2 (M+H) |
| 252 | 2-fluoro-5-methyl-phenyl | CH₃ | 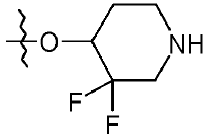 | 3 | 17 | 533.2 (M+H) |
| 253 | 2,5-difluoro-phenyl | CH₃ | 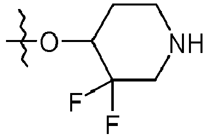 | 3 | 17 | 537.2 (M+H) |
| 254 | 2-fluoro-phenyl | CH₃ | 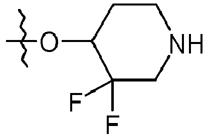 | 3 | 39 | 519.2 (M+H) |
| 255 | 5-chloro-2-fluoro-phenyl | CH₃ | 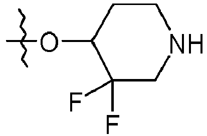 | 3 | 40 | 571.1 (M+H), CP |
| 256 | 2-fluoro-5-methoxyphenyl | CH₃ | 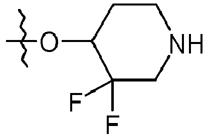 | 3 | 56 | 549.2 (M+H) |
| 257 | 2-chloro-5-methoxyphenyl | CH₃ | 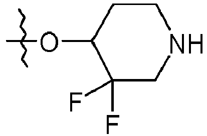 | 3 | 61 | 565.2 (M+H), CP |
| 258 | 5-chloro-2-cyano-phenyl | CH₃ | 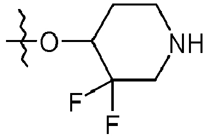 | 3 | 25 | 560.1 (M+H), CP |
| 259 | 2-cyano-5-methyl-phenyl | CH₃ | 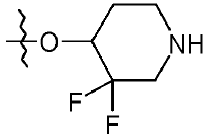 | 3 | 27 | 540.2 (M+H) |
| 260 | 2,5-dichloro-phenyl | CH₃ | 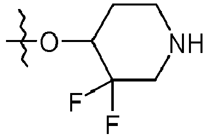 | 3 | 14 | 569.1 (M+H), CP |
| 261 | 5-chloro-2-fluoro-phenyl | CH₃ | 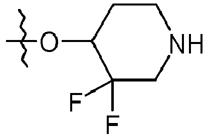 | 3 | 41 | 553.1 (M+H), CP |
| 262 | 2,5-difluoro-phenyl | H | 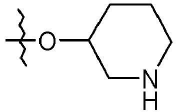 | 3 | 59 | 487.2 (M+H) |
| 263 | 2-fluoro-phenyl | H | 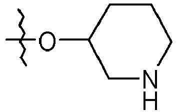 | 3 | 23 | 469.2 (M+H) |
| 264 | 2-fluoro-5-methyl-phenyl | H | 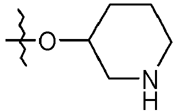 | 3 | 55 | 483.2 (M+H) |
| 265 | 5-chloro-2-fluoro-phenyl | H | 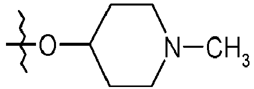 | 3 | 71 | 517.2 (M+H), CP |
| 266 | 2,5-dichloro-phenyl | H | 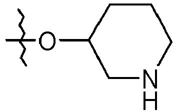 | 3 | 3 | 519.2 (M+H), CP |
| 267 | 2-fluoro-5-methoxyphenyl | CH₃ | 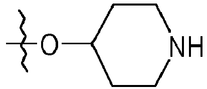 | 3 | 73 | 513.3 (M+H) |
| 268 | 2,5-difluoro-phenyl | CH₃ | 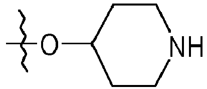 | 3 | 99 | 501.2 (M+H) |
| 269 | 5-chloro-2-fluoro-phenyl | H | 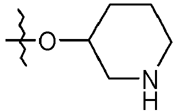 | 3 | 59 | 503.2 (M+H), CP |
| 270 | 2-fluoro-5-methyl-phenyl | CH₃ | 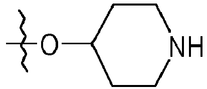 | 3 | 8 | 497.3 (M+H) |
| 271 | 2-fluoro-5-methyl-phenyl | H | 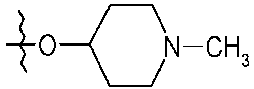 | 3 | 4 | 497.2 (M+H) |
| 272 | 2,5-difluoro-phenyl | CH₃ | 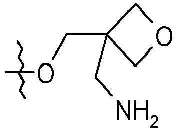 | 3 | 34 | 515.3 (M-H) |
| 273 | 2-cyano-5-methyl-phenyl | CH₃ | 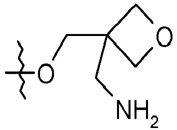 | 3 | 30 | 518.3 (M-H) |
| 274 | 2-fluoro-5-methyl-phenyl | CH₃ | 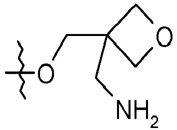 | 3 | 32 | 511.3 (M-H) |
| 275 | 5-chloro-2-fluoro-phenyl | CH₃ | 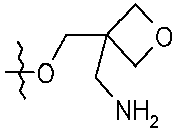 | 3 | 35 | 531.2 (M-H), CP |
| 276 | 2-fluoro-5-methoxyphenyl | CH₃ | 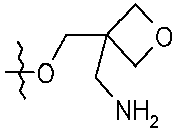 | 3 | 34 | 527.2 (M-H) |
| 277 | 2-chloro-5-methoxyphenyl | CH₃ | 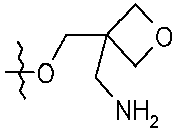 | 3 | 35 | 543.2 (M-H), CP |
| 278 | 2,5-dichloro-phenyl | CH₃ | 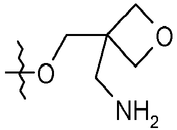 | 3 | 23 | 547.2 (M-H), CP |
| 279 | 5-chloro-2-cyano-phenyl | CH₃ | 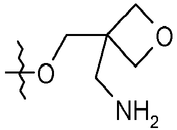 | 3 | 23 | 538.2 (M-H), CP |
| 280 | 2-fluoro-phenyl | CH₃ | 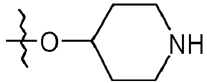 | 3 | 6 | 483.2 (M+H) |
| 281 | 2,5-dichloro-phenyl | CH₃ | 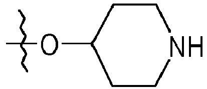 | 3 | 2 | 533.1 (M+H), CP |
| 282 | 5-chloro-2-fluoro-phenyl | CH₃ | 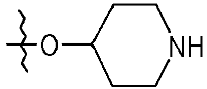 | 3 | 70 | 517.2 (M+H), CP |
| 283 | 2-chloro-5-methoxyphenyl | H | 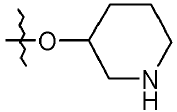 | 3 | 76 | 515.2 (M+H), CP |
| 284 | 2-chloro-5-methoxyphenyl | CH₃ | 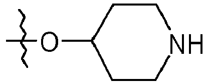 | 3 | 74 | 529.2 (M+H), CP |
| 285 | 5-cyano-2-methyl-phenyl | H | 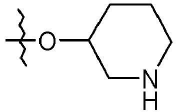 | 3 | 67 | 490.2 (M+H) |
| 286 | 5-cyano-2-methyl-phenyl | CH₃ | 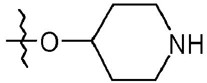 | 3 | 59 | 504.3 (M+H) |
| 287 | 2-chloro-5-methoxyphenyl | H | 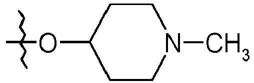 | 3 | 35 | 529.2 (M+H), CP |
| 288 | 2-fluoro-5-methyl-phenyl | CH₃ | 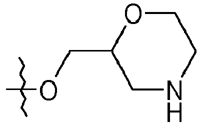 | 3 | 55 | 513.3 (M+H) |
| 289 | 2-fluoro-phenyl | CH₃ | 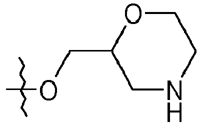 | 3 | 91 | 499.3 (M+H) |
| 290 | 2-chloro-5-methoxyphenyl | CH₃ | 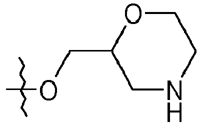 | 3 | 59 | 545.3 (M+H), CP |
| 291 | 5-chloro-2-fluoro-phenyl | CH₃ | 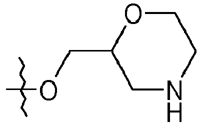 | 3 | 23 | 533.2 (M+H), CP |
| 292 | 2,5-difluoro-phenyl | CH₃ | 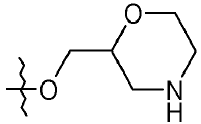 | 3 | 92 | 517.3 (M+H), CP |
| 293 | 2-cyano-5-methyl-phenyl | CH₃ | 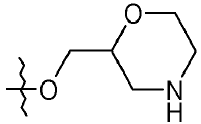 | 3 | 24 | 520.3 (M+H) |
| 294 | 2-fluoro-5-methoxyphenyl | CH₃ | 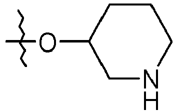 | 3 | 9 | 513.3 (M+H) |
| 295 | 2-fluoro-5-methoxyphenyl | CH₃ | 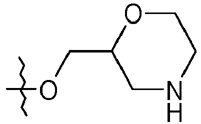 | 3 | 5 | 529.3 (M+H) |
| 296 | 2,5-difluoro-phenyl | CH₃ | 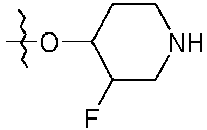 | 3 | 58 | 519.1 (M+H) |
| 297 | 2-fluoro-phenyl | CH₃ | 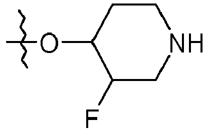 | 3 | 62 | 501.2 (M+H) |
| 298 | 2-fluoro-5-methyl-phenyl | CH₃ | 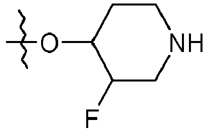 | 3 | 71 | 515.2 (M+H) |
| 299 | 5-chloro-2-cyano-phenyl | CH₃ | 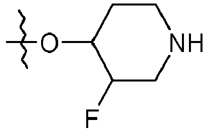 | 3 | 16 | 542.1 (M+H), CP |
| 300 | 2-cyano-5-methyl-phenyl | CH₃ | 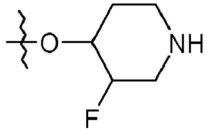 | 3 | 69 | 522.2 (M+H) |
| 301 | 5-chloro-2-fluoro-phenyl | CH₃ | 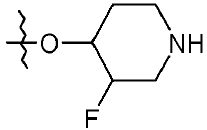 | 3 | 60 | 535.1 (M+H), CP |
| 302 | 2-fluoro-5-methoxyphenyl | CH₃ | 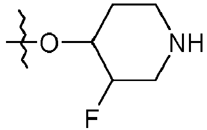 | 3 | 77 | 531.2 (M+H) |
| 303 | 2,5-dichloro-phenyl | CH₃ | 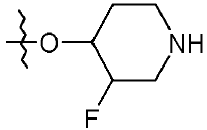 | 3 | 26 | 551.1 (M+H), CP |
| 304 | 2-chloro-5-methoxyphenyl | CH₃ | 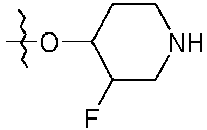 | 3 | 78 | 547.2 (M+H), CP |
| 305 | 5-chloro-2-fluoro-phenyl | H | 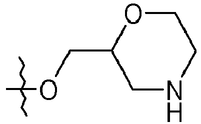 | 3 | | 519.1 (M+H), CP |
| 306 | 2-chloro-5-methoxyphenyl | H | 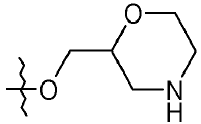 | 3 | 61 | 531.1 (M+H), CP |
| 307 | 2-fluoro-phenyl | H | 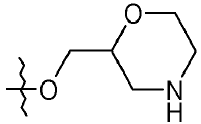 | 3 | 60 | 485.2 (M+H) |
| 308 | 2-fluoro-5-methyl-phenyl | H | 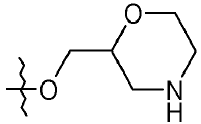 | 3 | 43 | 499.2 (M+H) |
| 309 | 2-fluoro-5-methoxyphenyl | H | 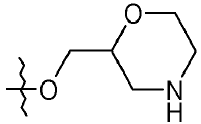 | 3 | 45 | 515.2 (M+H) |
| 310 | 2,5-dichloro-phenyl | NH₂ | 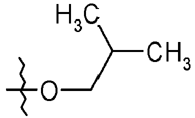 | 7 | 26 | 507.1 (M+H), CP |
| 311 | 2,5-difluoro-phenyl | H | 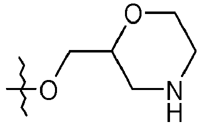 | 3 | 59 | 503.2 (M+H) |
| 312 | 2,5-dichloro-phenyl | H | 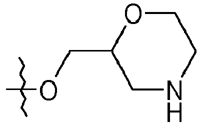 | 3 | 12 | 535.1 (M+H), CP |
| 313 | 2,5-dichloro-phenyl | CH₃ | 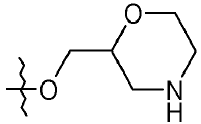 | 3 | 23 | 549.2 (M+H), CP |
| 314 | 2-cyano-5-methyl-phenyl | H | 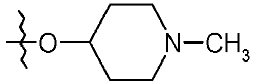 | 3 | 15 | 502.3 (M-H) |
| 315 | 2-cyano-5-methyl-phenyl | H | 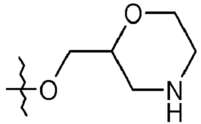 | 3 | 5 | 506.3 (M+H) |
| 316 | 2-chlorophenyl | NH₂ | 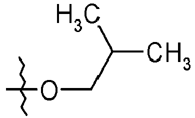 | 7 | 29 | 473.2 (M+H), CP |
| 317 | 2-fluoro-5-methyl-phenyl | NH₂ | 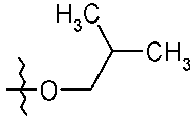 | 7 | 46 | 471.2 (M+H) |
| 318 | 2-chloro-4-fluoro-phenyl | NH₂ | 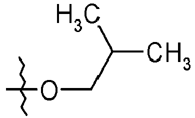 | 7 | 7 | 491.2 (M+H), CP |
| 319 | 2-chloro-5-methoxyphenyl | CH₃ | 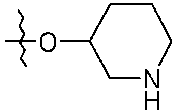 | 3 | 95 | 529.2 (M+H), CP |
| 320 | 2-cyano-5-methyl-phenyl | CH₃ | 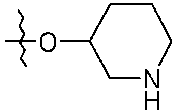 | 3 | 99 | 504.4 (M+H) |
| 321 | 2,5-difluoro-phenyl | CH₃ | 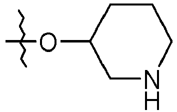 | 3 | 63 | 501.3 (M+H) |
| 322 | 2,5-dichloro-phenyl | CH₃ | 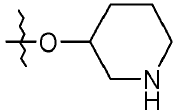 | 3 | 74 | 533.3 (M+H), CP |
| 323 | 2-fluoro-phenyl | CH₃ | 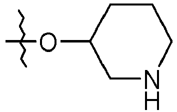 | 3 | 68 | 483.3 (M+H) |
| 324 | 2-chloro-3-fluoro-phenyl | NH₂ | 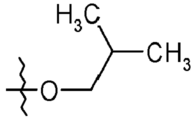 | 7 | 27 | 491.2 (M+H), CP |
| 325 | 2-chloro-4,5-difluoro-phenyl | NH₂ | 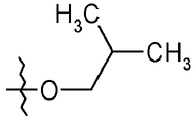 | 7 | 38 | 509.2 (M+H), CP |
| 326 | 2-fluoro-5-methoxyphenyl | NH₂ | 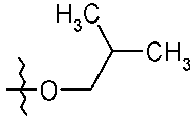 | 7 | 26 | 487.2 (M+H) |
| 327 | 3-chloro-2-fluoro-phenyl | NH₂ | 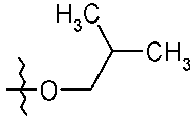 | 7 | 26 | 491.2 (M+H), CP |
| 328 | 2,5-difluoro-phenyl | NH₂ | 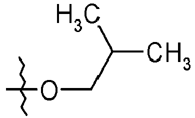 | 7 | 24 | 475.2 (M+H) |
| 329 | 2,4,5-trifluoro-phenyl | NH₂ | 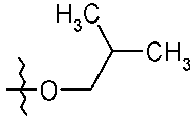 | 7 | 32 | 493.1 (M+H) |
| 330 | 2,5-dichloro-thiophen-3-yl | NH₂ | 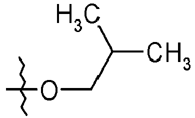 | 7 | 3 | 513.1 (M+H), CP |
| 331 | 2-fluoro-phenyl | NH₂ | 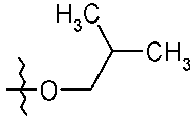 | 7 | 26 | 457.2 (M+H) |
| 332 | 2-chloro-5-methoxxy-phenyl | NH₂ | 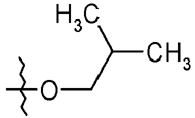 | 7 | 25 | 503.1 (M+H), CP |
| 333 | 2-cyano-5-methyl-phenyl | NH₂ | 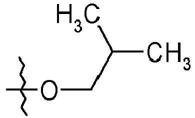 | 7 | 10 | 478.2 (M+H) |
| 334 | 5-chloro-2-fluoro-phenyl | NH₂ | 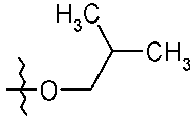 | 7 | 27 | 491.1 (M+H), CP |
| 335 | 2,5-dichloro-phenyl | NH₂ | 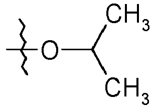 | 7 | 3 | 493.1 (M+H), CP |
| 336 | 2-cyano-5-methoxyphenyl | NH₂ | 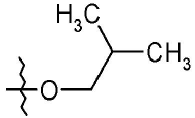 | 7 | 19 | 494.2 (M+H) |
| 337 | 2-chloro-3,5-difluoro-phenyl | NH₂ | 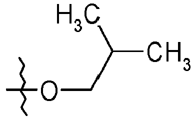 | 7 | 30 | 509.1 (M+H), CP |
| 338 | 2-fluoro-5-methyl-phenyl | CH₃ | 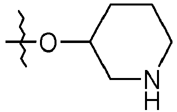 | 3 | 67 | 495.2 (M-H) |
| 339 | 2-fluoro-5-chloro-phenyl | CH₃ | 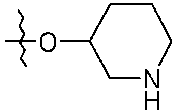 | 3 | 93 | 515.2 (M-H) |
| 340 | 2-fluoro-5-methyl-phenyl | CH₃ | 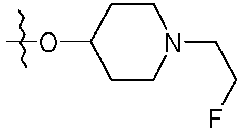 | 3 | 10 | 543.4 (M+H) |
| 341 | 2-chloro-5-methoxyphenyl | CH₃ | 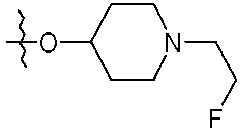 | 3 | 20 | 573.2 (M-H) |
| 342 | 2,5-dichloro-phenyl | CH₃ | 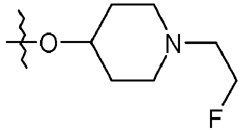 | 3 | 3 | 579.3 (M+H), CP |
| 343 | 2-cyano-5-methyl-phenyl | CH₃ | 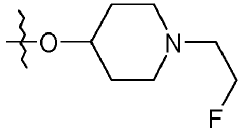 | 3 | 21 | 550.2 (M+H) |
| 344 | 2,5-difluoro-phenyl | CH₃ | 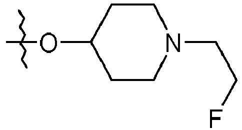 | 3 | 13 | 547.4 (M+H) |
| 345 | 2-chloro-5-methoxyphenyl | CH₃ | 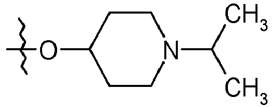 | 3 | 92 | 571.4 (M+H), CP |
| 346 | 5-chloro-2-fluoro-phenyl | CH₃ | 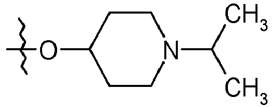 | 3 | 88 | 559.3 (M+H), CP |
| 347 | 2-fluoro-5-methoxyphenyl | CH₃ | 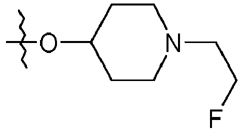 | 3 | 21 | 559.4 (M+H) |
| 348 | 2-fluoro-phenyl | CH₃ | 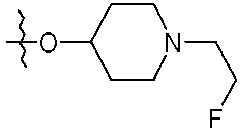 | 3 | 20 | 529.3 (M+H) |
| 349 | 2,5-difluoro-phenyl | CH₃ | 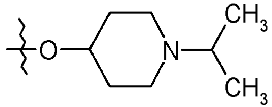 | 3 | 93 | 543.0 (M+H) |
| 350 | 2-cyano-5-methyl-phenyl | CH₃ | 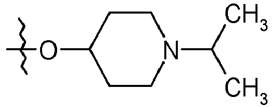 | 3 | 93 | 546.3 (M+H) |
| 351 | 2,5-dichloro-phenyl | CH₃ | 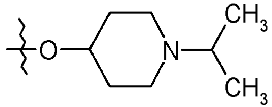 | 3 | 66 | 575.2 (M+H), CP |
| 352 | 2-fluoro-phenyl | CH₃ | 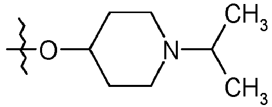 | 3 | 74 | 525.3 (M+H) |
| 353 | 2-fluoro-5-methyl-phenyl | CH₃ | 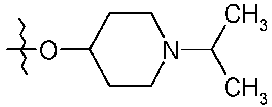 | 3 | 91 | 539.3 (M+H) |
| 354 | 5-chloro-2-cyano-phenyl | NH₂ | 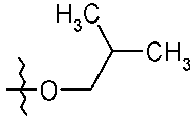 | 7 | 3 | 498.1 (M+H), CP |
| 355 | 2,5-dichloro-phenyl | NH₂ | 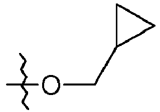 | 7 | 8 | 505.2 (M+H), CP |
| 356 | 5-chloro-2-fluoro-phenyl | CH₃ | 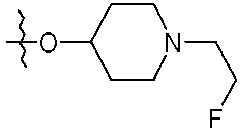 | 3 | 5 | 563.3 (M+H), CP |
| 357 | 2-fluoro-5-methoxyphenyl | CH₃ | 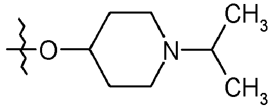 | 3 | 36 | 555.3 (M+H) |
| 358 | 2-chloro-5-methoxyphenyl | CH₃ | 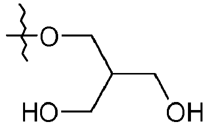 | 3 | 19 | 534.2 (M+H) |
| 359 | 5-chloro-2-fluoro-phenyl | CH₃ | 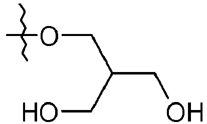 | 3 | 15 | 522.2 (M+H), CP |
| 360 | 2-fluoro-phenyl | CH₃ | 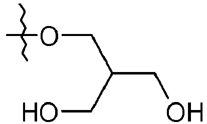 | 3 | 13 | 488.2 (M+H) |
| 361 | 2-fluoro-5-methyl-phenyl | CH₃ | 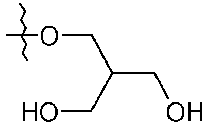 | 3 | 18 | 502.2 (M+H) |
| 362 | 2-chloro-5-methoxyphenyl | CH₃ | 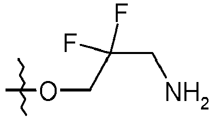 | 3 | 8 | 539.2 (M+H), CP |
| 363 | 2-fluoro-phenyl | CH₃ | 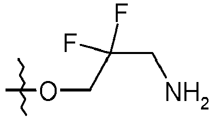 | 3 | 8 | 493.2 (M+H) |
| 364 | 2-fluoro-5-methyl-phenyl | CH₃ | 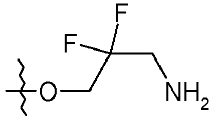 | 3 | 7 | 507.1 (M+H) |
| 365 | 2-fluoro-5-methoxyphenyl | CH₃ | 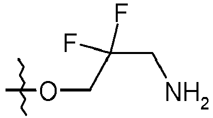 | 3 | 15 | 523.2 (M+H) |
| 366 | 2,5-difluoro-phenyl | CH₃ | 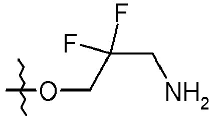 | 3 | 33 | 511.1 (M+H) |
| 367 | 5-chloro-2-fluoro-phenyl | CH₃ | 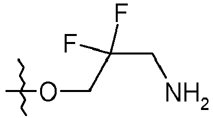 | 3 | 16 | 527.1 (M+H), CP |
| 368 | 2,5-dichloro-phenyl | CH₃ | 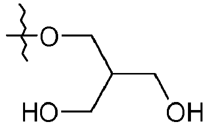 | 3 | 6 | 538.0 (M+H), CP |
| 369 | 2-fluoro-5-methoxyphenyl | CH₃ | 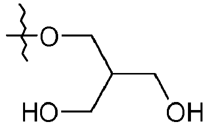 | 3 | 12 | 518.2 (M+H) |
| 370 | 2,5-difluoro-phenyl | CH₃ | 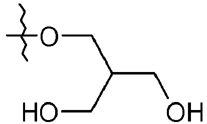 | 3 | 7 | 506.1 (M+H) |
| 371 | 2-cyano-5-methyl-phenyl | CH₃ | 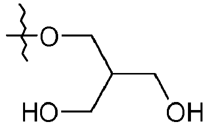 | 3 | 13 | 509.2 (M+H) |
| 372 | 2,5-difluoro-phenyl | CH₃ | 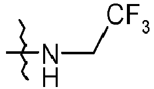 | 3 | 12 | 499.1 (M+H) |
| 373 | 5-chloro-2-fluoro-phenyl | CH₃ | 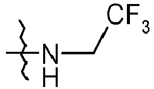 | 3 | 17 | 515.1 (M+H) |
| 374 | 2-fluoro-5-methyl-phenyl | CH₃ | 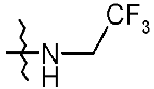 | 3 | 35 | 495.1 (M+H) |
| 375 | 2,5-dichloro-phenyl | CH₃ | 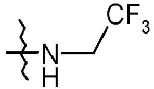 | 3 | 10 | 531.1 (M+H), CP |
| 376 | 2-cyano-5-methyl-phenyl | CH₃ | 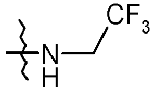 | 3 | 31 | 502.2 (M+H) |
| 377 | 2-fluoro-5-methyl-phenyl | CH₃ | 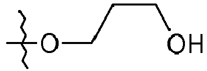 | 3 | 13 | 472.2 (M+H) |
| 378 | 2,5-dichloro-phenyl | CH₃ | 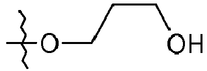 | 3 | 2 | 508.1 (M+H), CP |
| 379 | 2-fluoro-5-methoxyphenyl | CH₃ | 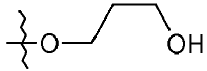 | 3 | 11 | 488.2 (M+H) |
| 380 | 2-cyano-5-methyl-phenyl | CH₃ | 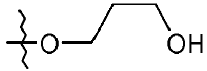 | 3 | 9 | 479.2 (M+H) |
| 381 | 2,5-difluoro-phenyl | CH₃ | 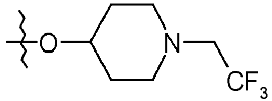 | 3 | 45 | 583.2 (M+H) |
| 382 | 5-chloro-2-fluoro-phenyl | CH₃ | 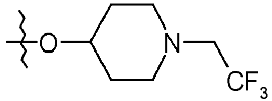 | 3 | 49 | 599.1 (M+H) |
| 383 | 2-chloro-5-methoxyphenyl | CH₃ | 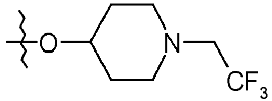 | 3 | 34 | 611.1 (M+H), CP |
| 384 | 2-fluoro-phenyl | CH₃ | 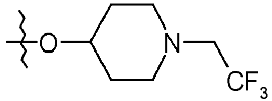 | 3 | 32 | 565.2 (M+H) |
| 385 | 2-fluoro-5-methyl-phenyl | CH₃ | 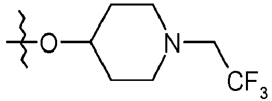 | 3 | 24 | 579.2 (M+H) |
| 386 | 2,5-dichloro-phenyl | CH₃ | 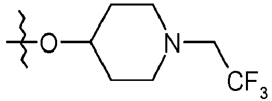 | 3 | 21 | 615.1 (M+H), CP |
| 387 | 2-fluoro-5-methoxyphenyl | CH₃ | 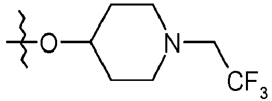 | 3 | 35 | 595.2 (M+H) |
| 388 | 2-cyano-5-methyl-phenyl | CH₃ | 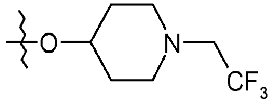 | 3 | 48 | 586.2 (M+H) |
| 389 | 2-fluoro-phenyl | CH₃ | 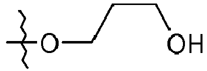 | 3 | 10 | 458.1 (M+H) |
| 390 | 2,5-difluoro-phenyl | CH₃ | 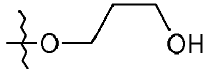 | 3 | 17 | 476.2 (M+H) |
| 391 | 5-chloro-2-fluoro-phenyl | CH₃ | 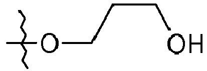 | 3 | 10 | 492.1 (M+H), CP |
| 392 | 2-chloro-5-methoxyphenyl | CH₃ | 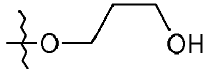 | 3 | 11 | 504.1 (M+H), CP |
| 393 | 2,5-difluoro-phenyl | H | 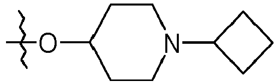 | 3 | 22 | 541.2 (M+H) |
| 394 | 2,5-difluoro-phenyl | H | 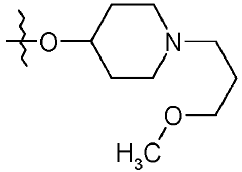 | 3 | 29 | 559.2 (M+H) |
| 395 | 2,5-difluoro-phenyl | H | 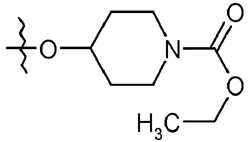 | 3 | 13 | 559.1 (M+H) |
| 396 | 2,5-difluoro-phenyl | H | 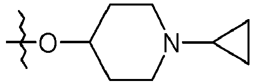 | 3 | 5 | 527.1 (M+H) |
| 397 | 2,5-difluoro-phenyl | CH₃ | 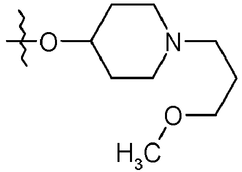 | 3 | 20 | 573.1 (M+H) |
| 398 | 2,5-difluoro-phenyl | CH₃ | 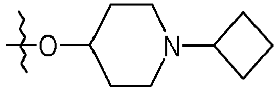 | 3 | 27 | 555.1 (M+H) |
| 399 | 5-chloro-2-fluoro-phenyl | CH₃ | 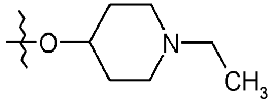 | 3 | 21 | 545.1 (M+H) |
| 400 | 2,5-difluoro-phenyl | CH₃ | 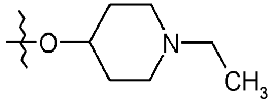 | 3 | 37 | 529.1 (M+H) |
| 401 | 2-chloro-5-methoxyphenyl | CH₃ | 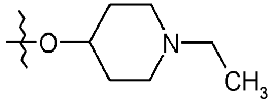 | 3 | 26 | 557.1 (M+H), CP |
| 402 | 2-fluoro-phenyl | CH₃ | 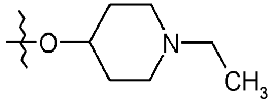 | 3 | 60 | 511.1 (M+H) |
| 403 | 2-fluoro-5-methyl-phenyl | CH₃ | 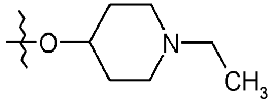 | 3 | 47 | 525.2 (M+H) |
| 404 | 2-fluoro-5-methoxyphenyl | CH₃ | 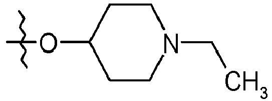 | 3 | 34 | 541.1 (M+H) |
| 405 | 2,5-dichloro-phenyl | CH₃ | 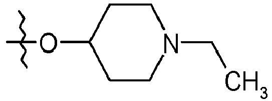 | 3 | 42 | 561.1 (M+H), CP |
| 406 | 5-chloro-2-fluoro-phenyl | CH₃ | 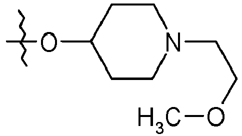 | 3 | 62 | 575.1 (M+H), CP |
| 407 | 2,5-difluoro-phenyl | CH₃ | 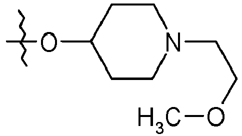 | 3 | 62 | 559.1 (M+H) |
| 408 | 2-fluoro-5-methyl--phenyl | CH₃ | 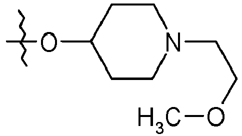 | 3 | 63 | 555.2 (M+H) |
| 409 | 2-fluoro-phenyl | CH₃ | 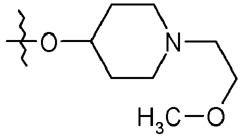 | 3 | 31 | 541.1 (M+H) |
| 410 | 2-fluoro-5-methoxyphenyl | CH₃ | 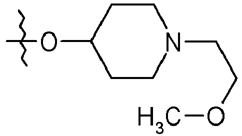 | 3 | 49 | 571.2 (M+H) |
| 411 | 5-chloro-2-cyano-phenyl | CH₃ | 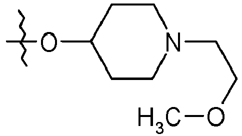 | 3 | 45 | 582.1 (M+H), CP |
| 412 | 2,5-difluoro-phenyl | CH₃ | 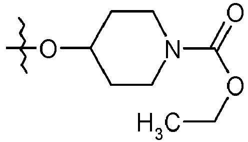 | 3 | 8 | 573.1 (M+H) |
| 413 | 2,5-difluoro-phenyl | NH₂ | 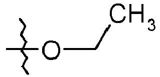 | 7 | 37 | 447.0 (M+H) |
| 414 | 5-chloro-2-fluoro-phenyl | NH₂ | 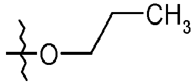 | 7 | 28 | 477.0 (M+H), CP |
| 415 | 2,5-difluoro-phenyl | NH₂ | 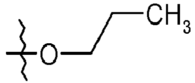 | 7 | 57 | 461.0 (M+H) |
| 416 | 5-chloro-2-fluoro-phenyl | NH₂ | 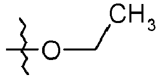 | 7 | 18 | 463.0 (M+H), CP |
| 417 | 2-cyano-5-methyl-phenyl | CH₃ | 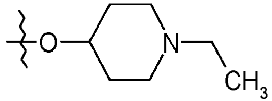 | 3 | 41 | 532.1 (M+H) |
| 418 | 5-chloro-2-fluoro-phenyl | CH₃ | 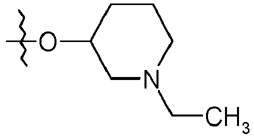 | 3 | 27 | 545.1 (M+H), CP |
| 419 | 2-chloro-5-methoxyphenyl | CH₃ | 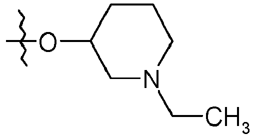 | 3 | 12 | 557.1 (M+H), CP |
| 420 | 2-fluoro-phenyl | CH₃ | 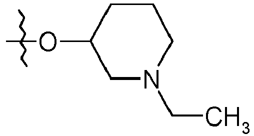 | 3 | 25 | 511.1 (M+H) |
| 421 | 2-fluoro-5-methyl-phenyl | CH₃ | 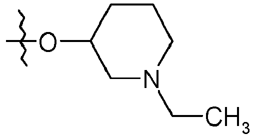 | 3 | 30 | 525.2 (M+H) |
| 422 | 2,5-dichloro-phenyl | CH₃ | 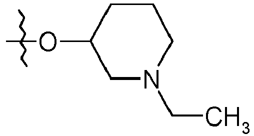 | 3 | 10 | 561.0 (M+H), CP |
| 423 | 2,5-difluoro-phenyl | CH₃ | 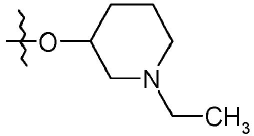 | 3 | 20 | 529.1 (M+H) |
| 424 | 2-fluoro-5-methoxyphenyl | CH₃ | 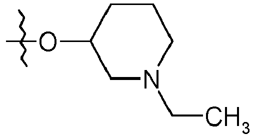 | 3 | 3 | 541.1 (M+H) |
| 425 | 2,5-dichloro-phenyl | NH₂ | 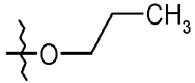 | 7 | 23 | 493.0 (M+H), CP |
| 426 | 2,5-dichloro-phenyl | NH₂ | 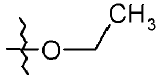 | 7 | 12 | 479.0 (M+H), CP |
| 427 | 2-cyano-5-methyl-phenyl | CH₃ | 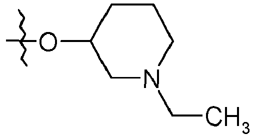 | 3 | 6 | 532.1 (M+H) |
| 428 | 2-fluoro-5-methoxyphenyl | CH₃ | 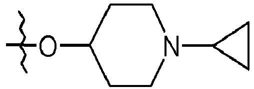 | 3 | 20 | 553.1 (M+H) |
| 429 | 5-chloro-2-fluoro-phenyl | CH₃ | 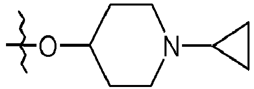 | 3 | 32 | 557.1 (M+H), CP |
| 430 | 2-chloro-5-methoxyphenyl | CH₃ | 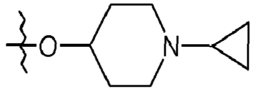 | 3 | 23 | 569.1 (M+H), CP |
| 431 | 2-fluoro-5-methyl-phenyl | CH₃ | 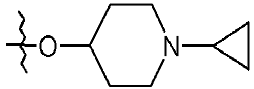 | 3 | 17 | 537.1 (M+H) |
| 432 | 2,5-dichloro-phenyl | CH₃ | 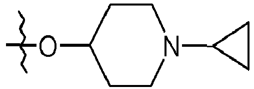 | 3 | 29 | 573.0 (M+H), CP |
| 433 | 2,5-difluoro-phenyl | CH₃ | 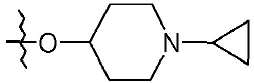 | 3 | 40 | 541.1 (M+H) |

| | | | | | | |
|---|---|---|---|---|---|---|
| (a) Cesium carbonate was employed instead of sodium hydride. (b) Water was employed as starting material. (c) Benzhydrylidene-[6-chloro-1-(tetrahydro-pyran-2-yl)-4-(2,2,2-trifluoro-ethoxy)-1H-pyrazolo[3,4-d]pyrimidin-3-yl]-amine was employed. | | | | | | |

### Exemplary NMR data of example compounds

### Example 346

¹H-NMR (DMSO-d₆): δ (ppm) = 1.31 (d, J = 6.6 Hz, 6H), 2.02-2.14 (m, 1H), 2.24-2.34 (m, 2H), 2.57 (s, 2H), 3.12-3.41 (m, 3H), 3.45-3.57 (m, 3H), 5.56-5.86 (m, 1H), 7.27 (dd, J = 3.8, 8.8 Hz, 2H), 7.52 (t, J = 8.8 Hz, 1H), 7.76-7.82 (m, 1H), 7.84-7.89 (m, 1H), 8.33 (dd, J = 8.7, 11.3 Hz, 2H), 9.73-10.01 (m, 1H), 11.13 (d, J = 5.6 Hz, 1H), 13.48 (br, 1H).

### Example 429

¹H-NMR (DMSO-d₆): δ (ppm) = 0.87 (br, s, 2H), = 0.98 (br, s, 2H), 1.83-1.96 (m, 1H), 2.07-2.18 (m, 1H), 2.29-2.39 (m, 1H), 2.50 (s, 3H), 2.61 (br, 2H), 2.83-3.15 (m, 1H), 3.30-3.42, 3.45-3.59 (m, 1H), 3.61-3.68 (m, 1H), 5.59-5.79 (m, 1H), 7.27 (d, J = 8.5 Hz, 2H), 7.52 (t, J = 9.2 Hz, 1H), 7.77-7.83 (m, 1H), 7.87 (dd, J = 2,7, 6.0 Hz, 1H), 8.30 (d, J = 8.3 Hz, 2H), 9.01 (br, 1H), 11.13 (s, 1H), 13.47 (br, 1H)

### Example 433

¹H-NMR (DMSO-d₆): δ (ppm) = 0.87 (br, s, 2H), 0.97 (br, s, 2H), 1.82-1.96 (m, 1H), 2.05-2.18 (m, 1H), 2.29-2.39 (m, 1H), 2.50 (s, 3H), 2.61 (br, 1H), 2.83-3.15 (m, 1H), 3.45-3.71 (m, 3H), 5.58-5.78 (m, 1H), 7.26 (d, J = 8.4 Hz, 2H), 7.49-7.56 (m, 1H), 7.57-7.64 (m, 1H), 7.69-7.75 (m, 1H), 8.30 (d, J = 8.3 Hz, 2H), 8.92 (br, 1H), 11.12 (s, 1H), 13.46 (s, 1H).

### Analogously to the procedures described in the examples above, the example compounds of the formula Ic

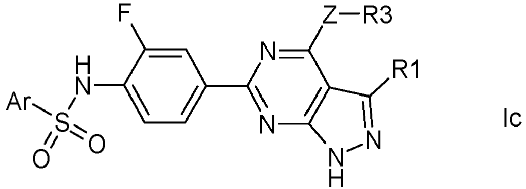

listed in Table 2 were synthesized, employing 2-fluoro-4-(4,4,5,5-tetramethyl-[1,3,2]dioxaborolan-2-yl)-phenylamine instead of 4-(4,4,5,5-tetramethyl-[1,3,2]dioxaborolan-2-yl)-phenylamine as starting material. In the formulae of the groups -Z-R3 in Table 2 the line crossed with the symbol 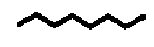 represents the free bond via which the group -Z-R3 is bonded to the carbon atom in the 4-position of the pyrazolo[3,4-d]pyrimidine ring system. I.e., in the formula of the complete molecule the terminal endpoint of the line crossed with the said symbol ends at the carbon atom in the 4-position of the pyrazolo[3,4-d]pyrimidine ring system. In example 442, 3-hydroxy-azetidine-1-carboxylic acid tert-butyl ester was employed as starting material. Deprotections were generally performed using either hydrogen chloride in Diox and/or iPrOH or TFA in DCM, for example a 1:1 mixture of TFA and DCM. In the column "Synthesis" the number of the example is specified in analogy to which the synthesis was performed. The ionization method in the MS characterization was ES+. CP means chloro pattern in the mass spectrum.

**Table 2. Example compounds of the formula Ic**

| Example no. | Ar | R1 | 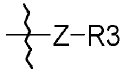 | Synthesis | Yield (%) | MS (m/e) |
|---|---|---|---|---|---|---|
| 434 | 2,5-dichloro-phenyl | CH₃ | 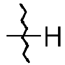 | 1 | 35 | 452.0 (M+H), CP |
| 435 | 5-chloro-2-fluoro-phenyl | H | 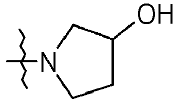 | 2 | 21 | 507.0 (M+H), CP |
| 436 | 5-chloro-2-fluoro-phenyl | H | 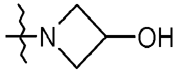 | 2 | 30 | 493.0 (M+H), CP |
| 437 | 2,5-dichloro-phenyl | H | 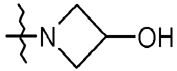 | 2 | 11 | 508.9 (M+H), CP |
| 438 | 2,5-dichloro-phenyl | H | 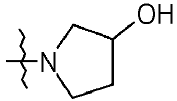 | 2 | 22 | 523.0 (M+H), CP |
| 439 | 2,5-dichloro-phenyl | H | 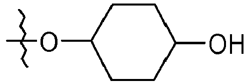 | 3 | 42 | 551.9 (M+H), CP |
| 440 | 5-chloro-2-fluoro-phenyl | H | 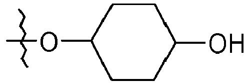 | 3 | 42 | 536.0 (M+H), CP |
| 441 | 5-chloro-2-fluoro-phenyl | H | 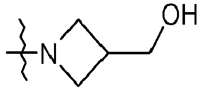 | 2 | 14 | 507.2 (M+H), CP |
| 442 | 5-chloro-2-fluoro-phenyl | H | 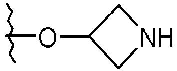 | 3 | 13 | 493.0 (M+H), CP |
| 443 | 5-chloro-2-fluoro-phenyl | H | 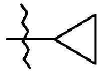 | 6 | 27 | 462.0 (M+H), CP |

### Example 444: N-[4-(3-Amino-4-isopropoxy-1H-pyrazolo[3,4-d]pyrimidin-6-yl)-2-fluoro-phenyl]-5-chloro-2-fluoro-benzenesulfonamide

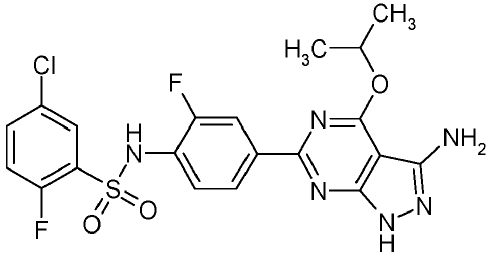

The title compound was prepared in 29% yield analogously to the procedure described in example 7 employing benzhydrylidene-[6-chloro-1-(tetrahydro-pyran-2-yl)-4-(2,2,2-trifluoro-ethoxy)-1H-pyrazolo[3,4-d]pyrimidin-3-yl]-amine and 5-chloro-2-fluoro-N-[2-fluoro-4-(4,4,5,5-tetramethyl-[1,3,2]dioxaborolan-2-yl)-phenyl]-benzenesulfonamide in step (v). Replacement of the 2,2,2-trifluoro-ethoxy group by an isopropoxy group occurred in the course of the treatment with isopropanol in step (vi).

MS (ES+): m/e = 495.09 (M+H), chloro pattern.

### Example 445: N-[4-(3-Amino-1H-pyrazolo[3,4-d]pyrimidin-6-yl)-phenyl]-5-chloro-2,4-difluoro-benzenesulfonamide

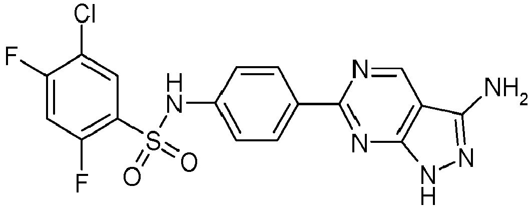

(i) 4-Hydroxy-2-(4-nitrophenyl)pyrimidine-5-carbonitrile
   To a solution of 3.14 g of 2-cyano-3-ethoxy-acrylic acid ethyl ester (US 2824121) and 3 g of 4-nitrobenzimidamide in 50 ml of ethanol, 14 ml of a sodium ethoxide solution (20% in ethanol) were slowly added. The reaction mixture was heated to reflux for 1 h. After cooling to RT and dilution with water, the reaction mixture was acidified with half-concentrated aqueous hydrochloric acid to pH 1. The organic solvents were removed under reduced pressure and the precipitating product was collected by filtration as a brown solid. Yield: 3.0 g.
(ii) 4-Chloro-2-(4-nitrophenyl)pyrimidine-5-carbonitrile
   To a solution of 3.0 g of 4-hydroxy-2-(4-nitrophenyl)pyrimidine-5-carbonitrile in 18 ml of phosphorus oxychloride, 1.6 ml of dimethyl-phenyl-amine were added. The reaction mixture was heated to reflux for 1 h, then cooled to RT and concentrated under reduced pressure. After addition of ice water and dilution with DCM, saturated aqueous sodium hydrogencarbonate solution was added and the mixture was extracted with DCM (3 x 200 ml). The combined organic layers were dried over magnesium sulfate and the solvents were removed under reduced pressure. The crude product was purified by chromatography on silica gel eluting with a gradient of Hep/EtOAc. The fractions containing the product were combined and the solvent evaporated under reduced pressure. Yield: 3.2 g.
(iii) 6-(4-Nitrophenyl)-1H-pyrazolo[3,4-d]pyrimidin-3-amine
   To a solution of 1.5 g of 4-chloro-2-(4-nitrophenyl)pyrimidine-5-carbonitrile in 10 ml of iPrOH, 12.7 ml of a hydrazine solution (35% in iPrOH) were added and the reaction mixture was heated for 25 min to 80°C by using microwave irradiation (Biotage^{®} Initiator apparatus). The reaction mixture was cooled to RT and diluted with acetic acid (20%). The precipitated product was collected by filtration and used in the next reaction step without further purification. Yield: 867 mg.
(iv) tert-Butyl 3-(bis(tert-butoxycarbonyl)amino)-6-(4-nitrophenyl)pyrazolo[3,4-d]pyrimidine-1-carboxylate
   To a suspension of 867 mg of 6-(4-nitrophenyl)-1H-pyrazolo[3,4-d]pyrimidin-3-amine in 10 ml of DCM, 2.3 g of di-tert-butyl dicarbonate, 1.4 ml of triethylamine and 4 mg of dimethyl-pyridin-4-yl-amine were added. The mixture was stirred for 16 h at RT, then quenched by the addition of water and diluted with DCM. After separation of the organic layer, the aqueous layer was extracted with DCM (3 x 200 ml). The combined organic layers were dried over magnesium sulfate and the solvents were removed under reduced pressure. The crude product was purified by chromatography on silica gel eluting with a gradient of Hep/EtOAc. The fractions containing the product were combined and the solvent evaporated under reduced pressure. Yield: 1.4 g.
(v) tert-Butyl 6-(4-aminophenyl)-3-(bis(tert-butoxycarbonyl)amino)pyrazolo[3,4-d]pyrimidine-1-carboxylate
   To a solution of 1.4 g of tert-butyl 3-(bis(tert-butoxycarbonyl)amino)-6-(4-nitrophenyl)pyrazolo[3,4-d]pyrimidine-1-carboxylate obtained in the preceding step in 50 ml of EtOAc, 347 mg of Pd/C (10%) were added under argon and the suspension was stirred under an atmosphere of hydrogen (2 bar) for 16 h. The suspension was filtered over a plug of Celite^{®} and washed with EtOAc. The crude product was obtained after evaporation of the solvent as a brown solid and was dried under reduced pressure. Yield: 3.3 g.
(vi) N-[4-(3-Amino-1H-pyrazolo[3,4-d]pyrimidin-6-yl)-phenyl]-5-chloro-2,4-difluoro-benzenesulfonamide To a solution of 150 mg of tert-butyl 6-(4-aminophenyl)-3-(bis(tert-butoxycarbonyl)amino)pyrazolo[3,4-d]pyrimidine-1-carboxylate in 2.5 ml DCM and 25 µl pyridine, 140 mg of 5-chloro-2,4-difluoro-benzenesulfonyl chloride were added. After stirring the reaction mixture for 16 h at RT, the solvents were removed under reduced pressure. The residue was dissolved in 2 ml DCM and 0.5 ml of TFA and stirred for 6 h at RT. Then toluene was added and the solvents were removed under reduced pressure to yield a brown solid. This crude product was purified by preparative HPLC (C18 reverse phase column, elution with a water/MeCN gradient with 0.1% TFA). The fractions containing the product were lyophilized to yield the pure title compound in the form of its salt with trifluoroacetic acid. This solid was dissolved in 1 ml of a water/MeCN mixture, then 0.5 ml of a 1 M aqueous hydrochloric acid were added and the solution was again lyophilized to yield the title compound in the form of N-[4-(3-amino-1H-pyrazolo[3,4-d]pyrimidin-6-yl)-phenyl]-5-chloro-2,4-difluoro-benzenesulfonamide hydrochloride. Yield: 87 mg. ¹H-NMR (DMSO-d₆): δ (ppm) = 7.28 (d, J = 8.7 Hz, 2H), 7.83 (t, J = 10.3 Hz, 1H), 8.10 (t, J = 8.5 Hz, 1H), 8.27 (d, J = 8.7 Hz, 2H), 9.17 (s, 1H), 11.18 (s, 1H). MS (ES+): m/e = 436.9 (M+H), chloro pattern.

### Example 446: N-[4-(3-Amino-1H-pyrazolo[3,4-d]pyrimidin-6-yl)-phenyl]-5-chloro-2-cyano-benzenesulfonamide

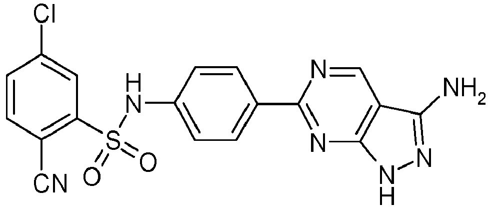

(i) tert-Butyl 3-(bis(tert-butoxycarbonyl)amino)-6-[4-[(5-chloro-2-cyano-phenyl)sulfonylamino]phenyl]pyrazolo[3,4-d]pyrimidine-1-carboxylate
   To a solution of 3 g of tert-butyl 6-(4-aminophenyl)-3-(bis(tert-butoxycarbonyl)amino)pyrazolo[3,4-d]pyrimidine-1-carboxylate (example 445, step (v)) in 50 ml DCM and 1.4 ml pyridine, 1.4 g of 5-chloro-2-cyano-benzenesulfonyl chloride were added. After stirring the reaction mixture for 16 h at RT, the solvents were removed under reduced pressure. The crude product was purified by chromatography on silica gel eluting with a gradient of Hep/EtOAc. The fractions containing the product were combined and the solvent evaporated under reduced pressure. Yield: 2.7 g.
(ii) N-[4-(3-Amino-1H-pyrazolo[3,4-d]pyrimidin-6-yl)-phenyl]-5-chloro-2-cyano-benzenesulfonamide
   1.6 g of tert-butyl 3-(bis(tert-butoxycarbonyl)amino)-6-[4-[(5-chloro-2-cyano-phenyl)sulfonylamino]phenyl]pyrazolo[3,4-d]pyrimidine-1-carboxylate were dissolved in 20 ml DCM and 1.7 ml of TFA. The reaction mixture was stirred for 16 h at RT, then diluted with water and neutralized with a saturated aqueous sodium hydrogencarbonate solution. The precipitated crude product was collected by filtration, washed with EtOAc and recrystallized from ethanol. Yield: 625 mg.

¹H-NMR (DMSO-d₆): δ (ppm) = 7.25 (d, J = 8.8 Hz, 2H), 7.59 (d, J = 8.5 Hz, 1H), 8.07 (s, 1H), 8.12 (d, J = 8.5 Hz, 1H), 8.33 (d, J = 8.8 Hz, 2H), 9.13 (s, 1H), 11.19 (s, 1H).

MS (ES-): m/e = 424.2 (M-H), chloro pattern.

### Example 447: N-[4-(3-Amino-4-methyl-1H-pyrazolo[3,4-d]pyrimidin-6-yl)-phenyl]-5-chloro-2,4-difluoro-benzenesulfonamide

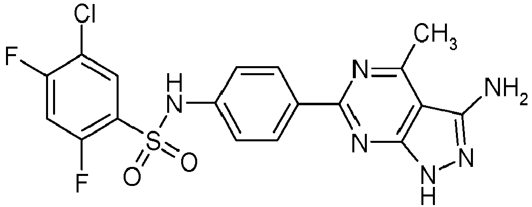

(i) 6-Methyl-2-(4-nitro-phenyl)-4-oxo-4,5-dihydro-pyrimidine-5-carbonitrile
   To a solution of 5.5 g of 2-cyano-3-ethoxy-but-2-enoic acid ethyl ester (US 2824121) and 5 g of 4-nitrobenzimidamide in 200 ml of ethanol, 23.5 ml of a sodium ethoxide solution (20% in ethanol) were slowly added. The reaction mixture was heated to reflux for 1 h. After cooling to RT and dilution with water, the reaction mixture was acidified with half-concentrated aqueous hydrochloric acid to pH 1. The organic solvents were removed under reduced pressure and the precipitating product was collected by filtration as a brown solid. Yield: 7.2 g.
(ii) 4-Chloro-6-methyl-2-(4-nitro-phenyl)-pyrimidine-5-carbonitrile
   To a solution of 5.3 g of 6-methyl-2-(4-nitro-phenyl)-4-oxo-4,5-dihydro-pyrimidine-5-carbonitrile in 71 ml of phosphorus oxychloride, 2.6 ml of dimethyl-phenyl-amine were added. The reaction mixture was heated to reflux for 1 h, then cooled to RT and concentrated under reduced pressure. After addition of ice water and dilution with DCM, saturated aqueous sodium hydrogencarbonate solution was added and the mixture was extracted with DCM (3 x 200 ml). The combined organic layers were dried over magnesium sulfate and the solvents were removed under reduced pressure. The crude product was purified by chromatography on silica gel eluting with a gradient of Hep/EtOAc. The fractions containing the product were combined and the solvent evaporated under reduced pressure. Yield: 3.8 g.
(iii) 4-Methyl-6-(4-nitro-phenyl)-1H-pyrazolo[3,4-d]pyrimidin-3-ylamine
   To a solution of 3.9 g of 4-chloro-6-methyl-2-(4-nitro-phenyl)-pyrimidine-5-carbonitrile in 25 ml of iPrOH, 24.5 ml of a hydrazine solution (35% in iPrOH) were added and the reaction mixture was heated for 10 min to 80°C by using microwave irradiation (Biotage^{®} Initiator apparatus). The reaction mixture was cooled to RT and diluted with acetic acid (20%). The precipitated product was collected by filtration and used in the next reaction step without further purification. Yield: 3.4 g.
(iv) tert-Butyl 3-(bis(tert-butoxycarbonyl)amino)-4-methyl-6-(4-nitrophenyl)pyrazolo[3,4-d]pyrimidine-1-carboxylate
   To a suspension of 3.4 g of 4-methyl-6-(4-nitro-phenyl)-1H-pyrazolo[3,4-d]pyrimidin-3-ylamine in 105 ml of DCM, 8.3 g of di-tert-butyl dicarbonate, 5.3 ml of triethylamine and 16 mg of dimethyl-pyridin-4-yl-amine were added. The mixture was stirred for 16 h at RT, then quenched by the addition of water and diluted with DCM. After separation of the organic layer, the aqueous layer was extracted with DCM (3 x 200 ml). The combined organic layers were dried over magnesium sulfate and the solvents were removed under reduced pressure. The crude product was purified by chromatography on silica gel eluting with a gradient of Hep/EtOAc. The fractions containing the product were combined and the solvent evaporated under reduced pressure. Yield: 6.5 g.
(v) tert-Butyl 6-(4-aminophenyl)-3-(bis(tert-butoxycarbonyl)amino)-4-methyl-pyrazolo[3,4-d]pyrimidine-1-carboxylate
   To a solution of 5.6 g of tert-butyl 3-(bis(tert-butoxycarbonyl)amino)-4-methyl-6-(4-nitrophenyl)pyrazolo[3,4-d]pyrimidine-1-carboxylate obtained in the preceding step in 85 ml of EtOAc, 559 mg of Pd/C (10%) were added under argon and the suspension was stirred under an atmosphere of hydrogen (2 bar) for 16 h. The suspension was filtered over a plug of Celite^{®} and washed with EtOAc. The crude product was obtained after evaporation of the solvent as a brown solid and was dried under reduced pressure. Yield: 5.0 g.
(vi) N-[4-(3-Amino-4-methyl-1H-pyrazolo[3,4-d]pyrimidin-6-yl)-phenyl]-5-chloro-2,4-difluoro-benzenesulfonamide
   To a solution of 250 mg of tert-butyl 6-(4-aminophenyl)-3-(bis(tert-butoxycarbonyl)amino)-4-methyl-pyrazolo[3,4-d]pyrimidine-1-carboxylate in 2.5 ml DCM and 75 µl pyridine, 114 mg of 5-chloro-2,4-difluoro-benzenesulfonyl chloride were added. After stirring the reaction mixture for 16 h at RT, the solvents were removed under reduced pressure. The residue was dissolved in 2 ml DCM and 0.5 ml of TFA and stirred for 6 h at RT. Then toluene was added and the solvents were removed under reduced pressure to yield a brown solid. This crude product was purified by preparative HPLC (C18 reverse phase column, elution with a water/MeCN gradient with 0.1% TFA). The fractions containing the product were lyophilized to yield the pure title compound in the form of its salt with trifluoroacetic acid. Yield: 65 mg.

¹H-NMR (DMSO-d₆): δ (ppm) = 2.77 (s, 3H), 7.27 (d, J = 8.7 Hz, 2H), 7.83 (t, J = 10.3 Hz, 1H), 8.09 (t, J = 8.5 Hz, 1H), 8.26 (d, J = 8.5 Hz, 2H), 11.16 (s, 1H).

MS(ES+): m/e = 451.0 (M+H), chloro pattern.

### Example 448: N-[4-(3-Amino-4-cyclopropyl-1H-pyrazolo[3,4-d]pyrimidin-6-yl)phenyl]-5-chloro-2,4-difluoro-benzenesulfonamide

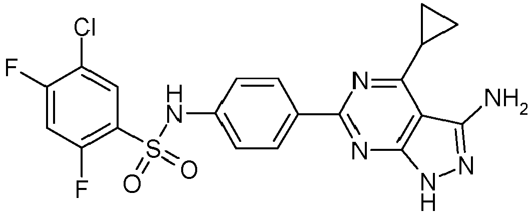

(i) 2-Cyano-3-cyclopropyl-3-hydroxy-acrylic acid ethyl ester
   To a solution of 11.0 g of cyano-acetic acid ethyl ester in 100 ml of MeCN, 9.0 g of anhydrous magnesium chloride were added at 0°C. After 10 min, 26.1 ml of triethylamine and after 1 h a solution of 10.0 g of cyclopropanecarbonyl chloride in 30 ml of DCM were added dropwise to the reaction mixture. After stirring for an additional 1 h, the reaction mixture was acidified with half-concentrated aqueous hydrochloric acid to pH 1, and the mixture was extracted with DCM (3 x 200 ml). The combined organic layers were washed with water, dried over magnesium sulfate and the solvents were removed under reduced pressure. The crude product was purified by crystallization from Hep/EtOAc to yield a crystalline solid. Yield: 10.1 g.
(ii) 2-Cyano-3-cyclopropyl-3-ethoxy-acrylic acid ethyl ester
   To a solution of 10.1 g of 2-cyano-3-cyclopropyl-3-hydroxy-acrylic acid ethyl ester in 200 ml of MeCN, 18.1 g of cesium carbonate were added at 0°C, followed by dropwise addition of 7.26 ml of trifluoromethanesulfonic acid ethyl ester. After 1 h the reaction mixture was allowed to warm to RT and stirred for 16 h. Then the reaction mixture was quenched with a saturated aqueous sodium hydrogencarbonate solution (15 ml) and filtered through a Chem Elut^{®} cartridge by eluting with EtOAc. The filtrate was concentrated under reduced pressure and the obtained crude product was used in the next reaction step. Yield: 12 g.
(iii) 4-Cyclopropyl-6-hydroxy-2-(4-nitrophenyl)pyrimidine-5-carbonitrile
   To a solution of 9.3 g of 2-cyano-3-cyclopropyl-3-ethoxy-acrylic acid ethyl ester and 3.7 g of 4-nitrobenzimidamide in 200 ml of ethanol, 31 ml of a sodium ethoxide solution (20% in ethanol) were slowly added. The reaction mixture was heated to reflux for 1 h. After cooling to RT and dilution with water, the reaction mixture was acidified with half-concentrated aqueous hydrochloric acid to pH 1. The organic solvents were removed under reduced pressure and the precipitating product was collected by filtration as a brown solid. Yield: 4.1 g.
(iv) 4-Chloro-6-cyclopropyl-2-(4-nitrophenyl)pyrimidine-5-carbonitrile
   To a solution of 4.1 g of 4-cyclopropyl-6-hydroxy-2-(4-nitrophenyl)pyrimidine-5-carbonitrile in 24.4 ml of phosphorus oxychloride, 1.9 ml of dimethyl-phenyl-amine were added. The reaction mixture was heated to reflux for 1 h, then cooled to RT and concentrated under reduced pressure. After addition of ice water and dilution with DCM, saturated aqueous sodium hydrogencarbonate solution was added and the mixture was extracted with DCM (3 x 100 ml). The combined organic layers were dried over magnesium sulfate and the solvents were removed under reduced pressure. The crude product was purified by chromatography on silica gel eluting with a gradient of Hep/EtOAc. The fractions containing the product were combined and the solvent evaporated under reduced pressure. Yield: 2.8 g.
(v) 4-Cyclopropyl-6-(4-nitrophenyl)-1 H-pyrazolo[3,4-d]pyrimidin-3-amine
   To a solution of 2.0 g of 4-chloro-6-cyclopropyl-2-(4-nitrophenyl)pyrimidine-5-carbonitrile in 18 ml of iPrOH, 18 ml of a hydrazine solution (35% in iPrOH) were added and the reaction mixture was heated for 15 min to 100°C by using microwave irradiation (Biotage^{®} Initiator apparatus). The reaction mixture was cooled to RT and diluted with acetic acid (20%). The precipitated product was collected by filtration and used in the next reaction step without further purification. Yield: 1.9 g.
(iv) tert-Butyl 3-(bis(tert-butoxycarbonyl)amino)-4-cyclopropyl-6-(4-nitrophenyl)pyrazolo[3,4-d]pyrimidine-1-carboxylate
   To a suspension of 890 mg of 4-cyclopropyl-6-(4-nitrophenyl)-1H-pyrazolo[3,4-d]pyrimidin-3-amine in 20 ml of DCM, 2 g of di-tert-butyl dicarbonate, 1.2 ml of triethylamine and 4 mg of dimethyl-pyridin-4-yl-amine were added. The mixture was stirred for 16 h at RT, then quenched by the addition of water and diluted with DCM. After separation of the organic layer, the aqueous layer was extracted with DCM (3 x 200 ml). The combined organic layers were dried over magnesium sulfate and the solvents were removed under reduced pressure. The crude product was purified by chromatography on silica gel eluting with a gradient of Hep/EtOAc. The fractions containing the product were combined and the solvent evaporated under reduced pressure. Yield: 1.3 g.
(v) tert-Butyl 6-(4-aminophenyl)-3-(bis(tert-butoxycarbonyl)amino)-4-cyclopropyl-pyrazolo[3,4-d]pyrimidine-1-carboxylate
   To a solution of 1.4 g of tert-butyl 3-(bis(tert-butoxycarbonyl)amino)-4-cyclopropyl-6-(4-nitrophenyl)pyrazolo[3,4-d]pyrimidine-1-carboxylate obtained in the preceding step in 8 ml of EtOAc, 139 mg of Pd/C (10%) were added under argon and the suspension was stirred under an atmosphere of hydrogen (2 bar) for 16 h. The suspension was filtered over a plug of Celite^{®} and washed with EtOAc. The crude product was obtained after evaporation of the solvent as a brown solid and was dried under reduced pressure. Yield: 1.3 g.
(vi) N-[4-(3-Amino-4-methyl-1H-pyrazolo[3,4-d]pyrimidin-6-yl)-phenyl]-5-chloro-2,4-difluoro-benzenesulfonamide
   To a solution of 250 mg of tert-butyl 6-(4-aminophenyl)-3-(bis(tert-butoxycarbonyl)amino)-4-cyclopropyl-pyrazolo[3,4-d]pyrimidine-1-carboxylate in 2 ml DCM and 43 µl pyridine, 67 mg of 5-chloro-2,4-difluoro-benzenesulfonyl chloride were added. After stirring the reaction mixture for 16 h at RT, the solvents were removed under reduced pressure. The residue was dissolved in 2 ml DCM and 1 ml of TFA and stirred for 1 h at RT. Then toluene was added and the solvents were removed under reduced pressure to yield a brown solid. This crude product was purified by preparative HPLC (C18 reverse phase column, elution with a water/MeCN gradient with 0.1% TFA). The fractions containing the product were lyophilized to yield the pure title compound in the form of its salt with trifluoroacetic acid. Yield: 40 mg.

¹H-NMR (DMSO-d₆): δ (ppm) = 1.11 (m, 2H), 1.28 (m, 2H), 2.66 (m, 1H), 7.20 (d, J = 8.5 Hz, 2H), 7.79 (t, J = 8.5 Hz, 1H), 8.03 (t, J = 8.5 Hz, 1H), 8.21 (d, J = 8.5 Hz, 2H), 11.02 (s, 1H).

MS (ES+): m/e = 477.2 (M+H), chloro pattern.

### Example 449: 5-Chloro-2,4-difluoro-N-[4-(3-methyl-1H-pyrazolo[3,4-d]pyrimidin-6-yl)-phenyl]-benzenesulfonamide

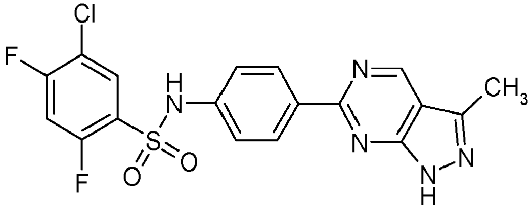

(i) 5-Chloro-2,4-difluoro-N-[4-(4,4,5,5-tetramethyl-[1,3,2]dioxaborolan-2-yl)phenyl]benzenesulfonamide To a solution of 500 mg of 4-(4,4,5,5-tetramethyl-[1,3,2]dioxaborolan-2-yl)-phenylamine in 5 ml of DCM and 0.2 ml of pyridine, 564 mg of 5-chloro-2,4-difluoro-benzenesulfonyl chloride were added, and the reaction mixture was stirred for 16 h at RT. Then the solvents were removed under reduced pressure and the crude product was purified by chromatography on silica gel eluting with a gradient of Hep/EtOAc. The fractions containing the product were combined and the solvent evaporated under reduced pressure. Yield: 1.0 g.
(ii) 5-Chloro-2,4-difluoro-N-[4-(3-methyl-1H-pyrazolo[3,4-d]pyrimidin-6-yl)-phenyl]-benzenesulfonamide
   A solution of 59 mg of 6-chloro-3-methyl-1-tetrahydropyran-2-yl-pyrazolo[3,4-d]pyrimidine, 102 mg 5-chloro-2,4-difluoro-N-[4-(4,4,5,5-tetramethyl-[1,3,2]dioxaborolan-2-yl)phenyl]benzenesulfonamide and 232 mg of cesium carbonate in 1.5 ml of Diox and 0.2 ml of water was purged with argon. Then 13 mg of BDFP were added and the reaction mixture was heated to 100°C. After 2 h, the reaction mixture was cooled to RT and diluted with water. After filtration through a Chem Elut^{®} cartridge by eluting with EtOAc, the solvents were removed under reduced pressure. The residue was dissolved in 2 ml of iPrOH and 2 ml of HCI in Diox (4M) at RT. After 15 min the reaction mixture was diluted with 20 ml of toluene and the solvents were removed under reduced pressure. The residue was purified by preparative HPLC (C18 reverse phase column, elution with a water/MeCN gradient with 0.1% TFA). The fractions containing the product were lyophilized to yield the pure title compound in the form of its salt with trifluoroacetic acid. Yield: 31 mg ¹H-NMR (DMSO-d₆): δ (ppm) = 2.56 (s, 3H), 7.28 (d, J = 8.7 Hz, 2H), 7.85 (t, J = 10.3 Hz, 1H), 8.09 (t, J = 8.5 Hz, 1H), 8.36 (d, J = 8.5 Hz, 2H), 9.34 (s, 1H), 11.16 (s, 1H). MS (ES+): m/e = 436.0 (M+H), chloro pattern.

### Example 450: 2,5-Dichloro-N-{4-[3-methyl-4-(2-oxa-6-aza-spiro[3.4]oct-6-yl)-1H-pyrazolo[3,4-d]pyrimidin-6-yl]-phenyl}-benzenesulfonamide

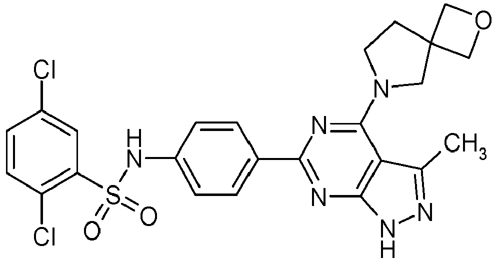

(i) 2,5-Dichloro-N-[4-(4,4,5,5-tetramethyl-[1,3,2]dioxaborolan-2-yl)-phenyl]benzenesulfonamide
   To a solution of 10 g of 4-(4,4,5,5-tetramethyl-[1,3,2]dioxaborolan-2-yl)-phenylamine in 100 ml DCM and 4 ml pyridine, 11.7 g of 2,5-dichloro-benzenesulfonyl chloride were added, and the reaction mixture was stirred for 16 h at RT. Then the solvents were removed under reduced pressure and the crude product was purified by chromatography on silica gel eluting with a gradient of Hep/EtOAc. The fractions containing the product were combined and the solvent evaporated under reduced pressure. Yield: 17.9 g.
(ii) 7-(6-Chloro-3-methyl-1-tetrahydropyran-2-yl-pyrazolo[3,4-d]pyrimidin-4-yl)-2-oxa-6-azaspiro[3.4]octane
   To a solution of 300 mg of 4,6-dichloro-3-methyl-1-tetrahydropyran-2-yl-pyrazolo[3,4-d]pyrimidine (WO 2011/140338) and 0.4 ml triethylamine in 5 ml MeCN, 118 mg of 2-oxa-6-aza-spiro[3.4]octane were added. The reaction mixture was stirred for 5 h at RT, quenched with a saturated aqueous sodium hydrogencarbonate solution (3 ml) and filtered through a Chem Elut^{®} cartridge by eluting with EtOAc. The filtrate was concentrated under reduced pressure and the obtained crude product was used in the next reaction step. Yield: 309 mg.
(iii) 2,5-Dichloro-N-{4-[3-methyl-4-(2-oxa-6-aza-spiro[3.4]oct-6-yl)-1H-pyrazolo[3,4-d]pyrimidin-6-yl]-phenyl}-benzenesulfonamide
   A solution of 155 mg of 7-(6-chloro-3-methyl-1-tetrahydropyran-2-yl-pyrazolo[3,4-d]pyrimidin-4-yl)-2-oxa-7-azaspiro[3.4]octane, 182 mg 2,5-dichloro-N-[4-(4,4,5,5-tetramethyl-[1,3,2]dioxaborolan-2-yl)-phenyl]-benzenesulfonamide and 415 mg of cesium carbonate in 5 ml of Diox and 1.6 ml of water was purged with argon. Then 28 mg of BDFP were added and the reaction mixture was heated to 100°C. After 3 h, the reaction mixture was cooled to RT and diluted with water. After filtration through a Chem Elut^{®} cartridge by eluting with EtOAc, the solvents were removed under reduced pressure. The residue was dissolved in 3 ml of DCM and 0.5 ml of TFA at RT. After 2 h the reaction mixture was diluted with 20 ml of toluene and the solvents were removed under reduced pressure. The residue was purified by preparative HPLC (C18 reverse phase column, elution with a water/MeCN gradient with 0.1% TFA). The fractions containing the product were lyophilized to yield the pure title compound in the form of its salt with trifluoroacetic acid. Yield: 39 mg.

¹H-NMR (DMSO-d₆): δ (ppm) = 2.33 (t, J = 6.6 Hz, 2H), 2.65 (s, 3H), 3.85 (s, 2H), 4.06 (s, 2H), 4.55 (d, J = 5.9 Hz, 2H), 4.67 (d, J = 5.9 Hz, 2H), 7.21 (d, J = 8.5 Hz, 2H), 7.75 - 7.67 (m, 2H), 8.05 (s, 1H), 8.27 (d, J = 8.5 Hz, 2H), 11.16 (s, 1H).

MS (ES+): m/e = 545.2 (M+H), chloro pattern.

### Analogously to the procedures described in the examples above, the example compounds of the formula Ib

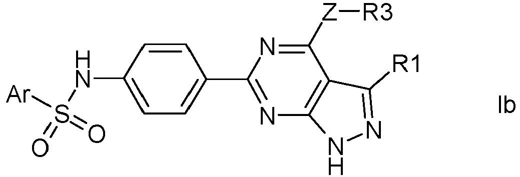

listed in Table 3 were synthesized. In the formulae of the groups -Z-R3 in Table 3 the line crossed with the symbol 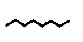 represents the free bond via which the group -Z-R3 is bonded to the carbon atom in the 4-position of the pyrazolo[3,4-d]pyrimidine ring system. I.e., in the formula of the complete molecule the terminal endpoint of the line crossed with the said symbol ends at the carbon atom in the 4-position of the pyrazolo[3,4-d]pyrimidine ring system. In the column "Synthesis" the number of the example is specified in analogy to which the synthesis was performed. The ionization method in the MS characterization was ES+ if the specified ion is M+H, and ES- if the specified ion is M-H. CP means chloro pattern, BP means bromo pattern in the mass spectrum.

**Table 3. Example compounds of the formula Ib**

| Example no. | Ar | R1 | 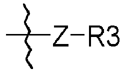 | Synthesis | MS (m/e) |
|---|---|---|---|---|---|
| 451 | 5-chloro-2,4- difl uoro-phenyl | NH₂ | 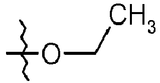 | 7 | 481.0 (M+H), CP |
| 452 | 2,5-dichloro-phenyl | NH₂ | 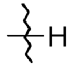 | 445 | 435.1 (M+H), CP |
| 453 | 2-cyano-5-methyl-phenyl | NH₂ | 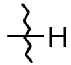 | 445 | 406.1 (M+H) |
| 454 | 3-cyano-4-fluoro-phenyl | NH₂ | 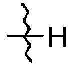 | 445 | 410.1 (M+H) |
| 455 | 2-fluoro-5-methyl-phenyl | NH₂ | 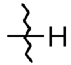 | 445 | 399.1 (M+H) |
| 456 | 3-chloro-2-cyano-phenyl | NH₂ | 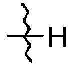 | 445 | 426.1 (M+H), CP |
| 457 | 3-cyano-phenyl | NH₂ | 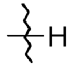 | 445 | 392.1 (M+H) |
| 458 | 8-chloro-3,4-dihydro-2H-benzo[b][1,4]dioxepin-7-yl | NH₂ | 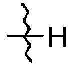 | 445 | 473.2 (M+H), CP |
| 459 | 8-bromo-3,4-dihydro-2H-benzo[b][1,4]dioxepin-7-yl | NH₂ | 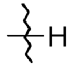 | 445 | 517.2 (M+H), BP |
| 460 | 5-chloro-1,3-dimethyl-1H-pyrazol-4-yl | NH₂ | 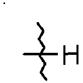 | 445 | 419.1 (M+H), CP |
| 461 | 2-chloro-5-methoxyphenyl | NH₂ | 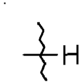 | 445 | 431.0 (M+H), CP |
| 462 | 2-chloro-3,5-difluoro-phenyl | NH₂ | 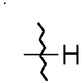 | 445 | 437.1 (M+H), CP |
| 463 | 2,5-dichloro-thiophen-3-yl | NH₂ | 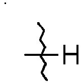 | 445 | 441.1 (M+H), CP |
| 464 | 2,5-dichloro-phenyl | NH₂ | 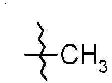 | 447 | 449.1 (M+H), CP |
| 465 | 5-chloro-2-cyano-phenyl | NH₂ | 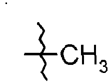 | 447 | 440.2 (M+H), CP |
| 466 | 5-chloro-2-fluoro-phenyl | NH₂ | 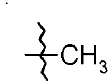 | 447 | 433.1 (M+H), CP |
| 467 | 2-chloro-5-methoxyphenyl | NH₂ | 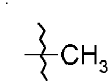 | 447 | 445.0 (M+H), CP |
| 468 | 2-cyano-5-methyl-phenyl | NH₂ | 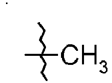 | 447 | 420.2 (M+H) |
| 469 | 3-cyano-4-fluoro-phenyl | NH₂ | 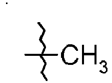 | 447 | 424.2 (M+H) |
| 470 | 3-chloro-2-cyano-phenyl | NH₂ | 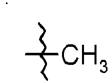 | 447 | 440.2 (M+H), CP |
| 471 | 3-cyano-phenyl | NH₂ | 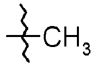 | 447 | 406.1 (M+H) |
| 472 | 2-fluoro-5-methyl-phenyl | NH₂ | 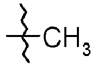 | 447 | 413.2 (M+H) |
| 473 | 5-cyano-2-methyl-phenyl | NH₂ | 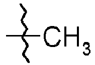 | 447 | 420.2 (M+H) |
| 474 | 2-cyano-3-fluoro-phenyl | NH₂ | 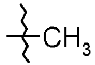 | 447 | 424.2 (M+H) |
| 475 | 5-chloro-1,3-dimethyl-1 H-pyrazol-4-yl | NH₂ | 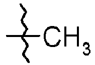 | 447 | 433.1 (M+H), CP |
| 476 | 8-chloro-3,4-dihydro-2H-benzo[b][1,4]dioxepin-7-yl | NH₂ | 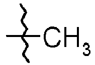 | 447 | 485.2 (M-H), CP |
| 477 | 8-bromo-3,4-dihydro-2H-benzo[b][1,4]dioxepin-7-yl | NH₂ | 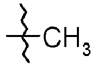 | 447 | 531.1 (M+H), BP |
| 478 | 7-chloro-2,3-dihydro-benzo[1,4]dioxin-6-yl | NH₂ | 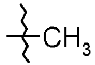 | 447 | 473.2 (M+H), CP |
| 479 | 2-chloro-3,5-difluoro-phenyl | NH₂ | 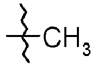 | 447 | 451.1 (M+H), CP |
| 480 | 2-chloro-3,5-difluoro-phenyl | NH₂ | 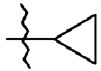 | 448 | 477.1 (M+H), CP |
| 481 | 2-chloro-4,5-difluoro-phenyl | NH₂ | 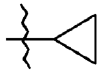 | 448 | 477.1 (M+H), CP |
| 482 | 3-chloro-2-fluoro-phenyl | NH₂ | 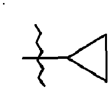 | 448 | 459.1 (M+H), CP |
| 483 | 2-chloro-4-fluoro-phenyl | NH₂ | 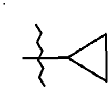 | 448 | 459.1 (M+H), CP |
| 484 | 2,4,5-trifluoro-phenyl | NH₂ | 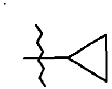 | 448 | 461.2 (M+H) |
| 485 | 2-fluoro-phenyl | NH₂ | 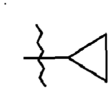 | 448 | 425.2 (M+H) |
| 486 | 2,5-difluoro-phenyl | NH₂ | 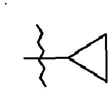 | 448 | 443.2 (M+H) |
| 487 | 5-chloro-2-fluoro-phenyl | NH₂ | 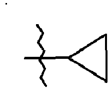 | 448 | 459.1 (M+H), CP |
| 488 | 2-fluoro-5-methyl-phenyl | NH₂ | 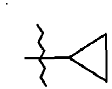 | 448 | 439.2 (M+H) |
| 489 | 2,5-dichloro-phenyl | NH₂ | 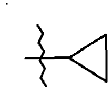 | 448 | 475.1 (M+H), CP |
| 490 | 2-cyano-phenyl | CH₃ | 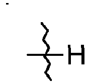 | 449 | 391.0 (M+H) |
| 491 | 2-chloro-5-methoxyphenyl | CH₃ | 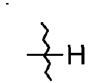 | 449 | 430.0 (M+H), CP |
| 492 | 5-chloro-2-cyano-phenyl | CH₃ | 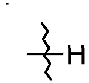 | 449 | 425.0 (M+H), CP |
| 493 | 3-cyano-4-fluoro-phenyl | CH₃ | 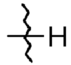 | 449 | 409.0 (M+H) |
| 494 | 3-cyano-phenyl | CH₃ | 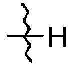 | 449 | 391.3 (M+H) |
| 495 | 5-cyano-2-methyl-phenyl | CH₃ | 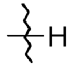 | 449 | 405.2 (M+H) |
| 496 | 2,5-dichloro-phenyl | CH₃ | 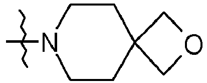 | 450 | 559.2 (M+H), CP |
| 497 | 2,5-dichloro-phenyl | CH₃ | 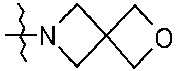 | 450 | 531.2 (M+H), CP |
| 498 | 2,5-dichloro-phenyl | CH₃ | 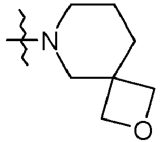 | 450 | 559.2 (M+H), CP |
| 499 | 5-chloro-2-cyano-phenyl | CH₃ | 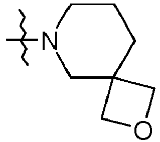 | 450 | 550.4 (M+H), CP |
| 500 | 2,5-dichloro-thiophen-3-yl | NH₂ | 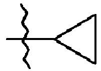 | 448 | 480.9 (M+H), CP |
| 501 | 5-chloro-2,4-difluoro-phenyl | NH₂ | 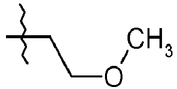 | 448 | 495.0 (M+H), CP |
| 502 | 2-chloro-5-methoxyphenyl | NH₂ | 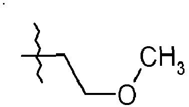 | 448 | 489.1 (M+H), CP |
| 503 | 2-fluoro-5-methyl-phenyl | NH₂ | 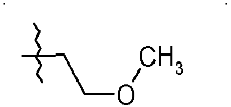 | 448 | 457.1 (M+H) |
| 504 | 2-fluoro-5-methoxyphenyl | NH₂ | 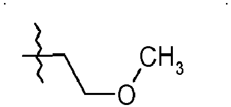 | 448 | 473.1 (M+H) |
| 505 | 2,5-dichloro-phenyl | NH₂ | 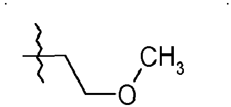 | 448 | 493.0 (M+H), CP |
| 506 | 5-chloro-2-fluoro-phenyl | NH₂ | 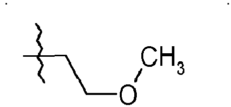 | 448 | 477.0 (M+H), CP |
| 507 | 2-chloro-4-fluoro-phenyl | NH₂ | 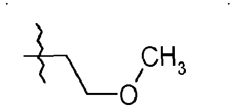 | 448 | 477.0 (M+H), CP |
| 508 | 2,5-difluoro-phenyl | NH₂ | 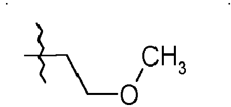 | 448 | 461.1 (M+H) |
| 509 | 2,5-dichloro-thiophen-3-yl | NH₂ | 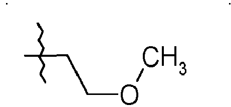 | 448 | 497.1 (M-H), CP |
| 510 | 2-chloro-4,5-difluoro-phenyl | NH₂ | 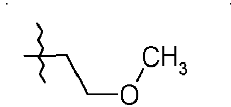 | 448 | 495.0 (M+H), CP |
| 511 | 3-chloro-2-fluoro-phenyl | NH₂ | 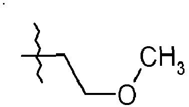 | 448 | 477.0 (M+H), CP |
| 512 | 2-fluoro-phenyl | NH₂ | 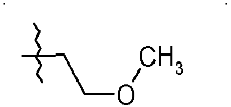 | 448 | 443.1 (M+H) |
| 513 | 2-cyano-5-methyl-phenyl | NH₂ | 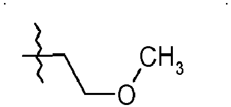 | 448 | 464.1 (M+H) |
| 514 | 2-chloro-4,5-difluoro-phenyl | NH₂ | 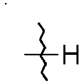 | 445 | 437.0 (M+H), CP |
| 515 | 3-chloro-2-fluoro-phenyl | NH₂ | 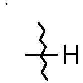 | 445 | 419.0 (M+H), CP |
| 516 | 2,4,5-trifluoro-phenyl | NH₂ | 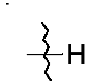 | 445 | 421.1 (M+H) |
| 517 | 2-fluoro-phenyl | NH₂ | 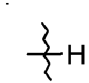 | 445 | 385.1 (M+H) |
| 518 | 2,4,5-trifluoro-phenyl | NH₂ | 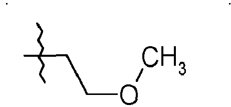 | 448 | 479.1 (M+H) |
| 519 | 2,5-difluoro-phenyl | NH₂ | 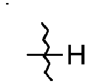 | 445 | 403.1 (M+H) |
| 520 | 2-cyano-5-methoxyphenyl | NH₂ | 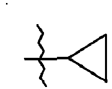 | 448 | 462.1 (M+H) |
| 521 | 2-cyano-5-methoxyphenyl | NH₂ | 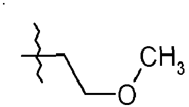 | 448 | 480.1 (M+H) |
| 522 | 2-cyano-5-methoxyphenyl | NH₂ | 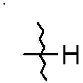 | 445 | 422.1 (M+H) |
| 523 | 2-chloro-3-fluoro-phenyl | NH₂ | 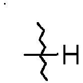 | 445 | 419.1 (M+H), CP |
| 524 | 2-chloro-phenyl | NH₂ | 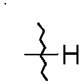 | 445 | 401.1 (M+H), CP |
| 525 | 5-chloro-2-fluoro-phenyl | NH₂ | 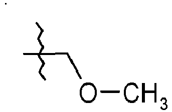 | 448 | 463.1 (M+H), CP |
| 526 | 2-chloro-4-fluoro-phenyl | NH₂ | 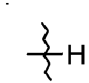 | 445 | 419.0 (M+H), CP |
| 527 | 2-fluoro-5-methoxyphenyl | NH₂ | 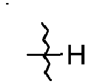 | 445 | 415.1 (M+H) |
| 528 | 2-fluoro-5-methyl-phenyl | NH₂ | 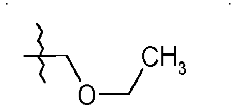 | 448 | 457.1 (M+H) |
| 529 | 2,5-dichloro-phenyl | NH₂ | 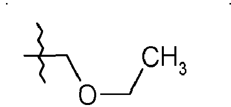 | 448 | 493.1 (M+H), CP |
| 530 | 3-chloro-2,6-difluoro-phenyl | NH₂ | 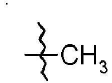 | 447 | 451.0 (M+H), CP |
| 531 | 3-chloro-2,6-difluoro-phenyl | NH₂ | 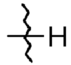 | 445 | 437.0 (M+H), CP |

### Exemplary NMR data of example compounds

### Example 519

¹H-NMR (DMSO-d₆): δ (ppm) = 7.27 (d, J = 8.8 Hz, 2H), 7.52 (m, 1H), 7.51 (m, 1H), 7.70 (m, 1H), 8.27 (d, J = 8.8 Hz, 2H), 9.14 (s, 1H), 11.13 (s, 1H).

### Example 520

¹H-NMR (DMSO-d₆): δ (ppm) = 1.14 (m, 2H), 1.31 (m, 2H), 2.71 (m, 1H), 3.88 (s, 3H), 7.23 (d, J = 8.8 Hz, 2H), 7.33 (m, 1H), 7.52 (d, J = 2.5 Hz, 1H), 8.00 (d, J = 8.8 Hz), 8.23 (d, J = 8.8 Hz, 2H), 11.02 (s, 1H).

### Example 527

¹H-NMR (DMSO-d₆): δ (ppm) = 3.88 (s, 3H), 7.21 (m, 1H), 7.26 (d, J = 8.8 Hz, 2H), 7.33 (m, 1H), 8.27 (d, J = 8.8 Hz, 2H), 9.13 (s, 1H), 11.10 (s, 1H).

### Example 531

¹H-NMR (DMSO-d₆): δ (ppm) = 7.30 (d, J = 8.8 Hz, 2H), 7.38 (t, J = 8.8 Hz, 1H), 7.94 (m, 1H), 8.31 (d, J = 8.8 Hz, 2H), 9.14 (s, 1H), 11.24 (s, 1H).

### Example 532: 2-[4-(3-Methyl-1H-pyrazolo[3,4-d]pyrimidin-6-yl)-phenylsulfamoyl]-benzamide

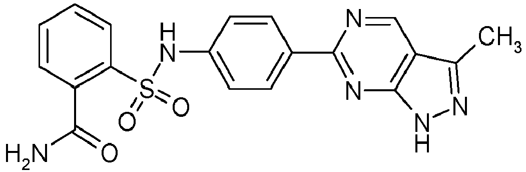

The title compound was isolated as a further product in example 490.

MS (ES+): m/e = 409.0 (M+H).

### Example 533: 2-Chloro-N-[2-fluoro-4-(3-methyl-1H-pyrazolo[3,4-d]pyrimidin-6-yl)-phenyl]-5-methoxy-benzenesulfonamide

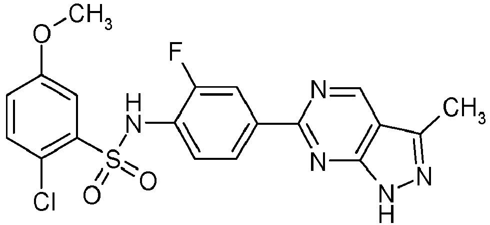

The title compound was prepared analogously to the procedure described in example 449, employing 2-chloro-N-[2-fluoro-4-(4,4,5,5-tetramethyl-[1,3,2]dioxaborolan-2-yl)-phenyl]-5-methoxy-benzenesulfonamide instead of 5-chloro-2,4-difluoro-N-[4-(4,4,5,5-tetramethyl-[1,3,2]dioxaborolan-2-yl)phenyl]benzenesulfonamide.

MS (ES+): m/e = 448.0 (M+H), chloro pattern.

### Example 534: 2,5-Dichloro-N-[2-methoxy-4-(3-methyl-1H-pyrazolo[3,4-d]pyrimidin-6-yl)-phenyl]-benzenesulfonamide

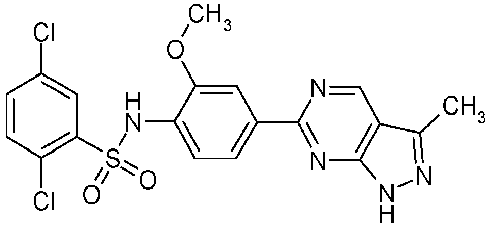

The title compound was prepared analogously to the procedure described in example 449, employing 2,5-dichloro-N-[2-methoxy-4-(4,4,5,5-tetramethyl-[1,3,2]dioxaborolan-2-yl)-phenyl]-benzenesulfonamide instead of 5-chloro-2,4-difluoro-N-[4-(4,4,5,5-tetramethyl-[1,3,2]dioxaborolan-2-yl)phenyl]benzenesulfonamide.

MS (ES+): m/e = 464.2 (M+H), chloro pattern.

### Example 535: 2,5-Dichloro-N-[4-(3-methyl-1H-pyrazolo[3,4-d]pyrimidin-6-yl)-2-trifluoromethoxyphenyl]-benzenesulfonamide

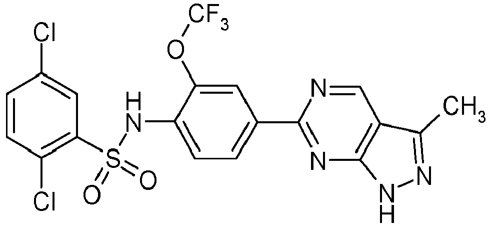

The title compound was prepared analogously to the procedure described in example 449, employing 2,5-dichloro-N-[4-(4,4,5,5-tetramethyl-[1,3,2]dioxaborolan-2-yl)-2-trifluoromethoxyphenyl]-benzenesulfonamide instead of 5-chloro-2,4-difluoro-N-[4-(4,4,5,5-tetramethyl-[1,3,2]dioxaborolan-2-yl)phenyl]benzenesulfonamide.

MS (ES+): m/e = 518.0 (M+H), chloro pattern.

### Example 536: 3-Hydroxy-cyclobutanecarboxylic acid {6-[4-(2,5-dichloro-benzenesulfonylamino)-phenyl]-4-methyl-1H-pyrazolo[3,4-d]pyrimidin-3-yl}-amide

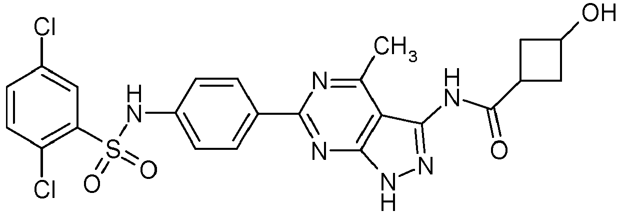

To a solution of 100 mg of N-[4-(3-amino-4-methyl-1H-pyrazolo[3,4-d]pyrimidin-6-yl)-phenyl]-2,5-dichloro-benzenesulfonamide and 23 mg of 3-hydroxycyclobutanecarboxylic acid in 1.5 ml of DMF, 0.1 ml of triethylamine and 45 mg of bis(2-oxo-3-oxazolidinyl)phosphonic chloride (BOP-Cl) were added at RT. After stirring for 16 h the solvents were removed and the residue was purified by preparative HPLC (C18 reverse phase column, elution with a water/MeCN gradient with 0.1% TFA). The fractions containing the product were lyophilized to yield the pure title compound in the form of its salt with trifluoroacetic acid. Yield: 3 mg.

MS (ES+): m/e = 547.2 (M+H), chloro pattern.

Analogously to the procedure described in the example 536, the example compounds of the formula Id 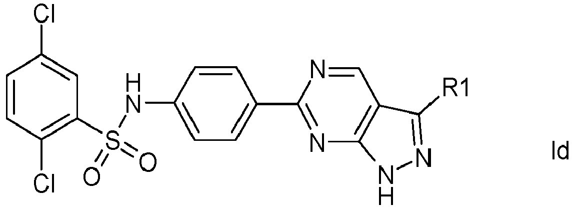 listed in Table 4 were synthesized. In the formulae of the groups -R1 in Table 4 the line crossed with the symbol 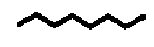 represents the free bond via which the group -R1 is bonded to the carbon atom in the 3-position of the pyrazolo[3,4-d]pyrimidine ring system. I.e., in the formula of the complete molecule the terminal endpoint of the line crossed with the said symbol ends at the carbon atom in the 3-position of the pyrazolo[3,4-d]pyrimidine ring system. The ionization method in the MS characterization was ES+. CP means chloro pattern in the mass spectrum.

**Table 4. Example compounds of the formula Id**

| Example no. | 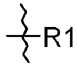 | MS (m/e) |
|---|---|---|
| 537 | 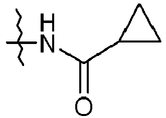 | 503.1 (M+H), CP |
| 538 | 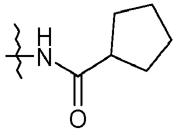 | 531.1 (M+H), CP |
| 539 | 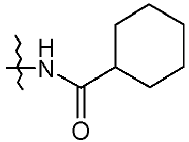 | 545.1 (M+H), CP |
| 540 | 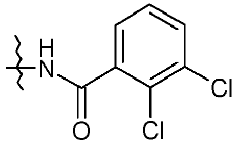 | 607.0 (M+H), CP |
| 541 | 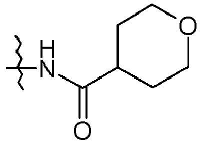 | 547.1 (M+H), CP |
| 542 | 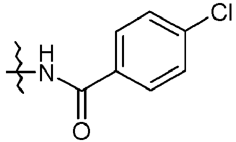 | 573.1 (M+H), CP |
| 543 | 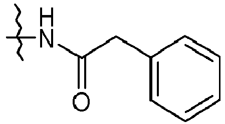 | 553.2 (M+H), CP |
| 544 | 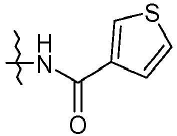 | 545.1 (M+H), CP |

### Example 545: N-[6-[4-[(2,5-Dichlorophenyl)sulfonylamino]phenyl]-4-methyl-1H-pyrazolo[3,4-d]pyrimidin-3-yl]tetrahydropyran-4-carboxamide

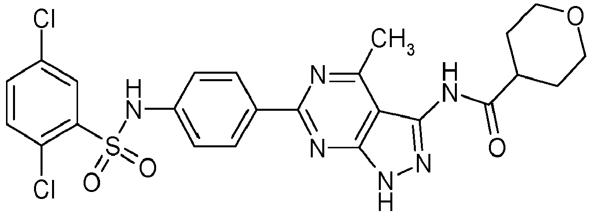

To a solution of 100 mg of N-[4-(3-amino-4-methyl-1H-pyrazolo[3,4-d]pyrimidin-6-yl)-phenyl]-2,5-dichloro-benzenesulfonamide in 2 ml pyridine, 26 mg of tetrahydropyran-4-carbonyl chloride were added and the reaction mixture was stirred for 16 h at RT. Then the solvents under reduced pressure and the crude product was purified by preparative HPLC (C18 reversed phase column, elution with a water/MeCN gradient with 0.1% TFA). The fractions containing the product were lyophilized to yield the pure title compound in the form of its salt with trifluoroacetic acid. Yield: 12 mg.

MS (ES+): m/e = 561.2 (M+H), chloro pattern.

### Example 546: N-[6-[4-[(2,5-Dichlorophenyl)sulfonylamino]phenyl]-1H-pyrazolo[3,4-d]pyrimidin-3-yl]piperidine-4-carboxamide

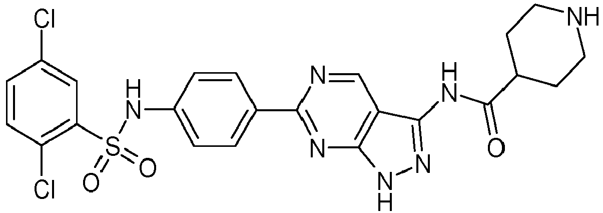

The title compound was prepared analogously to the procedure described in example 545, employing 1-tert-butoxycarbonyl-piperidine-4-carboxylic acid in the presence of bis(2-oxo-3-oxazolidinyl)phosphonic chloride (BOP-Cl) as coupling agent instead of tetrahydropyran-4-carbonyl chloride and deprotecting with TFA.

MS (ES+): m/e = 546.3 (M+H), chloro pattern.

### Example 547: 2,5-Dichloro-N-(4-{4-methyl-3-[(tetrahydro-pyran-4-ylmethyl)-amino]-1H-pyrazolo[3,4-d]pyrimidin-6-yl}-phenyl)-benzenesulfonamide

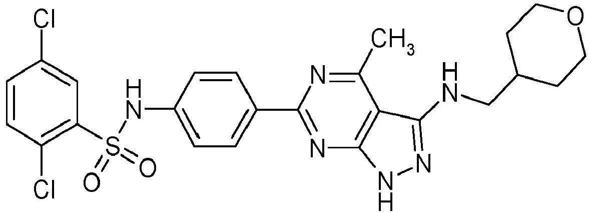

To a solution of 100 mg of N-[4-(3-amino-4-methyl-1H-pyrazolo[3,4-d]pyrimidin-6-yl)-phenyl]-2,5-dichloro-benzenesulfonamide and 20 mg of tetrahydropyran-4-carbaldehyde in 1 ml of methanol, 22 mg of sodium borohydride and 2 µl of acetic acid were added at RT. After stirring for 16 h, water was added and the reaction mixture was filtered through a Chem Elut^{®} cartridge by eluting with EtOAc. After removal of the solvents under reduced pressure, the residue was purified by preparative HPLC (C18 reversed phase column, elution with a water/MeCN gradient with 0.1% TFA). The fractions containing the product were lyophilized to yield the pure title compound in the form of its salt with trifluoroacetic acid. Yield: 9 mg.

MS (ES+): m/e = 547.2 (M+H), chloro pattern.

### Example 548: N-{4-[3-(Benzyl-amino]-1H-pyrazolo[3,4-d]pyrimidin-6-yl]-phenyl}-2,5-dichloro-benzenesulfonamide

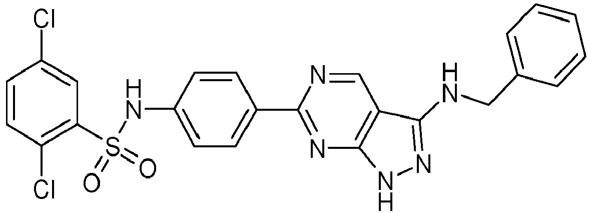

The title compound was prepared analogously to the procedure described in example 547, employing benzaldehyde instead of tetrahydropyran-4-carbaldehyde.

MS (ES+): m/e = 525.2 (M+H), chloro pattern.

### Example 549: N-[4-(3-Amino-4-hydroxy-1H-pyrazolo[4,3-c]pyridin-6-yl)phenyl]-5-chloro-2,4-difluoro-benzenesulfonamide

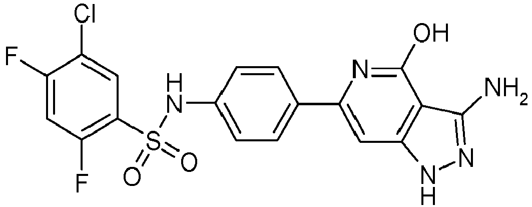

(i) tert-Butyl N-[4-[3,3-bis(methylsulfanyl)prop-2-enoyl]phenyl]carbamate
   To a suspension of 2.45 g of sodium tert-butylate in 40 ml of toluene, 3 g of tert-butyl N-(4-acetylphenyl)carbamate and 0.77 ml of carbon disulfide were added at 0°C. After 4 h at 0°C, the mixture was stirred for 16 h at RT. Then the solvents were removed under reduced pressure and the residue dissolved in 40 ml of dry methanol. After addition of 1.6 ml of methyl iodide, the reaction mixture was heated under reflux for 30 min, then cooled to RT and quenched by the addition of 50 ml of water. The precipitated product was collected by filtration, dried under reduced pressure and purified by chromatography on silica gel eluting with a gradient of Hep/EtOAc. The fractions containing the product were combined and the solvent evaporated under reduced pressure. Yield: 431 mg.
(ii) tert-Butyl N-[4-(3-cyano-4-methylsulfanyl-2-oxo-3H-pyridin-6-yl)phenyl]carbamate
   To 3.5 ml of iPrOH, 56 mg of sodium hydride (60% in mineral oil) were added. After 10 min at RT, 431 mg of tert-butyl N-[4-[3,3-bis(methylsulfanyl)prop-2-enoyl]phenyl]carbamate and 107 mg of 2-cyanoacetamide were added and the mixture was heated under reflux for 4 h. Then, after cooling to RT, water was added and the reaction mixture was neutralized by addition of diluted hydrochloric acid. The precipitated product was collected by filtration and dried under reduced pressure. Yield: 386 mg.
(iii) 3-Amino-6-(4-aminophenyl)-1H-pyrazolo[4,3-c]pyridin-4-ol hydrochloride
   To a solution of 386 mg of tert-butyl N-[4-(3-cyano-4-methylsulfanyl-2-oxo-3H-pyridin-6-yl)phenyl]carbamate in 2 ml of iPrOH, 2 ml of a hydrazine solution (35% in iPrOH) were added and the reaction mixture was heated for 40 min to 110°C by using microwave irradiation (Biotage^{®} Initiator apparatus). The reaction mixture was cooled to RT and diluted with acetic acid (20%). The precipitated intermediate product tert-butyl N-[4-(3-amino-4-hydroxy-1H-pyrazolo[4,3-c]pyridin-6-yl)phenyl]carbamate was collected by filtration, dried under reduced pressure and dissolved in 10 ml of a ethanolic solution of hydrochloric acid (8M). After stirring for 30 min at RT the reaction mixture was diluted with toluene (100 ml) and the solvents were removed under reduced pressure. The residue was co-distilled additional two times with toluene. After drying under reduced pressure the product was pure enough for the next reaction step. Yield: 319 mg.
(iv) N-[4-(3-Amino-4-hydroxy-1H-pyrazolo[4,3-c]pyridin-6-yl)phenyl]-5-chloro-2,4-difluoro-benzenesulfonamide
   To a solution of 166 mg of 3-amino-6-(4-aminophenyl)-1H-pyrazolo[4,3-c]pyridin-4-ol hydrochloride in 5 ml of DCM and 145 µl of pyridine, 148 mg of 5-chloro-2,4-difluoro-benzenesulfonyl chloride were added. After stirring the reaction mixture for 16 h at RT, the solvents were removed under reduced pressure. This crude product was purified by preparative HPLC (C18 reverse phase column, elution with a water/MeCN gradient with 0.1% TFA). The fractions containing the product were lyophilized to yield the pure title compound in the form of its salt with trifluoroacetic acid. Yield: 17 mg.

¹H-NMR (DMSO-d₆): δ (ppm) = 6.35 (s, 1H), 7.17 (d, J = 8.7 Hz, 2H), 7.62 (d, J = 8.7 Hz, 2H), 7.85 (t, J = 7.2, 1H), 8.09 (t, J = 7.2 Hz, 1H), 11.16 (s, 1H).

MS (ES-): m/e = 450.1 (M-H), chloro pattern.

### Example 550: N-[4-(3-Amino-4-hydroxy-1H-pyrazolo[4,3-c]pyridin-6-yl)phenyl]-2,5-dichloro-benzenesulfonamide

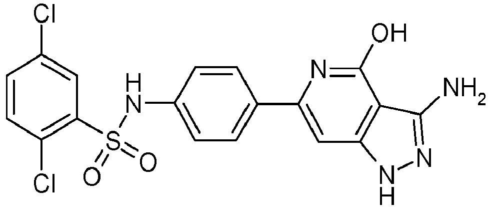

The title compound was prepared analogously to the procedure described in example 549, employing 2,5-dichloro-benzenesulfonyl chloride instead of 5-chloro-2,4-difluoro-benzenesulfonyl chloride.

MS (ES+): m/e = 450.1 (M+H), chloro pattern.

### Example 551: 2,5-Dichloro-N-[6-[4-[(2,5-dichlorophenyl)sulfonylamino]phenyl]-4-hydroxy-1H-pyrazolo[4,3-c]pyridin-3-yl]benzenesulfonamide

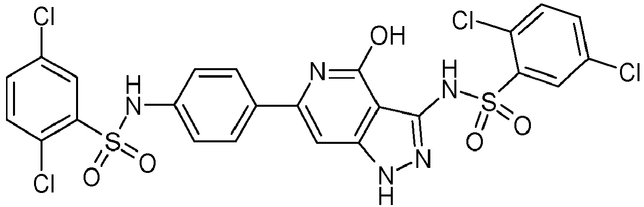

The title compound was isolated as a further product in example 550.

MS (ES-): m/e = 656.2, (M-H), chloro pattern.

### Example 552: N-[4-(3-Amino-4-hydroxy-1H-pyrazolo[4,3-c]pyridin-6-yl)phenyl]-5-chloro-2-cyano-benzenesulfonamide

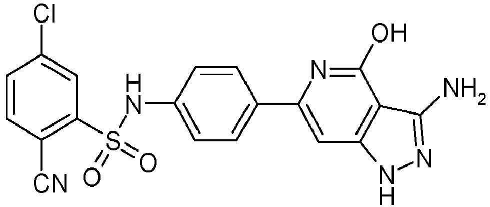

The title compound was prepared analogously to the procedure described in example 549, employing 5-chloro-2-cyano-benzenesulfonyl chloride instead of 5-chloro-2,4-difluoro-benzenesulfonyl chloride.

MS (ES+): m/e = 441.1 (M+H), chloro pattern.

### Example 553: N-[4-(3-Amino-1H-pyrazolo[4,3-c]pyridin-6-yl)phenyl]-2-cyano-5-methyl-benzenesulfonamide

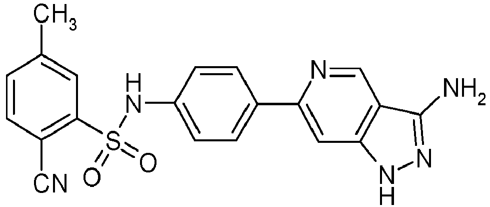

(i) tert-Butyl N-[4-(4-chloro-5-cyano-2-pyridyl)phenyl]carbamate
   A solution of 1.0 g of 4,6-dichloropyridine-3-carbonitrile, 1.8 g of tert-butyl N-[4-(4,4,5,5-tetramethyl-[1,3,2]dioxaborolan-2-yl)phenyl]carbamate and 5.6 g of cesium carbonate in 35 ml of Diox and 6 ml of water was purged with argon. Then 338 mg of BDFP were added and the reaction mixture was heated to 100°C. After 5 h, the reaction mixture was cooled to RT and diluted with water. After filtration through a Chem Elut^{®} cartridge by eluting with EtOAc, the solvents were removed under reduced pressure. The crude product was purified by chromatography on silica gel eluting with a gradient of Hep/EtOAc. The fractions containing the product were combined and the solvent evaporated under reduced pressure. Yield: 2.1 g.
(ii) 6-(4-Aminophenyl)-1H-pyrazolo[4,3-c]pyridin-3-amine hydrochloride
   To a solution of 2.1 g of tert-butyl N-[4-(4-chloro-5-cyano-2-pyridyl)phenyl]carbamate in 20 ml of iPrOH, 19.4 ml of a hydrazine solution (35% in iPrOH) were added and the reaction mixture was heated for 25 min to 80°C by using microwave irradiation (Biotage^{®} Initiator apparatus). The reaction mixture was cooled to RT and diluted with acetic acid (20%). The precipitated intermediate product tert-butyl N-[4-(3-amino-1H-pyrazolo[4,3-c]pyridin-6-yl)phenyl]carbamate was collected by filtration, dried under reduced pressure and dissolved in 10 ml of a ethanolic solution of hydrochloric acid (8M). After stirring for 30 min at RT the reaction mixture was diluted with toluene (100 ml) and the solvents were removed under reduced pressure. The residue was co-distilled additional two times with toluene. After drying under reduced pressure the product was pure enough for the next reaction step. Yield: 1.7 g.
(iii) N-[4-(3-amino-1H-pyrazolo[4,3-c]pyridin-6-yl)phenyl]-2-cyano-5-methyl-benzenesulfonamide To a solution of 150 mg of 6-(4-aminophenyl)-1H-pyrazolo[4,3-c]pyridin-3-amine hydrochloride in 3 ml of DCM and 138 µl of pyridine, 123 mg of 2-cyano-5-methyl-benzenesulfonyl chloride were added. After stirring the reaction mixture for 16 h at RT, the solvents were removed under reduced pressure. This crude product was purified by preparative HPLC (C18 reverse phase column, elution with a water/MeCN gradient). The fractions containing the product were lyophilized to yield the pure title compound. Yield: 22 mg.

MS (ES+): m/e = 405.2 (M+H).

### Analogously to the procedure described in the example 553, the example compounds of the formula le

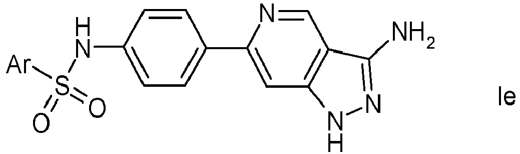

listed in Table 5 were synthesized, employing the respective sulfonyl chloride instead of 2-cyano-5-methyl-benzenesulfonyl chloride. The ionization method in the MS characterization was ES+. CP means chloro pattern in the mass spectrum.

**Table 5. Example compounds of the formula le**

| Example no. | Ar | MS (m/e) |
|---|---|---|
| 554 | 5-chloro-2-cyano-phenyl | 425.3 (M+H), CP |
| 555 | 5-chloro-2,4-difluoro-phenyl | 436.1 (M+H), CP |
| 556 | 2,5-dichloro-phenyl | 434.1 (M+H), CP |
| 557 | 2-chloro-3,5-difluoro-phenyl | 436.1 (M+H), CP |
| 558 | 2,5-dichloro-thiophen-3-yl | 440.1 (M+H), CP |
| 559 | 5-chloro-2-fluoro-phenyl | 418.1 (M+H), CP |

### Example 560: 2,5-Dichloro-N-[6-[4-[(2,5-dichlorophenyl)sulfonylamino]phenyl]-1H-pyrazolo[4,3-c]pyridin-3-yl]benzenesulfonamide

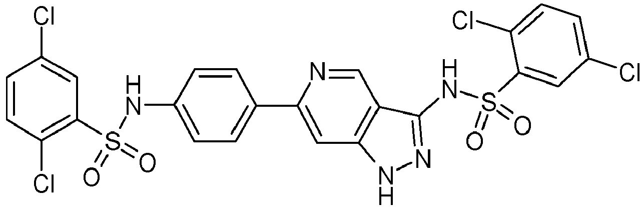

The title compound was isolated as a further product in example 556.

MS (ES+): m/e = 642.0 (M+H), chloro pattern.

### Example 561: 2-Cyano-5-methyl-N-[4-(3-methyl-1H-pyrazolo[4,3-c]pyridin-6-yl)phenyl]benzenesulfonamide

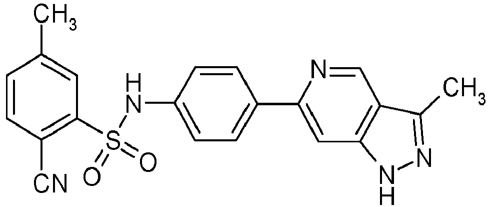

(i) tert-Butyl N-[4-(5-acetyl-4-chloro-2-pyridyl)phenyl]carbamate
   A solution of 200 mg of 1-(4,6-dichloro-3-pyridyl)ethanone, 335 mg of tert-butyl N-[4-(4,4,5,5-tetramethyl-[1,3,2]dioxaborolan-2-yl)phenyl]carbamate and 1.0 g of cesium carbonate in 6 ml of Diox and 1 ml of water was purged with argon. Then 62 mg of BDFP were added and the reaction mixture was heated to 100°C. After 2 h, the reaction mixture was cooled to RT and diluted with water. After filtration through a Chem Elut^{®} cartridge by eluting with EtOAc, the solvents were removed under reduced pressure. The crude product was purified by chromatography on silica gel eluting with a gradient of Hep/EtOAc. The fractions containing the product were combined and the solvent evaporated under reduced pressure. Yield: 290 mg.
(ii) 4-(3-Methyl-1H-pyrazolo[4,3-c]pyridin-6-yl)aniline hydrochloride
   To a solution of 290 mg of tert-butyl N-[4-(5-acetyl-4-chloro-2-pyridyl)phenyl]carbamate in 3 ml of iPrOH, 2.8 ml of a hydrazine solution (35% in iPrOH) were added and the reaction mixture was heated for 15 min to 80°C by using microwave irradiation (Biotage^{®} Initiator apparatus). The reaction mixture was cooled to RT and diluted with acetic acid (20%). The precipitated intermediate product tert-butyl N-[4-(3-methyl-1H-pyrazolo[4,3-c]pyridin-6-yl)phenyl]carbamate was collected by filtration, dried under reduced pressure and dissolved in 5 ml of a ethanolic solution of hydrochloric acid (8M). After stirring for 1 h at RT the reaction mixture was diluted with toluene (100 ml) and the solvents were removed under reduced pressure. The residue was co-distilled additional two times with toluene. After drying under reduced pressure the product was pure enough for the next reaction step. Yield: 170 mg.
(iii) 2-Cyano-5-methyl-N-[4-(3-methyl-1H-pyrazolo[4,3-c]pyridin-6-yl)phenyl]benzenesulfonamide
   To a solution of 170 mg of 6-(4-aminophenyl)-1H-pyrazolo[4,3-c]pyridin-3-amine hydrochloride in 4 ml of DCM and 180 µl of pyridine, 140 mg of 2-cyano-5-methyl-benzenesulfonyl chloride were added. After stirring the reaction mixture for 16 h at RT, the solvents were removed under reduced pressure. This crude product was purified by preparative HPLC (C18 reverse phase column, elution with a water/MeCN gradient). The fractions containing the product were lyophilized to yield the pure title compound. Yield: 44 mg.

¹H-NMR (DMSO-d₆): δ (ppm) = 2.48 (s, 3H), 2.57 (s, 3H), 7.23 (d, J = 8.9 Hz, 2H), 7.65 (d, J = 8.9 Hz, 1H), 7.86 (s, 1H), 7.96 (d, J = 8.9 Hz, 2H), 8.02 (d, J = 8.9 Hz, 2H), 9.12 (s, 1H), 10.98 (s, 1H). MS (ES+): m/e = 404.2 (M+H).

### Example 562: 5-Chloro-2-cyano-N-[4-(3-methyl-1H-pyrazolo[4,3-c]pyridin-6-yl)phenyl]benzenesulfonamide

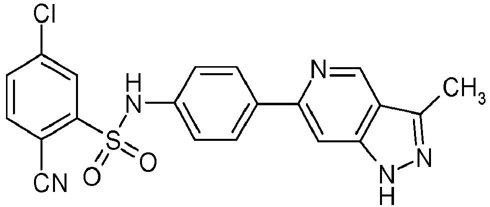

The title compound was prepared analogously to the procedure described in example 561, employing 5-chloro-2-cyano-benzenesulfonyl chloride instead of 2-cyano-5-methyl-benzenesulfonyl chloride.

MS (ES+): m/e = 424.1 (M+H), chloro pattern.

### Example 563: 2,5-Dichloro-N-[4-(1H-pyrazolo[4,3-c]pyridin-6-yl)phenyl]benzenesulfonamide

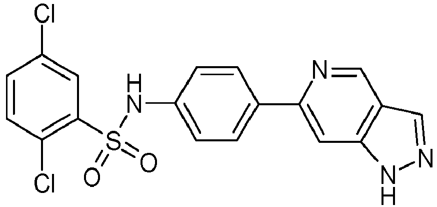

(i) 2,5-Dichloro-N-[4-(4-chloro-5-formyl-2-pyridyl)phenyl]benzenesulfonamide A solution of 60 mg of 4,6-dichloropyridine-3-carbaldehyde, 146 mg of 2,5-dichloro-N-[4-(4,4,5,5-tetramethyl-[1,3,2]dioxaborolan-2-yl)phenyl]benzenesulfonamide and 333 mg of cesium carbonate in 2 ml of Diox and 0.3 ml of water was purged with argon. Then 20 mg of BDFP were added and the reaction mixture was heated to 100°C. After 2 h, the reaction mixture was cooled to RT and diluted with water. After filtration through a Chem Elut^{®} cartridge by eluting with EtOAc, the solvents were removed under reduced pressure. The crude product was used in the next reaction step without further purification. Yield: 190 mg.
(ii) 2,5-Dichloro-N-[4-(1H-pyrazolo[4,3-c]pyridin-6-yl)phenyl]benzenesulfonamide To a solution of 190 mg of 2,5-dichloro-N-[4-(4-chloro-5-formyl-2-pyridyl)phenyl]benzenesulfonamide in 3 ml of iPrOH, 1.45 ml of a hydrazine solution (35% in iPrOH) were added and the reaction mixture was heated for 40 min to 120°C by using microwave irradiation (Biotage^{®} Initiator apparatus). The reaction mixture was cooled to RT and diluted with acetic acid (20%). The precipitate was collected by filtration and purified by preparative HPLC (C18 reverse phase column, elution with a water/MeCN gradient with 0.1% TFA). The fractions containing the product were lyophilized to yield the pure title compound in the form of its salt with trifluoroacetic acid. Yield: 12 mg.

¹H-NMR (DMSO-d₆): δ (ppm) = 7.30 (d, J = 8.7 Hz, 2H), 7.70 (d, J = 8.7 Hz, 1H), 7.74 (m, 2H), 7.96 (d, J = 8.7 Hz, 2H), 8.03 (s, 1H), 8.12 (s, 1H), 8.40 (s, 1H), 9.33 (s, 1H), 10.98 (s, 1H).

MS (ES+): m/e = 419.1 (M+H), chloro pattern.

### Example 564: 5-Cyano-2-methyl-N-[4-(1H-pyrazolo[4,3-c]pyridin-6-yl)-phenyl]-benzenesulfonamide

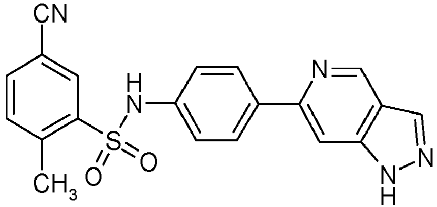

The title compound was prepared analogously to the procedure described in example 563, employing 5-cyano-2-methyl-N-[4-(4,4,5,5-tetramethyl-[1,3,2]dioxaborolan-2-yl)-phenyl]-benzenesulfonamide instead of 2,5-dichloro-N-[4-(4,4,5,5-tetramethyl-[1,3,2]dioxaborolan-2-yl)phenyl]benzenesulfonamide.

MS (ES+): m/e = 390.2 (M+H).

### Example 565: 1-[(4-Chloro-phenyl)methyl]-3-[6-[4-[(2,5-dichlorophenyl)sulfonylamino]phenyl]-1H-pyrazolo[3,4-d]pyrimidin-3-yl]urea

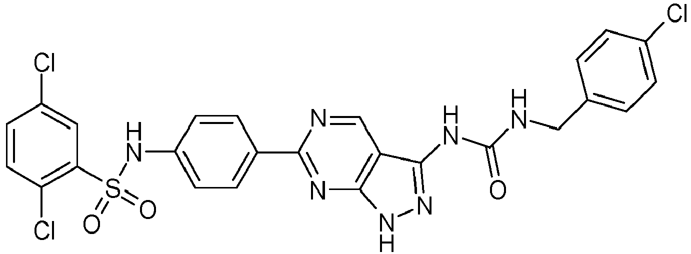

To a solution of 80 mg of N-[4-(3-amino-1H-pyrazolo[3,4-d]pyrimidin-6-yl)-phenyl]-2,5-dichloro-benzenesulfonamide in 2 ml Diox, 31 mg of 1-chloro-4-isocyanatomethyl-benzene and 21 mg 1,3-dimethylimidazolidin-2-one were added and the reaction mixture was stirred for 16 h at RT. Then the reaction mixture was concentrated under reduced pressure and the crude product was purified by preparative HPLC (C18 reversed phase column, elution with a water/MeCN gradient with 0.1% TFA). The fractions containing the product were lyophilized to yield the pure title compound in the form of its salt with trifluoroacetic acid. Yield: 102 mg.

MS (ES+): m/e = 602.1 (M+H), chloro pattern.

### Example 566: 1-[6-[4-[(2,5-Dichlorophenyl)sulfonylamino]phenyl]-1H-pyrazolo[3,4-d]pyrimidin-3-yl]-3-(tetrahydropyran-4-ylmethyl)urea

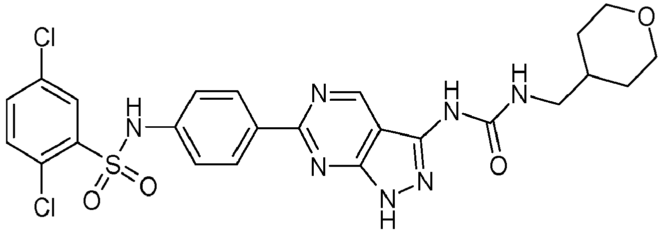

The title compound was prepared analogously to the procedure described in example 565.

MS (ES+): m/e = 576.2 (M+H), chloro pattern.

### Example 567: 2,5-Dichloro-N-[4-[3-(diethylamino)-1H-pyrazolo[3,4-d]pyrimidin-6-yl]phenyl]benzenesulfonamide

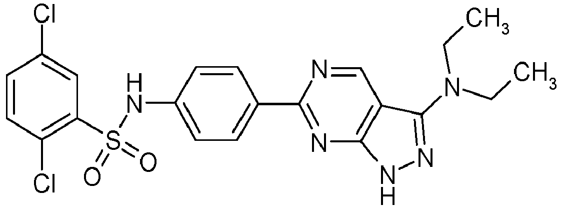

To a solution of 80 mg of N-[4-(3-amino-1H-pyrazolo[3,4-d]pyrimidin-6-yl)-phenyl]-2,5-dichloro-benzenesulfonamide, 41 mg of acetaldehyde in 5 ml of 1,2-dichloroethane, 22 mg of sodium triacetoxyborohydride and 2 µl of acetic acid were added at RT. After stirring for 16 h, water was added and the reaction mixture was filtered through a Chem Elut^{®} cartridge by eluting with EtOAc. After removal of the solvents under reduced pressure, the residue was purified by preparative HPLC (C18 reversed phase column, elution with a water/MeCN gradient with 0.1% TFA). The fractions containing the product were lyophilized to yield the pure title compound in the form of its salt with trifluoroacetic acid. Yield: 15 mg.

MS (ES+): m/e = 491.1 (M+H), chloro pattern.

### Example 568: N-[4-(3-Amino-4-trifluoromethyl-1H-pyrazolo[3,4-d]pyrimidin-6-yl)-phenyl]-5-chloro-2-fluoro-benzenesulfonamide

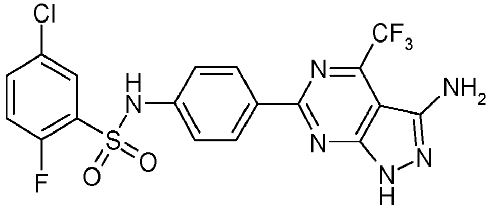

(i) Ethyl 2-cyano-4,4,4-trifluoro-3-hydroxybut-2-enoate
   To a solution of 19.2 ml of trifluoroacetic acid anhydride in 300 ml of DCM, 12.3 ml of cyano-acetic acid ethyl ester were added. Then 40 ml of triethylamine were added dropwise at 0°C and the reaction mixture was allowed to warm to RT and stirred for 1 h. The reaction mixture was acidified with half-concentrated aqueous hydrochloric acid to pH 1, and the mixture was extracted with DCM (3 x 200 ml). The combined organic layers were washed with water, dried over magnesium sulfate and the solvents were removed under reduced pressure. The crude product was used in the subsequent reaction step. Yield: 30 g.
(ii) Ethyl 3-chloro-2-cyano-4,4,4-trifluorobut-2-enoate
   To a solution of 30 g of ethyl 2-cyano-4,4,4-trifluoro-3-hydroxybut-2-enoate in 300 ml of DCM, 64 ml of oxalyl chloride were slowly added dropwise at 0°C. Then the reaction mixture was allowed to warm to RT, stirred for 1 h and 0.3 ml of pyridine were added. The reaction mixture was heated to reflux for 4 h, then cooled to RT and poured into 500 ml of ice water. The organic phase was separated and the aqueous phase was extracted with DCM (2 × 100 ml). The combined organic layers were dried over magnesium sulfate and the solvents were removed under reduced pressure. The obtained crude product was used in the subsequent reaction step. Yield: 28 g.
(iii) 4-Hydroxy-2-(4-nitrophenyl)-6-(trifluoromethyl)-pyrimidine-5-carbonitrile
   To a mixture of 21 g of ethyl 3-chloro-2-cyano-4,4,4-trifluorobut-2-enoate and 7.6 g of 4-nitrobenzimidamide in 300 ml of water, 31 ml of a aqueous sodium hydroxide solution (2M) were added. The reaction mixture was stirred at RT for 4 h, then diluted with 200 ml of water, acidified with half-concentrated aqueous hydrochloric acid to pH 3 and extracted with EtOAc (3 x 500 ml). The combined organic layers were dried over magnesium sulfate and the solvents were removed under reduced pressure. The crude product was purified by chromatography on silica gel eluting with a gradient of EtOAc/methanol. Yield: 2.7 g.
(iv) 4-Chloro-2-(4-nitrophenyl)-6-(trifluoromethyl)-pyrimidine-5-carbonitrile
   To a solution of 660 mg of 4-hydroxy-2-(4-nitrophenyl)-6-(trifluoromethyl)-pyrimidine-5-carbonitrile in 2.92 ml of phosphorus oxychloride, 0.3 ml of dimethyl-phenyl-amine were added. The reaction mixture was heated to reflux for 30 minh, then cooled to RT and concentrated under reduced pressure. After addition of ice water and dilution with DCM, saturated aqueous sodium hydrogencarbonate solution was added and the mixture was extracted with DCM (3 x 100 ml). The combined organic layers were dried over magnesium sulfate and the solvents were removed under reduced pressure. The obtained crude product was used in the subsequent reaction without further purification. Yield: 700 mg.
(v) 6-(4-Nitrophenyl)-4-(trifluoromethyl)-1H-pyrazolo[3,4-d]pyrimidin-3-amine
   To a solution of 700 mg of 4-chloro-2-(4-nitrophenyl)-6-(trifluoromethyl)-pyrimidine-5-carbonitrile in 10 ml of iPrOH, 2.4 ml of hydrazine hydrate (64% in water) were added and the reaction mixture was heated for 4 h to 100°C. The reaction mixture was cooled to RT and diluted with acetic acid (20%). The precipitated product was collected by filtration and purified by preparative HPLC (C18 reverse phase column, elution with a water/MeCN gradient with 0.1% TFA). The fractions containing the product were lyophilized to yield the pure title compound in the form of its salt with trifluoroacetic acid. Yield: 85 mg.
(vi) tert-Butyl 3-(bis(tert-butoxycarbonyl)amino)-6-(4-nitrophenyl)-4-(trifluoromethyl)-pyrazolo[3,4-d]pyrimidine-1-carboxylate
   To a suspension of 85 mg of 6-(4-nitrophenyl)-4-(trifluoromethyl)-1H-pyrazolo[3,4-d]pyrimidin-3-amine in 30 ml of DCM, 2 g of di-tert-butyl dicarbonate, 0.1 ml of triethylamine and 3 mg of dimethyl-pyridin-4-yl-amine were added. The mixture was stirred for 16 h at RT, then quenched by the addition of water and diluted with DCM. After separation of the organic layer, the aqueous layer was extracted with DCM (3 x 200 ml). The combined organic layers were dried over magnesium sulfate and the solvents were removed under reduced pressure. The obtained crude product was used without further purification in the next reaction. Yield: 170 mg.
(vii) tert-Butyl 6-(4-aminophenyl)-3-(bis(tert-butoxycarbonyl)amino)-4-(trifluoromethyl)-pyrazolo[3,4-d]pyrimidine-1-carboxylate
   To a solution of 170 mg of tert-butyl 3-(bis(tert-butoxycarbonyl)amino)-6-(4-nitrophenyl)-4-(trifluoromethyl)-pyrazolo[3,4-d]pyrimidine-1-carboxylate obtained in the preceding step in 40 ml of EtOAc, 30 mg of Pd/C (10%) were added under argon and the suspension was stirred under an atmosphere of hydrogen (2 bar) for 1 h. The suspension was filtered over a plug of Celite^{®} and washed with EtOAc. The crude product was obtained after evaporation of the solvent as a brown solid and was dried under reduced pressure. Yield: 160 mg.
(viii) N-[4-(3-Amino-4-trifluoromethyl-1H-pyrazolo[3,4-d]pyrimidin-6-yl)-phenyl]-5-chloro-2-fluoro-benzenesulfonamide
   To a solution of 160 mg of tert-butyl 6-(4-aminophenyl)-3-(bis(tert-butoxycarbonyl)amino)-4-trifluoromethyl)-pyrazolo[3,4-d]pyrimidine-1-carboxylate in 5 ml DCM and 43 µl pyridine, 63 mg of 5-chloro-2-fluoro-benzenesulfonyl chloride were added. After stirring the reaction mixture for 16 h at RT, the solvents were removed under reduced pressure. The residue was dissolved in 10 ml DCM and 1 ml of TFA and stirred for 2 h at RT. Then toluene was added and the solvents were removed under reduced pressure to yield a brown solid. This crude product was purified by preparative HPLC (C18 reverse phase column, elution with a water/MeCN gradient with 0.1% TFA). The fractions containing the product were lyophilized to yield the pure title compound in the form of its salt with trifluoroacetic acid. Yield: 23 mg.

MS (ES+): m/e = 487.0 (M+H), chloro pattern.

### Analogously to the procedure described in example 568, the example compounds of the formula If

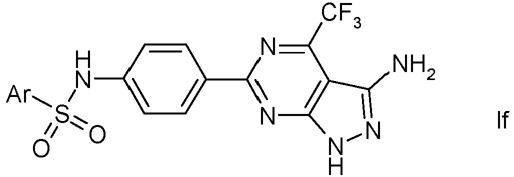

listed in Table 6 were synthesized, employing the respective sulfonyl chloride instead of 5-chloro-2-fluoro-benzenesulfonyl chloride. The ionization method in the MS characterization was ES+. CP means chloro pattern in the mass spectrum.

**Table 6. Example compounds of the formula If**

| Example no. | Ar | MS (m/e) |
|---|---|---|
| 569 | 2-cyano-5-methyl-phenyl | 474.1 (M+H) |
| 570 | 5-chloro-2-cyano-phenyl | 494.0 (M+H), CP |
| 571 | 2-cyano-5-methoxy-phenyl | 490.1 (M+H) |
| 572 | 2,5-dichloro-thiophen-3-yl | 508.9 (M+H), CP |
| 573 | 2,3,5-trifluoro-phenyl | 489.1 (M+H) |
| 574 | 5-chloro-2,4-difluoro-phenyl | 505.0 (M+H), CP |
| 575 | 2-fluoro-phenyl | 453.1 (M+H) |
| 576 | 2-chloro-3,5-difluoro-phenyl | 505.0 (M+H), CP |
| 577 | 2-chloro-4-fluoro-phenyl | 487.0 (M+H), CP |
| 578 | 3-chloro-2-fluoro-phenyl | 487.0 (M+H), CP |
| 579 | 2,5-dichloro-phenyl | 503.0 (M+H), CP |
| 580 | 2-fluoro-5-methyl-phenyl | 467.1 (M+H) |
| 581 | 2-chloro-5-methoxy-phenyl | 499.0 (M+H), CP |
| 582 | 2,5-difluoro-phenyl | 471.0 (M+H) |
| 583 | 2-chloro-phenyl | 469.0 (M+H), CP |
| 584 | 2,4,5-trifluoro-phenyl | 489.1 (M+H) |

### Analogously to the procedures described in the examples above, the example compounds of the formula Ib

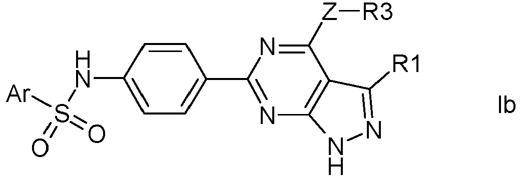

listed in Table 7 were synthesized. In the formulae of the groups -Z-R3 in Table 7 the line crossed with the symbol 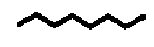 represents the free bond via which the group -Z-R3 is bonded to the carbon atom in the 4-position of the pyrazolo[3,4-d]pyrimidine ring system. I.e., in the formula of the complete molecule the terminal endpoint of the line crossed with the said symbol ends at the carbon atom in the 4-position of the pyrazolo[3,4-d]pyrimidine ring system. In the column "Synthesis" the number of the example is specified in analogy to which the synthesis was performed. The ionization method in the MS characterization was ES+. CP means chloro pattern in the mass spectrum.

**Table 7. Example compounds of the formula Ib**

| Example no. | Ar | R1 | 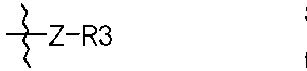 | Syn-thesis | Yield (%) | MS (m/e) |
|---|---|---|---|---|---|---|
| 585 | 2-fluoro-phenyl | CH₃ | 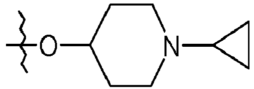 | 3 (a) | 31 | 523.2 (M+H) |
| 586 | 2-cyano-5-methyl-phenyl | CH₃ | 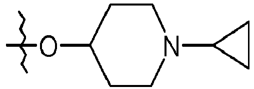 | 3 (a) | 6 | 544.2 (M+H) |
| 587 | 5-chloro-2-fluoro-phenyl | CH₃ | 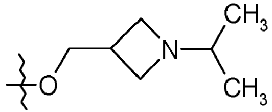 | 3 (a) | 29 | 545.1 (M+H), CP |
| 588 | 2-chloro-5-methoxy-phenyl | CH₃ | 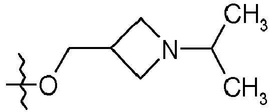 | 3 (a) | 7 | 557.1 (M+H), CP |
| 589 | 2-fluoro-5-methyl-phenyl | CH₃ | 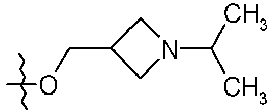 | 3 (a) | 25 | 525.1 (M+H) |
| 590 | 2,5-dichloro-phenyl | CH₃ | 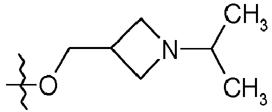 | 3 (a) | 18 | 561.0 (M+H), CP |
| 591 | 2-fluoro-phenyl | CH₃ | 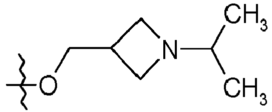 | 3 (a) | 22 | 511.1 (M+H) |
| 592 | 2,5-difluoro-phenyl | CH₃ | 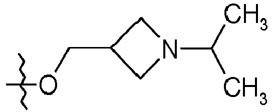 | 3 (a) | 24 | 529.1 (M+H) |
| 593 | 2-fluoro-5-methoxy-phenyl | CH₃ | 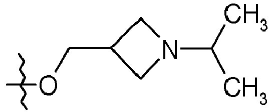 | 3 (a) | 21 | 541.1 (M+H) |
| 594 | 5-chloro-2,4-difluoro-phenyl | NH₂ | 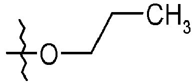 | 7 | 28 | 495.0 (M+H), CP |
| 595 | 2-fluoro-5-methyl-phenyl | NH₂ | 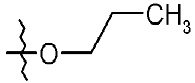 | 7 | 29 | 457.1 (M+H) |
| 596 | 2-fluoro-5-methyl-phenyl | NH₂ | 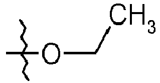 | 7 | 24 | 443.1 (M+H) |
| 597 | 5-chloro-2-fluoro-phenyl | CH₃ | 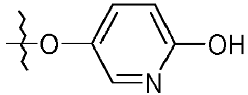 | 3 (a) | 13 | 527.0 (M+H), CP |
| 598 | 2-fluoro-phenyl | CH₃ | 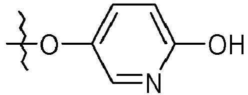 | 3 (a) | 10 | 493.0 (M+H) |
| 599 | 2-chloro-5-methoxy-phenyl | CH₃ | 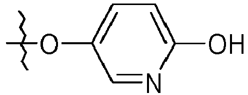 | 3 (a) | 9 | 539.0 (M+H), CP |
| 600 | 2-fluoro-5-methyl-phenyl | CH₃ | 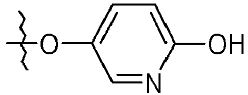 | 3 (a) | 7 | 507.1 (M+H) |
| 601 | 2,5-dichloro-phenyl | CH₃ | 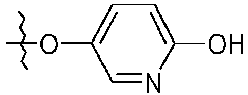 | 3 (a) | 5 | 543.2 (M+H), CP |
| 602 | 2-fluoro-5-methoxy-phenyl | CH₃ | 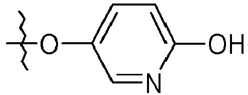 | 3 (a) | 15 | 523.3 (M+H) |
| 603 | 2-cyano-5-methyl-phenyl | CH₃ | 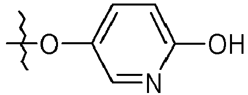 | 3 (a) | 8 | 514.1 (M+H) |
| 604 | 2,5-difluoro-phenyl | CH₃ | 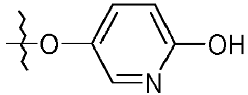 | 3 (a) | 13 | 511.2 (M+H) |
| 605 | 2-chloro-4,5-difluoro-phenyl | NH₂ | 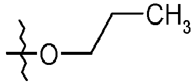 | 7 | 34 | 495.0 (M+H), CP |
| 606 | 2-fluoro-phenyl | NH₂ | 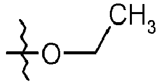 | 7 | 22 | 429.0 (M+H) |
| 607 | 2-fluoro-phenyl | NH₂ | 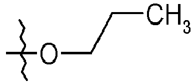 | 7 | 32 | 443.1 (M+H) |
| 608 | 2-fluoro-5-methoxy-phenyl | NH₂ | 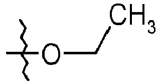 | 7 | 33 | 459.2 (M+H) |
| 609 | 2-fluoro-5-methoxy-phenyl | NH₂ | 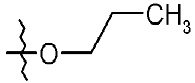 | 7 | 29 | 473.3 (M+H) |
| 610 | 2-chloro-5-methoxy-phenyl | NH₂ | 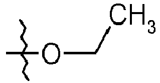 | 7 | 36 | 475.3 (M+H), CP |
| 611 | 2-chloro-5-methoxy-phenyl | NH₂ | 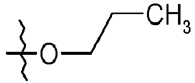 | 7 | 34 | 489.2 (M+H), CP |
| 612 | 2,4,5-trifluoro-phenyl | NH₂ | 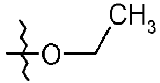 | 7 | 32 | 465.0 (M+H) |
| 613 | 2,4,5-trifluoro-phenyl | NH₂ | 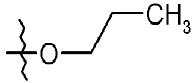 | 7 | 46 | 479.0 (M+H) |
| 614 | 2-chloro-4,5-difluoro-phenyl | NH₂ | 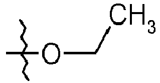 | 7 | 35 | 481.1 (M+H), CP |
| 615 | 5-chloro-2-fluoro-phenyl | CH₃ | 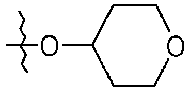 | 3 (a) | 37 | 518.2 (M+H), CP |
| 616 | 2-fluoro-phenyl | CH₃ | 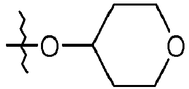 | 3 (a) | 53 | 484.2 (M+H) |
| 617 | 2,5-dichloro-phenyl | CH₃ | 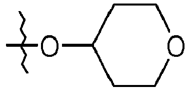 | 3 (a) | 24 | 534.2 (M+H), CP |
| 618 | 5-chloro-2-fluoro-phenyl | CH₃ | 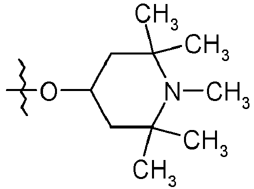 | 3 (a) | 29 | 587.3 (M+H), CP |
| 619 | 2-fluoro-phenyl | CH₃ | 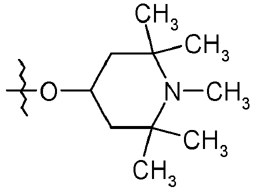 | 3 (a) | 36 | 553.4 (M+H) |
| 620 | 2,5-dichloro-phenyl | CH₃ | 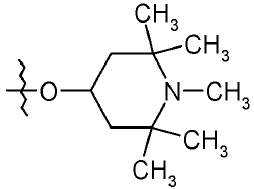 | 3 (a) | 36 | 603.3 (M+H), CP |
| 621 | 2,5-difluoro-phenyl | CH₃ | 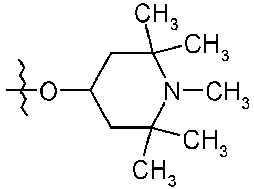 | 3 (a) | 36 | 571.3 (M+H) |
| 622 | 2,5-difluoro-phenyl | CH₃ | 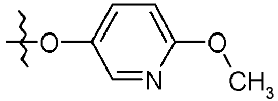 | 3 (a) | 28 | 525.2 (M+H) |
| 623 | 2,5-dichloro-phenyl | CH₃ | 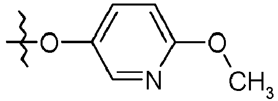 | 3 (a) | 10 | 557.2 (M+H), CP |
| 624 | 2-fluoro-phenyl | CH₃ | 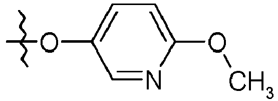 | 3 (a) | 37 | 507.3 (M+H) |
| 625 | 2,5-difluoro-phenyl | CH₃ | 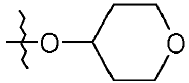 | 3 (a) | 54 | 502.2 (M+H) |
| 626 | 5-chloro-2-fluoro-phenyl | CH₃ | 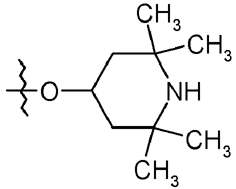 | 3 (a) | 5 | 573.3 (M+H), CP |
| Example no. | Ar | R1 | 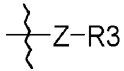 | Syn-thesis | Yield (%) | MS (m/e) |
| 627 | 2-fluoro-phenyl | CH₃ | 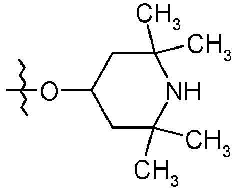 | 3 (a) | 4 | 539.3 (M+H) |
| 628 | 2-chloro-5-methoxy-phenyl | CH₃ | 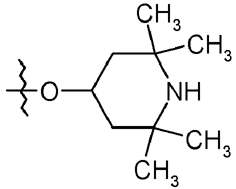 | 3 (a) | 9 | 585.3 (M+H), CP |
| 629 | 2,5-dichloro-phenyl | CH₃ | 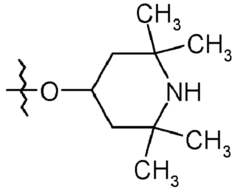 | 3 (a) | 41 | 589.3 (M+H), CP |
| 630 | 2-fluoro-5-methoxy-phenyl | CH₃ | 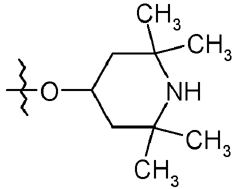 | 3 (a) | 3 | 569.4 (M+H) |
| 631 | 2-fluoro-5-methyl-phenyl | CH₃ | 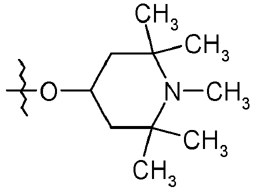 | 3 (a) | 37 | 567.4 (M+H) |
| 632 | 2-chloro-5-methoxy-phenyl | CH₃ | 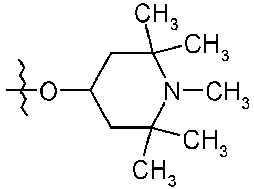 | 3 (a) | 36 | 599.4 (M+H), CP |
| 633 | 2-fluoro-5-methoxy-phenyl | CH₃ | 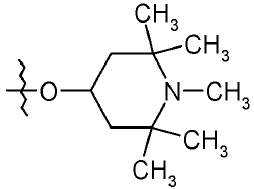 | 3 (a) | 31 | 583.4 (M+H) |
| 634 | 5-chloro-2-fluoro-phenyl | CH₃ | 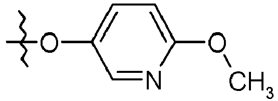 | 3 (a) | 2 | 541.2 (M+H), CP |
| 635 | 2,5-dichloro-phenyl | CH₃ | 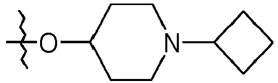 | 3 | 23 | 587.2 (M+H), CP |
| 636 | 5-chloro-2,4-difluoro-phenyl | CH₃ | 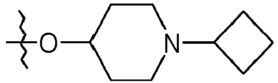 | 3 | 25 | 589.3 (M+H), CP |
| 637 | 5-chloro-2-fluoro-phenyl | CH₃ | 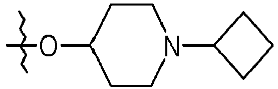 | 3 | 24 | 571.3 (M+H), CP |
| 638 | 2-chloro-3,5-difluoro-phenyl | CH₃ | 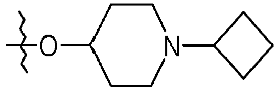 | 3 | 22 | 589.3 (M+H), CP |
| 639 | 2,4,5-trifluoro-phenyl | CH₃ | 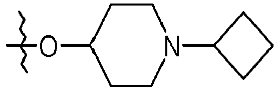 | 3 | 28 | 573.3 (M+H) |
| 640 | 2-chloro-4,5-difluoro-phenyl | CH₃ | 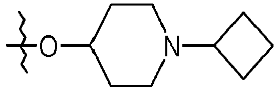 | 3 | 30 | 589.3 (M+H), CP |
| 641 | 2-cyano-5-methoxy-phenyl | CH₃ | 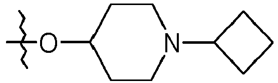 | 3 | 12 | 574.3 (M+H) |
| 642 | 2-fluoro-5-methoxy-phenyl | CH₃ | 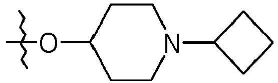 | 3 | 23 | 567.3 (M+H) |
| 643 | 2-chloro-5-methoxy-phenyl | CH₃ | 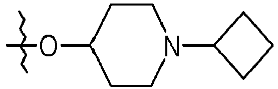 | 3 | 17 | 583.3 (M+H), CP |
| 644 | 2,5-difluoro-phenyl | CH₃ | 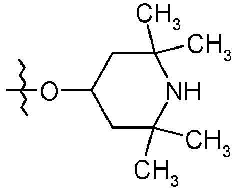 | 3 (a) | 28 | 557.3 (M+H) |
| 645 | 2-cyano-5-methyl-phenyl | CH₃ | 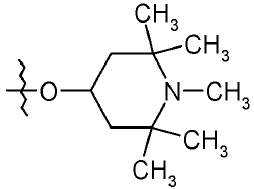 | 3 (a) | 9 | 574.3 (M+H) |
| 646 | 5-chloro-2-fluoro-phenyl | CH₃ | 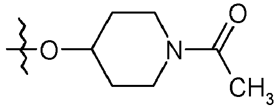 | 3 (a) | 12 | 559.3 (M+H), CP |
| 647 | 2-cyano-5-methyl-phenyl | CH₃ | 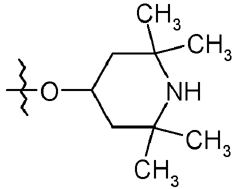 | 3 (a) | 8 | 560.4 (M+H) |
| 648 | 2-fluoro-5-methyl-phenyl | CH₃ | 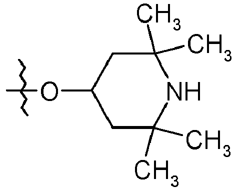 | 3 (a) | 12 | 553.4 (M+H) |
| 649 | 5-chloro-2-fluoro-phenyl | CH₃ | 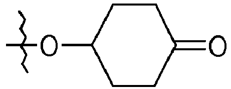 | 3 (a) | 12 | 530.2 (M+H), CP |
| 650 | 2,5-dichloro-phenyl | CH₃ | 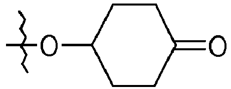 | 3 (a) | 21 | 546.2 (M+H), CP |
| 651 | 2-chloro-5-methoxy-phenyl | CH₃ | 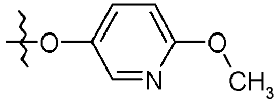 | 3 (a) | 12 | 553.3 (M+H), CP |
| 652 | 2-cyano-5-methyl-phenyl | CH₃ | 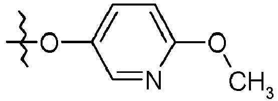 | 3 (a) | 4 | 528.3 (M+H) |
| 653 | 2-fluoro-5-methoxy-phenyl | CH₃ | 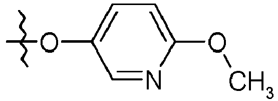 | 3 (a) | 9 | 537.3 (M+H) |
| 654 | 2-fluoro-5-methyl-phenyl | CH₃ | 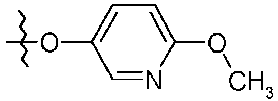 | 3 (a) | 13 | 521.3 (M+H) |
| 655 | 2-fluoro-phenyl | CH₃ | 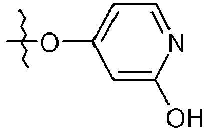 | 3 (a) | 3 | 493.2 (M+H) |
| 656 | 2-fluoro-5-methyl-phenyl | CH₃ | 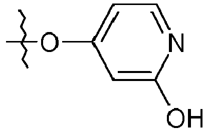 | 3 (a) | 6 | 507.2 (M+H) |
| 657 | 2-cyano-5-methyl-phenyl | CH₃ | 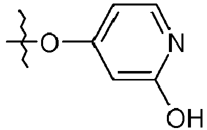 | 3 (a) | 5 | 514.3 (M+H) |
| 658 | 2-fluoro-5-methoxy-phenyl | CH₃ | 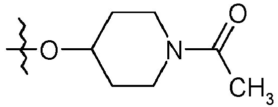 | 3 (a) | 4 | 555.3 (M+H) |
| 659 | 2-cyano-5-methyl-phenyl | CH₃ | 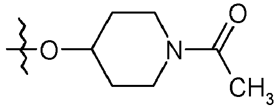 | 3 (a) | 7 | 546.3 (M+H) |
| 660 | 2-chloro-5-methoxy-phenyl | CH₃ | 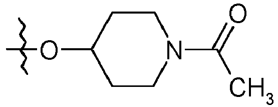 | 3 (a) | 26 | 571.3 (M+H), CP |
| 661 | 2,5-difluoro-phenyl | CH₃ | 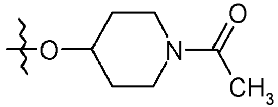 | 3 (a) | 2 | 543.3 (M+H) |
| 662 | 2-fluoro-5-methoxy-phenyl | CH₃ | 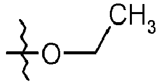 | 3 (a) | 4 | 458.3 (M+H) |
| 663 | 2-fluoro-phenyl | CH₃ | 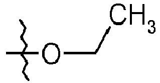 | 3 (a) | 9 | 428.3 (M+H) |
| 664 | 2,5-dichloro-phenyl | CH₃ | 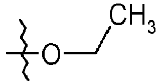 | 3 (a) | 4 | 478.2 (M+H), CP |
| 665 | 2-fluoro-5-methoxy-phenyl | CH₃ | 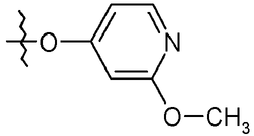 | 3 (a) | 33 | 537.3 (M+H) |
| 666 | 5-chloro-2-fluoro-phenyl | CH₃ | 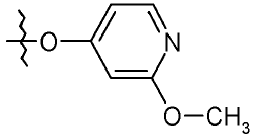 | 3 (a) | 21 | 541.3 (M+H), CP |
| 667 | 2-cyano-5-methyl-phenyl | CH₃ | 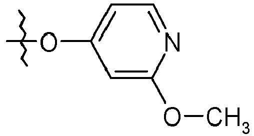 | 3 (a) | 12 | 528.4 (M+H) |
| 668 | 2,5-difluoro-phenyl | CH₃ | 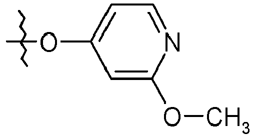 | 3 (a) | 34 | 525.3 (M+H) |
| 669 | 2,5-dichloro-phenyl | CH₃ | 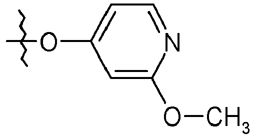 | 3 (a) | 3 | 557.2 (M+H), CP |
| 670 | 2-chloro-5-methoxy-phenyl | CH₃ | 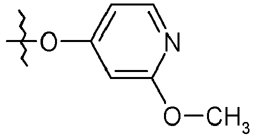 | 3 (a) | 3 | 553.3 (M+H), CP |
| 671 | 2-fluoro-phenyl | CH₃ | 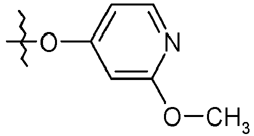 | 3 (a) | 4 | 505.4 (M+H) |
| 672 | 2-fluoro-5-methyl-phenyl | CH₃ | 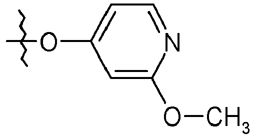 | 3 (a) | 4 | 519.4 (M+H) |
| 673 | 2-cyano-5-methyl-phenyl | CH₃ | 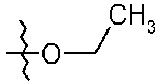 | 3 (a) | 3 | 449.3 (M+H) |
| 674 | 2,5-difluoro-phenyl | CH₃ | 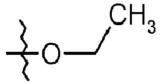 | 3 (a) | 2 | 446.2 (M+H) |
| 675 | 2-chloro-5-methoxy-phenyl | CH₃ | 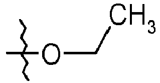 | 3 (a) | 5 | 474.2 (M+H), CP |
| 676 | 5-chloro-2-fluoro-phenyl | CH₃ | 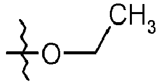 | 3 (a) | 6 | 462.2 (M+H), CP |
| 677 | 2-fluoro-5-methyl-phenyl | CH₃ | 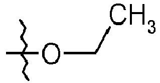 | 3 (a) | 4 | 442.2 (M+H) |
| 678 | 2-fluoro-5-methoxy-phenyl | CH₃ | 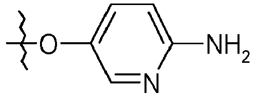 | 3 (a) | 35 | 522.2 (M+H) |
| 679 | 5-chloro-2-fluoro-phenyl | CH₃ | 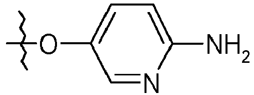 | 3 (a) | 30 | 526.2 (M+H), CP |
| 680 | 2-fluoro-phenyl | CH₃ | 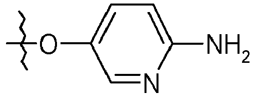 | 3 (a) | 28 | 492.2 (M+H) |
| 681 | 2-chloro-5-methoxy-phenyl | CH₃ | 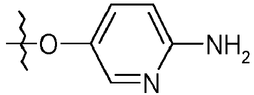 | 3 (a) | 36 | 538.2 (M+H), CP |
| 682 | 2,5-difluoro-phenyl | CH₃ | 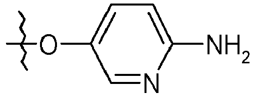 | 3 (a) | 17 | 510.2 (M+H) |
| 683 | 2-fluoro-5-methyl-phenyl | CH₃ | 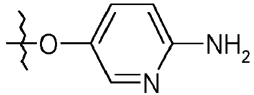 | 3 (a) | 34 | 506.2 (M+H) |
| 684 | 2,5-dichloro-phenyl | CH₃ | 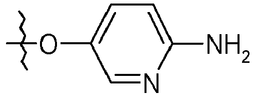 | 3 (a) | 12 | 542.2 (M+H), CP |
| 685 | 2-cyano-5-methyl-phenyl | CH₃ | 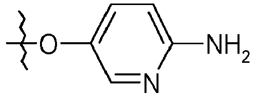 | 3 (a) | 20 | 513.3 (M+H) |
| 686 | 2-fluoro-5-methyl-phenyl | CH₃ | 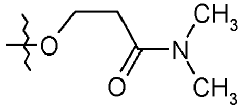 | 3 (a) | 37 | 513.3 (M+H) |
| 687 | 5-chloro-2-fluoro-phenyl | CH₃ | 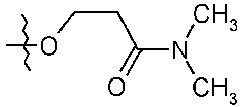 | 3 (a) | 21 | 533.2 (M+H), CP |
| 688 | 2-fluoro-phenyl | CH₃ | 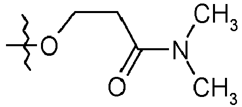 | 3 (a) | 46 | 499.3 (M+H) |
| 689 | 2-chloro-5-methoxy-phenyl | CH₃ | 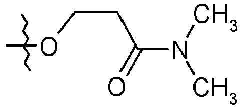 | 3 (a) | 25 | 545.3 (M+H), CP |
| 690 | 2,5-dichloro-phenyl | CH₃ | 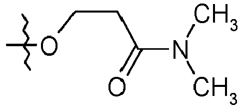 | 3 (a) | 26 | 549.2 (M+H), CP |
| 691 | 2,5-difluoro-phenyl | CH₃ | 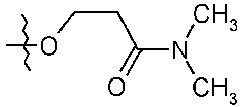 | 3 (a) | 54 | 517.2 (M+H) |
| 692 | 2-fluoro-5-methoxy-phenyl | CH₃ | 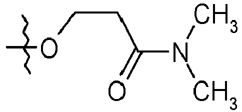 | 3 (a) | 37 | 529.3 (M+H) |
| 693 | 2-fluoro-5-methyl-phenyl | CH₃ | 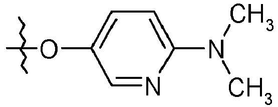 | 3 (a) | 28 | 534.3 (M+H) |
| 694 | 5-chloro-2-fluoro-phenyl | CH₃ | 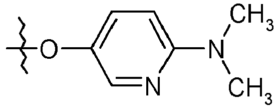 | 3 (a) | 40 | 554.2 (M+H), CP |
| 695 | 2-fluoro-phenyl | CH₃ | 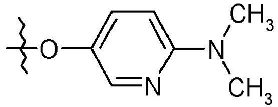 | 3 (a) | 60 | 520.2 (M+H) |
| 696 | 2,5-dichloro-phenyl | CH₃ | 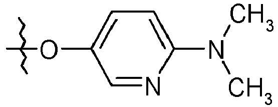 | 3 (a) | 39 | 570.2 (M+H), CP |
| 697 | 2,5-difluoro-phenyl | CH₃ | 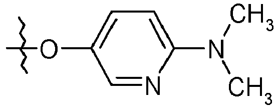 | 3 (a) | 43 | 538.2 (M+H) |
| 698 | 2-fluoro-5-methoxy-phenyl | CH₃ | 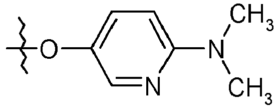 | 3 (a) | 55 | 550.2 (M+H) |
| 699 | 2-chloro-5-methoxy-phenyl | CH₃ | 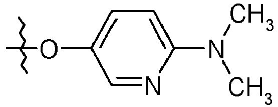 | 3 (a) | 49 | 566.2 (M+H), CP |

| | | | | | | |
|---|---|---|---|---|---|---|
| (a) In step (ii) of the procedure of example 3, potassium hydroxide and dimethyl sulfoxide were employed instead of sodium hydride and tetrahydrofuran. | | | | | | |

### Exemplary NMR data of example compounds

### Example 597

¹H-NMR (DMSO-d₆): δ (ppm) = 2.54 (s, 1H), 2.60 (s, 3H), 6.44-6.50 (m, 1H), 7.22 (d, J = 8.8 Hz, 2H), 7.49 (t, J = 9.5 Hz, 1H), 7.60-7.67 (m, 2H), 7.74-7.79 (m, 1H), 7.87 (dd, J = 2.7, 6.0 Hz, 1H), 8.08 (d, J = 8.8 Hz, 2H), 11.11 (s, 1H), 13.63 (s, 1H).

### Example 600

¹H-NMR (DMSO-d₆): δ (ppm) = 2.32 (s, 3H), 2.54 (s, 1H), 2.60 (s, 3H), ), 6.44-6.49 (m, 1H), 7.20 (d, J = 8.8 Hz, 2H), 7.27 (dd, J = 8.4, 10.3 Hz, 1H), 7.44-7.48 (m, 1H), 7.60-7.65 (m, 2H), 7.71 (dd, J = 2.1, 6.9 Hz, 1H), 8.05 (d, J = 8.8 Hz, 2H), 10.92 (s, 1H), 13.61 (s, 1H).

### Example 646

¹H-NMR (DMSO-d₆): δ (ppm) = 1.70-1.91 (m, 2H), 1.94-2.14 (m, 2H), 2.05 (s, 3H), 2.51 (s, 3H), 3.48-3.72 (m, 4H), 5.69-5.76 (m, 1H), 7.27 (d, J = 8.8 Hz, 2H), 7.51 (t, J = 9.2 Hz, 1H), 7.76-7.82 (m, 1H), 7.87 (dd, J = 2.6, 6.0 Hz, 1H), 8.31 (d, J = 8.8 Hz, 2H), 11.10 (s, 1H), 13.42 (br, 1H).

### Example 658

¹H-NMR (DMSO-d₆): δ (ppm) = 1.70-1.90 (m, 2H), 1.95-2.12 (m, 2H), 2.05 (s, 3H), 2.52 (s, 3H), 3.49-3.72 (m, 4H), 3.78 (s, 3H), 5.68-5.75 (m, 1H), 7.20-7.25 (m, 1H), 7.26 (d, J = 8.8 Hz, 2H), 7.31-7.38 (m, 2H), 8.29 (d, J = 8.8 Hz, 2H), 10.96 (s, 1H), 13.41 (br, 1H).

### Example 661

¹H-NMR (DMSO-d₆): δ (ppm) = 1.70-1.90 (m, 2H), 1.95-2.13 (m, 2H), 2.05 (s, 3H), 2.51 (s, 3H), 3.50-3.72 (m, 4H), 5.69-5.75 (m, 1H)), 7.27 (d, J = 8.8 Hz, 2H), 7.52 (dt, J = 4.2, 9.2 Hz, 1H), 7.56-7.63 (m, 1H), 7.69-7.74 (m, 1H), 8.29 (d, J = 8.8 Hz, 2H), 11.10 (s, 1H), 13.42 (s, 1H).

### Example 1 - Expression of SGK1 in erythroid lineage.

FIG. 1 shows the expression of SGK1 in human CD34+ cells from healthy donor. SGK1 expression was measured by Western blot. Total cell lysates from human erythroid CD34+ cells were prepared, and the total protein concentration was determined using a Bradford protein assay (ThermoFisher Scientific). Reduced and denaturated protein (40µg) was loaded and separated by SDS-PAGE (12% gel), blotted on nitrocellulose membranes (BioRad) and finally incubated with SGK1 antibody (Cell Signaling). Immunoreactive proteins were visualized by using an ECL^{®} (enhanced chemiluminescence) detection system (BioRad).

### Example 2 - Induction of fetal hemoglobin HbF by Compound 346 in human CD34+ cells during differentiation in vitro.

Mobilized CD34+ Human Stem/Progenitor Cells (HSPC) from healthy individuals were cultured for 3 days in a maintenance media consisting of X-VIVO 10 (VWR), 100 U/mL penicillin-streptomycin (ThermoFisher), 2mM L-glutamine (Fisher Scientific), 100 ng/mL Recombinant Human Stem Cell Factor (SCF), 100 ng/mL Recombinant Human Thrombopoietin (TPO) and 100 ng/mL Recombinant Human Flt-3 Ligand (Flt-3L) (all three from ThermoFisher). Cells were differentiated into erythroid cells using a three-step differentiation protocol developed by the Luc Douay group (Giarratana et al. 2005). In brief, CD34+ cells were cultured for 7 days in Step 1 media, consisting of Iscove's modified Dulbecco's medium (IMDM) (ThermoFisher) supplemented with 1X GlutaMAX, 100 U/mL penicillin-streptomycin (ThermoFisher), 5% human AB+plasma, 330 ug/mL human holo-transferrin, 10 ug/mL human insulin, 2 U/mL heparin, 1 uM/mL hydrocortisone (Sigma-Aldrich), 3 U/mL recombinant human erythropoietin (EPO) (ThermoFisher), 100 ng/mL SCF (ThermoFisher) and 5 ng/mL interleukin 3 (IL-3) (SigmaAldrich). On day 7, cells were transferred to step 2 media, a step 1 media without hydrocortisone and IL-3 and cultured for 3-4 days. Then cells were cultured for 8-9 days in step 3 media, a step 2 media without SCF. During the entire process of differentiation, mobilized CD34+ HSPC were exposed to Compound 346. To determine the percentage of HbF-positive cells (F-cells), differentiated cells were fixed and permeabilized using a fixation kit (ThermoFisher). Cells were stained with PE-Cy7-conjugated anti-CD235 or PE-conjugated anti-CD71 antibodies. HbF levels were detected using Allophycocyanin (APC)-conjugated anti-HbF antibody (ThermoFisher). The acquisition of stained cells was performed on BD FACSCanto^{™} and the analysis was run using FlowJo^{™} Software. Data shows the frequency of F-cells increased when CD34+ cells were exposed to Compound 346, compared to vehicle (DMSO), after 21 days of differentiation (FIG. 2A). The increase of HbF+ cells is about 1.5-fold when cells are treated with Compound 346 compared to DMSO (FIG. 2B).

### Example 3 - Compound 346 and Hydroxyurea combination shows additive effect on fetal hemoglobin induction in human CD34+ cells during differentiation in vitro.

To assess the effect of the combination of Hydroxyurea (Sigma-Aldrich) and Compound 346, human CD34+ cells were incubated with Compound 346 or Hydroxyurea alone, or a combination of both during the differentiation process. After 14 days of differentiation, cells were fixed and stained with CD235a, CD71 and fetal hemoglobin antibodies for flowcytometry analysis. Data show that the combination of Hydroxyurea and Compound 346 leads to a synergetic effect in fetal hemoglobin induction compared to Compound 346 or Hydroxyurea alone (FIG.3A). The fold change in fetal hemoglobin expression is 1,5-fold for Compound 346 and Hydroxyurea alone, and 2-fold for a combination of both compared to control (Vehicle as DMSO) (FIG. 3B).

### Example 4 - Induction of fetal hemoglobin HbF by Compound 346 in human CD34+ cells from Sickle Cell donors during differentiation in vitro.

To measure the effect of Compound 346 on fetal hemoglobin induction in CD34+ cells from patients with sickle cell disease, circulating CD34+ progenitor cells were isolated from total blood obtained from sickle cell patients, by performing a positive selection for CD34+ cells with magnetic beads (Miltenyi Biotec). Purified CD34+ cells were differentiated for 14 days. After differentiation, cells were fixed and stained with CD235a, CD71 and fetal hemoglobin antibodies for flowcytometry analysis. Data show that Compound 346 induced fetal hemoglobin in CD34+ cells compared to control (Vehicle as DMSO), increasing the frequency of F-cells (FIG. 4A) with a 1.5-fold change (FIG. 4B).

### Example 5 - SGK1 target engagement in human CD34+ after exposure to Compound 346 in vitro.

To verify SGK1 target engagement by Compound 346, CD34+ cells from a healthy donor were exposed to Compound 346 at the indicated dose (µM), harvested and lysed at the indicated time points. Phosphorylation of SGK1 at threonine 256 (Thr256) was assessed by Western blot using antibody anti-pSGK1 (Thr256) (Thermofisher) and anti-SGK1 (Cell Signaling). Human CD34+ cells, cells were collected on Day 11 of differentiation then exposed to Compound 346. According to the time course, cells were collected and lysed using RIPA buffer mixed with a phosphatase and protease inhibitors cocktail (Thermofisher). Sample protein concentrations were measured with the Pierce BSA Protein Assay (ThermoFisher). The α-tubulin antibody (Cell Signaling) served as an internal control. Immunoreactive proteins were visualized by using an ECL^{®} (enhanced chemiluminescence) detection system (BioRad). Optical density was measured with ImageJ software (National Institutes of Health, Bethesda, MD) and the ratio of phospho-SGK1 to total SGK1 was calculated, normalized on α-tubulin, and plotted based on the densitometry measurements. Data show a decrease in p-SGK1 (Thr256) in CD34+ cells at 1h after exposure, confirming target engagement in SGK1 when cells are treated with Compound 346. Furthermore, SGK1 downstream pathway activation was verified by measuring phosphorylation of FOXO3 at Serine 315, a direct target of activated SGK1. Quantification of FOXO3 activation was assessed by Western Blot using antibodies anti-pFOXO3 (Ser315) and anti-FOXO3 (Cell Signaling). Densitometry measurement confirms a decrease in the phosphorylation of FOXO3 at Serine 315 in CD34+cells exposed to Compound 346, confirming the upstream inhibition of SGK1 due to exposure to Compound 346 (FIG.5).

### Example 6 - Induction of fetal hemoglobin HbF by Compound 346 in vitro prevents 21-day differentiated erythroid cells from sickling in hypoxia condition.

To evaluate the effect of Compound 346 in sickle erythroid cells sickling, a sickling assay was conducted. Circulating CD34+ progenitor cells were isolated from total blood obtained from sickle cell patients by performing a positive selection for CD34+ cells with magnetic beads (Miltenyi Biotec). Purified CD34+ cells were differentiated for 21 days. At the end of the differentiation, cells were incubated for 4 hours at 2% of oxygen in Hemox Buffer (Fisher Scientific) supplemented with 10mM Glutamine (Gibco) and 0.5% BSA (Sigma Aldrich). After exposure to hypoxia, cells were fixed with 25% of glutaraldehyde (Sigma Aldrich) and transferred to tubes for flowcytometry analysis, using a Amnis^{®} ImageStream^{®} flow cytometer to assess abnormal shaped cells. Cells were determined using RMS of brightfield image, and single cells using Area vs Aspect Ratio of brightfield image, and live cells were identified using a Live Dead NIR stain. Single living cells were analyzed using the following masking of the brightfield image: Skeleton (M1, Ch1, Thin), Object (M1, Ch1, Tight). Finally, the sickling factor was calculated using the following feature: Length_Skeleton (M1, CH1, Thin)/Width_(M1, Ch1, Tight). Cells with a sickle factor feature of 1.4 or higher were sickled. Cells with a sickle factor feature less than 1.4 were not sickled.

## Claims

1. A compound of the formula I, in any of its stereoisomeric forms or a mixture of stereoisomeric forms in any ratio, or a pharmaceutically acceptable salt thereof, 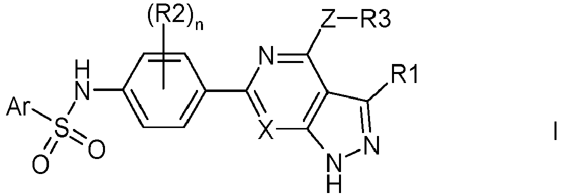 for use in the treatment of sickle cell disease,
wherein
Ar is selected from the series consisting of phenyl and a 5-membered or 6-membered, monocyclic, aromatic, heterocyclic group which comprises 1, 2 or 3 identical or different ring heteroatoms selected from the series consisting of nitrogen, oxygen and sulfur, and is bonded via a ring carbon atom, which all are unsubstituted or substituted by one or more identical or different substituents R5;
n is selected from the series consisting of 0, 1 and 2;
X is selected from the series consisting of N and CH;
Z is selected from the series consisting of a direct bond, O, S and N(R10);
R1 is selected from the series consisting of H, -N(R11)-R12, -N(R13)-C(O)-R14, -N(R13)-S(O)₂-R15, -N(R13)-C(O)-NH-R16, (C₁-C₄)-alkyl and -(C₁-C₄)-alkyl-O-R17;
R2 is selected from the series consisting of halogen, (C₁-C₄)-alkyl, -O-(C₁-C₄)-alkyl and-CN;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, (C₃-C₇)-cycloalkyl,-(C₁-C₄)-alkyl-(C₃-C₇)-cycloalkyl, -O-(C₁-C₄)-alkyl, -O-(C₃-C₇)-cycloalkyl, -O-(C₁-C₄)-alkyl-(C₃-C₇)-cycloalkyl, -C(O)-N(R6)-R7 and -CN,
and two groups R5 bonded to adjacent ring carbon atoms in Ar, together with the carbon atoms carrying them, can form a 5-membered to 8-membered, monocyclic, unsaturated ring which comprises 0, 1 or 2 identical or different ring heteroatoms selected from the series consisting of nitrogen, oxygen and sulfur, and which is unsubstituted or substituted by one or more identical or different substituents R8;
R6 and R7 are independently of one another selected from the series consisting of H and (C₁-C₄)-alkyl;
R8 is selected from the series consisting of halogen, (C₁-C₄)-alkyl, -O-(C₁-C₄)-alkyl and-CN;
R10 is selected from the series consisting of H and (C₁-C₄)-alkyl;
R11 and R12 are independently of one another selected from the series consisting of H, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl, wherein phenyl is unsubstituted or substituted by one or more identical or different substituents R50,
or R11 and R12, together with the nitrogen atom carrying them, form a 4-membered to 7-membered, monocyclic, saturated, heterocyclic group which, in addition to the nitrogen atom carrying R11 and R12, comprises 0 or 1 further ring heteroatom selected from the series consisting of nitrogen, oxygen and sulfur, and which is unsubstituted or substituted by one or more identical or different substituents selected from the series consisting of fluorine and (C₁-C₄)-alkyl;
R13 is selected from the series consisting of H, (C₁-C₄)-alkyl and (C₃-C₇)-cycloalkyl;
R14 and R16 are independently of one another selected from the series consisting of (C₁-C₈)-alkyl, (C₃-C₇)-cycloalkyl, -(C₁-C₄)-alkyl-(C₃-C₇)-cycloalkyl, phenyl, -(C₁-C₄)-alkylphenyl, Het2 and -(C₁-C₄)-alkyl-Het2, wherein (C₁-C₈)-alkyl and (C₃-C₇)-cycloalkyl all are unsubstituted or substituted by one or more identical or different substituents selected from the series consisting of-OH and -O-(C₁-C₄)-alkyl, and wherein phenyl and Het2 all are unsubstituted or substituted by one or more identical or different substituents R50;
R15 is selected from the series consisting of (C₁-C₈)-alkyl, phenyl and Het3, wherein phenyl and Het3 all are unsubstituted or substituted by one or more identical or different substituents R50;
R17 is selected from the series consisting of H and (C₁-C₄)-alkyl;
R30 is a 3-membered to 12-membered, monocyclic or bicyclic, saturated, partially unsaturated or aromatic, cyclic group which comprises 0, 1, 2 or 3 identical or different ring heteroatoms selected from the series consisting of nitrogen, oxygen and sulfur, which is unsubstituted or substituted by one or more identical or different substituents R32;
R31 is selected from the series consisting of halogen, -OH, -O-(C₁-C₄)-alkyl, -O-(C₃-C₇)-cycloalkyl, -O-(C₁-C₄)-alkyl-(C₃-C₇)-cycloalkyl, -N(R33)-R34, -CN and -C(O)-N(R35)-R36;
R32 is selected from the series consisting of halogen, (C₁-C₄)-alkyl, (C₃-C₇)-cycloalkyl,-(C₁-C₄)-alkyl-(C₃-C₇)-cycloalkyl, -(C₁-C₄)-alkyl-O-R37, -(C₁-C₄)-alkyl-N(R38)-R39, -(C₁-C₄)-alkyl-CN, -C(O)-(C₁-C₄)-alkyl, -CN, -OH, =O, -O-(C₁-C₄)-alkyl, -N(R40)-R41, -C(O)-O-(C₁-C₄)-alkyl and -C(O)-N(R42)-R43;
R33, R34, R35, R36, R37, R38, R39, R40, R41, R42 and R43 are independently of one another selected from the series consisting of H and (C₁-C₄)-alkyl;
R50 is selected from the series consisting of halogen, (C₁-C₄)-alkyl, -0-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen, oxygen and sulfur, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated, partially unsaturated or aromatic, heterocyclic group which comprises 1 or 2 identical or different ring heteroatoms selected from the series consisting of nitrogen, oxygen and sulfur, and is bonded via a ring carbon atom;
Het3 is a 5-membered or 6-membered, monocyclic, aromatic, heterocyclic group which comprises 1, 2 or 3 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

2. The compound of the formula I, in any of its stereoisomeric forms or a mixture of stereoisomeric forms in any ratio, or a pharmaceutically acceptable salt thereof, according to claim 1, for use in the treatment of sickle cell disease, wherein:
Ar is selected from the series consisting of phenyl and a 5-membered or 6-membered, monocyclic, aromatic, heterocyclic group which comprises 1 or 2 identical or different ring heteroatoms selected from the series consisting of nitrogen, oxygen and sulfur, and
is bonded via a ring carbon atom, which all are unsubstituted or substituted by one or more identical or different substituents R5;
n is selected from the series consisting of 0, 1 and 2;
X is selected from the series consisting of N and CH;
Z is selected from the series consisting of a direct bond, O, S and N(R10);
R1 is selected from the series consisting of H, -N(R11)-R12, -N(R13)-C(O)-R14, -N(R13)-S(O)₂-R15, -N(R13)-C(O)-NH-R16 and (C₁-C₄)-alkyl;
R2 is selected from the series consisting of halogen, (C₁-C₄)-alkyl, -0-(C₁-C₄)-alkyl and - CN;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, (C₃-C₇)-cycloalkyl, -O-(C₁-C₄)-alkyl, -O-(C₃-C₇)-cycloalkyl, -C(O)-N(R6)-R7 and -CN,
and two groups R5 bonded to adjacent ring carbon atoms in Ar, together with the carbon atoms carrying them, can form a 5-membered to 8-membered, monocyclic, unsaturated ring which comprises 0, 1 or 2 identical or different ring heteroatoms selected from the series consisting of nitrogen, oxygen and sulfur, and which is unsubstituted or substituted by one or more identical or different substituents R8;
R6 and R7 are independently of one another selected from the series consisting of H and (C₁-C₄)-alkyl;
R8 is selected from the series consisting of halogen and (C₁-C₄)-alkyl;
R10 is selected from the series consisting of H and (C₁-C₄)-alkyl;
R11 and R12 are independently of one another selected from the series consisting of H, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl, wherein phenyl is unsubstituted or substituted by one or more identical or different substituents R50;
R13 is selected from the series consisting of H and (C₁-C₄)-alkyl;
R14 and R16 are independently of one another selected from the series consisting of (C₁-C₈)-alkyl, (C₃-C₇)-cycloalkyl, -(C₁-C₄)-alkyl-(C₃-C₇)-cycloalkyl, phenyl, -(C₁-C₄)-alkylphenyl, Het2 and -(C₁-C₄)-alkyl-Het2, wherein (C₁-C₈)-alkyl and (C₃-C₇)-cycloalkyl all are unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R15 is selected from the series consisting of phenyl and Het3, wherein phenyl and Het3 all are unsubstituted or substituted by one or more identical or different substituents R50;
R30 is a 3-membered to 12-membered, monocyclic or bicyclic, saturated, partially unsaturated or aromatic, cyclic group which comprises 0, 1, 2 or 3 identical or different ring heteroatoms selected from the series consisting of nitrogen, oxygen and sulfur, which is unsubstituted or substituted by one or more identical or different substituents R32;
R31 is selected from the series consisting of halogen, -OH, -O-(C₁-C₄)-alkyl, -O-(C₃-C₇)-cycloalkyl, -O-(C₁-C₄)-alkyl-(C₃-C₇)-cycloalkyl, -N(R33)-R34 and -CN;
R32 is selected from the series consisting of halogen, (C₁-C₄)-alkyl, (C₃-C₇)-cycloalkyl, - (C₁-C₄)-alkyl-(C₃-C₇)-cycloalkyl, -(C₁-C₄)-alkyl-O-R37, -(C₁-C₄)-alkyl-N(R38)-R39, -(C₁-C₄)-alkyl-CN, -C(O)-(C₁-C₄)-alkyl, -CN, -OH, =O, -O-(C₁-C₄)-alkyl, -N(R40)-R41, -C(O)-O-(C₁-C₄)-alkyl and -C(O)-N(R42)-R43;
R33, R34, R37, R38, R39, R40, R41, R42 and R43 are independently of one another selected from the series consisting of H and (C₁-C₄)-alkyl;
R50 is selected from the series consisting of halogen, (C₁-C₄)-alkyl, -0-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen, oxygen and sulfur, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated, partially unsaturated or aromatic, heterocyclic group which comprises 1 or 2 identical or different ring heteroatoms selected from the series consisting of nitrogen, oxygen and sulfur, and is bonded via a ring carbon atom;
Het3 is a 5-membered or 6-membered, monocyclic, aromatic, heterocyclic group which comprises 1 or 2 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

3. The compound of the formula I, in any of its stereoisomeric forms or a mixture of stereoisomeric forms in any ratio, or a pharmaceutically acceptable salt thereof, according to claim 1 or 2, for use in the treatment of sickle cell disease, wherein
Ar is selected from the series consisting of phenyl and a 5-membered monocyclic, aromatic, heterocyclic group which comprises 1 or 2 identical or different ring heteroatoms selected from the series consisting of nitrogen and sulfur, and is bonded via a ring carbon atom, which all are unsubstituted or substituted by one or more identical or different substituents R5;
n is selected from the series consisting of 0, 1 and 2;
X is selected from the series consisting of N and CH;
Z is selected from the series consisting of a direct bond, O, S and N(R10);
R1 is selected from the series consisting of H, -N(R11)-R12, -N(R13)-C(O)-R14, -N(R13)-S(O)₂-R15, -N(R13)-C(O)-NH-R16 and (C₁-C₄)-alkyl;
R2 is selected from the series consisting of halogen, (C₁-C₄)-alkyl and -O-(C₁-C₄)-alkyl;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, (C₃-C₇)-cycloalkyl, -O-(C₁-C₄)-alkyl, -O-(C₃-C₇)-cycloalkyl and -CN,
and two groups R5 bonded to adjacent ring carbon atoms in Ar, together with the carbon atoms carrying them, can form a 5-membered to 7-membered, monocyclic, unsaturated ring which comprises 0, 1 or 2 oxygen atoms as ring heteroatoms, and which is unsubstituted or substituted by one or more identical or different substituents R8;
R8 is selected from the series consisting of halogen and (C₁-C₄)-alkyl;
R10 is selected from the series consisting of H and (C₁-C₄)-alkyl; one of the groups R11 and R12 is selected from the series consisting of hydrogen and (C₁-C₄)-alkyl, and the other of the groups R11 and R12 is selected from the series consisting of hydrogen, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl;
R13 is selected from the series consisting of H and (C₁-C₄)-alkyl;
R14 and R16 are independently of one another selected from the series consisting of (C₁-C₈)-alkyl, (C₃-C₇)-cycloalkyl, -(C₁-C₄)-alkyl-(C₃-C₇)-cycloalkyl, phenyl, -(C₁-C₄)-alkylphenyl, Het2 and -(C₁-C₄)-alkyl-Het2, wherein (C₁-C₈)-alkyl and (C₃-C₇)-cycloalkyl all are unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R15 is phenyl which is unsubstituted or substituted by one or more identical or different substituents R50;
R30 is a 3-membered to 12-membered, monocyclic or bicyclic, saturated, partially unsaturated or aromatic, cyclic group which comprises 0, 1, 2 or 3 identical or different ring heteroatoms selected from the series consisting of nitrogen and oxygen, which is unsubstituted or substituted by one or more identical or different substituents R32;
R31 is selected from the series consisting of halogen, -OH, -O-(C₁-C₄)-alkyl, -O-(C₃-C₇)-cycloalkyl, -O-(C₁-C₄)-alkyl-(C₃-C₇)-cycloalkyl and -N(R33)-R34;
R32 is selected from the series consisting of halogen, (C₁-C₄)-alkyl, (C₃-C₇)-cycloalkyl, - (C₁-C₄)-alkyl-(C₃-C₇)-cycloalkyl, -(C₁-C₄)-alkyl-O-R37, -(C₁-C₄)-alkyl-N(R38)-R39, -OH, =O, -O-(C₁-C₄)-alkyl and -N(R40)-R41;
R33, R34, R37, R38, R39, R40 and R41 are independently of one another selected from the series consisting of H and (C₁-C₄)-alkyl;
R50 is selected from the series consisting of halogen, (C₁-C₄)-alkyl, -0-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen and oxygen, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated, partially unsaturated or aromatic, heterocyclic group which comprises 1 or 2 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

4. The compound of the formula I, in any of its stereoisomeric forms or a mixture of stereoisomeric forms in any ratio, or a pharmaceutically acceptable salt thereof, according to any one of claims 1 to 3, for use in the treatment of sickle cell disease, wherein
Ar is phenyl, which is unsubstituted or substituted by one or more identical or different substituents R5;
n is selected from the series consisting of 0 and 1;
X is selected from the series consisting of N and CH;
Z is selected from the series consisting of a direct bond, O and N(R10);
R1 is selected from the series consisting of H, -N(R11)-R12, -N(R13)-C(O)-R14 and (C₁-C₄)-alkyl;
R2 is selected from the series consisting of halogen and -O-(C₁-C₄)-alkyl;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, -0-(C₁-C₄)-alkyl and - CN,
and two groups R5 bonded to adjacent ring carbon atoms in Ar, together with the carbon atoms carrying them, can form a 5-membered to 7-membered, monocyclic, unsaturated ring which comprises 0, 1 or 2 oxygen atoms as ring heteroatoms, and which is unsubstituted or substituted by one or more identical or different substituents R8;
R8 is selected from the series consisting of halogen and (C₁-C₄)-alkyl;
R10 is selected from the series consisting of H and (C₁-C₄)-alkyl;
one of the groups R11 and R12 is selected from the series consisting of hydrogen and (C₁-C₄)-alkyl, and the other of the groups R11 and R12 is selected from the series consisting of hydrogen, (C₁-C₄)-alkyl, -(C₁-C₄)-alkyl-(C₃-C₇)-cycloalkyl and -(C₁-C₄)-alkyl-Het1;
R13 is selected from the series consisting of H and (C₁-C₄)-alkyl;
R14 is selected from the series consisting of (C₃-C₇)-cycloalkyl, phenyl and Het2, wherein (C₃-C₇)-cycloalkyl is unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R30 is a 3-membered to 10-membered, monocyclic or bicyclic, saturated, partially unsaturated or aromatic, cyclic group which comprises 0, 1, 2 or 3 identical or different ring heteroatoms selected from the series consisting of nitrogen and oxygen, which is unsubstituted or substituted by one or more identical or different substituents R32;
R31 is selected from the series consisting of halogen, -OH, -O-(C₁-C₄)-alkyl, -O-(C₃-C₇)-cycloalkyl and -N(R33)-R34;
R32 is selected from the series consisting of halogen, (C₁-C₄)-alkyl, (C₃-C₇)-cycloalkyl,-(C₁-C₄)-alkyl-O-R37, -(C₁-C₄)-alkyl-N(R38)-R39, -OH, =O, -0-(C₁-C₄)-alkyl and -N(R40)-R41;
R33, R34, R37, R38, R39, R40 and R41 are independently of one another selected from the series consisting of H and (C₁-C₄)-alkyl;
R50 is selected from the series consisting of halogen, (C₁-C₄)-alkyl, -0-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen and oxygen, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated or aromatic, heterocyclic group which comprises 1 or 2 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

5. The compound of the formula I, in any of its stereoisomeric forms or a mixture of stereoisomeric forms in any ratio, or a pharmaceutically acceptable salt thereof, according to any one of claims 1 to 4, for use in the treatment of sickle cell disease, wherein,
Ar is phenyl, which is unsubstituted or substituted by one or more identical or different substituents R5;
n is selected from the series consisting of 0 and 1;
X is selected from the series consisting of N and CH;
Z is selected from the series consisting of a direct bond and O;
R1 is selected from the series consisting of H, -N(R11)-R12 and (C₁-C₄)-alkyl;
R2 is selected from the series consisting of halogen;
R3 is selected from the series consisting of H, R30 and -(C₁-C₄)-alkyl-R30;
R5 is selected from the series consisting of halogen, (C₁-C₄)-alkyl, -0-(C₁-C₄)-alkyl and - CN;
R11 and R12 are independently of one another selected from the series consisting of hydrogen and (C₁-C₄)-alkyl;
R30 is a 3-membered to 7-membered, monocyclic, saturated or aromatic, cyclic group which comprises 0, 1 or 2 identical or different ring heteroatoms selected from the series consisting of nitrogen and oxygen, which is unsubstituted or substituted by one or more identical or different substituents R32;
R32 is selected from the series consisting of halogen, (C₁-C₄)-alkyl, (C₃-C₇)-cycloalkyl, - (C₁-C₄)-alkyl-O-R37, -(C₁-C₄)-alkyl-N(R38)-R39, -OH and =O;
R37, R38 and R39 are independently of one another selected from the series consisting of H and (C₁-C₄)-alkyl;
wherein all cycloalkyl groups can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

6. The compound of the formula I, in any of its stereoisomeric forms or a mixture of stereoisomeric forms in any ratio, or a pharmaceutically acceptable salt thereof, according to any one of claims 1 to 5, for use in the treatment of sickle cell disease, wherein Z is a direct bond.

7. The compound of the formula I, in any of its stereoisomeric forms or a mixture of stereoisomeric forms in any ratio, or a pharmaceutically acceptable salt thereof, according to any one of claims 1 to 5, for use in the treatment of sickle cell disease, wherein Z is O.

8. The compound of the formula I, in any of its stereoisomeric forms or a mixture of stereoisomeric forms in any ratio, or a pharmaceutically acceptable salt thereof, according to any one of claims 1 to 7, for use in the treatment of sickle cell disease, wherein X is N.

9. The compound of the formula I, in any of its stereoisomeric forms or a mixture of stereoisomeric forms in any ratio, or a pharmaceutically acceptable salt thereof, according to any one of claims 1 to 7, for use in the treatment of sickle cell disease, wherein X is CH.

10. The compound of the formula I according to claim 1, for use in the treatment of sickle cell disease, which is selected from the group consisting of:
N-[4-(3-Amino-4-cyclopropyl-1H-pyrazolo[3,4-d]pyrimidin-6-yl)-phenyl]-2-cyano-5-methoxy-benzenesulfonamide,
N-[4-(3-Amino-1H-pyrazolo[3,4-d]pyrimidin-6-yl)-phenyl]-2,5-difluoro-benzenesulfonamide,
N-[4-(3-Amino-1H-pyrazolo[3,4-d]pyrimidin-6-yl)-phenyl]-5-chloro-2-cyano-benzenesulfonamide,
2-Chloro-N-{4-[4-(1-ethyl-piperidin-3-yloxy)-3-methyl-1H-pyrazolo[3,4-d]pyrimidin-6-yl]-phenyl}-5-methoxy-benzenesulfonamide,
5-Chloro-N-{4-[4-(1-ethyl-piperidin-3-yloxy)-3-methyl-1H-pyrazolo[3,4-d]pyrimidin-6-yl]-phenyl}-2-fluoro-benzenesulfonamide,
4-{6-[4-(2,5-Difluoro-benzenesulfonylamino)-phenyl]-3-methyl-1H-pyrazolo[3,4-d]pyrimidin-4-yloxy}-piperidine-1-carboxylic acid ethyl ester,
N-[4-(3-Amino-4-propoxy-1H-pyrazolo[3,4-d]pyrimidin-6-yl)-phenyl]-2,5-difluoro-benzenesulfonamide,
N-[4-(3-Amino-4-ethoxy-1H-pyrazolo[3,4-d]pyrimidin-6-yl)-phenyl]-5-chloro-2-fluoro-benzenesulfonamide,
N-[4-(3-Amino-4-propoxy-1H-pyrazolo[3,4-d]pyrimidin-6-yl)-phenyl]-5-chloro-2-fluoro-benzenesulfonamide,
N-[4-(3-Amino-4-ethoxy-1H-pyrazolo[3,4-d]pyrimidin-6-yl)-phenyl]-2,5-difluoro-benzenesulfonamide,
2-Fluoro-N-(4-{4-[1-(2-methoxy-ethyl)-piperidin-4-yloxy]-3-methyl-1H-pyrazolo[3,4-d]pyrimidin-6-yl}-phenyl)-5-methyl-benzenesulfonamide,
2,5-Difluoro-N-(4-{4-[1-(2-methoxy-ethyl)-piperidin-4-yloxy]-3-methyl-1H-pyrazolo[3,4-d]pyrimidin-6-yl}-phenyl)-benzenesulfonamide,
5-Chloro-2-fluoro-N-(4-{4-[1-(2-methoxy-ethyl)-piperidin-4-yloxy]-3-methyl-1H-pyrazolo[3,4-d]pyrimidin-6-yl}-phenyl)-benzenesulfonamide,
N-{4-[4-(1-Ethyl-piperidin-4-yloxy)-3-methyl-1H-pyrazolo[3,4-d]pyrimidin-6-yl]-phenyl}-2-fluoro-5-methoxy-benzenesulfonamide,
2,5-Dichloro-N-{4-[4-(1-ethyl-piperidin-4-yloxy)-3-methyl-1H-pyrazolo[3,4-d]pyrimidin-6-yl]-phenyl}-benzenesulfonamide,
N-{4-[4-(1-Ethyl-piperidin-4-yloxy)-3-methyl-1H-pyrazolo[3,4-d]pyrimidin-6-yl]-phenyl}-2-fluoro-5-methyl-benzenesulfonamide,
N-{4-[4-(1-Ethyl-piperidin-4-yloxy)-3-methyl-1H-pyrazolo[3,4-d]pyrimidin-6-yl]-phenyl}-2-fluoro-benzenesulfonamide,
5-Chloro-N-{4-[4-(1-ethyl-piperidin-4-yloxy)-3-methyl-1H-pyrazolo[3,4-d]pyrimidin-6-yl]-phenyl}-2-fluoro-benzenesulfonamide,
N-{4-[4-(1-Cyclobutyl-piperidin-4-yloxy)-3-methyl-1H-pyrazolo[3,4-d]pyrimidin-6-yl]-phenyl}-2,5-difluoro-benzenesulfonamide,
2,5-Difluoro-N-(4-{4-[1-(3-methoxy-propyl)-piperidin-4-yloxy]-3-methyl-1H-pyrazolo[3,4-d]pyrimidin-6-yl}-phenyl)-benzenesulfonamide,
5-Chloro-2-fluoro-N-{4-[4-(3-hydroxy-propoxy)-3-methyl-1H-pyrazolo[3,4-d]pyrimidin-6-yl]-phenyl}-benzenesulfonamide,
2,5-Difluoro-N-{4-[4-(1-isopropyl-piperidin-4-yloxy)-3-methyl-1H-pyrazolo[3,4-d]pyrimidin-6-yl]-phenyl}-benzenesulfonamide,
2-Fluoro-N-(4-{4-[1-(2-fluoro-ethyl)-piperidin-4-yloxy]-3-methyl-1H-pyrazolo[3,4-d]pyrimidin-6-yl}-phenyl)-benzenesulfonamide,
5-Chloro-2-fluoro-N-{4-[4-(1-isopropyl-piperidin-4-yloxy)-3-methyl-1H-pyrazolo[3,4-d]pyrimidin-6-yl]-phenyl}-benzenesulfonamide,
2,5-Difluoro-N-(4-{4-[1-(2-fluoro-ethyl)-piperidin-4-yloxy]-3-methyl-1H-pyrazolo[3,4-d]pyrimidin-6-yl}-phenyl)-benzenesulfonamide,
N-[4-(3-Amino-4-isopropoxy-1H-pyrazolo[3,4-d]pyrimidin-6-yl)-phenyl]-2,5-dichloro-benzenesulfonamide,
N-[4-(3-Amino-4-isobutoxy-1H-pyrazolo[3,4-d]pyrimidin-6-yl)-phenyl]-2,5-difluoro-benzenesulfonamide,
N-[4-(3-Amino-4-isobutoxy-1H-pyrazolo[3,4-d]pyrimidin-6-yl)-phenyl]-2-fluoro-5-methoxy-benzenesulfonamide,
2,5-Dichloro-N-{4-[3-methyl-4-(piperidin-3-yloxy)-1H-pyrazolo[3,4-d]pyrimidin-6-yl]-phenyl}-benzenesulfonamide,
2,5-Difluoro-N-{4-[3-methyl-4-(piperidin-3-yloxy)-1H-pyrazolo[3,4-d]pyrimidin-6-yl]-phenyl}-benzenesulfonamide,
2-Fluoro-5-methyl-N-{4-[3-methyl-4-(morpholin-2-ylmethoxy)-1H-pyrazolo[3,4-d]pyrimidin-6-yl]-phenyl}-benzenesulfonamide,
N-{4-[4-(3-Aminomethyl-oxetan-3-ylmethoxy)-1H-pyrazolo[3,4-d]pyrimidin-6-yl]-phenyl}-5-chloro-2-fluoro-benzenesulfonamide,
N-[4-(3-Amino-4-ethoxymethyl-1H-pyrazolo[3,4-d]pyrimidin-6-yl)-phenyl]-2-fluoro-5-methyl-benzenesulfonamide,
N-[4-(3-Amino-4-trifluoromethyl-1H-pyrazolo[3,4-d]pyrimidin-6-yl)-phenyl]-2,5-difluoro-benzenesulfonamide,
2-Fluoro-N-{4-[4-(piperidin-4-yloxy)-1H-pyrazolo[3,4-d]pyrimidin-6-yl]-phenyl}-benzenesulfonamide,
N-[4-(3-Amino-1H-pyrazolo[3,4-d]pyrimidin-6-yl)-phenyl]-2-fluoro-5-methoxy-benzenesulfonamide,
N-[4-(3-Amino-4-methoxymethyl-1H-pyrazolo[3,4-d]pyrimidin-6-yl)-phenyl]-5-chloro-2-fluoro-benzenesulfonamide,
N-{4-[4-(3-Amino-propoxy)-3-methyl-1H-pyrazolo[3,4-d]pyrimidin-6-yl]-phenyl}-5-chloro-2-fluoro-benzenesulfonamide,
N-[4-(3-Amino-1H-pyrazolo[3,4-d]pyrimidin-6-yl)-phenyl]-2,5-difluoro-benzenesulfonamide,
N-[4-(3-Amino-1H-pyrazolo[3,4-d]pyrimidin-6-yl)-phenyl]-2,4,5-trifluoro-benzenesulfonamide,
N-[4-(3-Amino-1H-pyrazolo[3,4-d]pyrimidin-6-yl)-phenyl]-2-chloro-4,5-difluoro-benzenesulfonamide,
N-{4-[3-Amino-4-(2,2,2-trifluoro-ethoxy)-1H-pyrazolo[3,4-d]pyrimidin-6-yl]-phenyl}-2-cyano-5-methyl-benzenesulfonamide,
N-[4-(3-Amino-4-trifluoromethyl-1H-pyrazolo[3,4-d]pyrimidin-6-yl)-phenyl]-5-chloro-2-fluoro-benzenesulfonamide,
N-{4-[3-Amino-4-(2-methoxy-ethyl)-1H-pyrazolo[3,4-d]pyrimidin-6-yl]-phenyl}-2-cyano-5-methyl-benzenesulfonamide,
2-Cyano-5-methyl-N-{4-[4-(2,2,2-trifluoro-ethoxy)-1H-pyrazolo[3,4-d]pyrimidin-6-yl]-phenyl}-benzenesulfonamide,
N-[4-(3-Amino-4-cyclopropyl-1H-pyrazolo[3,4-d]pyrimidin-6-yl)-phenyl]-2,4,5-trifluoro-benzenesulfonamide,
N-[4-(3-Amino-4-cyclopropyl-1H-pyrazolo[3,4-d]pyrimidin-6-yl)-phenyl]-2-fluoro-benzenesulfonamide,
N-[4-(3-Amino-4-cyclopropyl-1H-pyrazolo[3,4-d]pyrimidin-6-yl)-phenyl]-2,5-difluoro-benzenesulfonamide,
N-[4-(3-Amino-4-methoxy-1H-pyrazolo[3,4-d]pyrimidin-6-yl)-phenyl]-5-chloro-2-fluoro-benzenesulfonamide,
N-[4-(3-Amino-4-methoxy-1H-pyrazolo[3,4-d]pyrimidin-6-yl)-phenyl]-5-chloro-2-cyano-benzenesulfonamide,
N-[4-(3-Amino-4-methyl-1H-pyrazolo[3,4-d]pyrimidin-6-yl)-phenyl]-2-chloro-3,5-difluoro-benzenesulfonamide,
2-Cyano-N-{4-[4-(4-hydroxy-cyclohexyloxy)-1H-pyrazolo[3,4-d]pyrimidin-6-yl]-phenyl}-5-methoxy-benzenesulfonamide,
N-[4-(3-Amino-1H-pyrazolo[4,3-c]pyridin-6-yl)-phenyl]-5-chloro-2,4-difluoro-benzenesulfonamide,
5-Chloro-2-cyano-N-{4-[4-(4-hydroxy-cyclohexyloxy)-3-methyl-1H-pyrazolo[3,4-d]pyrimidin-6-yl]-phenyl}-benzenesulfonamide,
N-{4-[4-(1-Cyclopropyl-piperidin-4-yloxy)-3-methyl-1H-pyrazolo[3,4-d]pyrimidin-6-yl]-phenyl}-2,5-difluoro-benzenesulfonamide,
5-Chloro-N-{4-[4-(1-cyclopropyl-piperidin-4-yloxy)-3-methyl-1H-pyrazolo[3,4-d]pyrimidin-6-yl]-phenyl}-2-fluoro-benzenesulfonamide,
N-{4-[4-(1-Acetyl-piperidin-4-yloxy)-3-methyl-1H-pyrazolo[3,4-d]pyrimidin-6-yl]-phenyl}-2-fluoro-5-methoxy-benzenesulfonamide,
N-{4-[4-(1-Acetyl-piperidin-4-yloxy)-3-methyl-1H-pyrazolo[3,4-d]pyrimidin-6-yl]-phenyl}-2,5-difluoro-benzenesulfonamide,
N-{4-[4-(1-Acetyl-piperidin-4-yloxy)-3-methyl-1H-pyrazolo[3,4-d]pyrimidin-6-yl]-phenyl}-5-chloro-2-fluoro-benzenesulfonamide,
5-Chloro-2-fluoro-N-{4-[4-(6-hydroxy-pyridin-3-yloxy)-3-methyl-1H-pyrazolo[3,4-d]pyrimidin-6-yl]-phenyl}-benzenesulfonamide, and
2-Fluoro-N-{4-[4-(6-hydroxy-pyridin-3-yloxy)-3-methyl-1H-pyrazolo[3,4-d]pyrimidin-6-yl]-phenyl}-5-methyl-benzenesulfonamide,
or a pharmaceutically acceptable salt thereof.

11. The compound of the formula I according to claim 1, for use in the treatment of sickle cell disease, which is 5-Chloro-2-fluoro-N-{4-[4-(1-isopropyl-piperidin-4-yloxy)-3-methyl-1H-pyrazolo[3,4-d]pyrimidin-6-yl]-phenyl}-benzenesulfonamide: 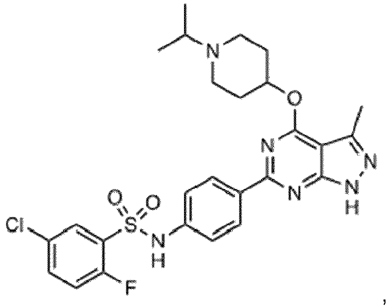 or a pharmaceutically acceptable salt thereof.

12. The compound of the formula I according to any one of claims 1 to 11, or a pharmaceutically acceptable salt thereof, for use in the treatment of sickle cell disease in combination with at least one additional therapeutic agent.

13. The compound of the formula I according to claim 12, or a pharmaceutically acceptable salt thereof, for use in the treatment of sickle cell disease, wherein the at least one additional therapeutic agent is selected from the group consisting of hydroxyurea, L-glutamine, voxelotor and crizanlizumab.

14. A pharmaceutical composition, comprising the compound of any one of claims 1 to 13 or a pharmaceutically acceptable salt thereof, and a pharmaceutically acceptable carrier, for use in the treatment of sickle cell disease.

15. Use of a compound of the formula I, in any of its stereoisomeric forms or a mixture of stereoisomeric forms in any ratio, or a pharmaceutically acceptable salt thereof, 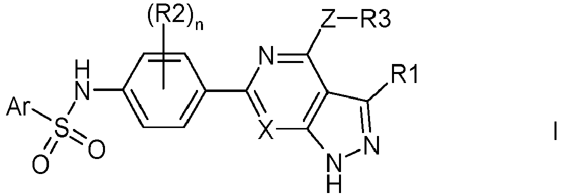 in the treatment of sickle cell disease,
wherein
Ar is selected from the series consisting of phenyl and a 5-membered or 6-membered, monocyclic, aromatic, heterocyclic group which comprises 1, 2 or 3 identical or different ring heteroatoms selected from the series consisting of nitrogen, oxygen and sulfur, and is bonded via a ring carbon atom, which all are unsubstituted or substituted by one or more identical or different substituents R5;
n is selected from the series consisting of 0, 1 and 2;
X is selected from the series consisting of N and CH;
Z is selected from the series consisting of a direct bond, O, S and N(R10);
R1 is selected from the series consisting of H, -N(R11)-R12, -N(R13)-C(O)-R14, -N(R13)-S(O)₂-R15, -N(R13)-C(O)-NH-R16, (C₁-C₄)-alkyl and -(C₁-C₄)-alkyl-O-R17;
R2 is selected from the series consisting of halogen, (C₁-C₄)-alkyl, -O-(C₁-C₄)-alkyl and - CN;
R3 is selected from the series consisting of H, (C₁-C₈)-alkyl, R30 and -(C₁-C₄)-alkyl-R30,
wherein (C₁-C₈)-alkyl is unsubstituted or substituted by one or more identical or different substituents R31;
R5 is selected from the series consisting of halogen, (C₁-C₄)-alkyl, (C₃-C₇)-cycloalkyl,-(C₁-C₄)-alkyl-(C₃-C₇)-cycloalkyl, -O-(C₁-C₄)-alkyl, -O-(C₃-C₇)-cycloalkyl, -O-(C₁-C₄)-alkyl-(C₃-C₇)-cycloalkyl, -C(O)-N(R6)-R7 and -CN,
and two groups R5 bonded to adjacent ring carbon atoms in Ar, together with the carbon atoms carrying them, can form a 5-membered to 8-membered, monocyclic, unsaturated ring which comprises 0, 1 or 2 identical or different ring heteroatoms selected from the series consisting of nitrogen, oxygen and sulfur, and which is unsubstituted or substituted by one or more identical or different substituents R8;
R6 and R7 are independently of one another selected from the series consisting of H and (C₁-C₄)-alkyl;
R8 is selected from the series consisting of halogen, (C₁-C₄)-alkyl, -O-(C₁-C₄)-alkyl and-CN;
R10 is selected from the series consisting of H and (C₁-C₄)-alkyl;
R11 and R12 are independently of one another selected from the series consisting of H, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl, wherein phenyl is unsubstituted or substituted by one or more identical or different substituents R50,
or R11 and R12, together with the nitrogen atom carrying them, form a 4-membered to 7-membered, monocyclic, saturated, heterocyclic group which, in addition to the nitrogen atom carrying R11 and R12, comprises 0 or 1 further ring heteroatom selected from the series consisting of nitrogen, oxygen and sulfur, and which is unsubstituted or substituted by one or more identical or different substituents selected from the series consisting of fluorine and (C₁-C₄)-alkyl;
R13 is selected from the series consisting of H, (C₁-C₄)-alkyl and (C₃-C₇)-cycloalkyl;
R14 and R16 are independently of one another selected from the series consisting of (C₁-C₈)-alkyl, (C₃-C₇)-cycloalkyl, -(C₁-C₄)-alkyl-(C₃-C₇)-cycloalkyl, phenyl, -(C₁-C₄)-alkylphenyl, Het2 and -(C₁-C₄)-alkyl-Het2, wherein (C₁-C₈)-alkyl and (C₃-C₇)-cycloalkyl all are unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R15 is selected from the series consisting of (C₁-C₈)-alkyl, phenyl and Het3, wherein phenyl and Het3 all are unsubstituted or substituted by one or more identical or different substituents R50;
R17 is selected from the series consisting of H and (C₁-C₄)-alkyl;
R30 is a 3-membered to 12-membered, monocyclic or bicyclic, saturated, partially unsaturated or aromatic, cyclic group which comprises 0, 1, 2 or 3 identical or different ring heteroatoms selected from the series consisting of nitrogen, oxygen and sulfur, which is unsubstituted or substituted by one or more identical or different substituents R32;
R31 is selected from the series consisting of halogen, -OH, -O-(C₁-C₄)-alkyl, -O-(C₃-C₇)-cycloalkyl, -O-(C₁-C₄)-alkyl-(C₃-C₇)-cycloalkyl, -N(R33)-R34, -CN and -C(O)-N(R35)-R36;
R32 is selected from the series consisting of halogen, (C₁-C₄)-alkyl, (C₃-C₇)-cycloalkyl, - (C₁-C₄)-alkyl-(C₃-C₇)-cycloalkyl, -(C₁-C₄)-alkyl-O-R37, -(C₁-C₄)-alkyl-N(R38)-R39, -(C₁-C₄)-alkyl-CN, -C(O)-(C₁-C₄)-alkyl, -CN, -OH, =O, -O-(C₁-C₄)-alkyl, -N(R40)-R41, -C(O)-O-(C₁-C₄)-alkyl and -C(O)-N(R42)-R43;
R33, R34, R35, R36, R37, R38, R39, R40, R41, R42 and R43 are independently of one another selected from the series consisting of H and (C₁-C₄)-alkyl;
R50 is selected from the series consisting of halogen, (C₁-C₄)-alkyl, -O-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen, oxygen and sulfur, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated, partially unsaturated or aromatic, heterocyclic group which comprises 1 or 2 identical or different ring heteroatoms selected from the series consisting of nitrogen, oxygen and sulfur, and is bonded via a ring carbon atom;
Het3 is a 5-membered or 6-membered, monocyclic, aromatic, heterocyclic group which comprises 1, 2 or 3 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

16. The Use according to claim 15, wherein:
Ar is selected from the series consisting of phenyl and a 5-membered or 6-membered, monocyclic, aromatic, heterocyclic group which comprises 1 or 2 identical or different ring heteroatoms selected from the series consisting of nitrogen, oxygen and sulfur, and is bonded via a ring carbon atom, which all are unsubstituted or substituted by one or more identical or different substituents R5;
n is selected from the series consisting of 0, 1 and 2;
X is selected from the series consisting of N and CH;
Z is selected from the series consisting of a direct bond, O, S and N(R10);
R1 is selected from the series consisting of H, -N(R11)-R12, -N(R13)-C(O)-R14, -N(R13)-S(O)₂-R15, -N(R13)-C(O)-NH-R16 and (C₁-C₄)-alkyl;
R2 is selected from the series consisting of halogen, (C₁-C₄)-alkyl, -O-(C₁-C₄)-alkyl and-CN;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, (C₃-C₇)-cycloalkyl, -O-(C₁-C₄)-alkyl, -O-(C₃-C₇)-cycloalkyl, -C(O)-N(R6)-R7 and -CN,
and two groups R5 bonded to adjacent ring carbon atoms in Ar, together with the carbon atoms carrying them, can form a 5-membered to 8-membered, monocyclic, unsaturated ring which comprises 0, 1 or 2 identical or different ring heteroatoms selected from the series consisting of nitrogen, oxygen and sulfur, and which is unsubstituted or substituted by one or more identical or different substituents R8;
R6 and R7 are independently of one another selected from the series consisting of H and (C₁-C₄)-alkyl;
R8 is selected from the series consisting of halogen and (C₁-C₄)-alkyl;
R10 is selected from the series consisting of H and (C₁-C₄)-alkyl;
R11 and R12 are independently of one another selected from the series consisting of H, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl, wherein phenyl is unsubstituted or substituted by one or more identical or different substituents R50;
R13 is selected from the series consisting of H and (C₁-C₄)-alkyl;
R14 and R16 are independently of one another selected from the series consisting of (C₁-C₈)-alkyl, (C₃-C₇)-cycloalkyl, -(C₁-C₄)-alkyl-(C₃-C₇)-cycloalkyl, phenyl, -(C₁-C₄)-alkylphenyl, Het2 and -(C₁-C₄)-alkyl-Het2, wherein (C₁-C₈)-alkyl and (C₃-C₇)-cycloalkyl all are unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R15 is selected from the series consisting of phenyl and Het3, wherein phenyl and Het3 all are unsubstituted or substituted by one or more identical or different substituents R50;
R30 is a 3-membered to 12-membered, monocyclic or bicyclic, saturated, partially unsaturated or aromatic, cyclic group which comprises 0, 1, 2 or 3 identical or different ring heteroatoms selected from the series consisting of nitrogen, oxygen and sulfur, which is unsubstituted or substituted by one or more identical or different substituents R32;
R31 is selected from the series consisting of halogen, -OH, -O-(C₁-C₄)-alkyl, -O-(C₃-C₇)-cycloalkyl, -O-(C₁-C₄)-alkyl-(C₃-C₇)-cycloalkyl, -N(R33)-R34 and -CN;
R32 is selected from the series consisting of halogen, (C₁-C₄)-alkyl, (C₃-C₇)-cycloalkyl,-(C₁-C₄)-alkyl-(C₃-C₇)-cycloalkyl, -(C₁-C₄)-alkyl-O-R37, -(C₁-C₄)-alkyl-N(R38)-R39, -(C₁-C₄)-alkyl-CN, -C(O)-(C₁-C₄)-alkyl, -CN, -OH, =O, -O-(C₁-C₄)-alkyl, -N(R40)-R41, -C(O)-O-(C₁-C₄)-alkyl and -C(O)-N(R42)-R43;
R33, R34, R37, R38, R39, R40, R41, R42 and R43 are independently of one another selected from the series consisting of H and (C₁-C₄)-alkyl;
R50 is selected from the series consisting of halogen, (C₁-C₄)-alkyl, -O-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen, oxygen and sulfur, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated, partially unsaturated or aromatic, heterocyclic group which comprises 1 or 2 identical or different ring heteroatoms selected from the series consisting of nitrogen, oxygen and sulfur, and is bonded via a ring carbon atom;
Het3 is a 5-membered or 6-membered, monocyclic, aromatic, heterocyclic group which comprises 1 or 2 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

17. The use according to claim 15 or 16, wherein
Ar is selected from the series consisting of phenyl and a 5-membered monocyclic, aromatic, heterocyclic group which comprises 1 or 2 identical or different ring heteroatoms selected from the series consisting of nitrogen and sulfur, and is bonded via a ring carbon atom, which all are unsubstituted or substituted by one or more identical or different substituents R5;
n is selected from the series consisting of 0, 1 and 2;
X is selected from the series consisting of N and CH;
Z is selected from the series consisting of a direct bond, O, S and N(R10);
R1 is selected from the series consisting of H, -N(R11)-R12, -N(R13)-C(O)-R14, -N(R13)-S(O)₂-R15, -N(R13)-C(O)-NH-R16 and (C₁-C₄)-alkyl;
R2 is selected from the series consisting of halogen, (C₁-C₄)-alkyl and -O-(C₁-C₄)-alkyl;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, (C₃-C₇)-cycloalkyl, -O-(C₁-C₄)-alkyl, -O-(C₃-C₇)-cycloalkyl and -CN,
and two groups R5 bonded to adjacent ring carbon atoms in Ar, together with the carbon atoms carrying them, can form a 5-membered to 7-membered, monocyclic, unsaturated ring which comprises 0, 1 or 2 oxygen atoms as ring heteroatoms, and which is unsubstituted or substituted by one or more identical or different substituents R8;
R8 is selected from the series consisting of halogen and (C₁-C₄)-alkyl;
R10 is selected from the series consisting of H and (C₁-C₄)-alkyl;
one of the groups R11 and R12 is selected from the series consisting of hydrogen and (C₁-C₄)-alkyl, and the other of the groups R11 and R12 is selected from the series consisting of hydrogen, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl;
R13 is selected from the series consisting of H and (C₁-C₄)-alkyl;
R14 and R16 are independently of one another selected from the series consisting of (C₁-C₈)-alkyl, (C₃-C₇)-cycloalkyl, -(C₁-C₄)-alkyl-(C₃-C₇)-cycloalkyl, phenyl, -(C₁-C₄)-alkylphenyl, Het2 and -(C₁-C₄)-alkyl-Het2, wherein (C₁-C₈)-alkyl and (C₃-C₇)-cycloalkyl all are unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R15 is phenyl which is unsubstituted or substituted by one or more identical or different substituents R50;
R30 is a 3-membered to 12-membered, monocyclic or bicyclic, saturated, partially unsaturated or aromatic, cyclic group which comprises 0, 1, 2 or 3 identical or different ring heteroatoms selected from the series consisting of nitrogen and oxygen, which is unsubstituted or substituted by one or more identical or different substituents R32;
R31 is selected from the series consisting of halogen, -OH, -O-(C₁-C₄)-alkyl, -O-(C₃-C₇)-cycloalkyl, -O-(C₁-C₄)-alkyl-(C₃-C₇)-cycloalkyl and -N(R33)-R34;
R32 is selected from the series consisting of halogen, (C₁-C₄)-alkyl, (C₃-C₇)-cycloalkyl, - (C₁-C₄)-alkyl-(C₃-C₇)-cycloalkyl, -(C₁-C₄)-alkyl-O-R37, -(C₁-C₄)-alkyl-N(R38)-R39, -OH, =O, -O-(C₁-C₄)-alkyl and -N(R40)-R41;
R33, R34, R37, R38, R39, R40 and R41 are independently of one another selected from the series consisting of H and (C₁-C₄)-alkyl;
R50 is selected from the series consisting of halogen, (C₁-C₄)-alkyl, -O-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen and oxygen, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated, partially unsaturated or aromatic, heterocyclic group which comprises 1 or 2 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

18. The use according to any one of claims 15 to 17, wherein
Ar is phenyl, which is unsubstituted or substituted by one or more identical or different substituents R5;
n is selected from the series consisting of 0 and 1;
X is selected from the series consisting of N and CH;
Z is selected from the series consisting of a direct bond, O and N(R10);
R1 is selected from the series consisting of H, -N(R11)-R12, -N(R13)-C(O)-R14 and (C₁-C₄)-alkyl;
R2 is selected from the series consisting of halogen and -O-(C₁-C₄)-alkyl;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, -O-(C₁-C₄)-alkyl and - CN,
and two groups R5 bonded to adjacent ring carbon atoms in Ar, together with the carbon atoms carrying them, can form a 5-membered to 7-membered, monocyclic, unsaturated ring which comprises 0, 1 or 2 oxygen atoms as ring heteroatoms, and which is unsubstituted or substituted by one or more identical or different substituents R8;
R8 is selected from the series consisting of halogen and (C₁-C₄)-alkyl;
R10 is selected from the series consisting of H and (C₁-C₄)-alkyl;
one of the groups R11 and R12 is selected from the series consisting of hydrogen and (C₁-C₄)-alkyl, and the other of the groups R11 and R12 is selected from the series consisting of hydrogen, (C₁-C₄)-alkyl, -(C₁-C₄)-alkyl-(C₃-C₇)-cycloalkyl and -(C₁-C₄)-alkyl-Het1;
R13 is selected from the series consisting of H and (C₁-C₄)-alkyl;
R14 is selected from the series consisting of (C₃-C₇)-cycloalkyl, phenyl and Het2, wherein (C₃-C₇)-cycloalkyl is unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R30 is a 3-membered to 10-membered, monocyclic or bicyclic, saturated, partially unsaturated or aromatic, cyclic group which comprises 0, 1, 2 or 3 identical or different ring heteroatoms selected from the series consisting of nitrogen and oxygen, which is unsubstituted or substituted by one or more identical or different substituents R32;
R31 is selected from the series consisting of halogen, -OH, -O-(C₁-C₄)-alkyl, -O-(C₃-C₇)-cycloalkyl and -N(R33)-R34;
R32 is selected from the series consisting of halogen, (C₁-C₄)-alkyl, (C₃-C₇)-cycloalkyl, - (C₁-C₄)-alkyl-O-R37, -(C₁-C₄)-alkyl-N(R38)-R39, -OH, =O, -O-(C₁-C₄)-alkyl and -N(R40)-R41;
R33, R34, R37, R38, R39, R40 and R41 are independently of one another selected from the series consisting of H and (C₁-C₄)-alkyl;
R50 is selected from the series consisting of halogen, (C₁-C₄)-alkyl, -O-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen and oxygen, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated or aromatic, heterocyclic group which comprises 1 or 2 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

19. The use according to any one of claims 15 to 18, wherein
Ar is phenyl, which is unsubstituted or substituted by one or more identical or different substituents R5;
n is selected from the series consisting of 0 and 1;
X is selected from the series consisting of N and CH;
Z is selected from the series consisting of a direct bond and O;
R1 is selected from the series consisting of H, -N(R11)-R12 and (C₁-C₄)-alkyl;
R2 is selected from the series consisting of halogen;
R3 is selected from the series consisting of H, R30 and -(C₁-C₄)-alkyl-R30;
R5 is selected from the series consisting of halogen, (C₁-C₄)-alkyl, -O-(C₁-C₄)-alkyl and - CN;
R11 and R12 are independently of one another selected from the series consisting of hydrogen and (C₁-C₄)-alkyl;
R30 is a 3-membered to 7-membered, monocyclic, saturated or aromatic, cyclic group which comprises 0, 1 or 2 identical or different ring heteroatoms selected from the series consisting of nitrogen and oxygen, which is unsubstituted or substituted by one or more identical or different substituents R32;
R32 is selected from the series consisting of halogen, (C₁-C₄)-alkyl, (C₃-C₇)-cycloalkyl, - (C₁-C₄)-alkyl-O-R37, -(C₁-C₄)-alkyl-N(R38)-R39, -OH and =O;
R37, R38 and R39 are independently of one another selected from the series consisting of H and (C₁-C₄)-alkyl;
wherein all cycloalkyl groups can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

20. The use according to any one of claims 15 to 19, wherein Z is a direct bond.

21. The use according to any one of claims 15 to 19, wherein Z is O.

22. The use according to any one of claims 15 to 21, wherein X is N.

23. The use according to any one of claims 15 to 21, wherein X is CH.

24. The use according to claim 15, wherein the compound of the formula I is selected from the group consisting of:
N-[4-(3-Amino-4-cyclopropyl-1H-pyrazolo[3,4-d]pyrimidin-6-yl)-phenyl]-2-cyano-5-methoxy-benzenesulfonamide,
N-[4-(3-Amino-1H-pyrazolo[3,4-d]pyrimidin-6-yl)-phenyl]-2,5-difluoro-benzenesulfonamide,
N-[4-(3-Amino-1H-pyrazolo[3,4-d]pyrimidin-6-yl)-phenyl]-5-chloro-2-cyano-benzenesulfonamide,
2-Chloro-N-{4-[4-(1-ethyl-piperidin-3-yloxy)-3-methyl-1H-pyrazolo[3,4-d]pyrimidin-6-yl]-phenyl}-5-methoxy-benzenesulfonamide,
5-Chloro-N-{4-[4-(1-ethyl-piperidin-3-yloxy)-3-methyl-1H-pyrazolo[3,4-d]pyrimidin-6-yl]-phenyl}-2-fluoro-benzenesulfonamide,
4-{6-[4-(2,5-Difluoro-benzenesulfonylamino)-phenyl]-3-methyl-1H-pyrazolo[3,4-d]pyrimidin-4-yloxy}-piperidine-1-carboxylic acid ethyl ester,
N-[4-(3-Amino-4-propoxy-1H-pyrazolo[3,4-d]pyrimidin-6-yl)-phenyl]-2,5-difluoro-benzenesulfonamide,
N-[4-(3-Amino-4-ethoxy-1H-pyrazolo[3,4-d]pyrimidin-6-yl)-phenyl]-5-chloro-2-fluoro-benzenesulfonamide,
N-[4-(3-Amino-4-propoxy-1H-pyrazolo[3,4-d]pyrimidin-6-yl)-phenyl]-5-chloro-2-fluoro-benzenesulfonamide,
N-[4-(3-Amino-4-ethoxy-1H-pyrazolo[3,4-d]pyrimidin-6-yl)-phenyl]-2,5-difluoro-benzenesulfonamide,
2-Fluoro-N-(4-{4-[1-(2-methoxy-ethyl)-piperidin-4-yloxy]-3-methyl-1H-pyrazolo[3,4-d]pyrimidin-6-yl}-phenyl)-5-methyl-benzenesulfonamide,
2,5-Difluoro-N-(4-{4-[1-(2-methoxy-ethyl)-piperidin-4-yloxy]-3-methyl-1H-pyrazolo[3,4-d]pyrimidin-6-yl}-phenyl)-benzenesulfonamide,
5-Chloro-2-fluoro-N-(4-{4-[1-(2-methoxy-ethyl)-piperidin-4-yloxy]-3-methyl-1H-pyrazolo[3,4-d]pyrimidin-6-yl}-phenyl)-benzenesulfonamide,
N-{4-[4-(1-Ethyl-piperidin-4-yloxy)-3-methyl-1H-pyrazolo[3,4-d]pyrimidin-6-yl]-phenyl}-2-fluoro-5-methoxy-benzenesulfonamide,
2,5-Dichloro-N-{4-[4-(1-ethyl-piperidin-4-yloxy)-3-methyl-1H-pyrazolo[3,4-d]pyrimidin-6-yl]-phenyl}-benzenesulfonamide,
N-{4-[4-(1-Ethyl-piperidin-4-yloxy)-3-methyl-1H-pyrazolo[3,4-d]pyrimidin-6-yl]-phenyl}-2-fluoro-5-methyl-benzenesulfonamide,
N-{4-[4-(1-Ethyl-piperidin-4-yloxy)-3-methyl-1H-pyrazolo[3,4-d]pyrimidin-6-yl]-phenyl}-2-fluoro-benzenesulfonamide,
5-Chloro-N-{4-[4-(1-ethyl-piperidin-4-yloxy)-3-methyl-1H-pyrazolo[3,4-d]pyrimidin-6-yl]-phenyl}-2-fluoro-benzenesulfonamide,
N-{4-[4-(1-Cyclobutyl-piperidin-4-yloxy)-3-methyl-1H-pyrazolo[3,4-d]pyrimidin-6-yl]-phenyl}-2,5-difluoro-benzenesulfonamide,
2,5-Difluoro-N-(4-{4-[1-(3-methoxy-propyl)-piperidin-4-yloxy]-3-methyl-1H-pyrazolo[3,4-d]pyrimidin-6-yl}-phenyl)-benzenesulfonamide,
5-Chloro-2-fluoro-N-{4-[4-(3-hydroxy-propoxy)-3-methyl-1H-pyrazolo[3,4-d]pyrimidin-6-yl]-phenyl}-benzenesulfonamide,
2,5-Difluoro-N-{4-[4-(1-isopropyl-piperidin-4-yloxy)-3-methyl-1H-pyrazolo[3,4-d]pyrimidin-6-yl]-phenyl}-benzenesulfonamide,
2-Fluoro-N-(4-{4-[1-(2-fluoro-ethyl)-piperidin-4-yloxy]-3-methyl-1H-pyrazolo[3,4-d]pyrimidin-6-yl}-phenyl)-benzenesulfonamide,
5-Chloro-2-fluoro-N-{4-[4-(1-isopropyl-piperidin-4-yloxy)-3-methyl-1H-pyrazolo[3,4-d]pyrimidin-6-yl]-phenyl}-benzenesulfonamide,
2,5-Difluoro-N-(4-{4-[1-(2-fluoro-ethyl)-piperidin-4-yloxy]-3-methyl-1H-pyrazolo[3,4-d]pyrimidin-6-yl}-phenyl)-benzenesulfonamide,
N-[4-(3-Amino-4-isopropoxy-1H-pyrazolo[3,4-d]pyrimidin-6-yl)-phenyl]-2,5-dichloro-benzenesulfonamide,
N-[4-(3-Amino-4-isobutoxy-1H-pyrazolo[3,4-d]pyrimidin-6-yl)-phenyl]-2,5-difluoro-benzenesulfonamide,
N-[4-(3-Amino-4-isobutoxy-1H-pyrazolo[3,4-d]pyrimidin-6-yl)-phenyl]-2-fluoro-5-methoxy-benzenesulfonamide,
2,5-Dichloro-N-{4-[3-methyl-4-(piperidin-3-yloxy)-1H-pyrazolo[3,4-d]pyrimidin-6-yl]-phenyl}-benzenesulfonamide,
2,5-Difluoro-N-{4-[3-methyl-4-(piperidin-3-yloxy)-1H-pyrazolo[3,4-d]pyrimidin-6-yl]-phenyl}-benzenesulfonamide,
2-Fluoro-5-methyl-N-{4-[3-methyl-4-(morpholin-2-ylmethoxy)-1H-pyrazolo[3,4-d]pyrimidin-6-yl]-phenyl}-benzenesulfonamide,
N-{4-[4-(3-Aminomethyl-oxetan-3-ylmethoxy)-1H-pyrazolo[3,4-d]pyrimidin-6-yl]-phenyl}-5-chloro-2-fluoro-benzenesulfonamide,
N-[4-(3-Amino-4-ethoxymethyl-1H-pyrazolo[3,4-d]pyrimidin-6-yl)-phenyl]-2-fluoro-5-methyl-benzenesulfonamide,
N-[4-(3-Amino-4-trifluoromethyl-1H-pyrazolo[3,4-d]pyrimidin-6-yl)-phenyl]-2,5-difluoro-benzenesulfonamide,
2-Fluoro-N-{4-[4-(piperidin-4-yloxy)-1H-pyrazolo[3,4-d]pyrimidin-6-yl]-phenyl}-benzenesulfonamide,
N-[4-(3-Amino-1H-pyrazolo[3,4-d]pyrimidin-6-yl)-phenyl]-2-fluoro-5-methoxy-benzenesulfonamide,
N-[4-(3-Amino-4-methoxymethyl-1H-pyrazolo[3,4-d]pyrimidin-6-yl)-phenyl]-5-chloro-2-fluoro-benzenesulfonamide,
N-{4-[4-(3-Amino-propoxy)-3-methyl-1H-pyrazolo[3,4-d]pyrimidin-6-yl]-phenyl}-5-chloro-2-fluoro-benzenesulfonamide,
N-[4-(3-Amino-1H-pyrazolo[3,4-d]pyrimidin-6-yl)-phenyl]-2,5-difluoro-benzenesulfonamide,
N-[4-(3-Amino-1H-pyrazolo[3,4-d]pyrimidin-6-yl)-phenyl]-2,4,5-trifluoro-benzenesulfonamide,
N-[4-(3-Amino-1H-pyrazolo[3,4-d]pyrimidin-6-yl)-phenyl]-2-chloro-4,5-difluoro-benzenesulfonamide,
N-{4-[3-Amino-4-(2,2,2-trifluoro-ethoxy)-1H-pyrazolo[3,4-d]pyrimidin-6-yl]-phenyl}-2-cyano-5-methyl-benzenesulfonamide,
N-[4-(3-Amino-4-trifluoromethyl-1H-pyrazolo[3,4-d]pyrimidin-6-yl)-phenyl]-5-chloro-2-fluoro-benzenesulfonamide,
N-{4-[3-Amino-4-(2-methoxy-ethyl)-1H-pyrazolo[3,4-d]pyrimidin-6-yl]-phenyl}-2-cyano-5-methyl-benzenesulfonamide,
2-Cyano-5-methyl-N-{4-[4-(2,2,2-trifluoro-ethoxy)-1H-pyrazolo[3,4-d]pyrimidin-6-yl]-phenyl}-benzenesulfonamide,
N-[4-(3-Amino-4-cyclopropyl-1H-pyrazolo[3,4-d]pyrimidin-6-yl)-phenyl]-2,4,5-trifluoro-benzenesulfonamide,
N-[4-(3-Amino-4-cyclopropyl-1H-pyrazolo[3,4-d]pyrimidin-6-yl)-phenyl]-2-fluoro-benzenesulfonamide,
N-[4-(3-Amino-4-cyclopropyl-1H-pyrazolo[3,4-d]pyrimidin-6-yl)-phenyl]-2,5-difluoro-benzenesulfonamide,
N-[4-(3-Amino-4-methoxy-1H-pyrazolo[3,4-d]pyrimidin-6-yl)-phenyl]-5-chloro-2-fluoro-benzenesulfonamide,
N-[4-(3-Amino-4-methoxy-1H-pyrazolo[3,4-d]pyrimidin-6-yl)-phenyl]-5-chloro-2-cyano-benzenesulfonamide,
N-[4-(3-Amino-4-methyl-1H-pyrazolo[3,4-d]pyrimidin-6-yl)-phenyl]-2-chloro-3,5-difluoro-benzenesulfonamide,
2-Cyano-N-{4-[4-(4-hydroxy-cyclohexyloxy)-1H-pyrazolo[3,4-d]pyrimidin-6-yl]-phenyl}-5-methoxy-benzenesulfonamide,
N-[4-(3-Amino-1 H-pyrazolo[4,3-c]pyridin-6-yl)-phenyl]-5-chloro-2,4-difluoro-benzenesulfonamide,
5-Chloro-2-cyano-N-{4-[4-(4-hydroxy-cyclohexyloxy)-3-methyl-1H-pyrazolo[3,4-d]pyrimidin-6-yl]-phenyl}-benzenesulfonamide,
N-{4-[4-(1-Cyclopropyl-piperidin-4-yloxy)-3-methyl-1H-pyrazolo[3,4-d]pyrimidin-6-yl]-phenyl}-2,5-difluoro-benzenesulfonamide,
5-Chloro-N-{4-[4-(1-cyclopropyl-piperidin-4-yloxy)-3-methyl-1H-pyrazolo[3,4-d]pyrimidin-6-yl]-phenyl}-2-fluoro-benzenesulfonamide,
N-{4-[4-(1-Acetyl-piperidin-4-yloxy)-3-methyl-1H-pyrazolo[3,4-d]pyrimidin-6-yl]-phenyl}-2-fluoro-5-methoxy-benzenesulfonamide,
N-{4-[4-(1-Acetyl-piperidin-4-yloxy)-3-methyl-1H-pyrazolo[3,4-d]pyrimidin-6-yl]-phenyl}-2,5-difluoro-benzenesulfonamide,
N-{4-[4-(1-Acetyl-piperidin-4-yloxy)-3-methyl-1H-pyrazolo[3,4-d]pyrimidin-6-yl]-phenyl}-5-chloro-2-fluoro-benzenesulfonamide,
5-Chloro-2-fluoro-N-{4-[4-(6-hydroxy-pyridin-3-yloxy)-3-methyl-1H-pyrazolo[3,4-d]pyrimidin-6-yl]-phenyl}-benzenesulfonamide, and
2-Fluoro-N-{4-[4-(6-hydroxy-pyridin-3-yloxy)-3-methyl-1H-pyrazolo[3,4-d]pyrimidin-6-yl]-phenyl}-5-methyl-benzenesulfonamide,
or a pharmaceutically acceptable salt thereof.

25. The use according to claim 15, wherein the compound of the formula I is 5-Chloro-2-fluoro-N-{4-[4-(1-isopropyl-piperidin-4-yloxy)-3-methyl-1H-pyrazolo[3,4-d]pyrimidin-6-yl]-phenyl}-benzenesulfonamide: 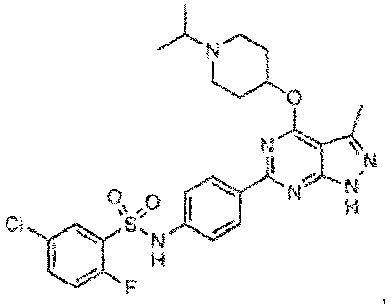 or a pharmaceutically acceptable salt thereof.

26. A method for treating sickle cell disease, comprising administering a compound of the formula I, in any of its stereoisomeric forms or a mixture of stereoisomeric forms in any ratio, or a pharmaceutically acceptable salt thereof, 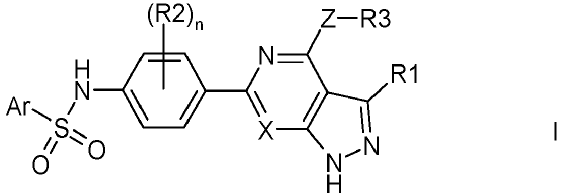 wherein
Ar is selected from the series consisting of phenyl and a 5-membered or 6-membered, monocyclic, aromatic, heterocyclic group which comprises 1, 2 or 3 identical or different ring heteroatoms selected from the series consisting of nitrogen, oxygen and sulfur, and is bonded via a ring carbon atom, which all are unsubstituted or substituted by one or more identical or different substituents R5;
n is selected from the series consisting of 0, 1 and 2;
X is selected from the series consisting of N and CH;
Z is selected from the series consisting of a direct bond, O, S and N(R10);
R1 is selected from the series consisting of H, -N(R11)-R12, -N(R13)-C(O)-R14, -N(R13)-S(O)₂-R15, -N(R13)-C(O)-NH-R16, (C₁-C₄)-alkyl and -(C₁-C₄)-alkyl-O-R17;
R2 is selected from the series consisting of halogen, (C₁-C₄)-alkyl, -O-(C₁-C₄)-alkyl and - CN;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, (C₃-C₇)-cycloalkyl,-(C₁-C₄)-alkyl-(C₃-C₇)-cycloalkyl, -O-(C₁-C₄)-alkyl, -O-(C₃-C₇)-cycloalkyl, -O-(C₁-C₄)-alkyl-(C₃-C₇)-cycloalkyl, -C(O)-N(R6)-R7 and -CN,
and two groups R5 bonded to adjacent ring carbon atoms in Ar, together with the carbon atoms carrying them, can form a 5-membered to 8-membered, monocyclic, unsaturated ring which comprises 0, 1 or 2 identical or different ring heteroatoms selected from the series consisting of nitrogen, oxygen and sulfur, and which is unsubstituted or substituted by one or more identical or different substituents R8;
R6 and R7 are independently of one another selected from the series consisting of H and (C₁-C₄)-alkyl;
R8 is selected from the series consisting of halogen, (C₁-C₄)-alkyl, -O-(C₁-C₄)-alkyl and - CN;
R10 is selected from the series consisting of H and (C₁-C₄)-alkyl;
R11 and R12 are independently of one another selected from the series consisting of H, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl, wherein phenyl is unsubstituted or substituted by one or more identical or different substituents R50,
or R11 and R12, together with the nitrogen atom carrying them, form a 4-membered to 7-membered, monocyclic, saturated, heterocyclic group which, in addition to the nitrogen atom carrying R11 and R12, comprises 0 or 1 further ring heteroatom selected from the series consisting of nitrogen, oxygen and sulfur, and which is unsubstituted or substituted by one or more identical or different substituents selected from the series consisting of fluorine and (C₁-C₄)-alkyl;
R13 is selected from the series consisting of H, (C₁-C₄)-alkyl and (C₃-C₇)-cycloalkyl;
R14 and R16 are independently of one another selected from the series consisting of (C₁-C₈)-alkyl, (C₃-C₇)-cycloalkyl, -(C₁-C₄)-alkyl-(C₃-C₇)-cycloalkyl, phenyl, -(C₁-C₄)-alkylphenyl, Het2 and -(C₁-C₄)-alkyl-Het2, wherein (C₁-C₈)-alkyl and (C₃-C₇)-cycloalkyl all are unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R15 is selected from the series consisting of (C₁-C₈)-alkyl, phenyl and Het3, wherein phenyl and Het3 all are unsubstituted or substituted by one or more identical or different substituents R50;
R17 is selected from the series consisting of H and (C₁-C₄)-alkyl;
R30 is a 3-membered to 12-membered, monocyclic or bicyclic, saturated, partially unsaturated or aromatic, cyclic group which comprises 0, 1, 2 or 3 identical or different ring heteroatoms selected from the series consisting of nitrogen, oxygen and sulfur, which is unsubstituted or substituted by one or more identical or different substituents R32;
R31 is selected from the series consisting of halogen, -OH, -O-(C₁-C₄)-alkyl, -O-(C₃-C₇)-cycloalkyl, -O-(C₁-C₄)-alkyl-(C₃-C₇)-cycloalkyl, -N(R33)-R34, -CN and -C(O)-N(R35)-R36;
R32 is selected from the series consisting of halogen, (C₁-C₄)-alkyl, (C₃-C₇)-cycloalkyl,-(C₁-C₄)-alkyl-(C₃-C₇)-cycloalkyl, -(C₁-C₄)-alkyl-O-R37, -(C₁-C₄)-alkyl-N(R38)-R39, -(C₁-C₄)-alkyl-CN, -C(O)-(C₁-C₄)-alkyl, -CN, -OH, =O, -O-(C₁-C₄)-alkyl, -N(R40)-R41, -C(O)-O-(C₁-C₄)-alkyl and -C(O)-N(R42)-R43;
R33, R34, R35, R36, R37, R38, R39, R40, R41, R42 and R43 are independently of one another selected from the series consisting of H and (C₁-C₄)-alkyl;
R50 is selected from the series consisting of halogen, (C₁-C₄)-alkyl, -O-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen, oxygen and sulfur, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated, partially unsaturated or aromatic, heterocyclic group which comprises 1 or 2 identical or different ring heteroatoms selected from the series consisting of nitrogen, oxygen and sulfur, and is bonded via a ring carbon atom;
Het3 is a 5-membered or 6-membered, monocyclic, aromatic, heterocyclic group which comprises 1, 2 or 3 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

27. The method according to claim 26, wherein:
Ar is selected from the series consisting of phenyl and a 5-membered or 6-membered, monocyclic, aromatic, heterocyclic group which comprises 1 or 2 identical or different ring heteroatoms selected from the series consisting of nitrogen, oxygen and sulfur, and is bonded via a ring carbon atom, which all are unsubstituted or substituted by one or more identical or different substituents R5;
n is selected from the series consisting of 0, 1 and 2;
X is selected from the series consisting of N and CH;
Z is selected from the series consisting of a direct bond, O, S and N(R10);
R1 is selected from the series consisting of H, -N(R11)-R12, -N(R13)-C(O)-R14, -N(R13)-S(O)₂-R15, -N(R13)-C(O)-NH-R16 and (C₁-C₄)-alkyl;
R2 is selected from the series consisting of halogen, (C₁-C₄)-alkyl, -O-(C₁-C₄)-alkyl and - CN;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, (C₃-C₇)-cycloalkyl, -O-(C₁-C₄)-alkyl, -O-(C₃-C₇)-cycloalkyl, -C(O)-N(R6)-R7 and -CN,
and two groups R5 bonded to adjacent ring carbon atoms in Ar, together with the carbon atoms carrying them, can form a 5-membered to 8-membered, monocyclic, unsaturated ring which comprises 0, 1 or 2 identical or different ring heteroatoms selected from the series consisting of nitrogen, oxygen and sulfur, and which is unsubstituted or substituted by one or more identical or different substituents R8;
R6 and R7 are independently of one another selected from the series consisting of H and (C₁-C₄)-alkyl;
R8 is selected from the series consisting of halogen and (C₁-C₄)-alkyl;
R10 is selected from the series consisting of H and (C₁-C₄)-alkyl;
R11 and R12 are independently of one another selected from the series consisting of H, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl, wherein phenyl is unsubstituted or substituted by one or more identical or different substituents R50;
R13 is selected from the series consisting of H and (C₁-C₄)-alkyl;
R14 and R16 are independently of one another selected from the series consisting of (C₁-C₈)-alkyl, (C₃-C₇)-cycloalkyl, -(C₁-C₄)-alkyl-(C₃-C₇)-cycloalkyl, phenyl, -(C₁-C₄)-alkylphenyl, Het2 and -(C₁-C₄)-alkyl-Het2, wherein (C₁-C₈)-alkyl and (C₃-C₇)-cycloalkyl all are unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R15 is selected from the series consisting of phenyl and Het3, wherein phenyl and Het3 all are unsubstituted or substituted by one or more identical or different substituents R50;
R30 is a 3-membered to 12-membered, monocyclic or bicyclic, saturated, partially unsaturated or aromatic, cyclic group which comprises 0, 1, 2 or 3 identical or different ring heteroatoms selected from the series consisting of nitrogen, oxygen and sulfur, which is unsubstituted or substituted by one or more identical or different substituents R32;
R31 is selected from the series consisting of halogen, -OH, -O-(C₁-C₄)-alkyl, -O-(C₃-C₇)-cycloalkyl, -O-(C₁-C₄)-alkyl-(C₃-C₇)-cycloalkyl, -N(R33)-R34 and -CN;
R32 is selected from the series consisting of halogen, (C₁-C₄)-alkyl, (C₃-C₇)-cycloalkyl, - (C₁-C₄)-alkyl-(C₃-C₇)-cycloalkyl, -(C₁-C₄)-alkyl-O-R37, -(C₁-C₄)-alkyl-N(R38)-R39, -(C₁-C₄)-alkyl-CN, -C(O)-(C₁-C₄)-alkyl, -CN, -OH, =O, -O-(C₁-C₄)-alkyl, -N(R40)-R41, -C(O)-O-(C₁-C₄)-alkyl and -C(O)-N(R42)-R43;
R33, R34, R37, R38, R39, R40, R41, R42 and R43 are independently of one another selected from the series consisting of H and (C₁-C₄)-alkyl;
R50 is selected from the series consisting of halogen, (C₁-C₄)-alkyl, -O-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen, oxygen and sulfur, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated, partially unsaturated or aromatic, heterocyclic group which comprises 1 or 2 identical or different ring heteroatoms selected from the series consisting of nitrogen, oxygen and sulfur, and is bonded via a ring carbon atom;
Het3 is a 5-membered or 6-membered, monocyclic, aromatic, heterocyclic group which comprises 1 or 2 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

28. The method according to claim 26 or 27, wherein
Ar is selected from the series consisting of phenyl and a 5-membered monocyclic, aromatic, heterocyclic group which comprises 1 or 2 identical or different ring heteroatoms selected from the series consisting of nitrogen and sulfur, and is bonded via a ring carbon atom, which all are unsubstituted or substituted by one or more identical or different substituents R5;
n is selected from the series consisting of 0, 1 and 2;
X is selected from the series consisting of N and CH;
Z is selected from the series consisting of a direct bond, O, S and N(R10);
R1 is selected from the series consisting of H, -N(R11)-R12, -N(R13)-C(O)-R14, -N(R13)-S(O)₂-R15, -N(R13)-C(O)-NH-R16 and (C₁-C₄)-alkyl;
R2 is selected from the series consisting of halogen, (C₁-C₄)-alkyl and -O-(C₁-C₄)-alkyl;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, (C₃-C₇)-cycloalkyl, -O-(C₁-C₄)-alkyl, -O-(C₃-C₇)-cycloalkyl and -CN,
and two groups R5 bonded to adjacent ring carbon atoms in Ar, together with the carbon atoms carrying them, can form a 5-membered to 7-membered, monocyclic, unsaturated ring which comprises 0, 1 or 2 oxygen atoms as ring heteroatoms, and which is unsubstituted or substituted by one or more identical or different substituents R8;
R8 is selected from the series consisting of halogen and (C₁-C₄)-alkyl;
R10 is selected from the series consisting of H and (C₁-C₄)-alkyl;
one of the groups R11 and R12 is selected from the series consisting of hydrogen and (C₁-C₄)-alkyl, and the other of the groups R11 and R12 is selected from the series consisting of hydrogen, (C₁-C₄)-alkyl, (C₃-C₇)-cycloalkyl, -(C₁-C₄)-alkyl-(C₃-C₇)-cycloalkyl, Het1, -(C₁-C₄)-alkyl-Het1 and -(C₁-C₄)-alkyl-phenyl;
R13 is selected from the series consisting of H and (C₁-C₄)-alkyl;
R14 and R16 are independently of one another selected from the series consisting of (C₁-C₈)-alkyl, (C₃-C₇)-cycloalkyl, -(C₁-C₄)-alkyl-(C₃-C₇)-cycloalkyl, phenyl, -(C₁-C₄)-alkylphenyl, Het2 and -(C₁-C₄)-alkyl-Het2, wherein (C₁-C₈)-alkyl and (C₃-C₇)-cycloalkyl all are unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R15 is phenyl which is unsubstituted or substituted by one or more identical or different substituents R50;
R30 is a 3-membered to 12-membered, monocyclic or bicyclic, saturated, partially unsaturated or aromatic, cyclic group which comprises 0, 1, 2 or 3 identical or different ring heteroatoms selected from the series consisting of nitrogen and oxygen, which is unsubstituted or substituted by one or more identical or different substituents R32;
R31 is selected from the series consisting of halogen, -OH, -O-(C₁-C₄)-alkyl, -O-(C₃-C₇)-cycloalkyl, -O-(C₁-C₄)-alkyl-(C₃-C₇)-cycloalkyl and -N(R33)-R34;
R32 is selected from the series consisting of halogen, (C₁-C₄)-alkyl, (C₃-C₇)-cycloalkyl, - (C₁-C₄)-alkyl-(C₃-C₇)-cycloalkyl, -(C₁-C₄)-alkyl-O-R37, -(C₁-C₄)-alkyl-N(R38)-R39, -OH, =O, -O-(C₁-C₄)-alkyl and -N(R40)-R41;
R33, R34, R37, R38, R39, R40 and R41 are independently of one another selected from the series consisting of H and (C₁-C₄)-alkyl;
R50 is selected from the series consisting of halogen, (C₁-C₄)-alkyl, -O-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen and oxygen, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated, partially unsaturated or aromatic, heterocyclic group which comprises 1 or 2 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

29. The method according to any one of claims 26 to 28, wherein
Ar is phenyl, which is unsubstituted or substituted by one or more identical or different substituents R5;
n is selected from the series consisting of 0 and 1;
X is selected from the series consisting of N and CH;
Z is selected from the series consisting of a direct bond, O and N(R10);
R1 is selected from the series consisting of H, -N(R11)-R12, -N(R13)-C(O)-R14 and (C₁-C₄)-alkyl;
R2 is selected from the series consisting of halogen and -O-(C₁-C₄)-alkyl;
R3 is selected from the series consisting of H, (C₁-C₈)-alkyl, R30 and -(C₁-C₄)-alkyl-R30, wherein (C₁-C₈)-alkyl is unsubstituted or substituted by one or more identical or different substituents R31;
R5 is selected from the series consisting of halogen, (C₁-C₄)-alkyl, -O-(C₁-C₄)-alkyl and - CN,
and two groups R5 bonded to adjacent ring carbon atoms in Ar, together with the carbon atoms carrying them, can form a 5-membered to 7-membered, monocyclic, unsaturated ring which comprises 0, 1 or 2 oxygen atoms as ring heteroatoms, and which is unsubstituted or substituted by one or more identical or different substituents R8;
R8 is selected from the series consisting of halogen and (C₁-C₄)-alkyl;
R10 is selected from the series consisting of H and (C₁-C₄)-alkyl;
one of the groups R11 and R12 is selected from the series consisting of hydrogen and (C₁-C₄)-alkyl, and the other of the groups R11 and R12 is selected from the series consisting of hydrogen, (C₁-C₄)-alkyl, -(C₁-C₄)-alkyl-(C₃-C₇)-cycloalkyl and -(C₁-C₄)-alkyl-Het1;
R13 is selected from the series consisting of H and (C₁-C₄)-alkyl;
R14 is selected from the series consisting of (C₃-C₇)-cycloalkyl, phenyl and Het2, wherein (C₃-C₇)-cycloalkyl is unsubstituted or substituted by one or more identical or different substituents selected from the series consisting of -OH and -O-(C₁-C₄)-alkyl, and wherein phenyl and Het2 all are unsubstituted or substituted by one or more identical or different substituents R50;
R30 is a 3-membered to 10-membered, monocyclic or bicyclic, saturated, partially unsaturated or aromatic, cyclic group which comprises 0, 1, 2 or 3 identical or different ring heteroatoms selected from the series consisting of nitrogen and oxygen, which is unsubstituted or substituted by one or more identical or different substituents R32;
R31 is selected from the series consisting of halogen, -OH, -O-(C₁-C₄)-alkyl, -O-(C₃-C₇)-cycloalkyl and -N(R33)-R34;
R32 is selected from the series consisting of halogen, (C₁-C₄)-alkyl, (C₃-C₇)-cycloalkyl, - (C₁-C₄)-alkyl-O-R37, -(C₁-C₄)-alkyl-N(R38)-R39, -OH, =O, -O-(C₁-C₄)-alkyl and -N(R40)-R41;
R33, R34, R37, R38, R39, R40 and R41 are independently of one another selected from the series consisting of H and (C₁-C₄)-alkyl;
R50 is selected from the series consisting of halogen, (C₁-C₄)-alkyl, -O-(C₁-C₄)-alkyl and -CN;
Het1 is a 4-membered to 7-membered, monocyclic, saturated, heterocyclic group which comprises 1 or 2 identical or different ring heteroatoms selected from the series consisting of nitrogen and oxygen, and is bonded via a ring carbon atom, and which is unsubstituted or substituted by one or more identical or different substituents selected from the series consisting of fluorine and (C₁-C₄)-alkyl;
Het2 is a 4-membered to 7-membered, monocyclic, saturated or aromatic, heterocyclic group which comprises 1 or 2 identical or different ring heteroatoms selected from the series consisting of nitrogen, oxygen and sulfur, and is bonded via a ring carbon atom;
wherein all cycloalkyl groups, independently of any other substituents which can be present on a cycloalkyl group, can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

30. The method according to any one of claims 26 to 29, wherein
Ar is phenyl, which is unsubstituted or substituted by one or more identical or different substituents R5;
n is selected from the series consisting of 0 and 1;
X is selected from the series consisting of N and CH;
Z is selected from the series consisting of a direct bond and O;
R1 is selected from the series consisting of H, -N(R11)-R12 and (C₁-C₄)-alkyl;
R2 is selected from the series consisting of halogen;
R3 is selected from the series consisting of H, R30 and -(C₁-C₄)-alkyl-R30;
R5 is selected from the series consisting of halogen, (C₁-C₄)-alkyl, -O-(C₁-C₄)-alkyl and - CN;
R11 and R12 are independently of one another selected from the series consisting of hydrogen and (C₁-C₄)-alkyl;
R30 is a 3-membered to 7-membered, monocyclic, saturated or aromatic, cyclic group which comprises 0, 1 or 2 identical or different ring heteroatoms selected from the series consisting of nitrogen and oxygen, which is unsubstituted or substituted by one or more identical or different substituents R32;
R32 is selected from the series consisting of halogen, (C₁-C₄)-alkyl, (C₃-C₇)-cycloalkyl,-(C₁-C₄)-alkyl-O-R37, -(C₁-C₄)-alkyl-N(R38)-R39, -OH and =O;
R37, R38 and R39 are independently of one another selected from the series consisting of H and (C₁-C₄)-alkyl;
wherein all cycloalkyl groups can be substituted by one or more identical or different substituents selected from the series consisting of fluorine and (C₁-C₄)-alkyl;
wherein all alkyl groups, independently of any other substituents which can be present on an alkyl group, can be substituted by one or more fluorine substituents.

31. The method according to any one of claims 26 to 30, wherein Z is a direct bond.

32. The method according to any one of claims 26 to 30, wherein Z is O.

33. The method according to any one of claims 26 to 32, wherein X is N.

34. The method according to any one of claims 26 to 32, wherein X is CH.

35. The method according to claim 26, wherein the compound of the formula I is selected from the group consisting of:
N-[4-(3-Amino-4-cyclopropyl-1H-pyrazolo[3,4-d]pyrimidin-6-yl)-phenyl]-2-cyano-5-methoxy-benzenesulfonamide,
N-[4-(3-Amino-1H-pyrazolo[3,4-d]pyrimidin-6-yl)-phenyl]-2,5-difluoro-benzenesulfonamide,
N-[4-(3-Amino-1H-pyrazolo[3,4-d]pyrimidin-6-yl)-phenyl]-5-chloro-2-cyano-benzenesulfonamide,
2-Chloro-N-{4-[4-(1-ethyl-piperidin-3-yloxy)-3-methyl-1H-pyrazolo[3,4-d]pyrimidin-6-yl]-phenyl}-5-methoxy-benzenesulfonamide,
5-Chloro-N-{4-[4-(1-ethyl-piperidin-3-yloxy)-3-methyl-1H-pyrazolo[3,4-d]pyrimidin-6-yl]-phenyl}-2-fluoro-benzenesulfonamide,
4-{6-[4-(2,5-Difluoro-benzenesulfonylamino)-phenyl]-3-methyl-1H-pyrazolo[3,4-d]pyrimidin-4-yloxy}-piperidine-1-carboxylic acid ethyl ester,
N-[4-(3-Amino-4-propoxy-1H-pyrazolo[3,4-d]pyrimidin-6-yl)-phenyl]-2,5-difluoro-benzenesulfonamide,
N-[4-(3-Amino-4-ethoxy-1H-pyrazolo[3,4-d]pyrimidin-6-yl)-phenyl]-5-chloro-2-fluoro-benzenesulfonamide,
N-[4-(3-Amino-4-propoxy-1H-pyrazolo[3,4-d]pyrimidin-6-yl)-phenyl]-5-chloro-2-fluoro-benzenesulfonamide,
N-[4-(3-Amino-4-ethoxy-1H-pyrazolo[3,4-d]pyrimidin-6-yl)-phenyl]-2,5-difluoro-benzenesulfonamide,
2-Fluoro-N-(4-{4-[1-(2-methoxy-ethyl)-piperidin-4-yloxy]-3-methyl-1H-pyrazolo[3,4-d]pyrimidin-6-yl}-phenyl)-5-methyl-benzenesulfonamide,
2,5-Difluoro-N-(4-{4-[1-(2-methoxy-ethyl)-piperidin-4-yloxy]-3-methyl-1H-pyrazolo[3,4-d]pyrimidin-6-yl}-phenyl)-benzenesulfonamide,
5-Chloro-2-fluoro-N-(4-{4-[1-(2-methoxy-ethyl)-piperidin-4-yloxy]-3-methyl-1H-pyrazolo[3,4-d]pyrimidin-6-yl}-phenyl)-benzenesulfonamide,
N-{4-[4-(1-Ethyl-piperidin-4-yloxy)-3-methyl-1H-pyrazolo[3,4-d]pyrimidin-6-yl]-phenyl}-2-fluoro-5-methoxy-benzenesulfonamide,
2,5-Dichloro-N-{4-[4-(1-ethyl-piperidin-4-yloxy)-3-methyl-1H-pyrazolo[3,4-d]pyrimidin-6-yl]-phenyl}-benzenesulfonamide,
N-{4-[4-(1-Ethyl-piperidin-4-yloxy)-3-methyl-1H-pyrazolo[3,4-d]pyrimidin-6-yl]-phenyl}-2-fluoro-5-methyl-benzenesulfonamide,
N-{4-[4-(1-Ethyl-piperidin-4-yloxy)-3-methyl-1H-pyrazolo[3,4-d]pyrimidin-6-yl]-phenyl}-2-fluoro-benzenesulfonamide,
5-Chloro-N-{4-[4-(1-ethyl-piperidin-4-yloxy)-3-methyl-1H-pyrazolo[3,4-d]pyrimidin-6-yl]-phenyl}-2-fluoro-benzenesulfonamide,
N-{4-[4-(1-Cyclobutyl-piperidin-4-yloxy)-3-methyl-1H-pyrazolo[3,4-d]pyrimidin-6-yl]-phenyl}-2,5-difluoro-benzenesulfonamide,
2,5-Difluoro-N-(4-{4-[1-(3-methoxy-propyl)-piperidin-4-yloxy]-3-methyl-1H-pyrazolo[3,4-d]pyrimidin-6-yl}-phenyl)-benzenesulfonamide,
5-Chloro-2-fluoro-N-{4-[4-(3-hydroxy-propoxy)-3-methyl-1H-pyrazolo[3,4-d]pyrimidin-6-yl]-phenyl}-benzenesulfonamide,
2,5-Difluoro-N-{4-[4-(1-isopropyl-piperidin-4-yloxy)-3-methyl-1H-pyrazolo[3,4-d]pyrimidin-6-yl]-phenyl}-benzenesulfonamide,
2-Fluoro-N-(4-{4-[1-(2-fluoro-ethyl)-piperidin-4-yloxy]-3-methyl-1H-pyrazolo[3,4-d]pyrimidin-6-yl}-phenyl)-benzenesulfonamide,
5-Chloro-2-fluoro-N-{4-[4-(1-isopropyl-piperidin-4-yloxy)-3-methyl-1H-pyrazolo[3,4-d]pyrimidin-6-yl]-phenyl}-benzenesulfonamide,
2,5-Difluoro-N-(4-{4-[1-(2-fluoro-ethyl)-piperidin-4-yloxy]-3-methyl-1H-pyrazolo[3,4-d]pyrimidin-6-yl}-phenyl)-benzenesulfonamide,
N-[4-(3-Amino-4-isopropoxy-1H-pyrazolo[3,4-d]pyrimidin-6-yl)-phenyl]-2,5-dichloro-benzenesulfonamide,
N-[4-(3-Amino-4-isobutoxy-1H-pyrazolo[3,4-d]pyrimidin-6-yl)-phenyl]-2,5-difluoro-benzenesulfonamide,
N-[4-(3-Amino-4-isobutoxy-1H-pyrazolo[3,4-d]pyrimidin-6-yl)-phenyl]-2-fluoro-5-methoxy-benzenesulfonamide,
2,5-Dichloro-N-{4-[3-methyl-4-(piperidin-3-yloxy)-1H-pyrazolo[3,4-d]pyrimidin-6-yl]-phenyl}-benzenesulfonamide,
2,5-Difluoro-N-{4-[3-methyl-4-(piperidin-3-yloxy)-1H-pyrazolo[3,4-d]pyrimidin-6-yl]-phenyl}-benzenesulfonamide,
2-Fluoro-5-methyl-N-{4-[3-methyl-4-(morpholin-2-ylmethoxy)-1H-pyrazolo[3,4-d]pyrimidin-6-yl]-phenyl}-benzenesulfonamide,
N-{4-[4-(3-Aminomethyl-oxetan-3-ylmethoxy)-1H-pyrazolo[3,4-d]pyrimidin-6-yl]-phenyl}-5-chloro-2-fluoro-benzenesulfonamide,
N-[4-(3-Amino-4-ethoxymethyl-1H-pyrazolo[3,4-d]pyrimidin-6-yl)-phenyl]-2-fluoro-5-methyl-benzenesulfonamide,
N-[4-(3-Amino-4-trifluoromethyl-1H-pyrazolo[3,4-d]pyrimidin-6-yl)-phenyl]-2,5-difluoro-benzenesulfonamide,
2-Fluoro-N-{4-[4-(piperidin-4-yloxy)-1H-pyrazolo[3,4-d]pyrimidin-6-yl]-phenyl}-benzenesulfonamide,
N-[4-(3-Amino-1H-pyrazolo[3,4-d]pyrimidin-6-yl)-phenyl]-2-fluoro-5-methoxy-benzenesulfonamide,
N-[4-(3-Amino-4-methoxymethyl-1H-pyrazolo[3,4-d]pyrimidin-6-yl)-phenyl]-5-chloro-2-fluoro-benzenesulfonamide,
N-{4-[4-(3-Amino-propoxy)-3-methyl-1H-pyrazolo[3,4-d]pyrimidin-6-yl]-phenyl}-5-chloro-2-fluoro-benzenesulfonamide,
N-[4-(3-Amino-1H-pyrazolo[3,4-d]pyrimidin-6-yl)-phenyl]-2,5-difluoro-benzenesulfonamide,
N-[4-(3-Amino-1H-pyrazolo[3,4-d]pyrimidin-6-yl)-phenyl]-2,4,5-trifluoro-benzenesulfonamide,
N-[4-(3-Amino-1H-pyrazolo[3,4-d]pyrimidin-6-yl)-phenyl]-2-chloro-4,5-difluoro-benzenesulfonamide,
N-{4-[3-Amino-4-(2,2,2-trifluoro-ethoxy)-1H-pyrazolo[3,4-d]pyrimidin-6-yl]-phenyl}-2-cyano-5-methyl-benzenesulfonamide,
N-[4-(3-Amino-4-trifluoromethyl-1H-pyrazolo[3,4-d]pyrimidin-6-yl)-phenyl]-5-chloro-2-fluoro-benzenesulfonamide,
N-{4-[3-Amino-4-(2-methoxy-ethyl)-1H-pyrazolo[3,4-d]pyrimidin-6-yl]-phenyl}-2-cyano-5-methyl-benzenesulfonamide,
2-Cyano-5-methyl-N-{4-[4-(2,2,2-trifluoro-ethoxy)-1H-pyrazolo[3,4-d]pyrimidin-6-yl]-phenyl}-benzenesulfonamide,
N-[4-(3-Amino-4-cyclopropyl-1H-pyrazolo[3,4-d]pyrimidin-6-yl)-phenyl]-2,4,5-trifluoro-benzenesulfonamide,
N-[4-(3-Amino-4-cyclopropyl-1H-pyrazolo[3,4-d]pyrimidin-6-yl)-phenyl]-2-fluoro-benzenesulfonamide,
N-[4-(3-Amino-4-cyclopropyl-1H-pyrazolo[3,4-d]pyrimidin-6-yl)-phenyl]-2,5-difluoro-benzenesulfonamide,
N-[4-(3-Amino-4-methoxy-1H-pyrazolo[3,4-d]pyrimidin-6-yl)-phenyl]-5-chloro-2-fluoro-benzenesulfonamide,
N-[4-(3-Amino-4-methoxy-1H-pyrazolo[3,4-d]pyrimidin-6-yl)-phenyl]-5-chloro-2-cyano-benzenesulfonamide,
N-[4-(3-Amino-4-methyl-1H-pyrazolo[3,4-d]pyrimidin-6-yl)-phenyl]-2-chloro-3,5-difluoro-benzenesulfonamide,
2-Cyano-N-{4-[4-(4-hydroxy-cyclohexyloxy)-1H-pyrazolo[3,4-d]pyrimidin-6-yl]-phenyl}-5-methoxy-benzenesulfonamide,
N-[4-(3-Amino-1H-pyrazolo[4,3-c]pyridin-6-yl)-phenyl]-5-chloro-2,4-difluoro-benzenesulfonamide,
5-Chloro-2-cyano-N-{4-[4-(4-hydroxy-cyclohexyloxy)-3-methyl-1H-pyrazolo[3,4-d]pyrimidin-6-yl]-phenyl}-benzenesulfonamide,
N-{4-[4-(1-Cyclopropyl-piperidin-4-yloxy)-3-methyl-1H-pyrazolo[3,4-d]pyrimidin-6-yl]-phenyl}-2,5-difluoro-benzenesulfonamide,
5-Chloro-N-{4-[4-(1-cyclopropyl-piperidin-4-yloxy)-3-methyl-1H-pyrazolo[3,4-d]pyrimidin-6-yl]-phenyl}-2-fluoro-benzenesulfonamide,
N-{4-[4-(1-Acetyl-piperidin-4-yloxy)-3-methyl-1H-pyrazolo[3,4-d]pyrimidin-6-yl]-phenyl}-2-fluoro-5-methoxy-benzenesulfonamide,
N-{4-[4-(1-Acetyl-piperidin-4-yloxy)-3-methyl-1H-pyrazolo[3,4-d]pyrimidin-6-yl]-phenyl}-2,5-difluoro-benzenesulfonamide,
N-{4-[4-(1-Acetyl-piperidin-4-yloxy)-3-methyl-1H-pyrazolo[3,4-d]pyrimidin-6-yl]-phenyl}-5-chloro-2-fluoro-benzenesulfonamide,
5-Chloro-2-fluoro-N-{4-[4-(6-hydroxy-pyridin-3-yloxy)-3-methyl-1H-pyrazolo[3,4-d]pyrimidin-6-yl]-phenyl}-benzenesulfonamide, and
2-Fluoro-N-{4-[4-(6-hydroxy-pyridin-3-yloxy)-3-methyl-1H-pyrazolo[3,4-d]pyrimidin-6-yl]-phenyl}-5-methyl-benzenesulfonamide,
or a pharmaceutically acceptable salt thereof.

36. The method according to claim 26, wherein the compound of the formula I is 5-Chloro-2-fluoro-N-{4-[4-(1-isopropyl-piperidin-4-yloxy)-3-methyl-1H-pyrazolo[3,4-d]pyrimidin-6-yl]-phenyl}-benzenesulfonamide: 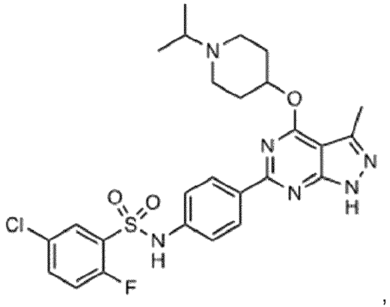 or a pharmaceutically acceptable salt thereof.

37. The method according to any one of claims 26 to 36, further comprising administering at least one additional therapeutic agent in combination with the compound of the formula I or a pharmaceutically acceptable salt thereof.

38. The method according to claim 37, wherein the at least one additional therapeutic agent is selected from the group consisting of hydroxyurea, L-glutamine, voxelotor and crizanlizumab.

39. Use of a SGK1 inhibitor, for increasing fetal hemoglobin (HbF) in erythrocytes.

40. Use of a SGK1 inhibitor, in the treatment of a β-hemoglobinopathy.

41. The use of claim 40, wherein the β-hemoglobinopathy is sickle cell disease or β-thalassemia.

42. The use of claim 41, wherein the β-hemoglobinopathy is sickle cell disease.

43. The use of any one of claims 39 to 42, wherein the SGK1 inhibitor is the compound of the formula I, in any of its stereoisomeric forms or a mixture of stereoisomeric forms in any ratio, or a pharmaceutically acceptable salt thereof, as defined in any one of claims 1 to 11.
